(19) Europäisches Patentamt
European Patent Office
Office européen des brevets

(11) **EP 3 327 034 A1**

(12) **EUROPEAN PATENT APPLICATION**

(43) Date of publication:
**30.05.2018   Bulletin 2018/22**

(21) Application number: **17205688.9**

(22) Date of filing: **25.04.2014**

(51) Int Cl.:
*C07K 16/22* (2006.01)        *C07K 16/28* (2006.01)
*A61K 39/00* (2006.01)        *A61K 39/395* (2006.01)
*A61K 9/00* (2006.01)          *C07K 16/40* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.04.2013   EP 13165741
15.01.2014   EP 14151318**

(62) Document number(s) of the earlier application(s) in
accordance with Art. 76 EPC:
**14721295.5 / 2 992 011**

(27) Previously filed application:
**25.04.2014 PCT/EP2014/058418**

(71) Applicant: **F. Hoffmann-La Roche AG
4070 Basel (CH)**

(72) Inventors:
• **HARTMANN, Guido
4070 Basel (CH)**

• **REGULA, Joerg Thomas
82377 Penzberg (DE)**
• **RUETH, Matthias
82377 Penzberg (DE)**
• **SCHAEFER, Wolfgang
82377 Penzberg (DE)**
• **SCHLOTHAUER, Tilman
82377 Penzberg (DE)**

(74) Representative: **Burger, Alexander
Roche Diagnostics GmbH
Patent Department (LPP.....6164)
P.O.Box 11 52
82372 Penzberg (DE)**

Remarks:
This application was filed on 06-12-2017 as a
divisional application to the application mentioned
under INID code 62.

(54) **FCRN-BINDING ABOLISHED ANTI-IGF-1R ANTIBODIES AND THEIR USE IN THE TREATMENT OF VASCULAR EYE DISEASES**

(57)     The invention provides anti-IGF-lR antibodies with abolished FcRn-binding and methods of using the same for the treatment of vascular eye diseases.

EP 3 327 034 A1

**Description**

**FIELD OF THE INVENTION**

The present invention relates to FcRn-binding silent antibodies against human insulin-like growth factor 1 receptor (IGF-1R), methods for their production, pharmaceutical compositions containing these antibodies, and uses thereof.

**BACKGROUND**

Insulin-like growth factor 1 receptor (IGF-1R, EC 2.7.10.1, CD 221 antigen) belongs to the family of transmembrane protein tyrosine kinases (LeRoith, D., et al., Endocrin. Rev. 16 (1995) 143-163; and Adams, T.E., et al., Cell. Mol. Life Sci. 57 (2000) 1050-1063). IGF-1R binds IGF-1 with high affinity and initiates the physiological response to this ligand in vivo. IGF-1R also binds to IGF-2, however with slightly lower affinity. IGF-1R overexpression promotes the neoplastic transformation of cells and there exists evidence that IGF-1R is involved in malignant transformation of cells and is therefore a useful target for the development of therapeutic agents for the treatment of cancer (Adams, T.E., et al., Cell. Mol. Life Sci. 57 (2000) 1050-1063).

Angiogenesis is implicated in the pathogenesis of a variety of disorders which include solid tumors, intraocular neovascular syndromes such as proliferative retinopathies or age-related macular degeneration (AMD), rheumatoid arthritis, and psoriasis (Folkman, J., et al., J. Biol. Chem. 267 (1992) 10931-10934; Klagsbrun, M., et al., Annu. Rev. Physiol. 53 (1991) 217-239; and Garner, A., Vascular diseases, in: Pathobiology of ocular disease, A dynamic approach, Garner, A., and Klintworth, G. K. (eds.), 2nd edition, Marcel Dekker, New York (1994), pp. 1625-1710).

Vascular eye diseases such as age related macular degeneration (AMD) and diabetic retinopathy (DR) are due to abnormal choroidal or retinal neovascularization, respectively. They are the leading causes of visual loss in industrialized nations. Since the retina consists of well-defined layers of neuronal, glial, and vascular elements, relatively small disturbances such as those seen in vascular proliferation or edema can lead to significant loss of visual function. Inherited retinal degenerations, such as Retinitis Pigmentosa (RP), are also associated with vascular abnormalities, such as arteriolar narrowing and vascular atrophy. They affect as many as 1 in 3,500 individuals and are characterized by progressive night blindness, visual field loss, optic nerve atrophy, arteriolar attenuation, and central loss of vision often progressing to complete blindness.

Ischemic retinopathies are characterized by loss or dysfunction of the retinal vasculature which results in a reduction of blood flow and hypoxia. The retina responds to hypoxia by generating signals to grow new blood vessels, but these new vessels are usually fragile and disorganized. It is the growth of these abnormal new vessels that creates most of the threat to vision since they can leak, lead to hemorrhage or lead to scarring that may end in retinal detachment. Current treatments for ischemic retinopathies seek to halt the growth of the pathological vessels but do not address the underlying ischemia that drives their growth. Furthermore, standard treatment for diabetic retinopathy, an ischemic retinopathy that affects millions, involves destruction of a portion of the retina with a laser in an attempt to stop new vessel growth and preserve central vision. Strategies have been employed to block the function of vascular endothelial growth factor (VEGF), a major promoter of vessel growth. In the short term, anti-VEGF therapy can improve vision, but it does not address the underlying ischemia and in fact may exacerbate this condition as it inhibits all vessel growth, including beneficial collaterals. There is also the serious concern of systemic exposure of these drugs in elderly and/or diabetic patients where new vessel growth may be required in ischemic brains, hearts or limbs.

Typically for ocular diseases often smaller antibody fragments like Fab or Fab$_2$ are used via intravitreal application as they have a low serum half-life and the risk of systemic toxicities is lower. However this smaller fragments typically have also lower intravitreal half-lives (e.g. due to the faster diffusion into serum) and have to be dosed typically more often.

Kim et al. (Kim, H., et al., Invest. Ophthalmol. Vis. Sci. 49 (2008) 2025-2029) report that except for the retinal pigment epithelial and choroid tissue, ocular tissues, including the ciliary body and iris, retina, conjunctiva, cornea, lens, and optic nerve bundle, showed the presence of FcRn transcript at the predicted size. The blood-ocular barrier showed FcRn receptor expression, indicating that IgG transport from ocular tissues to the blood system may use this receptor. Since the inner ocular tissues such as the retina are separated from the blood system by the blood-ocular barrier, one would not expect to detect a full-length antibody in the blood system only a short time after intravitreous injection. However, recent pharmacokinetic data from monkey and humans all indicate that intravitreous bevacizumab appears in the blood within hours after intravitreous injection. Therefore, it may be that the function of the FcRn receptor in the conjunctival lymphatic vessels is to act as an efflux receptor for the efficient elimination of antigen-antibody IgG complexes from the conjunctival space. Despite similar molecular weights, IgG (150 kDa) was detected in the aqueous humor; however, IgA (160 kDa) was not. The discrepancy between IgG and IgA penetration from the serum into the aqueous humor may be explained by the presence of the FcRn receptors, which are selective for IgGs.

Kim et al. further report (Kim, H., et al., Mol. Vis. 15 (2009) 2803-2812) that direct intravitreal injection has become a common approach for delivering therapeutic antibodies to the posterior segment of the eye for retina disorders. Both intravitreally administered bevacizumab (IgG) and chicken IgY overcame the inner limiting membrane barrier and diffused into the deeper retinal structures. After diffusing through the retina bevacizumab crossed the blood-retina barrier and leaked into the systemic circulation. The intraretinal chicken IgY was only localized along the abluminal side of the blood-retina barrier. Furthermore, the choroidal blood vessels were negative for the presence of chicken IgY. Physiologically relevant serum levels of bevacizumab after intravitreal administration, representing up to 30 % of the injected dose, were found. This suggests greater risk for systemic side effects than previously recognized. The blood-ocular barrier manifests a specific mechanism for transporting and clearing full-length IgGs into the systemic circulation. Their current study confirms the hypothesis that this mechanism is the neonatal Fc-receptor.

There is a need in the art for better means for treating and preventing various vascular eye diseases such as ischemic retinopathies.

In WO 2008/077546 antibodies against insulin-like growth factor I receptor and uses thereof are reported.

In WO 2009/126304 therapeutic combinations of anti-IGF-1R antibodies and other compounds are reported.

Magdelaine-Beuzelin, C., et al. report for therapeutic antibodies in ophthalmology that old is new again (MABS 2 (2010) 176-180).

Steinbrook, R., report the price of sight - Ranibizumab, Bevacizumab, and the treatment of macular degeneration (New Eng. J. Med.355 (2006) 1409-1412).

Kim, J.K., et al. report the mapping of the site on human IgG for binding of the MHC class I-related receptor, FcRn (Eur. J. Immunol. 29 (1999) 2819-2825).

Kuo, T.T., et al. report about the neonatal Fc receptor: from immunity to therapeutics (J. Clin. Immunol. 30 (2010) 777-789).

Kuo, T.T, et al. report about neonatal Fc receptor and IgG-based therapeutics (MABS 3 (2011) 422-438).

Medesan, C., et al. report the delineation of the amino acid residues involved in transcytosis and catabolism of mouse IgG1 (J. Immunol. 158 (1997) 2211-2217).

Qiao, S.-W., et al. report the dependence of antibody-mediated presentation of antigen on FcRn (Proc. Natl. Acad. Sci. USA 105 (2008) 9337-9342).

In WO 2006/031370 polypeptide variants with altered effector function are reported.

## SUMMARY

It has been found that anti-IGF-1R antibodies with abolished FcRn-binding can be used for the treatment of IGF-1R-related disorders in the eye (vascular eye diseases).

One aspect as reported herein is the use of an anti-IGF-1R antibody with abolished human neonatal Fc-receptor (FcRn) binding for inhibition of IGF-1R in the eye.

One aspect as reported herein is the use of an anti-IGF-1R antibody with abolished human neonatal Fc-receptor (FcRn) binding for the treatment of vascular eye diseases.

In one embodiment the anti-IGF-1R antibody has no (remaining) detectable FcRn binding using a surface plasmon resonance based determination method.

In one embodiment the anti-IGF-1R antibody binds to human FcRn with a $K_D$-value of more than 1.7 $\mu$M at pH 6, i.e. with low affinity.

In one embodiment of all aspects the antibody has the same or a shorter retention time on an FcRn affinity chromatography column as an antibody comprising an Fc-region with the mutations I253A/H310A/H435A or H310A/H433A/Y436A or L251D/L314D/L432D or combinations thereof (numbering according to Kabat EU index numbering system).

In one embodiment of all aspects the antibody has a retention time on an FcRn affinity chromatography column of three minutes or less.

In one embodiment the FcRn affinity column has the column dimensions of 50 mm x 5 mm, the bed height is 5 cm and the loading is 50 μg antibody. In one embodiment the equilibration buffer is 20 mM MES, with 150 mM NaCl, adjusted to pH 5.5, the elution buffer is 20 mM Tris/HCl, with 150 mM NaCl, adjusted to pH 8.8 and the elution is by applying 7.5 CV equilibration buffer, from 0 % to 100 % elution buffer in 30 CV, and thereafter 10 CV elution buffer.

In one embodiment the anti-IGF-1R antibody does not bind to a human FcRn column due to single or multiple point mutations in the CH2 and/or CH3 domain of the anti-IGF-1R antibody. In one embodiment the antibody has a comparable, i.e. within +/- 10 %, or shorter retention time on a FcRn column as an anti IGF-1R antibody with 4 point mutations (HC1: I253A, H310A, H435A; HC2: H310A).

In one embodiment the anti-IGF-1R antibody binds to human FcRn with about the same or lesser affinity than an antibody comprising an Fc-region with at least one of the mutations L251D, M252T, I253A, S254W, S254R, H310A, N434L, H435A, Y436A (numbering according to Kabat EU index numbering system).

In one embodiment the anti-IGF-1R antibody binds to human FcRn with about the same or lesser affinity than an antibody comprising an Fc-region with the mutations I253A/H310A/H435A or H310A/H433A/Y436A or L251D/L314D/L432D or combinations thereof (numbering according to Kabat EU index numbering system).

In one embodiment the anti-IGF-1R antibody binds to human FcRn with about the same or lesser affinity than an antibody with a heavy chain amino acid sequence of SEQ ID NO: 01 or SEQ ID NO: 96 and a light chain amino acid sequence of SEQ ID NO: 03, or than an antibody with a heavy chain amino acid sequence of SEQ ID NO: 02 or SEQ ID NO: 96 and a light chain amino acid sequence of SEQ ID NO: 04.

In one embodiment the anti-IGF-1R antibody has at least one of the mutations L251D, M252T, I253A, S254W, S254R, H310A, H433A, N434L, H435A, Y436A (numbering according to Kabat EU index numbering system) or a combination thereof.

In one embodiment the anti-IGF-1R antibody has at least one of the mutations L251D, M252T, I253A, S254W, S254R, H310A, H433A, N434L, H435A, Y436A (numbering according to Kabat EU index numbering system) in the first Fc-region polypeptide and at least one of the mutations L251D, M252T, I253A, S254W, S254R, H310A, H433A, N434L, H435A, Y436A (numbering according to Kabat EU index numbering system) in the second Fc-region polypeptide.

In one embodiment the anti-IGF-1R antibody has at least the mutations I253A/H310A/H435A or H310A/H433A/Y436A or L251D/L314D/L432D or combinations thereof (numbering according to Kabat EU index numbering system) in the first Fc-region polypeptide and at least one of the mutations L251D, M252T, I253A, S254W, S254R, H310A, H433A, N434G, N434L, H435A, Y436A (numbering according to Kabat EU index numbering system) in the second Fc-region polypeptide.

In one embodiment the anti-IGF-1R antibody has at least the mutations I253A/H310A/H435A or H310A/H433A/Y436A or L251D/L314D/L432D or combinations thereof (numbering according to Kabat EU index numbering system) in both Fc-region polypeptides.

In one embodiment the anti-IGF-1R antibody has

a) an antibody heavy chain comprising as HVRs amino acid residues 31-35 (HVR-H1), amino acid residues 50-66 (HVR-H2) and amino acid residues 99-107 (HVR-H3) of SEQ ID NO: 01 or SEQ ID NO: 02, and

b) an antibody light chain comprising as HVRs amino acid residues 24-34 (HVR-L1), amino acid residues 50-56 (HVR-L2) and amino acid residues 89-98 (HVR-L3) of SEQ ID NO: 03 or SEQ ID NO: 04.

In one preferred embodiment the anti-IGF-1R antibody has

a) an antibody heavy chain comprising as HVRs amino acid residues 31-35 (HVR-H1), amino acid residues 50-66 (HVR-H2) and amino acid residues 99-107 (HVR-H3) of SEQ ID NO: 01, and

b) an antibody light chain comprising as HVRs amino acid residues 24-34 (HVR-L1), amino acid residues 50-56 (HVR-L2) and amino acid residues 89-98 (HVR-L3) of SEQ ID NO: 03.

These are the sequences of AK18 (<IGF-1R> HUMAB Clone 18) which is described in detail in WO 2005/005635.

In another preferred embodiment the anti-IGF-1R antibody comprised a modified heavy chain variable domain (VH) and light chain variable domain (VL), and the respective HVRs, derived from AK18 (<IGF-1R> HUMAB Clone 18) with improved affinity as described in detail in WO 2013/041462. This affinity improved anti-IGF-1R antibodies in combination with the Fc-region mutations as described herein are especially useful for the treatment of the respective vascular eye and inflammatory diseases.

Therefore, in one preferred embodiment the anti-IGF-1R antibody has

a) an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3), or

b) an antibody heavy chain comprising as HVRs the amino acid sequence of SEQ ID NO: 64 (HVR-H1), the amino acid sequence of SEQ ID NO: 65 (HVR-H2), and the amino acid sequence of SEQ ID NO: 66 (HVR-H3), and an antibody light chain comprising as HVRs the amino acid sequence of SEQ ID NO: 67 (HVR-L1), the amino acid sequence of SEQ ID NO: 68 (HVR-L2), and the amino acid sequence of SEQ ID NO: 69 (HVR-L3), or

c) an antibody heavy chain comprising as HVRs the amino acid sequence of SEQ ID NO: 72 (HVR-H1), the amino acid sequence of SEQ ID NO: 73 (HVR-H2), and the amino acid sequence of SEQ ID NO: 74 (HVR-H3), and an antibody light chain comprising as HVRs the amino acid sequence of SEQ ID NO: 75 (HVR-L1), the amino acid sequence of SEQ ID NO: 76 (HVR-L2), and the amino acid sequence of SEQ ID NO: 77 (HVR-L3), or

d) an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3), or

e) an antibody heavy chain comprising as HVRs the amino acid sequence of SEQ ID NO: 88 (HVR-H1), the amino acid sequence of SEQ ID NO: 89 (HVR-H2), and the amino acid sequence of SEQ ID NO: 90 (HVR-H3), and an antibody light chain comprising as HVRs the amino acid sequence of SEQ ID NO: 91 (HVR-L1), the amino acid sequence of SEQ ID NO: 92 (HVR-L2), and the amino acid sequence of SEQ ID NO: 93 (HVR-L3).

Therefore, in another preferred embodiment the anti-IGF-1R antibody comprises

a) a heavy chain variable domain VH of SEQ ID NO: 62 and a light chain variable domain VL of SEQ ID NO: 63, or
b) a heavy chain variable domain VH of SEQ ID NO: 70 and a light chain variable domain VL of SEQ ID NO: 71, or
c) a heavy chain variable domain VH of SEQ ID NO: 78 and a light chain variable domain VL of SEQ ID NO: 79, or
d) a heavy chain variable domain VH of SEQ ID NO: 86 and a light chain variable domain VL of SEQ ID NO: 87, or
e) a heavy chain variable domain VH of SEQ ID NO: 94 and a light chain variable domain VL of SEQ ID NO: 95.

Therefore, in one preferred embodiment the anti-IGF-1R antibody has

an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3).

Therefore, in another preferred embodiment the anti-IGF-1R antibody has

an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3).

Therefore, in one preferred embodiment the anti-IGF-1R antibody comprises

a) a heavy chain variable domain VH of SEQ ID NO: 62 and a light chain variable domain VL of SEQ ID NO: 63.

Therefore, in another preferred embodiment the anti-IGF-1R antibody comprises

d) a heavy chain variable domain VH of SEQ ID NO: 86 and a light chain variable domain VL of SEQ ID NO: 87.

In one embodiment the anti-IGF-1R antibody has a heterodimeric Fc-region.
In one embodiment the antibody is an isolated antibody.
In one embodiment the antibody is a full-length antibody.
In one embodiment the antibody is a monoclonal antibody.
In one embodiment the antibody is a human, humanized or chimeric antibody.
In one embodiment the antibody comprises a VH sequence of SEQ ID NO: 05 or SEQ ID NO: 06.

In one embodiment the antibody comprises a VL sequence of SEQ ID NO: 07 or SEQ ID NO: 08.

In one embodiment the antibody comprises a VH sequence of SEQ ID NO: 05 and a VL sequence of SEQ ID NO: 07.

In one embodiment the antibody comprises a VH sequence of SEQ ID NO: 06 and a VL sequence of SEQ ID NO: 08.

In one embodiment the antibody comprises an Fc-region comprising a first Fc-region polypeptide and a second Fc-region polypeptide wherein

i) the first and the second Fc-region polypeptide comprise the mutation Y436A, or

ii) the first and the second Fc-region polypeptide comprise the mutations I253A, H310A and H435A, or

iii) the first and the second Fc-region polypeptide comprise the mutations H310A, H433A and Y436A, or

iv) the first and the second Fc-region polypeptide comprise the mutations L251D, L314D and L432D, or

v) the first and the second Fc-region polypeptide comprise the mutations L251S, L314S and L432S, or

vi) the first Fc-region polypeptide comprises the mutation Y436A and the second Fc-region polypeptide comprises

    a) the mutations I253A, H310A and H435A, or
    b) the mutations H310A, H433A and Y436A, or
    c) the mutations L251D, L314D and L432D, or
    d) the mutations L251S, L314S and L432S,

or

vii) the first Fc-region polypeptide comprises the mutations I253A, H310A and H435A and the second Fc-region polypeptide comprises

    a) the mutations H310A, H433A and Y436A, or
    b) the mutations L251D, L314D and L432D, or
    c) the mutations L251S, L314S and L432S,

or

viii) the first Fc-region polypeptide comprises the mutations H310A, H433A and Y436A and the second Fc-region polypeptide comprises

    a) the mutations L251D, L314D and L432D, or
    b) the mutations L251S, L314S and L432S,

or

ix) the first Fc-region polypeptide comprises the mutations L251D, L314D and L432D and the second Fc-region polypeptide comprises the mutations L251S, L314S and L432S.

In one embodiment of all aspects the antibody does not specifically bind to the human FcRn. In one embodiment of all aspects the antibody does not specifically bind to Staphylococcal protein A.

In one embodiment of all aspects the antibody does not specifically bind to the human FcRn. In one embodiment of all aspects the antibody does specifically bind to Staphylococcal protein A.

In one embodiment of all aspects the antibody comprises an Fc-region comprising a first and a second Fc-region polypeptide both of human IgG1 or human IgG4 subclass (derived from human origin) which comprise one or two of the mutations selected from i) the group I253A, H310A and H435A, or ii) the group H310A, H433A and Y436A, or iii) the group L251D, L314D and L432D (numbering according to Kabat EU index numbering system) in the first Fc-region polypeptide and one or two of the mutations selected from the group comprising the mutations L251D, I253A, H310A, L314D, L432D, H433A, H435A and Y436A (numbering according to Kabat EU index numbering system) in the second Fc-region polypeptide so that all of the mutations i) I253A, H310A and H435A, or ii) H310A, H433A and Y436A, or iii) L251D, L314D and L432D are comprised in the variant (human) IgG class Fc-region.

In one embodiment of all aspects the antibody comprises an Fc-region comprising a first and a second Fc-region polypeptide both of human IgG1 or human IgG4 subclass (i.e. derived from human origin), which comprise the mutations I253A/H310A/H435A or H310A/H433A/Y436A or L251D/L314D/L432D or combinations thereof in the Fc-region (numbering according to Kabat EU index numbering system), whereby i) all mutations are in the first or the second Fc-region polypeptide, or ii) one or two mutations are in the first Fc-region polypeptide and one or two mutations are in the second Fc-region polypeptide so that all of the mutations i) I253A, H310A and H435A, or ii) H310A, H433A and Y436A, or iii) L251D, L314D and L432D are comprised in the Fc-region.

In one embodiment of all aspects the antibody comprises an Fc-region comprising a first and a second Fc-region polypeptide both of human IgG1 or human IgG4 subclass (i.e. derived from human origin), which comprise the mutations I253A/H310A/H435A or H310A/H433A/Y436A or L251D/L314D/L432D in the first as well as in the second Fc-region polypeptide (numbering according to Kabat EU index numbering system), or which comprise the mutations I253A/H310A/H435A in the first Fc-region polypeptide and the mutations H310A/H433A/Y436A in the second Fc-region polypeptide (numbering according to Kabat EU index numbering system).

In one embodiment of all aspects the second Fc-region polypeptide further comprises the mutations Y349C, T366S, L368A and Y407V ("hole") and the first Fc-region polypeptide further comprises the mutations S354C and T366W ("knob").

In one embodiment of all aspects the Fc-region polypeptides are of the human IgG1 subclass. In one embodiment the first Fc-region polypeptide and the second Fc-region polypeptide further comprise the mutations L234A and L235A. In one embodiment the first Fc-region polypeptide and the second Fc-region polypeptide further comprise the mutation P329G.

In one embodiment of all aspects the Fc-region polypeptides are of the human IgG4 subclass. In one embodiment the first Fc-region polypeptide and the second Fc-region polypeptide further comprise the mutations S228P and L235E. In one embodiment the first Fc-region polypeptide and the second Fc-region polypeptide further comprise the mutation P329G.

One aspect as reported herein is method of treatment of patient suffering from ocular vascular diseases by administering an anti-IGF-1R antibody as reported herein to a patient in the need of such treatment.

In one embodiment the anti-IGF-1R antibody has no (remaining) detectable FcRn binding using a surface plasmon resonance based determination method.

In one embodiment the anti-IGF-1R antibody binds to human FcRn with a $K_D$-value of more than 1.7 $\mu$M at pH 6, i.e. with low affinity.

In one embodiment of all aspects the antibody has the same or a shorter retention time on an FcRn affinity chromatography column as an antibody comprising an Fc-region with the mutations I253A/H310A/H435A or H310A/H433A/Y436A or L251D/L314D/L432D or combinations thereof (numbering according to Kabat EU index numbering system).

In one embodiment of all aspects the antibody has a retention time on an FcRn affinity chromatography column of three minutes or less.

In one embodiment the FcRn affinity column has the column dimensions of 50 mm x 5 mm, the bed height is 5 cm and the loading is 50 $\mu$g antibody. In one embodiment the equilibration buffer is 20 mM MES, with 150 mM NaCl, adjusted to pH 5.5, the elution buffer is 20 mM Tris/HCl, with 150 mM NaCl, adjusted to pH 8.8 and the elution is by applying 7.5 CV equilibration buffer, from 0 % to 100 % elution buffer in 30 CV, and thereafter 10 CV elution buffer.

In one embodiment the anti-IGF-1R antibody does not bind to a human FcRn column due to single or multiple point mutations in the CH2 and/or CH3 domain of the anti-IGF-1R antibody. In one embodiment the antibody has a comparable, i.e. within +/- 10 %, or shorter retention time on a FcRn column as an anti IGF-1R antibody with 4 point mutations (HC1: I253A, H310A, H435A; HC2: H310A).

In one embodiment the anti-IGF-1R antibody binds to human FcRn with about the same or lesser affinity than an antibody comprising an Fc-region with at least one of the mutations L251D, M252T, I253A, S254W, S254R, H310A, N434L, H435A, Y436A (numbering according to Kabat EU index numbering system).

In one embodiment the anti-IGF-1R antibody binds to human FcRn with about the same or lesser affinity than an antibody comprising an Fc-region with the mutations I253A/H310A/H435A or H310A/H433A/Y436A or L251D/L314D/L432D or combinations thereof (numbering according to Kabat EU index numbering system).

In one embodiment the anti-IGF-1R antibody binds to human FcRn with about the same or lesser affinity than an antibody with a heavy chain amino acid sequence of SEQ ID NO: 01 or SEQ ID NO: 96 and a light chain amino acid sequence of SEQ ID NO: 03, or than an antibody with a heavy chain amino acid sequence of SEQ ID NO: 02 or SEQ ID NO: 96 and a light chain amino acid sequence of SEQ ID NO: 04.

In one embodiment the anti-IGF-1R antibody has at least one of the mutations L251D, M252T, I253A, S254W, S254R, H310A, H433A, N434L, H435A, Y436A (numbering according to Kabat EU index numbering system) or a combination thereof.

In one embodiment the anti-IGF-1R antibody has at least one of the mutations L251D, M252T, I253A, S254W, S254R, H310A, H433A, N434L, H435A, Y436A (numbering according to Kabat EU index numbering system) in the first Fc-region polypeptide and at least one of the mutations L251D, M252T, I253A, S254W, S254R, H310A, H433A, N434L, H435A, Y436A (numbering according to Kabat EU index numbering system) in the second Fc-region polypeptide.

In one embodiment the anti-IGF-1R antibody has at least the mutations I253A/H310A/H435A or H310A/H433A/Y436A or L251D/L314D/L432D or combinations thereof (numbering according to Kabat EU index numbering system) in the first Fc-region polypeptide and at least one of the mutations L251D, M252T, I253A, S254W, S254R, H310A, H433A, N434G, N434L, H435A, Y436A (numbering according to Kabat EU index numbering system) in the second Fc-region polypeptide.

In one embodiment the anti-IGF-1R antibody has at least the mutations I253A/H310A/H435A or H310A/H433A/Y436A or L251D/L314D/L432D or combinations thereof (numbering according to Kabat EU index numbering system) in both Fc-region polypeptides.

In one embodiment the anti-IGF-1R antibody has

a) an antibody heavy chain comprising as HVRs amino acid residues 31-35 (HVR-H1), amino acid residues 50-66 (HVR-H2) and amino acid residues 99-107 (HVR-H3) of SEQ ID NO: 01 or SEQ ID NO: 02, and

b) an antibody light chain comprising as HVRs amino acid residues 24-34 (HVR-L1), amino acid residues 50-56 (HVR-L2) and amino acid residues 89-98 (HVR-L3) of SEQ ID NO: 03 or SEQ ID NO: 04.

In one preferred embodiment the anti-IGF-1R antibody has

a) an antibody heavy chain comprising as HVRs amino acid residues 31-35 (HVR-H1), amino acid residues 50-66 (HVR-H2) and amino acid residues 99-107 (HVR-H3) of SEQ ID NO: 01, and

b) an antibody light chain comprising as HVRs amino acid residues 24-34 (HVR-L1), amino acid residues 50-56 (HVR-L2) and amino acid residues 89-98 (HVR-L3) of SEQ ID NO: 03.

These are the sequences of AK18 (<IGF-1R> HUMAB Clone 18) which is described in detail in WO 2005/005635.

In another preferred embodiment the anti-IGF-1R antibody comprised a modified heavy chain variable domain (VH) and light chain variable domain (VL), and the respective HVRs, derived from AK18 (<IGF-1R> HUMAB Clone 18) with improved affinity as described in detail in WO 2013/041462. This affinity improved anti-IGF-1R antibodies in combination with the Fc-region mutations as described herein are especially useful for the treatment of the respective vascular eye and inflammatory diseases.

Therefore, in one preferred embodiment the anti-IGF-1R antibody has

a) an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3), or

b) an antibody heavy chain comprising as HVRs the amino acid sequence of SEQ ID NO: 64 (HVR-H1), the amino acid sequence of SEQ ID NO: 65 (HVR-H2), and the amino acid sequence of SEQ ID NO: 66 (HVR-H3), and an antibody light chain comprising as HVRs the amino acid sequence of SEQ ID NO: 67 (HVR-L1), the amino acid sequence of SEQ ID NO: 68 (HVR-L2), and the amino acid sequence of SEQ ID NO: 69 (HVR-L3), or

c) an antibody heavy chain comprising as HVRs the amino acid sequence of SEQ ID NO: 72 (HVR-H1), the amino acid sequence of SEQ ID NO: 73 (HVR-H2), and the amino acid sequence of SEQ ID NO: 74 (HVR-H3), and an antibody light chain comprising as HVRs the amino acid sequence of SEQ ID NO: 75 (HVR-L1), the amino acid sequence of SEQ ID NO: 76 (HVR-L2), and the amino acid sequence of SEQ ID NO: 77 (HVR-L3), or

d) an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3), or

e) an antibody heavy chain comprising as HVRs the amino acid sequence of SEQ ID NO: 88 (HVR-H1), the amino acid sequence of SEQ ID NO: 89 (HVR-H2), and the amino acid sequence of SEQ ID NO: 90 (HVR-H3), and an antibody light chain comprising as HVRs the amino acid sequence of SEQ ID NO: 91 (HVR-L1), the amino acid sequence of SEQ ID NO: 92 (HVR-L2), and the amino acid sequence of SEQ ID NO: 93 (HVR-L3).

Therefore, in another preferred embodiment the anti-IGF-1R antibody comprises

a) a heavy chain variable domain VH of SEQ ID NO: 62 and a light chain variable domain VL of SEQ ID NO: 63, or
b) a heavy chain variable domain VH of SEQ ID NO: 70 and a light chain variable domain VL of SEQ ID NO: 71, or
c) a heavy chain variable domain VH of SEQ ID NO: 78 and a light chain variable domain VL of SEQ ID NO: 79, or
d) a heavy chain variable domain VH of SEQ ID NO: 86 and a light chain variable domain VL of SEQ ID NO: 87, or
e) a heavy chain variable domain VH of SEQ ID NO: 94 and a light chain variable domain VL of SEQ ID NO: 95.

Therefore, in one preferred embodiment the anti-IGF-1R antibody has

an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3).

Therefore, in another preferred embodiment the anti-IGF-1R antibody has

an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3).

Therefore, in one preferred embodiment the anti-IGF-1R antibody comprises

a) a heavy chain variable domain VH of SEQ ID NO: 62 and a light chain variable domain VL of SEQ ID NO: 63.

Therefore, in another preferred embodiment the anti-IGF-1R antibody comprises

d) a heavy chain variable domain VH of SEQ ID NO: 86 and a light chain variable domain VL of SEQ ID NO: 87.

In one embodiment the anti-IGF-1R antibody has a heterodimeric Fc-region.
In one embodiment the antibody is an isolated antibody.
In one embodiment the antibody is a full-length antibody.
In one embodiment the antibody is a monoclonal antibody.
In one embodiment the antibody is a human, humanized or chimeric antibody.
In one embodiment the antibody comprises a VH sequence of SEQ ID NO: 05 or SEQ ID NO: 06.
In one embodiment the antibody comprises a VL sequence of SEQ ID NO: 07 or SEQ ID NO: 08.
In one embodiment the antibody comprises a VH sequence of SEQ ID NO: 05 and a VL sequence of SEQ ID NO: 07.
In one embodiment the antibody comprises a VH sequence of SEQ ID NO: 06 and a VL sequence of SEQ ID NO: 08.
In one embodiment the antibody comprises an Fc-region comprising a first Fc-region polypeptide and a second Fc-region polypeptide wherein

i) the first and the second Fc-region polypeptide comprise the mutation Y436A, or

ii) the first and the second Fc-region polypeptide comprise the mutations I253A, H310A and H435A, or

iii) the first and the second Fc-region polypeptide comprise the mutations H310A, H433A and Y436A, or

iv) the first and the second Fc-region polypeptide comprise the mutations L251D, L314D and L432D, or

v) the first and the second Fc-region polypeptide comprise the mutations L251S, L314S and L432S, or

vi) the first Fc-region polypeptide comprises the mutation Y436A and the second Fc-region polypeptide comprises

a) the mutations I253A, H310A and H435A, or
b) the mutations H310A, H433A and Y436A, or
c) the mutations L251D, L314D and L432D, or
d) the mutations L251S, L314S and L432S,

or

vii) the first Fc-region polypeptide comprises the mutations I253A, H310A and H435A and the second Fc-region polypeptide comprises

a) the mutations H310A, H433A and Y436A, or
b) the mutations L251D, L314D and L432D, or
c) the mutations L251S, L314S and L432S,

or

viii) the first Fc-region polypeptide comprises the mutations H310A, H433A and Y436A and the second Fc-region polypeptide comprises

a) the mutations L251D, L314D and L432D, or
b) the mutations L251S, L314S and L432S,

or

ix) the first Fc-region polypeptide comprises the mutations L251D, L314D and L432D and the second Fc-region polypeptide comprises the mutations L251S, L314S and L432S.

In one embodiment of all aspects the antibody does not specifically bind to the human FcRn. In one embodiment of all aspects the antibody does not specifically bind to Staphylococcal protein A.

In one embodiment of all aspects the antibody does not specifically bind to the human FcRn. In one embodiment of all aspects the antibody does specifically bind to Staphylococcal protein A.

In one embodiment of all aspects the antibody comprises an Fc-region comprising a first and a second Fc-region polypeptide both of human IgG1 or human IgG4 subclass (derived from human origin) which comprise one or two of the mutations selected from i) the group I253A, H310A and H435A, or ii) the group H310A, H433A and Y436A, or iii) the group L251D, L314D and L432D (numbering according to Kabat EU index numbering system) in the first Fc-region polypeptide and one or two of the mutations selected from the group comprising the mutations L251D, I253A, H310A, L314D, L432D, H433A, H435A and Y436A (numbering according to Kabat EU index numbering system) in the second Fc-region polypeptide so that all of the mutations i) I253A, H310A and H435A, or ii) H310A, H433A and Y436A, or iii) L251D, L314D and L432D are comprised in the variant (human) IgG class Fc-region.

In one embodiment of all aspects the antibody comprises an Fc-region comprising a first and a second Fc-region polypeptide both of human IgG1 or human IgG4 subclass (i.e. derived from human origin), which comprise the mutations I253A/H310A/H435A or H310A/H433A/Y436A or L251D/L314D/L432D or combinations thereof in the Fc-region (numbering according to Kabat EU index numbering system), whereby i) all mutations are in the first or the second Fc-region polypeptide, or ii) one or two mutations are in the first Fc-region polypeptide and one or two mutations are in the second Fc-region polypeptide so that all of the mutations i) I253A, H310A and H435A, or ii) H310A, H433A and Y436A, or iii) L251D, L314D and L432D are comprised in the Fc-region.

In one embodiment of all aspects the antibody comprises an Fc-region comprising a first and a second Fc-region polypeptide both of human IgG1 or human IgG4 subclass (i.e. derived from human origin), which comprise the mutations I253A/H310A/H435A or H310A/H433A/Y436A or L251D/L314D/L432D in the first as well as in the second Fc-region polypeptide (numbering according to Kabat EU index numbering system), or which comprise the mutations I253A/H310A/H435A in the first Fc-region polypeptide and the mutations H310A/H433A/Y436A in the second Fc-region polypeptide (numbering according to Kabat EU index numbering system).

In one embodiment of all aspects the second Fc-region polypeptide further comprises the mutations Y349C, T366S, L368A and Y407V ("hole") and the first Fc-region polypeptide further comprises the mutations S354C and T366W ("knob").

In one embodiment of all aspects the Fc-region polypeptides are of the human IgG1 subclass. In one embodiment the first Fc-region polypeptide and the second Fc-region polypeptide further comprise the mutations L234A and L235A. In one embodiment the first Fc-region polypeptide and the second Fc-region polypeptide further comprise the mutation P329G.

In one embodiment of all aspects the Fc-region polypeptides are of the human IgG4 subclass. In one embodiment the first Fc-region polypeptide and the second Fc-region polypeptide further comprise the mutations S228P and L235E. In one embodiment the first Fc-region polypeptide and the second Fc-region polypeptide further comprise the mutation P329G.

One aspect as reported herein is an anti-IGF-1R antibody with abolished human neonatal Fc-receptor (FcRn) binding for intravitreal application.

One aspect as reported herein is an anti-IGF-1R antibody with abolished human neonatal Fc-receptor (FcRn) binding for the treatment of vascular eye diseases.

In one embodiment the anti-IGF-1R antibody has no (remaining) detectable FcRn binding using a surface plasmon resonance based determination method.

In one embodiment the anti-IGF-1R antibody binds to human FcRn with a $K_D$-value of more than 1.7 $\mu$M at pH 6, i.e. with low affinity.

In one embodiment of all aspects the antibody has the same or a shorter retention time on an FcRn affinity chromatography column as an antibody comprising an Fc-region with the mutations I253A/H310A/H435A or H310A/H433A/Y436A or L251D/L314D/L432D or combinations thereof (numbering according to Kabat EU index numbering system).

In one embodiment of all aspects the antibody has a retention time on an FcRn affinity chromatography column of three minutes or less.

In one embodiment the FcRn affinity column has the column dimensions of 50 mm x 5 mm, the bed height is 5 cm and the loading is 50 $\mu$g antibody. In one embodiment the equilibration buffer is 20 mM MES, with 150 mM NaCl, adjusted to pH 5.5, the elution buffer is 20 mM Tris/HCl, with 150 mM NaCl, adjusted to pH 8.8 and the elution is by applying 7.5 CV equilibration buffer, from 0 % to 100 % elution buffer in 30 CV, and thereafter 10 CV elution buffer.

In one embodiment the anti-IGF-1R antibody does not bind to a human FcRn column due to single or multiple point mutations in the CH2 and/or CH3 domain of the anti-IGF-1R antibody. In one embodiment the antibody has a comparable, i.e. within +/- 10 %, or shorter retention time on a FcRn column as an anti IGF-1R antibody with 4 point mutations (HC1: I253A, H310A, H435A; HC2: H310A).

In one embodiment the anti-IGF-1R antibody binds to human FcRn with about the same or lesser affinity than an antibody comprising an Fc-region with at least one of the mutations L251D, M252T, I253A, S254W, S254R, H310A, N434L, H435A, Y436A (numbering according to Kabat EU index numbering system).

In one embodiment the anti-IGF-1R antibody binds to human FcRn with about the same or lesser affinity than an antibody comprising an Fc-region with the mutations I253A/H310A/H435A or H310A/H433A/Y436A or L251D/L314D/L432D or combinations thereof (numbering according to Kabat EU index numbering system).

In one embodiment the anti-IGF-1R antibody binds to human FcRn with about the same or lesser affinity than an antibody with a heavy chain amino acid sequence of SEQ ID NO: 01 or SEQ ID NO: 96 and a light chain amino acid sequence of SEQ ID NO: 03, or than an antibody with a heavy chain amino acid sequence of SEQ ID NO: 02 or SEQ ID NO: 96 and a light chain amino acid sequence of SEQ ID NO: 04.

In one embodiment the anti-IGF-1R antibody has at least one of the mutations L251D, M252T, I253A, S254W, S254R, H310A, H433A, N434L, H435A, Y436A (numbering according to Kabat EU index numbering system) or a combination thereof.

In one embodiment the anti-IGF-1R antibody has at least one of the mutations L251D, M252T, I253A, S254W, S254R, H310A, H433A, N434L, H435A, Y436A (numbering according to Kabat EU index numbering system) in the first Fc-region polypeptide and at least one of the mutations L251D, M252T, I253A, S254W, S254R, H310A, H433A, N434L, H435A, Y436A (numbering according to Kabat EU index numbering system) in the second Fc-region polypeptide.

In one embodiment the anti-IGF-1R antibody has at least the mutations I253A/H310A/H435A or H310A/H433A/Y436A or L251D/L314D/L432D or combinations thereof (numbering according to Kabat EU index numbering system) in the first Fc-region polypeptide and at least one of the mutations L251D, M252T, I253A, S254W, S254R, H310A, H433A, N434G, N434L, H435A, Y436A (numbering according to Kabat EU index numbering system) in the second Fc-region polypeptide.

In one embodiment the anti-IGF-1R antibody has at least the mutations I253A/H310A/H435A or H310A/H433A/Y436A or L251D/L314D/L432D or combinations thereof (numbering according to Kabat EU index numbering system) in both Fc-region polypeptides.

In one embodiment the anti-IGF-1R antibody has

a) an antibody heavy chain comprising as HVRs amino acid residues 31-35 (HVR-H1), amino acid residues 50-66 (HVR-H2) and amino acid residues 99-107 (HVR-H3) of SEQ ID NO: 01 or SEQ ID NO: 02, and

b) an antibody light chain comprising as HVRs amino acid residues 24-34 (HVR-L1), amino acid residues 50-56 (HVR-L2) and amino acid residues 89-98 (HVR-L3) of SEQ ID NO: 03 or SEQ ID NO: 04.

In one preferred embodiment the anti-IGF-1R antibody has

a) an antibody heavy chain comprising as HVRs amino acid residues 31-35 (HVR-H1), amino acid residues 50-66 (HVR-H2) and amino acid residues 99-107 (HVR-H3) of SEQ ID NO: 01, and

b) an antibody light chain comprising as HVRs amino acid residues 24-34 (HVR-L1), amino acid residues 50-56 (HVR-L2) and amino acid residues 89-98 (HVR-L3) of SEQ ID NO: 03.

These are the sequences of AK18 (<IGF-1R> HUMAB Clone 18) which is described in detail in WO 2005/005635.
In another preferred embodiment the anti-IGF-1R antibody comprised a modified heavy chain variable domain (VH) and light chain variable domain (VL), and the respective HVRs, derived from AK18 (<IGF-1R> HUMAB Clone 18) with improved affinity as described in detail in WO 2013/041462. This affinity improved anti-IGF-1R antibodies in combination with the Fc-region mutations as described herein are especially useful for the treatment of the respective vascular eye and inflammatory diseases.
Therefore, in one preferred embodiment the anti-IGF-1R antibody has

a) an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3), or

b) an antibody heavy chain comprising as HVRs the amino acid sequence of SEQ ID NO: 64 (HVR-H1), the amino acid sequence of SEQ ID NO: 65 (HVR-H2), and the amino acid sequence of SEQ ID NO: 66 (HVR-H3), and an antibody light chain comprising as HVRs the amino acid sequence of SEQ ID NO: 67 (HVR-L1), the amino acid sequence of SEQ ID NO: 68 (HVR-L2), and the amino acid sequence of SEQ ID NO: 69 (HVR-L3), or

c) an antibody heavy chain comprising as HVRs the amino acid sequence of SEQ ID NO: 72 (HVR-H1), the amino acid sequence of SEQ ID NO: 73 (HVR-H2), and the amino acid sequence of SEQ ID NO: 74 (HVR-H3), and an antibody light chain comprising as HVRs the amino acid sequence of SEQ ID NO: 75 (HVR-L1), the amino acid sequence of SEQ ID NO: 76 (HVR-L2), and the amino acid sequence of SEQ ID NO: 77 (HVR-L3), or

d) an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3), or

e) an antibody heavy chain comprising as HVRs the amino acid sequence of SEQ ID NO: 88 (HVR-H1), the amino acid sequence of SEQ ID NO: 89 (HVR-H2), and the amino acid sequence of SEQ ID NO: 90 (HVR-H3), and an antibody light chain comprising as HVRs the amino acid sequence of SEQ ID NO: 91 (HVR-L1), the amino acid sequence of SEQ ID NO: 92 (HVR-L2), and the amino acid sequence of SEQ ID NO: 93 (HVR-L3).

Therefore, in another preferred embodiment the anti-IGF-1R antibody comprises

a) a heavy chain variable domain VH of SEQ ID NO: 62 and a light chain variable domain VL of SEQ ID NO: 63, or
b) a heavy chain variable domain VH of SEQ ID NO: 70 and a light chain variable domain VL of SEQ ID NO: 71, or
c) a heavy chain variable domain VH of SEQ ID NO: 78 and a light chain variable domain VL of SEQ ID NO: 79, or
d) a heavy chain variable domain VH of SEQ ID NO: 86 and a light chain variable domain VL of SEQ ID NO: 87, or
e) a heavy chain variable domain VH of SEQ ID NO: 94 and a light chain variable domain VL of SEQ ID NO: 95.

Therefore, in one preferred embodiment the anti-IGF-1R antibody has

an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3).

Therefore, in another preferred embodiment the anti-IGF-1R antibody has

an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3).

Therefore, in one preferred embodiment the anti-IGF-1R antibody comprises

a) a heavy chain variable domain VH of SEQ ID NO: 62 and a light chain variable domain VL of SEQ ID NO: 63.

Therefore, in another preferred embodiment the anti-IGF-1R antibody comprises

d) a heavy chain variable domain VH of SEQ ID NO: 86 and a light chain variable domain VL of SEQ ID NO: 87.

In one embodiment the anti-IGF-1R antibody has a heterodimeric Fc-region.
In one embodiment the antibody is an isolated antibody.
In one embodiment the antibody is a full-length antibody.
In one embodiment the antibody is a monoclonal antibody.
In one embodiment the antibody is a human, humanized or chimeric antibody.
In one embodiment the antibody comprises a VH sequence of SEQ ID NO: 05 or SEQ ID NO: 06.
In one embodiment the antibody comprises a VL sequence of SEQ ID NO: 07 or SEQ ID NO: 08.
In one embodiment the antibody comprises a VH sequence of SEQ ID NO: 05 and a VL sequence of SEQ ID NO: 07.
In one embodiment the antibody comprises a VH sequence of SEQ ID NO: 06 and a VL sequence of SEQ ID NO: 08.
In one embodiment the antibody comprises an Fc-region comprising a first Fc-region polypeptide and a second Fc-region polypeptide wherein

i) the first and the second Fc-region polypeptide comprise the mutation Y436A, or

ii) the first and the second Fc-region polypeptide comprise the mutations I253A, H310A and H435A, or

iii) the first and the second Fc-region polypeptide comprise the mutations H310A, H433A and Y436A, or

iv) the first and the second Fc-region polypeptide comprise the mutations L251D, L314D and L432D, or

v) the first and the second Fc-region polypeptide comprise the mutations L251S, L314S and L432S, or

vi) the first Fc-region polypeptide comprises the mutation Y436A and the second Fc-region polypeptide comprises

a) the mutations I253A, H310A and H435A, or
b) the mutations H310A, H433A and Y436A, or
c) the mutations L251D, L314D and L432D, or
d) the mutations L251S, L314S and L432S,

or

vii) the first Fc-region polypeptide comprises the mutations I253A, H310A and H435A and the second Fc-region polypeptide comprises

a) the mutations H310A, H433A and Y436A, or
b) the mutations L251D, L314D and L432D, or
c) the mutations L251S, L314S and L432S,

or

viii) the first Fc-region polypeptide comprises the mutations H310A, H433A and Y436A and the second Fc-region polypeptide comprises

a) the mutations L251D, L314D and L432D, or
b) the mutations L251S, L314S and L432S,

or

ix) the first Fc-region polypeptide comprises the mutations L251D, L314D and L432D and the second Fc-region polypeptide comprises the mutations L251S, L314S and L432S.

In one embodiment of all aspects the antibody does not specifically bind to the human FcRn. In one embodiment of all aspects the antibody does not specifically bind to Staphylococcal protein A.

In one embodiment of all aspects the antibody does not specifically bind to the human FcRn. In one embodiment of all aspects the antibody does specifically bind to Staphylococcal protein A.

In one embodiment of all aspects the antibody comprises an Fc-region comprising a first and a second Fc-region polypeptide both of human IgG1 or human IgG4 subclass (derived from human origin) which comprise one or two of the mutations selected from i) the group I253A, H310A and H435A, or ii) the group H310A, H433A and Y436A, or iii) the group L251D, L314D and L432D (numbering according to Kabat EU index numbering system) in the first Fc-region polypeptide and one or two of the mutations selected from the group comprising the mutations L251D, I253A, H310A, L314D, L432D, H433A, H435A and Y436A (numbering according to Kabat EU index numbering system) in the second Fc-region polypeptide so that all of the mutations i) I253A, H310A and H435A, or ii) H310A, H433A and Y436A, or iii) L251D, L314D and L432D are comprised in the variant (human) IgG class Fc-region.

In one embodiment of all aspects the antibody comprises an Fc-region comprising a first and a second Fc-region polypeptide both of human IgG1 or human IgG4 subclass (i.e. derived from human origin), which comprise the mutations I253A/H310A/H435A or H310A/H433A/Y436A or L251D/L314D/L432D or combinations thereof in the Fc-region (numbering according to Kabat EU index numbering system), whereby i) all mutations are in the first or the second Fc-region polypeptide, or ii) one or two mutations are in the first Fc-region polypeptide and one or two mutations are in the second Fc-region polypeptide so that all of the mutations i) I253A, H310A and H435A, or ii) H310A, H433A and Y436A, or iii) L251D, L314D and L432D are comprised in the Fc-region.

In one embodiment of all aspects the antibody comprises an Fc-region comprising a first and a second Fc-region polypeptide both of human IgG1 or human IgG4 subclass (i.e. derived from human origin), which comprise the mutations I253A/H310A/H435A or H310A/H433A/Y436A or L251D/L314D/L432D in the first as well as in the second Fc-region polypeptide (numbering according to Kabat EU index numbering system), or which comprise the mutations I253A/H310A/H435A in the first Fc-region polypeptide and the mutations H310A/H433A/Y436A in the second Fc-region polypeptide (numbering according to Kabat EU index numbering system).

In one embodiment of all aspects the second Fc-region polypeptide further comprises the mutations Y349C, T366S, L368A and Y407V ("hole") and the first Fc-region polypeptide further comprises the mutations S354C and T366W ("knob").

In one embodiment of all aspects the Fc-region polypeptides are of the human IgG1 subclass. In one embodiment the first Fc-region polypeptide and the second Fc-region polypeptide further comprise the mutations L234A and L235A. In one embodiment the first Fc-region polypeptide and the second Fc-region polypeptide further comprise the mutation P329G.

In one embodiment of all aspects the Fc-region polypeptides are of the human IgG4 subclass. In one embodiment the first Fc-region polypeptide and the second Fc-region polypeptide further comprise the mutations S228P and L235E. In one embodiment the first Fc-region polypeptide and the second Fc-region polypeptide further comprise the mutation P329G.

One aspect as reported herein is a pharmaceutical formulation comprising an anti-IGF-1R antibody with abolished human neonatal Fc-receptor (FcRn) binding and optionally a pharmaceutically acceptable carrier.

In one embodiment the anti-IGF-1R antibody has no (remaining) detectable FcRn binding using a surface plasmon resonance based determination method.

In one embodiment the anti-IGF-1R antibody binds to human FcRn with a $K_D$-value of more than 1.7 $\mu$M at pH 6, i.e. with low affinity.

In one embodiment of all aspects the antibody has the same or a shorter retention time on an FcRn affinity chromatography column as an antibody comprising an Fc-region with the mutations I253A/H310A/H435A or H310A/H433A/Y436A or L251D/L314D/L432D or combinations thereof (numbering according to Kabat EU index numbering system).

In one embodiment of all aspects the antibody has a retention time on an FcRn affinity chromatography column of three minutes or less.

In one embodiment the FcRn affinity column has the column dimensions of 50 mm x 5 mm, the bed height is 5 cm and the loading is 50 μg antibody. In one embodiment the equilibration buffer is 20 mM MES, with 150 mM NaCl, adjusted to pH 5.5, the elution buffer is 20 mM Tris/HCl, with 150 mM NaCl, adjusted to pH 8.8 and the elution is by applying 7.5 CV equilibration buffer, from 0 % to 100 % elution buffer in 30 CV, and thereafter 10 CV elution buffer.

In one embodiment the anti-IGF-1R antibody does not bind to a human FcRn column due to single or multiple point mutations in the CH2 and/or CH3 domain of the anti-IGF-1R antibody. In one embodiment the antibody has a comparable, i.e. within +/- 10 %, or shorter retention time on a FcRn column as an anti IGF-1R antibody with 4 point mutations (HC1: I253A, H310A, H435A; HC2: H310A).

In one embodiment the anti-IGF-1R antibody binds to human FcRn with about the same or lesser affinity than an antibody comprising an Fc-region with at least one of the mutations L251D, M252T, I253A, S254W, S254R, H310A, N434L, H435A, Y436A (numbering according to Kabat EU index numbering system).

In one embodiment the anti-IGF-1R antibody binds to human FcRn with about the same or lesser affinity than an antibody comprising an Fc-region with the mutations I253A/H310A/H435A or H310A/H433A/Y436A or L251D/L314D/L432D or combinations thereof (numbering according to Kabat EU index numbering system).

In one embodiment the anti-IGF-1R antibody binds to human FcRn with about the same or lesser affinity than an antibody with a heavy chain amino acid sequence of SEQ ID NO: 01 or SEQ ID NO: 96 and a light chain amino acid sequence of SEQ ID NO: 03, or than an antibody with a heavy chain amino acid sequence of SEQ ID NO: 02 or SEQ ID NO: 96 and a light chain amino acid sequence of SEQ ID NO: 04.

In one embodiment the anti-IGF-1R antibody has at least one of the mutations L251D, M252T, I253A, S254W, S254R, H310A, H433A, N434L, H435A, Y436A (numbering according to Kabat EU index numbering system) or a combination thereof.

In one embodiment the anti-IGF-1R antibody has at least one of the mutations L251D, M252T, I253A, S254W, S254R, H310A, H433A, N434L, H435A, Y436A (numbering according to Kabat EU index numbering system) in the first Fc-region polypeptide and at least one of the mutations L251D, M252T, I253A, S254W, S254R, H310A, H433A, N434L, H435A, Y436A (numbering according to Kabat EU index numbering system) in the second Fc-region polypeptide.

In one embodiment the anti-IGF-1R antibody has at least the mutations I253A/H310A/H435A or H310A/H433A/Y436A or L251D/L314D/L432D or combinations thereof (numbering according to Kabat EU index numbering system) in the first Fc-region polypeptide and at least one of the mutations L251D, M252T, I253A, S254W, S254R, H310A, H433A, N434G, N434L, H435A, Y436A (numbering according to Kabat EU index numbering system) in the second Fc-region polypeptide.

In one embodiment the anti-IGF-1R antibody has at least the mutations I253A/H310A/H435A or H310A/H433A/Y436A or L251D/L314D/L432D or combinations thereof (numbering according to Kabat EU index numbering system) in both Fc-region polypeptides.

In one embodiment the anti-IGF-1R antibody has

a) an antibody heavy chain comprising as HVRs amino acid residues 31-35 (HVR-H1), amino acid residues 50-66 (HVR-H2) and amino acid residues 99-107 (HVR-H3) of SEQ ID NO: 01 or SEQ ID NO: 02, and

b) an antibody light chain comprising as HVRs amino acid residues 24-34 (HVR-L1), amino acid residues 50-56 (HVR-L2) and amino acid residues 89-98 (HVR-L3) of SEQ ID NO: 03 or SEQ ID NO: 04.

In one preferred embodiment the anti-IGF-1R antibody has

a) an antibody heavy chain comprising as HVRs amino acid residues 31-35 (HVR-H1), amino acid residues 50-66 (HVR-H2) and amino acid residues 99-107 (HVR-H3) of SEQ ID NO: 01, and

b) an antibody light chain comprising as HVRs amino acid residues 24-34 (HVR-L1), amino acid residues 50-56 (HVR-L2) and amino acid residues 89-98 (HVR-L3) of SEQ ID NO: 03.

These are the sequences of AK18 (<IGF-1R> HUMAB Clone 18) which is described in detail in WO 2005/005635.

In another preferred embodiment the anti-IGF-1R antibody comprised a modified heavy chain variable domain (VH) and light chain variable domain (VL), and the respective HVRs, derived from AK18 (<IGF-1R> HUMAB Clone 18) with improved affinity as described in detail in WO 2013/041462. This affinity improved anti-IGF-1R antibodies in combination with the Fc-region mutations as described herein are especially useful for the treatment of the respective vascular eye and inflammatory diseases.

Therefore, in one preferred embodiment the anti-IGF-1R antibody has

a) an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3), or

b) an antibody heavy chain comprising as HVRs the amino acid sequence of SEQ ID NO: 64 (HVR-H1), the amino acid sequence of SEQ ID NO: 65 (HVR-H2), and the amino acid sequence of SEQ ID NO: 66 (HVR-H3), and an antibody light chain comprising as HVRs the amino acid sequence of SEQ ID NO: 67 (HVR-L1), the amino acid sequence of SEQ ID NO: 68 (HVR-L2), and the amino acid sequence of SEQ ID NO: 69 (HVR-L3), or

c) an antibody heavy chain comprising as HVRs the amino acid sequence of SEQ ID NO: 72 (HVR-H1), the amino acid sequence of SEQ ID NO: 73 (HVR-H2), and the amino acid sequence of SEQ ID NO: 74 (HVR-H3), and an antibody light chain comprising as HVRs the amino acid sequence of SEQ ID NO: 75 (HVR-L1), the amino acid sequence of SEQ ID NO: 76 (HVR-L2), and the amino acid sequence of SEQ ID NO: 77 (HVR-L3), or

d) an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3), or

e) an antibody heavy chain comprising as HVRs the amino acid sequence of SEQ ID NO: 88 (HVR-H1), the amino acid sequence of SEQ ID NO: 89 (HVR-H2), and the amino acid sequence of SEQ ID NO: 90 (HVR-H3), and an antibody light chain comprising as HVRs the amino acid sequence of SEQ ID NO: 91 (HVR-L1), the amino acid sequence of SEQ ID NO: 92 (HVR-L2), and the amino acid sequence of SEQ ID NO: 93 (HVR-L3).

Therefore, in another preferred embodiment the anti-IGF-1R antibody comprises

a) a heavy chain variable domain VH of SEQ ID NO: 62 and a light chain variable domain VL of SEQ ID NO: 63, or
b) a heavy chain variable domain VH of SEQ ID NO: 70 and a light chain variable domain VL of SEQ ID NO: 71, or
c) a heavy chain variable domain VH of SEQ ID NO: 78 and a light chain variable domain VL of SEQ ID NO: 79, or
d) a heavy chain variable domain VH of SEQ ID NO: 86 and a light chain variable domain VL of SEQ ID NO: 87, or
e) a heavy chain variable domain VH of SEQ ID NO: 94 and a light chain variable domain VL of SEQ ID NO: 95.

Therefore, in one preferred embodiment the anti-IGF-1R antibody has

an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3).

Therefore, in another preferred embodiment the anti-IGF-1R antibody has

an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3).

Therefore, in one preferred embodiment the anti-IGF-1R antibody comprises

a) a heavy chain variable domain VH of SEQ ID NO: 62 and a light chain variable domain VL of SEQ ID NO: 63.

Therefore, in another preferred embodiment the anti-IGF-1R antibody comprises

d) a heavy chain variable domain VH of SEQ ID NO: 86 and a light chain variable domain VL of SEQ ID NO: 87.

In one embodiment the anti-IGF-1R antibody has a heterodimeric Fc-region.
In one embodiment the antibody is an isolated antibody.
In one embodiment the antibody is a full-length antibody.
In one embodiment the antibody is a monoclonal antibody.

In one embodiment the antibody is a human, humanized or chimeric antibody.

In one embodiment the antibody comprises a VH sequence of SEQ ID NO: 05 or SEQ ID NO: 06.

In one embodiment the antibody comprises a VL sequence of SEQ ID NO: 07 or SEQ ID NO: 08.

In one embodiment the antibody comprises a VH sequence of SEQ ID NO: 05 and a VL sequence of SEQ ID NO: 07.

In one embodiment the antibody comprises a VH sequence of SEQ ID NO: 06 and a VL sequence of SEQ ID NO: 08.

In one embodiment the antibody comprises an Fc-region comprising a first Fc-region polypeptide and a second Fc-region polypeptide wherein

i) the first and the second Fc-region polypeptide comprise the mutation Y436A, or

ii) the first and the second Fc-region polypeptide comprise the mutations I253A, H310A and H435A, or

iii) the first and the second Fc-region polypeptide comprise the mutations H310A, H433A and Y436A, or

iv) the first and the second Fc-region polypeptide comprise the mutations L251D, L314D and L432D, or

v) the first and the second Fc-region polypeptide comprise the mutations L251S, L314S and L432S, or

vi) the first Fc-region polypeptide comprises the mutation Y436A and the second Fc-region polypeptide comprises

a) the mutations I253A, H310A and H435A, or
b) the mutations H310A, H433A and Y436A, or
c) the mutations L251D, L314D and L432D, or
d) the mutations L251S, L314S and L432S,

or

vii) the first Fc-region polypeptide comprises the mutations I253A, H310A and H435A and the second Fc-region polypeptide comprises

a) the mutations H310A, H433A and Y436A, or
b) the mutations L251D, L314D and L432D, or
c) the mutations L251S, L314S and L432S,

or

viii) the first Fc-region polypeptide comprises the mutations H310A, H433A and Y436A and the second Fc-region polypeptide comprises

a) the mutations L251D, L314D and L432D, or
b) the mutations L251S, L314S and L432S,

or

ix) the first Fc-region polypeptide comprises the mutations L251D, L314D and L432D and the second Fc-region polypeptide comprises the mutations L251S, L314S and L432S.

In one embodiment of all aspects the antibody does not specifically bind to the human FcRn. In one embodiment of all aspects the antibody does not specifically bind to Staphylococcal protein A.

In one embodiment of all aspects the antibody does not specifically bind to the human FcRn. In one embodiment of all aspects the antibody does specifically bind to Staphylococcal protein A.

In one embodiment of all aspects the antibody comprises an Fc-region comprising a first and a second Fc-region polypeptide both of human IgG1 or human IgG4 subclass (derived from human origin) which comprise one or two of the mutations selected from i) the group I253A, H310A and H435A, or ii) the group H310A, H433A and Y436A, or iii) the group L251D, L314D and L432D (numbering according to Kabat EU index numbering system) in the first Fc-region polypeptide and one or two of the mutations selected from the group comprising the mutations L251D, I253A, H310A, L314D, L432D, H433A, H435A and Y436A (numbering according to Kabat EU index numbering system) in the second Fc-region polypeptide so that all of the mutations i) I253A, H310A and H435A, or ii) H310A, H433A and Y436A, or iii) L251D, L314D and L432D are comprised in the variant (human) IgG class Fc-region.

In one embodiment of all aspects the antibody comprises an Fc-region comprising a first and a second Fc-region polypeptide both of human IgG1 or human IgG4 subclass (i.e. derived from human origin), which comprise the mutations I253A/H310A/H435A or H310A/H433A/Y436A or L251D/L314D/L432D or combinations thereof in the Fc-region (numbering according to Kabat EU index numbering system), whereby i) all mutations are in the first or the second Fc-region polypeptide, or ii) one or two mutations are in the first Fc-region polypeptide and one or two mutations are in the second Fc-region polypeptide so that all of the mutations i) I253A, H310A and H435A, or ii) H310A, H433A and Y436A, or iii) L251D, L314D and L432D are comprised in the Fc-region.

In one embodiment of all aspects the antibody comprises an Fc-region comprising a first and a second Fc-region polypeptide both of human IgG1 or human IgG4 subclass (i.e. derived from human origin), which comprise the mutations I253A/H310A/H435A or H310A/H433A/Y436A or L251D/L314D/L432D in the first as well as in the second Fc-region polypeptide (numbering according to Kabat EU index numbering system), or which comprise the mutations I253A/H310A/H435A in the first Fc-region polypeptide and the mutations H310A/H433A/Y436A in the second Fc-region polypeptide (numbering according to Kabat EU index numbering system).

In one embodiment of all aspects the second Fc-region polypeptide further comprises the mutations Y349C, T366S, L368A and Y407V ("hole") and the first Fc-region polypeptide further comprises the mutations S354C and T366W ("knob").

In one embodiment of all aspects the Fc-region polypeptides are of the human IgG1 subclass. In one embodiment the first Fc-region polypeptide and the second Fc-region polypeptide further comprise the mutations L234A and L235A. In one embodiment the first Fc-region polypeptide and the second Fc-region polypeptide further comprise the mutation P329G.

In one embodiment of all aspects the Fc-region polypeptides are of the human IgG4 subclass. In one embodiment the first Fc-region polypeptide and the second Fc-region polypeptide further comprise the mutations S228P and L235E. In one embodiment the first Fc-region polypeptide and the second Fc-region polypeptide further comprise the mutation P329G.

One aspect as reported herein is an anti-IGF-1R antibody with abolished human neonatal Fc-receptor (FcRn) binding.

In one embodiment the anti-IGF-1R antibody has no (remaining) detectable FcRn binding using a surface plasmon resonance based determination method.

In one embodiment the anti-IGF-1R antibody binds to human FcRn with a $K_D$-value of more than 1.7 $\mu$M at pH 6, i.e. with low affinity.

In one embodiment of all aspects the antibody has the same or a shorter retention time on an FcRn affinity chromatography column as an antibody comprising an Fc-region with the mutations I253A/H310A/H435A or H310A/H433A/Y436A or L251D/L314D/L432D or combinations thereof (numbering according to Kabat EU index numbering system).

In one embodiment of all aspects the antibody has a retention time on an FcRn affinity chromatography column of three minutes or less.

In one embodiment the FcRn affinity column has the column dimensions of 50 mm x 5 mm, the bed height is 5 cm and the loading is 50 $\mu$g antibody. In one embodiment the equilibration buffer is 20 mM MES, with 150 mM NaCl, adjusted to pH 5.5, the elution buffer is 20 mM Tris/HCl, with 150 mM NaCl, adjusted to pH 8.8 and the elution is by applying 7.5 CV equilibration buffer, from 0 % to 100 % elution buffer in 30 CV, and thereafter 10 CV elution buffer.

In one embodiment the anti-IGF-1R antibody does not bind to a human FcRn column due to single or multiple point mutations in the CH2 and/or CH3 domain of the anti-IGF-1R antibody. In one embodiment the antibody has a comparable, i.e. within +/- 10 %, or shorter retention time on a FcRn column as an anti IGF-1R antibody with 4 point mutations (HC1: I253A, H310A, H435A; HC2: H310A).

In one embodiment the anti-IGF-1R antibody binds to human FcRn with about the same or lesser affinity than an antibody comprising an Fc-region with at least one of the mutations L251D, M252T, I253A, S254W, S254R, H310A, N434L, H435A, Y436A (numbering according to Kabat EU index numbering system).

In one embodiment the anti-IGF-1R antibody binds to human FcRn with about the same or lesser affinity than an antibody comprising an Fc-region with the mutations I253A/H310A/H435A or H310A/H433A/Y436A or L251D/L314D/L432D or combinations thereof (numbering according to Kabat EU index numbering system).

In one embodiment the anti-IGF-1R antibody binds to human FcRn with about the same or lesser affinity than an antibody with a heavy chain amino acid sequence of SEQ ID NO: 01 or SEQ ID NO: 96 and a light chain amino acid sequence of SEQ ID NO: 03, or than an antibody with a heavy chain amino acid sequence of SEQ ID NO: 02 or SEQ ID NO: 96 and a light chain amino acid sequence of SEQ ID NO: 04.

In one embodiment the anti-IGF-1R antibody has at least one of the mutations L251D, M252T, I253A, S254W, S254R, H310A, H433A, N434L, H435A, Y436A (numbering according to Kabat EU index numbering system) or a combination thereof.

In one embodiment the anti-IGF-1R antibody has at least one of the mutations L251D, M252T, I253A, S254W, S254R, H310A, H433A, N434L, H435A, Y436A (numbering according to Kabat EU index numbering system) in the first Fc-region polypeptide and at least one of the mutations L251D, M252T, I253A, S254W, S254R, H310A, H433A, N434L, H435A, Y436A (numbering according to Kabat EU index numbering system) in the second Fc-region polypeptide.

In one embodiment the anti-IGF-1R antibody has at least the mutations I253A/H310A/H435A or H310A/H433A/Y436A or L251D/L314D/L432D or combinations thereof (numbering according to Kabat EU index numbering system) in the first Fc-region polypeptide and at least one of the mutations L251D, M252T, 1253A, S254W, S254R, H310A, H433A, N434G, N434L, H435A, Y436A

(numbering according to Kabat EU index numbering system) in the second Fc-region polypeptide.

In one embodiment the anti-IGF-1R antibody has at least the mutations I253A/H310A/H435A or H310A/H433A/Y436A or L251D/L314D/L432D or combinations thereof (numbering according to Kabat EU index numbering system) in both Fc-region polypeptides.

In one embodiment the anti-IGF-1R antibody has

a) an antibody heavy chain comprising as HVRs amino acid residues 31-35 (HVR-H1), amino acid residues 50-66 (HVR-H2) and amino acid residues 99-107 (HVR-H3) of SEQ ID NO: 01 or SEQ ID NO: 02, and

b) an antibody light chain comprising as HVRs amino acid residues 24-34 (HVR-L1), amino acid residues 50-56 (HVR-L2) and amino acid residues 89-98 (HVR-L3) of SEQ ID NO: 03 or SEQ ID NO: 04.

In one preferred embodiment the anti-IGF-1R antibody has

a) an antibody heavy chain comprising as HVRs amino acid residues 31-35 (HVR-H1), amino acid residues 50-66 (HVR-H2) and amino acid residues 99-107 (HVR-H3) of SEQ ID NO: 01, and

b) an antibody light chain comprising as HVRs amino acid residues 24-34 (HVR-L1), amino acid residues 50-56 (HVR-L2) and amino acid residues 89-98 (HVR-L3) of SEQ ID NO: 03.

These are the sequences of AK18 (<IGF-1R> HUMAB Clone 18) which is described in detail in WO 2005/005635.

In another preferred embodiment the anti-IGF-1R antibody comprised a modified heavy chain variable domain (VH) and light chain variable domain (VL), and the respective HVRs, derived from AK18 (<IGF-1R> HUMAB Clone 18) with improved affinity as described in detail in WO 2013/041462. This affinity improved anti-IGF-1R antibodies in combination with the Fc-region mutations as described herein are especially useful for the treatment of the respective vascular eye and inflammatory diseases.

Therefore, in one preferred embodiment the anti-IGF-1R antibody has

a) an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3), or

b) an antibody heavy chain comprising as HVRs the amino acid sequence of SEQ ID NO: 64 (HVR-H1), the amino acid sequence of SEQ ID NO: 65 (HVR-H2), and the amino acid sequence of SEQ ID NO: 66 (HVR-H3), and an antibody light chain comprising as HVRs the amino acid sequence of SEQ ID NO: 67 (HVR-L1), the amino acid sequence of SEQ ID NO: 68 (HVR-L2), and the amino acid sequence of SEQ ID NO: 69 (HVR-L3), or

c) an antibody heavy chain comprising as HVRs the amino acid sequence of SEQ ID NO: 72 (HVR-H1), the amino acid sequence of SEQ ID NO: 73 (HVR-H2), and the amino acid sequence of SEQ ID NO: 74 (HVR-H3), and an antibody light chain comprising as HVRs the amino acid sequence of SEQ ID NO: 75 (HVR-L1), the amino acid sequence of SEQ ID NO: 76 (HVR-L2), and the amino acid sequence of SEQ ID NO: 77 (HVR-L3), or

d) an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3), or

e) an antibody heavy chain comprising as HVRs the amino acid sequence of SEQ ID NO: 88 (HVR-H1), the amino acid sequence of SEQ ID NO: 89 (HVR-H2), and the amino acid sequence of SEQ ID NO: 90 (HVR-H3), and an antibody light chain comprising as HVRs the amino acid sequence of SEQ ID NO: 91 (HVR-L1), the amino acid sequence of SEQ ID NO: 92 (HVR-L2), and the amino acid sequence of SEQ ID NO: 93 (HVR-L3).

Therefore, in another preferred embodiment the anti-IGF-1R antibody comprises

a) a heavy chain variable domain VH of SEQ ID NO: 62 and a light chain variable domain VL of SEQ ID NO: 63, or
b) a heavy chain variable domain VH of SEQ ID NO: 70 and a light chain variable domain VL of SEQ ID NO: 71, or
c) a heavy chain variable domain VH of SEQ ID NO: 78 and a light chain variable domain VL of SEQ ID NO: 79, or
d) a heavy chain variable domain VH of SEQ ID NO: 86 and a light chain variable domain VL of SEQ ID NO: 87, or
e) a heavy chain variable domain VH of SEQ ID NO: 94 and a light chain variable domain VL of SEQ ID NO: 95.

Therefore, in one preferred embodiment the anti-IGF-1R antibody has

an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3).

Therefore, in another preferred embodiment the anti-IGF-1R antibody has

an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3).

Therefore, in one preferred embodiment the anti-IGF-1R antibody comprises

a) a heavy chain variable domain VH of SEQ ID NO: 62 and a light chain variable domain VL of SEQ ID NO: 63.

Therefore, in another preferred embodiment the anti-IGF-1R antibody comprises

d) a heavy chain variable domain VH of SEQ ID NO: 86 and a light chain variable domain VL of SEQ ID NO: 87.

In one embodiment the anti-IGF-1R antibody has a heterodimeric Fc-region.
In one embodiment the antibody is an isolated antibody.
In one embodiment the antibody is a full-length antibody.
In one embodiment the antibody is a monoclonal antibody.
In one embodiment the antibody is a human, humanized or chimeric antibody.
In one embodiment the antibody comprises a VH sequence of SEQ ID NO: 05 or SEQ ID NO: 06.
In one embodiment the antibody comprises a VL sequence of SEQ ID NO: 07 or SEQ ID NO: 08.
In one embodiment the antibody comprises a VH sequence of SEQ ID NO: 05 and a VL sequence of SEQ ID NO: 07.
In one embodiment the antibody comprises a VH sequence of SEQ ID NO: 06 and a VL sequence of SEQ ID NO: 08.
In one embodiment the antibody comprises an Fc-region comprising a first Fc-region polypeptide and a second Fc-region polypeptide wherein

i) the first and the second Fc-region polypeptide comprise the mutation Y436A, or

ii) the first and the second Fc-region polypeptide comprise the mutations I253A, H310A and H435A, or

iii) the first and the second Fc-region polypeptide comprise the mutations H310A, H433A and Y436A, or

iv) the first and the second Fc-region polypeptide comprise the mutations L251D, L314D and L432D, or

v) the first and the second Fc-region polypeptide comprise the mutations L251S, L314S and L432S, or

vi) the first Fc-region polypeptide comprises the mutation Y436A and the second Fc-region polypeptide comprises

    a) the mutations I253A, H310A and H435A, or
    b) the mutations H310A, H433A and Y436A, or
    c) the mutations L251D, L314D and L432D, or
    d) the mutations L251S, L314S and L432S,

or

vii) the first Fc-region polypeptide comprises the mutations I253A, H310A and H435A and the second Fc-region polypeptide comprises

    a) the mutations H310A, H433A and Y436A, or
    b) the mutations L251D, L314D and L432D, or
    c) the mutations L251S, L314S and L432S,

or

viii) the first Fc-region polypeptide comprises the mutations H310A, H433A and Y436A and the second Fc-region polypeptide comprises

    a) the mutations L251D, L314D and L432D, or
    b) the mutations L251S, L314S and L432S,

or

ix) the first Fc-region polypeptide comprises the mutations L251D, L314D and L432D and the second Fc-region polypeptide comprises the mutations L251S, L314S and L432S.

In one embodiment of all aspects the antibody does not specifically bind to the human FcRn. In one embodiment of all aspects the antibody does not specifically bind to Staphylococcal protein A.

In one embodiment of all aspects the antibody does not specifically bind to the human FcRn. In one embodiment of all aspects the antibody does specifically bind to Staphylococcal protein A.

In one embodiment of all aspects the antibody comprises an Fc-region comprising a first and a second Fc-region polypeptide both of human IgG1 or human IgG4 subclass (derived from human origin) which comprise one or two of the mutations selected from i) the group I253A, H310A and H435A, or ii) the group H310A, H433A and Y436A, or iii) the group L251D, L314D and L432D (numbering according to Kabat EU index numbering system) in the first Fc-region polypeptide and one or two of the mutations selected from the group comprising the mutations L251D, I253A, H310A, L314D, L432D, H433A, H435A and Y436A (numbering according to Kabat EU index numbering system) in the second Fc-region polypeptide so that all of the mutations i) I253A, H310A and H435A, or ii) H310A, H433A and Y436A, or iii) L251D, L314D and L432D are comprised in the variant (human) IgG class Fc-region.

In one embodiment of all aspects the antibody comprises an Fc-region comprising a first and a second Fc-region polypeptide both of human IgG1 or human IgG4 subclass (i.e. derived from human origin), which comprise the mutations I253A/H310A/H435A or H310A/H433A/Y436A or L251D/L314D/L432D or combinations thereof in the Fc-region (numbering according to Kabat EU index numbering system), whereby i) all mutations are in the first or the second Fc-region polypeptide, or ii) one or two mutations are in the first Fc-region polypeptide and one or two mutations are in the second Fc-region polypeptide so that all of the mutations i) I253A, H310A and H435A, or ii) H310A, H433A and Y436A, or iii) L251D, L314D and L432D are comprised in the Fc-region.

In one embodiment of all aspects the antibody comprises an Fc-region comprising a first and a second Fc-region polypeptide both of human IgG1 or human IgG4 subclass (i.e. derived from human origin), which comprise the mutations I253A/H310A/H435A or H310A/H433A/Y436A or L251D/L314D/L432D in the first as well as in the second Fc-region polypeptide (numbering according to Kabat EU index numbering system), or which comprise the mutations I253A/H310A/H435A in the first Fc-region polypeptide and the mutations H310A/H433A/Y436A in the second Fc-region polypeptide (numbering according to Kabat EU index numbering system).

In one embodiment of all aspects the second Fc-region polypeptide further comprises the mutations Y349C, T366S, L368A and Y407V ("hole") and the first Fc-region polypeptide further comprises the mutations S354C and T366W ("knob").

In one embodiment of all aspects the Fc-region polypeptides are of the human IgG1 subclass. In one embodiment the first Fc-region polypeptide and the second Fc-region polypeptide further comprise the mutations L234A and L235A. In one embodiment the first Fc-region polypeptide and the second Fc-region polypeptide further comprise the mutation P329G.

In one embodiment of all aspects the Fc-region polypeptides are of the human IgG4 subclass. In one embodiment the first Fc-region polypeptide and the second Fc-region polypeptide further comprise the mutations S228P and L235E. In one embodiment the first Fc-region polypeptide and the second Fc-region polypeptide further comprise the mutation P329G.

One aspect as reported herein is a nucleic acid encoding the anti-IGF-1R antibody as reported herein.

One aspect as reported herein is a host cell comprising the nucleic acid encoding the anti-IGF-1R antibody as reported herein.

One aspect as reported herein is a method for producing the anti-IGF-1R antibody as reported herein comprising culturing the host cell comprising the nucleic acid encoding the anti-IGF-1R antibody as reported herein.

## BRIEF DESCRIPTION OF THE FIGURES

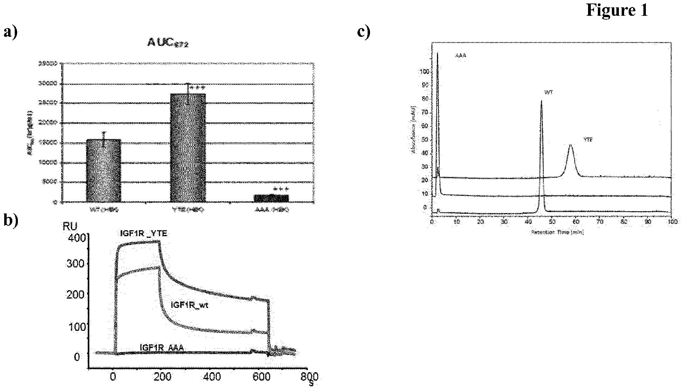

**Figure 1:** Antibodies engineered with respect to their ability to bind FcRn display prolonged (YTE) or shortened (AAA) in vivo half-lives, enhanced (YTE) or reduced FcRn binding (AAA) compared to the reference wild-type (wt) antibody in SPR analysis as well as enhanced or reduced retention time in FcRn column chromatography; a) PK data after single i.v. bolus application of 10 mg/kg into huFcRn transgenic male C57BL/6J mice +/- 276: AUC data for wild-type IgG as well as YTE and AAA Fc-modified IgGs; b) BIAcore sensorgram; c) FcRn affinity column elution; wild-type anti-IGF-1R antibody (reference), YTE-mutant of anti-IGF-1R antibody, AAA-mutant of anti-IGF-1R antibody.

**Figure 2:** Comparison of serum concentrations after intravenous application of antibodies IGF-1R 0033, 0035 and 0045.

**Figure 3:** Comparison of eye lysate concentration after intravitreal and intravenous application of antibody IGF-1R 0033.

**Figure 4:** Comparison of eye lysate concentration after intravitreal and intravenous application of antibody IGF-1R 0035.

**Figure 5:** Comparison of eye lysate concentration after intravitreal and intravenous application of antibody IGF-1R 0045.

## DETAILED DESCRIPTION OF EMBODIMENTS OF THE INVENTION

### I. DEFINITIONS

The term "about" denotes a range of +/- 20 % of the thereafter following numerical value. In one embodiment the term about denotes a range of +/- 10 % of the thereafter following numerical value. In one embodiment the term about denotes a range of +/- 5 % of the thereafter following numerical value.

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence alterations. In some embodiments, the number of amino acid alterations are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The term "alteration" denotes the mutation (substitution), insertion (addition), or deletion of one or more amino acid residues in a parent antibody or fusion polypeptide, e.g. a fusion polypeptide comprising at least an FcRn binding portion of an Fc-region, to obtain a modified antibody or fusion polypeptide. The term "mutation" denotes that the specified amino acid residue is substituted for a different amino acid residue. For example the mutation L234A denotes that the amino acid residue lysine at position 234 in an antibody Fc-region (polypeptide) is substituted by the amino acid residue alanine (substitution of lysine with alanine) (numbering according to the EU index).

The term "amino acid mutation" denotes the substitution of at least one existing amino acid residue with another different amino acid residue (= replacing amino acid residue). The replacing amino acid residue may be a "naturally occurring amino acid residue" and selected from the group consisting of alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

The term "amino acid insertion" denotes the (additional) incorporation of at least one amino acid residue at a predetermined position in an amino acid sequence. In one embodiment the insertion will be the insertion of one or two amino acid residues. The inserted amino acid residue(s) can be any naturally occurring or non-naturally occurring amino acid residue.

The term "amino acid deletion" denotes the removal of at least one amino acid residue at a predetermined position in an amino acid sequence.

The terms "anti-IGF-1R antibody" and "an antibody that binds to IGF-1R" refer to an antibody that is capable of binding to IGF-1R with sufficient affinity such that the antibody is useful as a diagnostic and/or therapeutic agent in targeting IGF-1R. In one embodiment, the extent of binding of an anti-IGF-1R antibody to an unrelated, non-IGF-1R protein is less than about 10 % of the binding of the antibody to IGF-1R as measured, e.g., by surface plasmon resonance (SPR).

The term "antibody" herein is used in the broadest sense and encompasses various antibody structures, including but not limited to monoclonal antibodies, multispecific antibodies (e.g. bispecific antibodies, trispecific antibodies), and antibody fragments so long as they exhibit the desired antigen- and/or protein A and/or FcRn-binding activity.

An "antibody that binds to the same epitope" as a reference antibody refers to an antibody that blocks binding of the reference antibody to its antigen in a competition assay by 50 % or more, and conversely, the reference antibody blocks binding of the antibody to its antigen in a competition assay by 50% or more.

The term "asymmetric Fc-region" denotes a pair of Fc-region polypeptides that have different amino acid residues at corresponding positions according to the Kabat EU index numbering system. The term "asymmetric Fc-region with respect to FcRn binding" denotes an Fc-region that consists of two polypeptide chains that have different amino acid residues at corresponding positions, whereby the positions are determined according to the Kabat EU index numbering system, whereby the different positions affect the binding of the Fc-region to the human neonatal Fc-receptor (FcRn). For the purpose herein the differences between the two polypeptide chains of the Fc-region in an "asymmetric Fc-region with respect to FcRn binding" do not include differences that have been introduced to facilitate the formation of heterodimeric Fc-regions, e.g. for the production of bispecific antibodies. These differences can also be asymmetric, i.e. the two chains have differences at non corresponding amino acid residues according to the Kabat EU index numbering system. These differences facilitate heterodimerization and reduce homodimerization. Examples of such differences are the so-called "knobs into holes" substitutions (see, e.g., US 7,695,936 and US 2003/0078385). The following knobs and holes substitutions in the individual polypeptide chains of an Fc-region of an IgG antibody of subclass IgG1 have been found to increase heterodimer formation: 1) Y407T in one chain and T366Y in the other chain; 2) Y407A in one chain and T366W in the other chain; 3) F405A in one chain and T394W in the other chain; 4) F405W in one chain and T394S in the other chain; 5) Y407T in one chain and T366Y in the other chain; 6) T366Y and F405A in one chain and T394W and Y407T in the other chain; 7) T366W and F405W in one chain and T394S and Y407A in the other chain; 8) F405W and Y407A in one chain and T366W and T394S in the other chain; and 9) T366W in one chain and T366S, L368A, and Y407V in the other chain, whereby the last listed is especially suited. In addition, changes creating new disulfide bridges between the two Fc-region polypeptide chains facilitate heterodimer formation (see, e.g., US 2003/0078385). The following substitutions resulting in appropriately spaced apart cysteine residues for the formation of new intra-chain disulfide bonds in the individual polypeptide chains of an Fc-region of an IgG antibody of subclass IgG1 have been found to increase heterodimer formation: Y349C in one chain and S354C in the other; Y349C in one chain and E356C in the other; Y349C in one chain and E357C in the other; L351C in one chain and S354C in the other; T394C in one chain and E397C in the other; or D399C in one chain and K392C in the other. Further examples of heterodimerization facilitating amino acid changes are the so-called "charge pair substitutions" (see, e.g., WO 2009/089004). The following charge pair substitutions in the individual polypeptide chains of an Fc-region of an IgG antibody of subclass IgG1 have been found to increase heterodimer formation: 1) K409D or K409E in one chain and D399K or D399R in the other chain; 2) K392D or K392E in one chain and D399K or D399R in the other chain; 3) K439D or K439E in one chain and E356K or E356R in the other chain; 4) K370D or K370E in one chain and E357K or E357R in the other chain; 5) K409D and K360D in one chain plus D399K and E356K in the other chain; 6) K409D and K370D in one chain plus D399K and E357K in the other chain; 7) K409D and K392D in one chain plus D399K, E356K, and E357K in the other chain; 8) K409D and K392D in one chain and D399K in the other chain; 9) K409D and K392D in one chain and D399K and E356K in the other chain; 10) K409D and K392D in one chain and D399K and D357K in the other chain; 11) K409D and K370D in one chain and D399K and D357K in the other chain; 12) D399K in one chain and K409D and K360D in the other chain; and 13) K409D and K439D in one chain and D399K and E356K on the other.

The term "binding to IGF-1R" denotes the binding of an antibody to IGF-1R in an in vitro assay, in one embodiment in a binding assay in which the antibody is bound to a surface and binding of IGF-1R to the antibody is measured by Surface Plasmon Resonance (SPR). Binding means a binding affinity ($K_D$) of $10^{-8}$ M or less, in some embodiments of $10^{-13}$ M to $10^{-8}$ M, in some embodiments of $10^{-13}$ to $10^{-9}$ M.

Binding to IGF-1R can be investigated by a BIAcore assay (GE Healthcare Biosensor AB, Uppsala, Sweden). The affinity of the binding is defined by the terms $k_a$ (rate constant for the association of the antibody from the antibody/antigen complex), $k_d$ (dissociation constant), and $K_D(k_d/k_a)$.

The binding of IGF-1 and IGF-2 to IGF-1R is also inhibited by the antibodies as reported herein. The inhibition is measured as $IC_{50}$ in an assay for binding of IGF-1/IGF-2 to IGF-1R on tumor cells. In such an assay, the amount of radiolabeled IGF-1 or IGF-2 or IGF-1R binding fragments thereof bound to the IGF-1R provided at the surface of the tumor cells (e.g. HT29) is measured without and with increasing concentrations of the antibody. The $IC_{50}$ values of the antibodies as reported herein for the binding of IGF-1 and IGF-2 to IGF-1R are no more than 2 nM and the ratio of the $IC_{50}$ values for binding of IGF-1/IGF-2 to IGF-1R is about 1:3 to 3:1. $IC_{50}$ values are measured as average or median values of at least three independent measurements. Single $IC_{50}$ values may be out of the scope. The amino acid sequence of the human IGF-1R is shown in SEQ ID NO: 11.

The term "chimeric" antibody refers to an antibody in which a portion of the heavy and/or light chain is derived from a particular source or species, while the remainder of the heavy and/or light chain is derived from a different source or species.

The term "CH2-domain" denotes the part of an antibody heavy chain polypeptide that extends approximately from EU position 231 to EU position 340 (EU numbering system according to Kabat). In one embodiment a CH2 domain has the amino acid sequence of SEQ ID NO: 09: APELLGG PSVFLFPPKP KDTLMISRTP EVTCVWDVS HEDPEVKFNW YVDGVEVHNA KTKPREEQ E STYRWSVLT VLHQDWLNGK EYKCKVSNKA LPAPIEKTIS KAK.

The term "CH3-domain" denotes the part of an antibody heavy chain polypeptide that extends approximately from EU position 341 to EU position 446. In one embodiment the CH3 domain has the amino acid sequence of SEQ ID NO: 10: GQPREPQ VYTLPPSRDE LTKNQVSLTC LVKGFYPSDI AVEWESNGQP ENNYKTTPPV LDSDGSFFLY SKLTVDKSRW QQGNVFSCSV MHEALHNHYT QKSLSLSPG.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$, respectively.

The term "comparable length" denotes that two polypeptides comprise the identical number of amino acid residues or can be different in length by one or more and up to 10 amino acid residues at most. In one embodiment the Fc-region polypeptides comprise the identical number of amino acid residues or differ by a number of from 1 to 10 amino acid residues. In one embodiment the Fc-region polypeptides comprise the identical number of amino acid residues or differ by a number of from 1 to 5 amino acid residues. In one embodiment the Fc-region polypeptides comprise the identical number of amino acid residues or differ by a number of from 1 to 3 amino acid residues.

The term "derived from" denotes that an amino acid sequence is derived from a parent amino acid sequence by introducing alterations at at least one position. Thus a derived amino acid sequence differs from the corresponding parent amino acid sequence at at least one corresponding position (numbering according to Kabat EU index numbering system for antibody Fc-regions). In one embodiment an amino acid sequence derived from a parent amino acid sequence differs by one to fifteen amino acid residues at corresponding positions. In one embodiment an amino acid sequence derived from a parent amino acid sequence differs by one to ten amino acid residues at corresponding positions. In one embodiment an amino acid sequence derived from a parent amino acid sequence differs by one to six amino acid residues at corresponding positions. Likewise a derived amino acid sequence has a high amino acid sequence identity to its parent amino acid sequence. In one embodiment an amino acid sequence derived from a parent amino acid sequence has 80 % or more amino acid sequence identity. In one embodiment an amino acid sequence derived from a parent amino acid sequence has 90 % or more amino acid sequence identity. In one embodiment an amino acid sequence derived from a parent amino acid sequence has 95 % or more amino acid sequence identity.

"Effector functions" refer to those biological activities attributable to the Fc-region of an antibody, which vary with the antibody class. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B-cell receptor); and B-cell activation.

An "effective amount" of an agent, e.g., a pharmaceutical formulation, refers to an amount effective, at dosages and for periods of time necessary, to achieve the desired therapeutic or prophylactic result.

The term "Fc-fusion polypeptide" denotes a fusion of a binding domain (e.g. an antigen binding domain such as a single chain antibody, or a polypeptide such as a ligand of a receptor) with an antibody Fc-region that exhibits the desired target- and/or protein A and/or FcRn-binding activity.

The term "Fc-region of human origin" denotes the C-terminal region of an immunoglobulin heavy chain of human origin that contains at least a part of the hinge region, the CH2 domain and the CH3 domain. In one embodiment, a human IgG heavy chain Fc-region extends from Cys226, or from Pro230, to the carboxyl-terminus of the heavy chain. In one embodiment the Fc-region has the amino acid sequence of SEQ ID NO: 14. However, the C-terminal lysine (Lys447) of the Fc-region may or may not be present. Unless otherwise specified herein, numbering of amino acid residues in the Fc-region or constant region is according to the EU numbering system, also called the EU index, as described in Kabat, E.A., et al., Sequences of Proteins of Immunological Interest, 5th ed., Public Health Service, National Institutes of Health, Bethesda, MD (1991), NIH Publication 91 3242. The Fc-region is composed of two heavy chain Fc-region polypeptides, which can be covalently linked to each other via the hinge region cysteine residues forming inter-polypeptide disulfide bonds.

The term "FcRn" denotes the human neonatal Fc-receptor. FcRn functions to salvage IgG from the lysosomal degradation pathway, resulting in reduced clearance and increased half-life. The FcRn is a heterodimeric protein consisting of two polypeptides: a 50 kDa class I major histocompatibility complex-like protein (a-FcRn) and a 15 kDa β2-microglobulin (β2m). FcRn binds with high affinity to the CH2-CH3 portion of the Fc-region of IgG. The interaction between IgG and FcRn is strictly pH dependent and occurs in a 1:2 stoichiometry, with one IgG binding to two FcRn molecules via its two heavy chains (Huber, A.H., et al., J. Mol. Biol. 230 (1993) 1077-1083). FcRn binding occurs in the endosome at acidic pH (pH < 6.5) and IgG is released at the neutral cell surface (pH of about 7.4). The pH-sensitive nature of the interaction facilitates the FcRn-mediated protection of IgGs pinocytosed into cells from intracellular degradation by binding to the receptor within the acidic environment of endosomes. FcRn then facilitates the recycling of IgG to the cell surface and subsequent release into the blood stream upon exposure of the FcRn-IgG complex to the neutral pH environment outside the cell.

The term "FcRn binding portion of an Fc-region" denotes the part of an antibody heavy chain polypeptide that extends approximately from EU position 243 to EU position 261 and approximately from EU position 275 to EU position 293 and approximately from EU position 302 to EU position 319 and approximately from EU position 336 to EU position 348 and approximately from EU position 367 to EU position 393 and EU position 408 and approximately from EU position 424 to EU position 440. In one embodiment one or more of the following amino acid residues according to the EU numbering of Kabat are altered F243, P244, P245 P, K246, P247, K248, D249, T250, L251, M252, 1253, S254, R255, T256, P257, E258, V259, T260, C261, F275, N276, W277, Y278, V279, D280, V282, E283, V284, H285, N286, A287, K288, T289, K290, P291, R292, E293, V302, V303, S304, V305, L306, T307, V308, L309, H310, Q311, D312, W313, L314, N315, G316, K317, E318, Y319, 1336, S337, K338, A339, K340, G341, Q342, P343, R344, E345, P346, Q347, V348, C367, V369, F372, Y373, P374, S375, D376, 1377, A378, V379, E380, W381, E382, S383, N384, G385, Q386, P387, E388, N389, Y391, T393, S408, S424, C425, S426, V427, M428, H429, E430, A431, L432, H433, N434, H435, Y436, T437, Q438, K439, and S440 (EU numbering).

"Framework" or "FR" refers to variable domain residues other than hypervariable region (HVR) residues. The FR of a variable domain generally consists of four FR domains: FR1, FR2, FR3, and FR4.Accordingly, the HVR and FR sequences generally appear in the following sequence in VH (or VL):FR1-H1(L1)-FR2-H2(L2)-FR3-H3(L3)-FR4.

The term "full length antibody" denotes an antibody having a structure substantially similar to a native antibody structure or having heavy chains that contain an Fc-region as defined herein. A full length antibody may comprise further domains, such as e.g. a scFv or a scFab conjugated to one or more of the chains of the full length antibody. These conjugates are also encompassed by the term full length antibody.

The terms "heterodimer" or "heterodimeric" denote a molecule that comprises two polypeptide chains (e.g. of comparable length), wherein the two polypeptide chains have an amino acid sequence that have at least one different amino acid residue in a corresponding position, whereby corresponding position is determined according to the EU index of Kabat.

The terms "homodimer" and "homodimeric" denote a molecule that comprises two polypeptide chains of comparable length, wherein the two polypeptide chains have an amino acid sequence that is identical in corresponding positions, whereby corresponding positions are determined according to the EU index of Kabat.

An antibody as reported herein can be homodimeric or heterodimeric with respect to its Fc-region which is determined with respect to mutations or properties in focus. For example, with respect to FcRn and/or protein A binding (i.e. the focused on properties) an Fc-region (antibody) is homodimeric (i.e. both heavy chain Fc-region polypeptides comprise these mutations) with respect to the mutations H310A, H433A and Y436A (these mutations are in focus with respect to FcRn and/or protein A binding property of the antibody) but at the same time heterodimeric with respect to the mutations Y349C, T366S, L368A and Y407V (these mutations are not in focus as these mutations are directed to the heterodimerization of the heavy chains and not to the FcRn/protein A binding properties) as well as the mutations S354C and T366W, respectively (the first set is comprised only in the first Fc-region polypeptide whereas the second set is comprised only in the second Fc-region polypeptide). Further for example, an antibody as reported herein can be heterodimeric with respect to the mutations I253A, H310A, H433A, H435A and Y436A (i.e. these mutations are directed all to the FcRn and/or protein A binding properties of the dimeric polypeptide), i.e. one Fc-region polypeptide comprises the mutations I253A, H310A and H435A, whereas the other Fc-region polypeptide comprises the mutations H310A, H433A and Y436A.

The terms "host cell", "host cell line", and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat, E.A.et al., Sequences of Proteins of Immunological Interest, 5th ed., Bethesda MD (1991), NIH Publication 91-3242, Vols. 1-3.In one embodiment, for the VL, the subgroup is subgroup kappa I as in Kabat et al., *supra.* In one embodiment, for the VH, the subgroup is subgroup III as in Kabat et al., *supra.*

The term "human Fc-region polypeptide" denotes an amino acid sequence which is identical to a "native" or "wild-type" human Fc-region polypeptide. The term "variant (human) Fc-region polypeptide" denotes an amino acid sequence which derived from a "native" or "wild-type" human Fc-region polypeptide by virtue of at least one "amino acid alteration". A "human Fc-region" is consisting of two human Fc-region polypeptides. A "variant (human) Fc-region" is consisting of two Fc-region polypeptides, whereby both can be variant (human) Fc-region polypeptides or one is a human Fc-region polypeptide and the other is a variant (human) Fc-region polypeptide. In one embodiment the human Fc-region polypeptide has the amino acid sequence of a human IgG1 Fc-region polypeptide of SEQ ID NO: 14, or of a human IgG2 Fc-region polypeptide of SEQ ID NO: 15, or of a human IgG3 Fc-region polypeptide of SEQ ID NO: 16, or of a human IgG4 Fc-region polypeptide of SEQ ID NO: 17. In one embodiment the Fc-region polypeptide is derived from an Fc-region polypeptide of SEQ ID NO: 14, or 15, or 16, or 17 and has at least one amino acid mutation compared to the Fc-region polypeptide of SEQ ID NO: 14, or 15, or 16, or 17. In one embodiment the Fc-region polypeptide comprises/has from about one to about ten amino acid mutations, and in one embodiment from about one to about five amino acid mutations. In one embodiment the Fc-region polypeptide has at least about 80 % homology with a human Fc-region polypeptide of SEQ ID NO: 14, or 15, or 16, or 17. In one embodiment the Fc-region polypeptide has least about 90 % homology with a human Fc-region polypeptide of SEQ ID NO: 14, or 15, or 16, or 17. In one embodiment the Fc-region polypeptide has at least about 95 % homology with a human Fc-region polypeptide of SEQ ID NO: 14, or 15, or 16, or 17.

The Fc-region polypeptide derived from a human Fc-region polypeptide of SEQ ID NO: 14, or 15, or 16, or 17 is defined by the amino acid alterations that are contained. Thus, for example, the term P329G denotes a human Fc-region polypeptide derived Fc-region polypeptide with the mutation of proline to glycine at amino acid position 329 relative to the human Fc-region polypeptide of SEQ ID NO: 14, or 15, or 16, or 17.

As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991) and is referred to as "numbering according to Kabat" herein. Specifically the Kabat numbering system (see pages 647-660) of Kabat, et al., Sequences of Proteins of Immunological Interest, 5th ed., Public Health Service, National Institutes of Health, Bethesda, MD (1991) is used for the light chain constant domain CL of kappa and lambda isotype and the Kabat EU index numbering system (see pages 661-723) is used for the constant heavy chain domains (CH1, Hinge, CH2 and CH3).

A human IgG1 Fc-region polypeptide has the following amino acid sequence:

DKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGF
YPSDIAVEWESNGQPENNYKTTPPVLDSDGSFFLYSKLTVDKSRWQQGNV
FSCSVMHEALHNHYTQKSLSLSPGK (SEQ ID NO: 14).

A human IgG1 Fc-region derived Fc-region polypeptide with the mutations L234A, L235A has the following amino acid sequence:

DKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGF
YPSDIAVEWESNGQPENNYKTTPPVLDSDGSFFLYSKLTVDKSRWQQGNV
FSCSVMHEALHNHYTQKSLSLSPGK (SEQ ID NO: 18).

A human IgG1 Fc-region derived Fc-region polypeptide with Y349C, T366S, L368A and Y407V mutations has the following amino acid sequence:

DKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALPAPIEKTISKAKGQPREPQVCTLPPSRDELTKNQVSLSCAVKGF
YPSDIAVEWESNGQPENNYKTTPPVLDSDGSFFLVSKLTVDKSRWQQGNV
FSCSVMHEALHNHYTQKSLSLSPGK (SEQ ID NO: 19).

A human IgG1 Fc-region derived Fc-region polypeptide with S354C, T366W mutations has the following amino acid sequence:

DKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALPAPIEKTISKAKGQPREPQVYTLPPCRDELTKNQVSLWCLVKG
FYPSDIAVEWESNGQPENNYKTTPPVLDSDGSFFLYSKLTVDKSRWQQGN
VFSCSVMHEALHNHYTQKSLSLSPGK (SEQ ID NO: 20).

A human IgG1 Fc-region derived Fc-region polypeptide with L234A, L235A mutations and Y349C, T366S, L368A, Y407V mutations has the following amino acid sequence:

DKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALPAPIEKTISKAKGQPREPQVCTLPPSRDELTKNQVSLSCAVKGF
YPSDIAVEWESNGQPENNYKTTPPVLDSDGSFFLVSKLTVDKSRWQQGNV
FSCSVMHEALHNHYTQKSLSLSPGK (SEQ ID NO: 21).

A human IgG1 Fc-region derived Fc-region polypeptide with a L234A, L235A and S354C, T366W mutations has the following amino acid sequence:

DKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALPAPIEKTISKAKGQPREPQVYTLPPCRDELTKNQVSLWCLVKG
FYPSDIAVEWESNGQPENNYKTTPPVLDSDGSFFLYSKLTVDKSRWQQGN
VFSCSVMHEALHNHYTQKSLSLSPGK (SEQ ID NO: 22).

A human IgG1 Fc-region derived Fc-region polypeptide with a P329G mutation has the following amino acid

sequence:

DKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALGAPIEKTISKAKGQPREPQVYTLPPSRDELTKNQVSLTCLVKG
FYPSDIAVEWESNGQPENNYKTTPPVLDSDGSFFLYSKLTVDKSRWQQGN
VFSCSVMHEALHNHYTQKSLSLSPGK (SEQ ID NO: 23).

A human IgG1 Fc-region derived Fc-region polypeptide with L234A, L235A mutations and P329G mutation has the following amino acid sequence:

DKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALGAPIEKTISKAKGQPREPQVYTLPPSRDELTKNQVSLTCLVKG
FYPSDIAVEWESNGQPENNYKTTPPVLDSDGSFFLYSKLTVDKSRWQQGN
VFSCSVMHEALHNHYTQKSLSLSPGK (SEQ ID NO: 24).

A human IgG1 Fc-region derived Fc-region polypeptide with a P329G mutation and Y349C, T366S, L368A, Y407V mutations has the following amino acid sequence:

DKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALGAPIEKTISKAKGQPREPQVCTLPPSRDELTKNQVSLSCAVKG
FYPSDIAVEWESNGQPENNYKTTPPVLDSDGSFFLVSKLTVDKSRWQQGN
VFSCSVMHEALHNHYTQKSLSLSPGK (SEQ ID NO: 25).

A human IgG1 Fc-region derived Fc-region polypeptide with a P329G mutation and S354C, T366W mutation has the following amino acid sequence:

DKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALGAPIEKTISKAKGQPREPQVYTLPPCRDELTKNQVSLWCLVKG
FYPSDIAVEWESNGQPENNYKTTPPVLDSDGSFFLYSKLTVDKSRWQQGN
VFSCSVMHEALHNHYTQKSLSLSPGK (SEQ ID NO: 26).

A human IgG1 Fc-region derived Fc-region polypeptide with L234A, L235A, P329G and Y349C, T366S, L368A, Y407V mutations has the following amino acid sequence:

DKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALGAPIEKTISKAKGQPREPQVCTLPPSRDELTKNQVSLSCAVKG
FYPSDIAVEWESNGQPENNYKTTPPVLDSDGSFFLVSKLTVDKSRWQQGN
VFSCSVMHEALHNHYTQKSLSLSPGK (SEQ ID NO: 27).

A human IgG1 Fc-region derived Fc-region polypeptide with L234A, L235A, P329G mutations and S354C, T366W mutations has the following amino acid sequence:

DKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALGAPIEKTISKAKGQPREPQVYTLPPCRDELTKNQVSLWCLVKG
FYPSDIAVEWESNGQPENNYKTTPPVLDSDGSFFLYSKLTVDKSRWQQGN
VFSCSVMHEALHNHYTQKSLSLSPGK (SEQ ID NO: 28).

A human IgG4 Fc-region polypeptide has the following amino acid sequence:

ESKYGPPCPSCPAPEFLGGPSVFLFPPKPKDTLMISRTPEVTCVVVDVSQED
PEVQFNWYVDGVEVHNAKTKPREEQFNSTYRVVSVLTVLHQDWLNGKEY
KCKVSNKGLPSSIEKTISKAKGQPREPQVYTLPPSQEEMTKNQVSLTCLVK
GFYPSDIAVEWESNGQPENNYKTTPPVLDSDGSFFLYSRLTVDKSRWQEGN
VFSCSVMHEALHNHYTQKSLSLSLGK (SEQ ID NO: 17).

A human IgG4 Fc-region derived Fc-region polypeptide with S228P and L235E mutations has the following amino acid sequence:

ESKYGPPCPPCPAPEFEGGPSVFLFPPKPKDTLMISRTPEVTCVVVDVSQED
PEVQFNWYVDGVEVHNAKTKPREEQFNSTYRVVSVLTVLHQDWLNGKEY
KCKVSNKGLPSSIEKTISKAKGQPREPQVYTLPPSQEEMTKNQVSLTCLVK
GFYPSDIAVEWESNGQPENNYKTTPPVLDSDGSFFLYSRLTVDKSRWQEGN
VFSCSVMHEALHNHYTQKSLSLSLGK (SEQ ID NO: 29).

A human IgG4 Fc-region derived Fc-region polypeptide with S228P, L235E mutations and P329G mutation has the following amino acid sequence:

ESKYGPPCPPCPAPEFEGGPSVFLFPPKPKDTLMISRTPEVTCVVVDVSQED
PEVQFNWYVDGVEVHNAKTKPREEQFNSTYRVVSVLTVLHQDWLNGKEY
KCKVSNKGLGSSIEKTISKAKGQPREPQVYTLPPSQEEMTKNQVSLTCLVK
GFYPSDIAVEWESNGQPENNYKTTPPVLDSDGSFFLYSRLTVDKSRWQEGN
VFSCSVMHEALHNHYTQKSLSLSLGK (SEQ ID NO: 30).

A human IgG4 Fc-region derived Fc-region polypeptide with S354C, T366W mutations has the following amino acid sequence:

ESKYGPPCPSCPAPEFLGGPSVFLFPPKPKDTLMISRTPEVTCVVVDVSQED
PEVQFNWYVDGVEVHNAKTKPREEQFNSTYRVVSVLTVLHQDWLNGKEY
KCKVSNKGLPSSIEKTISKAKGQPREPQVYTLPPCQEEMTKNQVSLWCLVK
GFYPSDIAVEWESNGQPENNYKTTPPVLDSDGSFFLYSRLTVDKSRWQEGN
VFSCSVMHEALHNHYTQKSLSLSLGK (SEQ ID NO: 31).

A human IgG4 Fc-region derived Fc-region polypeptide with Y349C, T366S, L368A, Y407V mutations has the following amino acid sequence:

ESKYGPPCPSCPAPEFLGGPSVFLFPPKPKDTLMISRTPEVTCVVVDVSQED
PEVQFNWYVDGVEVHNAKTKPREEQFNSTYRVVSVLTVLHQDWLNGKEY

KCKVSNKGLPSSIEKTISKAKGQPREPQVCTLPPSQEEMTKNQVSLSCAVK
GFYPSDIAVEWESNGQPENNYKTTPPVLDSDGSFFLVSRLTVDKSRWQEGN
VFSCSVMHEALHNHYTQKSLSLSLGK (SEQ ID NO: 32).

A human IgG4 Fc-region derived Fc-region polypeptide with a S228P, L235E and S354C, T366W mutations has the following amino acid sequence:

ESKYGPPCPPCPAPEFEGGPSVFLFPPKPKDTLMISRTPEVTCVVVDVSQED
PEVQFNWYVDGVEVHNAKTKPREEQFNSTYRVVSVLTVLHQDWLNGKEY
KCKVSNKGLPSSIEKTISKAKGQPREPQVYTLPPCQEEMTKNQVSLWCLVK
GFYPSDIAVEWESNGQPENNYKTTPPVLDSDGSFFLYSRLTVDKSRWQEGN
VFSCSVMHEALHNHYTQKSLSLSLGK (SEQ ID NO: 33).

A human IgG4 Fc-region derived Fc-region polypeptide with a S228P, L235E and Y349C, T366S, L368A, Y407V mutations has the following amino acid sequence:

ESKYGPPCPPCPAPEFEGGPSVFLFPPKPKDTLMISRTPEVTCVVVDVSQED
PEVQFNWYVDGVEVHNAKTKPREEQFNSTYRVVSVLTVLHQDWLNGKEY
KCKVSNKGLPSSIEKTISKAKGQPREPQVCTLPPSQEEMTKNQVSLSCAVK
GFYPSDIAVEWESNGQPENNYKTTPPVLDSDGSFFLVSRLTVDKSRWQEGN
VFSCSVMHEALHNHYTQKSLSLSLGK (SEQ ID NO: 34).

A human IgG4 Fc-region derived Fc-region polypeptide with a P329G mutation has the following amino acid sequence:

ESKYGPPCPSCPAPEFLGGPSVFLFPPKPKDTLMISRTPEVTCVVVDVSQED
PEVQFNWYVDGVEVHNAKTKPREEQFNSTYRVVSVLTVLHQDWLNGKEY
KCKVSNKGLGSSIEKTISKAKGQPREPQVYTLPPSQEEMTKNQVSLTCLVK
GFYPSDIAVEWESNGQPENNYKTTPPVLDSDGSFFLYSRLTVDKSRWQEGN
VFSCSVMHEALHNHYTQKSLSLSLGK (SEQ ID NO: 35).

A human IgG4 Fc-region derived Fc-region polypeptide with a P329G and Y349C, T366S, L368A, Y407V mutations has the following amino acid sequence:

ESKYGPPCPSCPAPEFLGGPSVFLFPPKPKDTLMISRTPEVTCVVVDVSQED
PEVQFNWYVDGVEVHNAKTKPREEQFNSTYRVVSVLTVLHQDWLNGKEY
KCKVSNKGLGSSIEKTISKAKGQPREPQVCTLPPSQEEMTKNQVSLSCAVK
GFYPSDIAVEWESNGQPENNYKTTPPVLDSDGSFFLVSRLTVDKSRWQEGN
VFSCSVMHEALHNHYTQKSLSLSLGK (SEQ ID NO: 36).

A human IgG4 Fc-region derived Fc-region polypeptide with a P329G and S354C, T366W mutations has the following amino acid sequence:

ESKYGPPCPSCPAPEFLGGPSVFLFPPKPKDTLMISRTPEVTCVVVDVSQED
PEVQFNWYVDGVEVHNAKTKPREEQFNSTYRVVSVLTVLHQDWLNGKEY
KCKVSNKGLGSSIEKTISKAKGQPREPQVYTLPPCQEEMTKNQVSLWCLVK
GFYPSDIAVEWESNGQPENNYKTTPPVLDSDGSFFLYSRLTVDKSRWQEGN
VFSCSVMHEALHNHYTQKSLSLSLGK (SEQ ID NO: 37).

A human IgG4 Fc-region derived Fc-region polypeptide with a S228P, L235E, P329G and Y349C, T366S, L368A, Y407V mutations has the following amino acid sequence:

ESKYGPPCPPCPAPEFEGGPSVFLFPPKPKDTLMISRTPEVTCVVVDVSQED
PEVQFNWYVDGVEVHNAKTKPREEQFNSTYRVVSVLTVLHQDWLNGKEY
KCKVSNKGLGSSIEKTISKAKGQPREPQVCTLPPSQEEMTKNQVSLSCAVK
GFYPSDIAVEWESNGQPENNYKTTPPVLDSDGSFFLVSRLTVDKSRWQEGN
VFSCSVMHEALHNHYTQKSLSLSLGK (SEQ ID NO: 38).

A human IgG4 Fc-region derived Fc-region polypeptide with a S228P, L235E, P329G and knob mutation has the following amino acid sequence:

ESKYGPPCPPCPAPEFEGGPSVFLFPPKPKDTLMISRTPEVTCVVVDVSQED
PEVQFNWYVDGVEVHNAKTKPREEQFNSTYRVVSVLTVLHQDWLNGKEY
KCKVSNKGLGSSIEKTISKAKGQPREPQVYTLPPCQEEMTKNQVSLWCLVK
GFYPSDIAVEWESNGQPENNYKTTPPVLDSDGSFFLYSRLTVDKSRWQEGN
VFSCSVMHEALHNHYTQKSLSLSLGK (SEQ ID NO: 39).

An alignment of the different human Fc-regions is shown below (EU numbering):

```
               2                    2
               3                    5
               0                    0
     IGG1    DKTHTCPPCP  APELLGGPSV  FLFPPKPKDT  LMISRTPEVT  CVVVDVSHED
     IGG2    ...VECPPCP  APP.VAGPSV  FLFPPKPKDT  LMISRTPEVT  CVVVDVSHED
     IGG3    DTPPPCPRCP  APELLGGPSV  FLFPPKPKDT  LMISRTPEVT  CVVVDVSHED
     IGG4    ...PPCPSCP  APEFLGGPSV  FLFPPKPKDT  LMISRTPEVT  CVVVDVSQED
             -- HINGE -|-- CH2 -----------------------------------
```

```
       IGG1    PEVKFNWYVD GVEVHNAKTK PREEQYNSTY RVVSVLTVLH QDWLNGKEYK
       IGG2    PEVQFNWYVD GVEVHNAKTK PREEQFNSTF RVVSVLTVVH QDWLNGKEYK
       IGG3    PEVQFKWYVD GVEVHNAKTK PREEQYNSTF RVVSVLTVLH QDWLNGKEYK
       IGG4    PEVQFNWYVD GVEVHNAKTK PREEQFNSTY RVVSVLTVLH QDWLNGKEYK
               -- CH2 -----------------------------------------------
```

```
       IGG1    CKVSNKALPA PIEKTISKAK GQPREPQVYT LPPSRDELTK NQVSLTCLVK
       IGG2    CKVSNKGLPA PIEKTISKTK GQPREPQVYT LPPSREEMTK NQVSLTCLVK
       IGG3    CKVSNKALPA PIEKTISKTK GQPREPQVYT LPPSREEMTK NQVSLTCLVK
       IGG4    CKVSNKGLPS SIEKTISKAK GQPREPQVYT LPPSQEEMTK NQVSLTCLVK
               -- CH2 ------- CH2 --|-- CH3 ------------------------
```

```
       IGG1    GFYPSDIAVE WESNGQPENN YKTTPPVLDS DGSFFLYSKL TVDKSRWQQG
       IGG2    GFYPSDISVE WESNGQPENN YKTTPPMLDS DGSFFLYSKL TVDKSRWQQG
       IGG3    GFYPSDIAVE WESSGQPENN YNTTPPMLDS DGSFFLYSKL TVDKSRWQQG
       IGG4    GFYPSDIAVE WESNGQPENN YKTTPPVLDS DGSFFLYSRL TVDKSRWQEG
               -- CH3 -----------------------------------------------
```

```
       IGG1    NVFSCSVMHE ALHNHYTQKS LSLSPGK
       IGG2    NVFSCSVMHE ALHNHYTQKS LSLSPGK
       IGG3    NIFSCSVMHE ALHNRFTQKS LSLSPGK
       IGG4    NVFSCSVMHE ALHNHYTQKS LSLSLGK
               -- CH3 ---------------------|
```

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., the CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR", as used herein, refers to each of the regions of an antibody variable domain which are hypervariable in sequence("complementarity determining regions" or "CDRs") and form structurally defined loops ("hypervariable loops"), and/or contain the antigen-contacting residues ("antigen contacts"). Generally, antibodies comprise six HVRs; three in the VH (HI, H2, H3), and three in the VL (L1, L2, L3).HVRs as denoted herein include

(a) hypervariable loops occurring at amino acid residues 26-32 (L1), 50-52 (L2), 91-96 (L3), 26-32 (H1), 53-55 (H2), and 96-101 (H3)(Chothia, C. and Lesk, A.M., J. Mol. Biol. 196 (1987) 901-917);
(b) CDRs occurring at amino acid residues 24-34 (L1), 50-56 (L2), 89-97 (L3), 31-35b(H1), 50-65 (H2), and 95-102 (H3)(Kabat, E.A.et al., Sequences of Proteins of Immunological Interest, 5th ed. Public Health Service, National Institutes of Health, Bethesda, MD(1991), NIH Publication 91-3242.);
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

In one embodiment, HVR residues comprise those identified elsewhere in the specification.

Unless otherwise indicated, HVR residues and other residues in the variable domain (e.g., FR residues) are numbered herein according to the Kabat EU index numbering system (Kabat et al., *supra*).

An "individual" or "subject" is a mammal. Mammals include, but are not limited to, domesticated animals (e.g. cows, sheep, cats, dogs, and horses), primates (e.g., humans and non-human primates such as monkeys), rabbits, and rodents (e.g., mice and rats).In certain embodiments, the individual or subject is a human.

An "isolated" antibody is one which has been separated from a component of its natural environment. In some embodiments, an antibody is purified to greater than 95 % or 99 % purity as determined by, for example, electrophoretic (e.g., SDS-PAGE, isoelectric focusing (IEF), capillary electrophoresis) or chromatographic (e.g., size exclusion chromatography, ion exchange or reverse phase HPLC).For review of methods for assessment of antibody purity, see, e.g., Flatman, S. et al., J. Chrom. B 848 (2007) 79-87.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-IGF-1R antibody" refers to one or more nucleic acid molecules encoding antibody heavy and light chains (or fragments thereof), including such nucleic acid molecule(s) in a single plasmid or separate plasmids, and such nucleic acid molecule(s) present at one or more locations in a host cell.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3).Similarly, from N- to C-terminus, each light chain has a variable region (VL), also called a variable light domain or a light chain variable domain, followed by a constant light (CL) domain. The light chain of an antibody may be assigned to one of two types, called kappa ($\kappa$) and lambda ($\lambda$), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2.The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087.The ALIGN-2 program is publicly available from Genentech, Inc., South San Francisco, California, or may be compiled from the source code. The ALIGN-2 program should be compiled for use on a UNIX operating system, including digital UNIX V4.0D.All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

The term "peptidic linker" as used herein denotes a peptide with amino acid sequences, which is in one embodiment of synthetic origin. The peptidic linker is in one embodiment a peptide with an amino acid sequence with a length of at least 30 amino acids, in one embodiment with a length of 32 to 50 amino acids. In one embodiment the peptidic linker is a peptide with an amino acid sequence with a length of 32 to 40 amino acids. In one embodiment the peptidic linker is (GxS)n with G = glycine, S = serine, (x = 3, n = 8, 9 or 10) or (x = 4 and n= 6, 7 or 8), in one embodiment with x = 4, n = 6 or 7, in one embodiment with x = 4, n = 7. In one embodiment the peptidic linker is (G4S)6G2.

The term "recombinant antibody", as used herein, denotes all antibodies (chimeric, humanized and human) that are prepared, expressed, created or isolated by recombinant means. This includes antibodies isolated from a host cell such as a NS0 or CHO cell or from an animal (e.g. a mouse) that is transgenic for human immunoglobulin genes or antibodies expressed using a recombinant expression plasmid transfected into a host cell. Such recombinant antibodies have variable and constant regions in a rearranged form. The recombinant antibodies as reported herein can be subjected to in vivo somatic hypermutation. Thus, the amino acid sequences of the VH and VL regions of the recombinant antibodies are sequences that, while derived from and related to human germ line VH and VL sequences, may not naturally exist within the human antibody germ line repertoire in vivo.

The term "IGF-1R" as used herein, refers to any native IGF-1R from any vertebrate source, including mammals such as primates (e.g. humans) and rodents (e.g., mice and rats), unless otherwise indicated. The term encompasses "full-length", unprocessed IGF-1R as well as any form of IGF-1R that results from processing in the cell. The term also encompasses naturally occurring variants of IGF-1R, e.g., splice variants or allelic variants. The amino acid sequence of human IGF-1R is shown in SEQ ID NO: 11.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The term "variable region" or "variable domain" refers to the domain of an antibody heavy or light chain that is involved in binding of the antibody to its antigen. The variable domains of the heavy chain and light chain (VH and VL, respectively) of an antibody generally have similar structures, with each domain comprising four framework regions (FRs) and three hypervariable regions (HVRs) (see, e.g., Kindt, T.J. et al. Kuby Immunology, 6th ed., W.H. Freeman and Co., N.Y. (2007), page 91). A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively (see, e.g., Portolano, S. et al., J. Immunol. 150 (1993) 880-887; Clackson, T. et al., Nature 352 (1991) 624-628)).

The term "vascular eye disease" includes, but is not limited to intraocular neovascular syndromes such as diabetic retinopathy, diabetic macular edema, retinopathy of prematurity, neovascular glaucoma, retinal vein occlusions, central retinal vein occlusions, macular degeneration, age-related macular degeneration, retinitis pigmentosa, retinal angiomatous proliferation, macular telangectasia, ischemic retinopathy, iris neovascularization, intraocular neovascularization, corneal neovascularization, retinal neovascularization, choroidal neovascularization, and retinal degeneration (see e.g. Garner, A., Vascular diseases, In: Pathobiology of ocular disease, A dynamic approach, Garner, A., and Klintworth, G.K., (eds.), 2nd edition, Marcel Dekker, New York (1994), pp. 1625-1710).

The term "plasmid", as used herein, refers to a nucleic acid molecule capable of propagating another nucleic acid to which it is linked. The term includes the plasmid as a self-replicating nucleic acid structure as well as the plasmid incorporated into the genome of a host cell into which it has been introduced. Certain plasmids are capable of directing the expression of nucleic acids to which they are operatively linked. Such plasmids are referred to herein as "expression plasmids".

The term "with (the) mutation IHH-AAA" as used herein refers to the combination of the mutations I253A (Ile253Ala), H310A (His310Ala), and H435A (His435Ala) and the term "with (the) mutation HHY-AAA" as used herein refers to the combination of the mutations H310A (His310Ala), H433A (His433Ala), and Y436A (Tyr436Ala) and the term "with (the) mutation YTE" as used herein refers to the combination of mutations M252Y (Met252Tyr), S254T (Ser254Thr), and T256E (Thr256Glu) in the constant heavy chain region of IgG1 or IgG4 subclass, wherein the numbering is according to the EU Index of Kabat.

The term "with (the) mutations P329G LALA" as used herein refers to the combination of the mutations L234A (Leu234Ala), L235A (Leu235Ala) and P329G (Pro329Gly) in the constant heavy chain region of IgG1 subclass, wherein the numbering is according to the EU Index of Kabat. The term "with (the) mutation SPLE" as used herein refers to the combination of the mutations S228P (Ser228Pro) and L235E (Leu235Glu) in the constant heavy chain region of IgG4 subclass, wherein the numbering is according to the EU Index of Kabat. The term "with (the) mutation SPLE and P329G" as used herein refers to the combination of the mutations S228P (Ser228Pro), L235E (Leu235Glu) and P329G (Pro329Gly) in the constant heavy chain region of IgG4 subclass, wherein the numbering is according to the EU Index of Kabat.

## II. COMPOSITIONS AND METHODS

The invention is based, at least in part, on the finding that anti-IGF-1R antibodies with abolished FcRn-binding can be used for the treatment of IGF-1R-related disorders in the eye (vascular eye diseases). In certain embodiments, antibodies that bind to IGF-1R with abolished FcRn-binding are provided. Antibodies as reported herein are useful, e.g., for the diagnosis or treatment of vascular eye diseases.

The invention is based, at least in part, on the finding that specific mutations or combinations of mutations which influence the binding of an immunoglobulin Fc-region to the neonatal Fc-receptor (FcRn), i.e. which reduce or even eliminate the binding of the Fc-region to the FcRn, do not simultaneously eliminate the binding of the Fc-region to Staphylococcal protein A. This has a profound effect on the purification process that can be employed as e.g. no specific and species limited affinity chromatography materials, such as e.g. KappaSelect™, which only binds to antibodies comprising a kappa light chain, are required. Thus, with the mutations as reported herein it is possible at the same time to reduce or even eliminate the binding to FcRn while maintaining the binding to protein A.

The invention is based, at least in part, on the finding that by using different mutations in each of the Fc-region polypeptides of an Fc-region a heterodimeric molecule, such as e.g. a bispecific antibody, can be provided that on the one hand has a reduced or even eliminated binding to FcRn but on the other hand maintains the ability to bind to protein A. This binding to protein A can be used to separate the heterodimeric antibody from homodimeric by-products. For example by combining the mutations I253A, H310A and H435A in one Fc-region polypeptide with the mutations H310A, H433A and Y436A in the other Fc-region polypeptide using the knobs-into-hole approach a heterodimeric Fc-region can be obtained that on the one hand does not bind to FcRn (both sets of mutations are silent with respect to the human FcRn) but maintains binding to Staphylococcal protein A (the Fc-region polypeptide with the mutations I253A, H310A and H435A does not bind to FcRn and does not bind to protein A, whereas the Fc-region polypeptide with the mutations H310A, H433A and Y436A does not bind to FcRn but does still bind to protein A). Thus, the standard protein A affinity chromatography can be used to remove the homodimeric hole-hole by-product as this no longer binds to protein A. Thus, by combining the knobs-into-holes approach with the mutations I253A, H310A and H435A in the hole chain and the mutations H310A, H433A and Y436A in the knobs chain the purification/separation of the heterodimeric knobs-into-holes product from the homodimeric hole-hole by-product can be facilitated.

The combination of mutations I253A, H310A, H435A, or L251D, L314D, L432D, or L251S, L314S, L432S result in a loss of the binding to protein A, whereas the combination of mutations I253A, H310A, H435A, or H310A, H433A, Y436A, or L251D, L314D, L432D result in a loss of the binding to the human neonatal Fc receptor.

The following table presents an exemplary overview of the amino acid residues in an Fc-region that are involved in interactions or have been changed to modify interactions.

| residue | interaction with | | KiH | | protein A binding | effect of mutations on FcRn binding |
| --- | --- | --- | --- | --- | --- | --- |
| | protein A | FcRn | knob | hole | | |
| Pro238 | | | | | | P238A increase |

(continued)

| residue | interaction with | | KiH | | protein A binding | effect of mutations on FcRn binding |
|---------|------------------|-----|-----|------|-------------------|--------------------------------------|
| | protein A | FcRn | knob | hole | | |
| Thr250 | | | | | | T250Q/M428L increase |
| Leu251 | main-chain contact | | | | | |
| Met252 | hydrophobic packing | | | | | M252W increase; M252Y increase; M252Y/T256Q increase; M252F/T256D increase; M252Y/S254T/T256E increase |
| Ile253 | main-chain contact; hydrogen bonding; significant binding reduction if mutated to Ala | interaction | | | | I253A reduction |
| Ser254 | polar interaction; hydrogen bonding | | | | | S254A reduction; M252Y/S254T/T256E increase |
| Arg255 | salt-bridge | | | | | R255A reduction |
| Thr256 | | | | | | T256A increase; T256Q increase; T256P increase; M252Y/T256Q reduction; M252F/T256D reduction; M252Y/S254T/T256E increase |
| Pro257 | | | | | | P257I/Q311I increase; P257I/N434H increase |
| Glu272 | | | | | | E272A increase |
| Asp280 | | | | | | D280K increase |
| His285 | | | | | | reduction |
| Lys288 | | | | | | K288A reduction; K288A/N434A increase |
| Val305 | | | | | | V305A increase |
| Thr307 | | | | | | T307A increase; T307A/E380A/N434A increase; T307Q/N434A increase; T307Q/N434S increase; T307Q/E380A/N434A increase |
| Val308 | | | | | | V308P/N434A increase |
| Leu309 | | | | | | L309A reduction |
| His310 | | interaction | | | | H310A reduction; H310Q/H433N reduction |
| Gln311 | polar or charged interaction | | | | | Q311A increase; P257I/Q311I increase |

(continued)

| residue | interaction with | | KiH | | protein A binding | effect of mutations on FcRn binding |
|---|---|---|---|---|---|---|
| | protein A | FcRn | knob | hole | | |
| Asp312 | | | | | | D312A increase |
| Leu314 | hydrophobic interaction | | | | | |
| Lys317 | | | | | | K317A increase |
| Ala339 | | | | | | A339T increase |
| Tyr349 | | | | Y349C | | |
| Ser354 | | | S354C | | | |
| Thr366 | | | T366W | T366S | | |
| Leu368 | | | | L368A | | |
| Asp376 | | | | | | D376A increase; D376V/N434H increase |
| Ala378 | | | | | | A378Q increase |
| Glu380 | salt-bridge | | | | | E380A increase E380A/N434A increase; T307A/E380A/N434A increase; T307Q/E380A/N434A increase |
| Glu382 | | | | | | E382A increase |
| Gly385 | | | | | | G385H increase; G385A/Q386P/N389S increase |
| Gln386 | | | | | | G385A/Q386P/N389S increase |
| Asn389 | | | | | | G385A/Q386P/N389S increase |
| Tyr407 | | | | Y407V | | |
| Ser415 | | | | | | S415A reduction |
| Ser424 | | | | | | S424A increase |
| Met428 | | | | | | M428L increase; T250Q/M428L increase |
| Leu432 | polar or charged interaction | | | | | |
| His433 | polar or charged interaction; salt-bridge | interaction | | | | H433A reduction; H310Q/H433N reduction; H433K/N434F/Y436H increase; H433R/N434Y/Y436 Hincrease; H433K/N434F increase |

(continued)

| residue | interaction with | | KiH | | protein A binding | effect of mutations on FcRn binding |
|---|---|---|---|---|---|---|
| | protein A | FcRn | knob | hole | | |
| Asn434 | hydrogen bonding; significant binding reduction if replaced by Ala | interaction | | | | N434W/Y/F/A/H increase; K288A/N434A increase; E380A/N434A increase; T307A/E380A/N434A increase; N434F/Y436H increase; H433K/N434F/Y436H increase; H433R/N434Y/Y436 Hincrease; H433K/N434F increase; P257I/N434H increase; D376V/N434H increase; T307Q/N434A increase; T307Q/N434S increase; V308P/N434A increase; T307Q/E380A/N434A increase |
| His435 | hydrophobic packing; significant binding reduction if mutated to Ala | interaction | | | H435R/Y436F eliminates binding to protein A | H435A reduction; H435R reduction |
| Tyr436 | hydrophobic packing; significant binding reduction if replaced by Ala | interaction | | | H435R/Y436F eliminates binding to protein A | Y436A reduction; N434F/Y436H increase; H433K/N434F/Y436H increase; H433R/N434Y/Y436 H increase |

The modifications/mutations as reported herein alter the binding specificity for one or more Fc receptors such as the human FcRn. At the same time some of the mutations which alter the binding to human FcRn do not alter the binding to protein A.

In one embodiment the mutations do alter or do substantially alter the serum half-life of the antibody as compared with a corresponding antibody that lacks the mutations. In one embodiment the mutations further do not alter or do not substantially alter the binding of the antibody to protein A as compared with a corresponding antibody that lacks these mutations.

**A. The neonatal Fc-receptor (FcRn)**

The neonatal Fc-receptor (FcRn) is important for the metabolic fate of antibodies of the IgG class in vivo. The FcRn functions to salvage wild-type IgG from the lysosomal degradation pathway, resulting in reduced clearance and increased half-life. It is a heterodimeric protein consisting of two polypeptides: a 50 kDa class I major histocompatibility complex-like protein ($\alpha$-FcRn) and a 15 kDa $\beta$2-microglobulin ($\beta$2m). FcRn binds with high affinity to the CH2-CH3 portion of the Fc-region of an antibody of the class IgG. The interaction between an antibody of the class IgG and the FcRn is pH dependent and occurs in a 1:2 stoichiometry, i.e. one IgG antibody molecule can interact with two FcRn molecules via its two heavy chain Fc-region polypeptides (see e.g. Huber, A.H., et al., J. Mol. Biol. 230 (1993) 1077-1083).

Thus, an IgGs in vitro FcRn binding properties/characteristics are indicative of its in vivo pharmacokinetic properties in the blood circulation.

In the interaction between the FcRn and the Fc-region of an antibody of the IgG class different amino acid residues of the heavy chain CH2- and CH3-domain are participating. The amino acid residues interacting with the FcRn are located approximately between EU position 243 and EU position 261, approximately between EU position 275 and EU position 293, approximately between EU position 302 and EU position 319, approximately between EU position 336 and EU position 348, approximately between EU position 367 and EU position 393, at EU position 408, and approximately between EU position 424 and EU position 440. More specifically the following amino acid residues according to the EU numbering of Kabat are involved in the interaction between the Fc-region and the FcRn: F243, P244, P245 P, K246, P247, K248, D249, T250, L251, M252, 1253, S254, R255, T256, P257, E258, V259, T260, C261, F275, N276, W277, Y278, V279, D280, V282, E283, V284, H285, N286, A287, K288, T289, K290, P291, R292, E293, V302, V303, S304, V305, L306, T307, V308, L309, H310, Q311, D312, W313, L314, N315, G316, K317, E318, Y319, 1336, S337, K338, A339, K340, G341, Q342, P343, R344, E345, P346, Q347, V348, C367, V369, F372, Y373, P374, S375, D376, 1377, A378, V379, E380, W381, E382, S383, N384, G385, Q386, P387, E388, N389, Y391, T393, S408, S424, C425, S426, V427, M428, H429, E430, A431, L432, H433, N434, H435, Y436, T437, Q438, K439, and S440.

Site-directed mutagenesis studies have proven that the critical binding sites in the Fc-region of IgGs for FcRn are Histidine 310, Histidine 435, and Isoleucine 253 and to a lesser extent Histidine 433 and Tyrosine 436 (see e.g. Kim, J.K., et al., Eur. J. Immunol. 29 (1999) 2819-2825; Raghavan, M., et al., Biochem. 34 (1995) 14649-146579; Medesan, C., et al., J Immunol. 158 (1997) 2211-2217).

Methods to increase IgG binding to FcRn have been performed by mutating IgG at various amino acid residues: Threonine 250, Methionine 252, Serine 254, Threonine 256, Threonine 307, Glutamic acid 380, Methionine 428, Histidine 433, and Asparagine 434 (see Kuo, T.T., et al., J. Clin. Immunol. 30 (2010) 777-789). In some cases antibodies with reduced half-life in the blood circulation are desired. For example, drugs for intravitreal application should have a long half-live in the eye and a short half-life in the circulation of the patient. Such antibodies also have the advantage of increased exposure to a disease site, e.g. in the eye.

Different mutations that influence the FcRn binding and therewith the half-live in the blood circulation are known. Fc-region residues critical to the mouse Fc-mouse FcRn interaction have been identified by site-directed mutagenesis (see e.g. Dall'Acqua, W.F., et al. J. Immunol 169 (2002) 5171-5180). Residues 1253, H310, H433, N434, and H435 (EU numbering according to Kabat) are involved in the interaction (Medesan, C., et al., Eur. J. Immunol. 26 (1996) 2533-2536; Firan, M., et al., Int. Immunol. 13 (2001) 993-1002; Kim, J.K., et al., Eur. J. Immunol. 24 (1994) 542-548). Residues 1253, H310, and H435 were found to be critical for the interaction of human Fc with murine FcRn (Kim, J.K., et al., Eur. J. Immunol. 29 (1999) 2819-2825). Residues M252Y, S254T, T256E have been described by Dall'Acqua et al. to improve FcRn binding by protein-protein interaction studies (Dall'Acqua, W.F., et al. J. Biol. Chem. 281 (2006) 23514-23524). Studies of the human Fc-human FcRn complex have shown that residues 1253, S254, H435, and Y436 are crucial for the interaction (Firan, M., et al., Int. Immunol. 13 (2001) 993-1002; Shields, R.L., et al., J. Biol. Chem. 276 (2001) 6591-6604). In Yeung, Y.A., et al. (J. Immunol. 182 (2009) 7667-7671) various mutants of residues 248 to 259 and 301 to 317 and 376 to 382 and 424 to 437 have been reported and examined. Exemplary mutations and their effect on FcRn binding are listed in the following table.

**Table.**

| mutation | effect on FcRn binding | half-live in the circulation | reference |
|---|---|---|---|
| H285 H310Q/H433N (murine IgG1) | reduced (murine) | reduced (in mouse) | Kim, J.K., Scand. J. Immunol. 40 (1994) 457-465 |
| I253A H310A H435A H436A (murine IgG1) | reduced (murine) | reduced (in mouse) | Ghetie, V. and Ward, E.S., Immunol. Today 18 (1997) 592-598 |
| T252L/T254S/T256F T252A/T254S/T256A (murine IgG1) | increased (murine) | increased (in mouse) | Ghetie, V. and Ward, E.S., Immunol. Today 18 (1997) 592-598 |
| I253A H310A H435A H436A H433A/N434Q (murine IgG1) | reduced (murine) | reduced (in mouse) | Medesan, C., et al., J. Immunol. 158 (1997) 2211-2217 |

(continued)

| mutation | effect on FcRn binding | half-live in the circulation | reference |
|---|---|---|---|
| I253A<br>H310A<br>H435A<br>H435R (human IgG1) | reduced H310A: <0.1 rel. binding to muFcRn (murine) | reduced (in mouse) | Kim, J.K., Eur. J. Immunol. 29 (1999) 2819-2825 |
| H433A (human IgG1) | 1.1 rel. binding to muFcRn, 0.4 rel. binding hu FcRn (murine) | | Kim, J.K., Eur. J. Immunol. 29 (1999) 2819-2825 |
| I253A<br>S254A<br>H435A<br>Y436A (human IgG1) | reduced <0.1 relative binding to huFcRn | reduced | Shields, R.L., et al., J. Biol. Chem. 276 (2001) 6591-6604 |
| R255A<br>K288A<br>L309A<br>S415A<br>H433A (human IgG1) | reduced (human) | reduced | Shields, R.L., et al., J. Biol. Chem. 276 (2001) 6591-6604 |
| P238A<br>T256A<br>E272A<br>V305A<br>T307A<br>Q311A<br>D312A<br>K317A<br>D376A<br>A378Q<br>E380A<br>E382A<br>S424A<br>N434A<br>K288A/N434A<br>E380A/N434A<br>T307A/E380A/N434A (human IgG1) | increased (human) | increased | Shields, R.L., et al., J. Biol. Chem. 276 (2001) 6591-6604 |
| H435A (humanized IgG1) | reduced <0.1 rel. binding to huFcRn | reduced | Firan, M., et al., Int. Immunol. 13 (2001) 993-1002 |

(continued)

| mutation | effect on FcRn binding | half-live in the circulation | reference |
|---|---|---|---|
| 1253A (no binding)<br>M252W<br>M252Y<br>M252Y/T256Q<br>  M252F/T256D<br>N434F/Y436H<br>M252Y/S254T/T256E<br>G385A/Q386P/N389S<br>H433K/N434F/Y436H<br>H433R/N434Y/Y436H<br>G385R/Q386T/P387R/N389P<br>M252Y/S254T/T256E/H433K<br>/N434F/Y436H<br>M252Y/S254T/T256E/G385R<br>/Q386T/P387R/N389P<br>(human IgG1) | increased (murine and human) | reduced (in mouse) | Dall'Acqua, J. Immunol. 169 (2002) 5171-5180 |
| M428L<br>T250Q/M428L (human IgG2) | increased (human) | increased (in monkey) | Hinton, P.R., et al., J. Biol. Chem. 279 (2004) 6213-6216 |
| M252Y/S254T/T256E +<br>H433K/N434F (human IgG) | increased (human) | increased (in mouse) | Vaccaro, C., et al., Nat. Biotechnol. 23 (2005) 1283-1288 |
| T307A/E380A/N434A<br>(chimeric IgG1) | increased | increased in transgenic mouse | Pop, L.M., et al., Int. Immunopha rmacol. 5 (2005) 1279-1290 |
| T250Q<br>E380A<br>M428L<br>N434A<br>K288A/N434A<br>E380A/N434A<br>T307A/E380A/N434A (human IgG1) | increased (human) | increased in transgenic mouse | Petkova, S.B., et al., Int. Immunol 18 (2006) 1759-1769 |
| I253A (human IgG1) | reduced (human) | reduced in transgenic mouse | Petkova, S.B., et al., Int. Immunol 18 (2006) 1759-1769 |
| S239D/A330L/I332E<br>M252Y/S254T/T256E<br>(humanized) | increased (human and Cynomolgus) | increased in Cynomolgus | Dall'Acqua, W.F., et al., J. Biol. Chem. 281 (2006) 23514-23524 |
| T250Q<br>M428L<br>T250Q/M428L (human IgG1) | increased (human) | increased in Rhesus apes | Hinton, P.R., et al., J. Immunol. 176 (2006) 346-356 |
| T250Q/M428L<br>P257I/Q311I (humanized IgG1) | increased (mouse and Cynomolgus) | no change in Cynomolgus increased in mouse | Datta-Mannan, A., et al., J. Biol. Chem. 282 (2007) 1709-1717 |

(continued)

| mutation | effect on FcRn binding | half-live in the circulation | reference |
|---|---|---|---|
| P257I/Q311I<br>P257I/N434H<br>D376V/N434H (humanized IgG1) | increased at pH 6 (human, Cynomolgus, mouse) | reduced in mice P257I/N434H reduced in Cynomolgus | Datta-Mannan, A., et al., Drug Metab. Dispos. 35 (2007) 86-94 |
| abrogate FcRn binding:<br>I253<br>H310<br>H433<br>H435<br>reduce FcRn binding:<br>Y436<br>increased FcRn binding:<br>T250<br>N252<br>S254<br>T256<br>T307<br>M428<br>N434 | increased and reduced | reducing the binding ability of IgG for FcRn reduces its serum persistence; a higher-affinity FcRn-IgG interaction prolongs the half-lives of IgG and Fc-coupled drugs in the serum | Ropeenian, D.C. and Akilesh, S., Nat. Rev. Immunol. 7 (2007) 715-725 |
| N434A<br>T307Q/N434A<br>T307Q/N434S<br>V308P/N434A<br>T307Q/E380A/N434A (human IgG1) | increased (Cynomolgus monkey) | increased in Cynomolgus monkey | Yeung, Y.A., et al., Cancer Res. 70 (2010) 3269-3277 |
| 256P<br>280K<br>339T<br>385H<br>428L<br>434W/Y/F/A/H (human IgG) | increased at neutral pH | | WO 2011/ 122011 |

It has been found that one mutation one-sided in one Fc-region polypeptide is sufficient to weaken the binding to an Fc receptor significantly. The more mutations are introduced into the Fc-region the weaker the binding to the FcRn becomes. But one-sided asymmetric mutations are not sufficient to completely inhibit FcRn binding. Mutations on both sides are necessary to completely inhibit FcRn binding.

Thus, the Fc-region is a heterodimer and the pairing of the first (heavy chain) Fc-region polypeptide and the second (heavy chain) Fc-region polypeptide to form a functional Fc-region results in the formation of a heterodimer.

The results of a symmetric engineering of an IgG1 Fc-region to influence FcRn binding is shown in the following table (alignment of mutations and retention time on an FcRn-affinity chromatography column).

**Table.**

| effector function influencing mutations | FcRn-binding influencing mutation 1 | FcRn-binding influencing mutation 2 | FcRn-binding influencing mutation 3 | FcRn-affinity column retention time [min] |
|---|---|---|---|---|
| L234A/L235A/P329G | --- | --- | --- | 45.3 |
| L234A/L235A/P329G | I253A | H310A | H435A | 2.3 |

(continued)

| effector function influencing mutations | FcRn-binding influencing mutation 1 | FcRn-binding influencing mutation 2 | FcRn-binding influencing mutation 3 | FcRn-affinity column retention time [min] |
|---|---|---|---|---|
| L234A/L235A/P329G | I253A | --- | --- | 2.7 |
| L234A/L235A/P329G | --- | H310A | --- | 2.4 |
| L234A/L235A/P329G | --- | --- | H435A | 2.7 |
| L234A/L235A/P329G | I253A | H310A | --- | 2.3 |
| L234A/L235A/P329G | I253A | --- | H435A | 2.3 |
| L234A/L235A/P329G | --- | H310A | H435A | 2.4 |
| L234A/L235A/P329G | --- | H310A | Y436A | 2.3 |
| L234A/L235A/P329G | H310A | H433A | Y436A | 2.4 |
| L234A/L235A/P329G | --- | --- | Y436A | 41.3 |

Retention times below 3 minutes correspond to no binding as the substance is in the flow-through (void peak).

The single mutation H310A is the most silent symmetrical mutation to delete any FcRn-binding.

The symmetric single mutation I253A and H435A result in a relative shift of retention time of 0.3 - 0.4 min. This can be generally regarded as a non-detectable binding.

The single mutation Y436A results in detectable interaction strength to the FcRn affinity column. Without being bound by this theory this mutation could have an FcRn mediated half-life which can be differentiated from a zero interaction such as the combination of the I253A, H310A and H435A mutations (IHH-AAA mutation).

The results obtained with a symmetrically modified anti-HER2 antibody are presented in the following table (see WO 2006/031370 for reference).

**Table.**

| mutation | retention time [min] |
|---|---|
| I253H | no binding |
| M252D | no binding |
| S254D | no binding |
| R255D | 41.4 |
| M252H | 43.6 |
| K288E | 45.2 |
| L309H | 45.5 |
| E258H | 45.6 |
| T256H | 46.0 |
| K290H | 46.2 |
| D98E | 46.2 |
| wild-type | 46.3 |
| K317H | 46.3 |
| Q311H | 46.3 |
| E430H | 46.4 |
| T307H | 47.0 |
| N434H | 52.0 |

The effect of the introduction of asymmetric FcRn-binding affecting mutations in the Fc-region has been exemplified with a bispecific antibody assembled using the knobs-into-holes technology (see e.g. US 7,695,936, US 2003/0078385; "hole chain" mutations: S354C/T366W, "knob chain" mutations: Y349C/T366S/L368A/Y407V). The effect of the asymmetrically introduced mutations on FcRn-binding can easily be determined using an FcRn affinity chromatography method. Antibodies that have a later elution from the FcRn affinity column, i.e. that have a longer retention time on the FcRn affinity column, have a longer half-life in vivo, and vice versa.

**Table.**

| FcRn affecting mutation | retention time on FcRn affinity column |
|---|---|
| one chain with M252Y/S254T/T256E | 56.2 min. |
| none | 51.8 min. |
| one chain with I253A or H435A | 48.8 min. |
| one chain with H310A | 48.4 min. |
| one chain with I253A/H435A or I253A/H310A or H310A/H435A | 48.0 min. |
| one chain with H310A/H433A/Y436A | 46.7 min. |
| one chain with I253A/H310A/H435A | 46.6 min. |
| one chain with L251D/L314D/L432D | 46.3 min. |
| first chain with I253A/H310A/H435A and second chain with H310A or H435A or I253A/H310A/H435A | no binding |

The effect of the introduction of asymmetric FcRn-binding affecting mutations in the Fc-region has further been exemplified with a monospecific anti-IGF-1R antibody assembled using the knobs-into-holes technology in order to allow the introduction of asymmetric mutations (see e.g. US 7,695,936, US 2003/0078385; "hole chain" mutations: S354C/T366W, "knob chain" mutations: Y349C/T366S/L368A/Y407V). The effect of the asymmetrically introduced mutations on FcRn-binding can easily be determined using an FcRn affinity chromatography method (see the following Table). Antibodies that have a later elution from the FcRn affinity column, i.e. that have a longer retention time on the FcRn affinity column, have a longer half-life in vivo, and vice versa.

**Table.**

| FcRn affecting mutation | retention time on FcRn affinity column |
|---|---|
| one chain with M252Y/S254T/T256E | 57.6 min. |
| none | 53.0 min. |
| one chain with H310A/H433A/Y436A | 42.4 min. |
| one chain with I253A/H310A/H435A | 42.0 min. |
| one chain with L251D/L314D/L432D | 40.9 min. |
| first chain with I253A/H310A/H435A and second chain with H310A or H435A or I253A/H310A/H435A | no binding |

The asymmetric IHH-AAA- and LLL-DDD-mutations (LLL-DDD-mutation = L251D, L314D and L432D) show weaker binding than the corresponding parent or wild-type antibody.

The symmetric HHY mutation (= combination of the mutations H310A, H433A and Y436A) results in an Fc-region that does no longer bind to the human FcRn whereas the binding to protein A is maintained (see Figures 11, 12, 13 and 14).

The effect of the introduction of asymmetric FcRn-binding affecting mutations in the Fc-region has further been exemplified with a monospecific anti-IGF-1R antibody (IGF-1R) assembled using the knobs-into-holes technology in order to allow the introduction of asymmetric mutations (see e.g. US 7,695,936, US 2003/0078385; "hole chain" mutations: S354C/T366W, "knob chain" mutations: Y349C/T366S/L368A/Y407V). The effect of the introduced mutations on FcRn-binding and protein A binding can easily be determined using an FcRn affinity chromatography method, a protein A affinity chromatography method and SPR-based methods (see the following Table).

| antibody | further mutation in knob chain | further mutation in hole chain | FcR binding affecting mutations | FcRn binding (SPR) | FcRn binding (column) | protein A binding (SPR) | protein A binding (column) |
|---|---|---|---|---|---|---|---|
| IGF-1R 0033 | none | none | none | yes | yes | n.d. | yes |
| IGF-1R 0034 | none | I253A H310A H435A | L234A L235A P329G | n.d. | yes | n.d. | yes |
| IGF-1R 0035 | none | H310A H433A Y436A | none | reduced | reduced | n.d. | yes |
| IGF-1R 0037 | none | L251D L314D L432D | L234A L235A P329G | n.d. | yes | n.d. | yes |
| IGF-1R 0036 | none | M252Y S254T T256E | L234A L235A P329G | n.d. | yes | n.d. | yes |
| IGF-1R 0045 | H310A H433A Y436A | H310A H433A Y436A | none | n.d. | n.d. | n.d. | yes |

One aspect as reported herein is an antibody comprising the combination of mutations in the Fc-region polypeptide(s) as reported herein.

The Fc-region in the antibody confers the above described characteristics. The antibody can be any antibody having a biological activity whose in vivo half-live shall be reduced or increased, i.e. whose in vivo half-live shall be clearly defined and tailor-made for its intended application.

## B. Vascular Eye diseases

Vascular eye diseases are any pathological condition characterized by altered or unregulated proliferation and invasion of new blood vessels into the structures of ocular tissues such as the retina or cornea.

In one embodiment the vascular eye disease is selected from the group consisting of wet age-related macular degeneration (wet AMD), dry age-related macular degeneration (dry AMD), diabetic macular edema (DME), cystoid macular edema (CME), non-proliferative diabetic retinopathy (NPDR), proliferative diabetic retinopathy (PDR), cystoid macular edema, vasculitis (e.g. central retinal vein occlusion), papilloedema, retinitis, conjunctivitis, uveitis, choroiditis, multifocal choroiditis, ocular histoplasmosis, blepharitis, dry eye (Sjogren's disease) and other ophthalmic diseases wherein the eye disease or disorder is associated with ocular neovascularization, vascular leakage, and/or retinal edema.

The anti-IGF-1R antibody according to the invention is useful in the prevention and treatment of wet AMD, dry AMD, CME, DME, NPDR, PDR, blepharitis, dry eye and uveitis, also in one preferred embodiment wet AMD, dry AMD, blepharitis, and dry eye, also in one preferred embodiment CME, DME, NPDR and PDR, also in one preferred embodiment blepharitis, and dry eye, in particular wet AMD and dry AMD, and also particularly wet AMD.

In some embodiments, the vascular eye disease is selected from the group consisting of wet age-related macular degeneration (wet AMD), macular edema, retinal vein occlusions, retinopathy of prematurity, and diabetic retinopathy.

Other diseases associated with corneal neovascularization include, but are not limited to, epidemic keratoconjunctivitis, Vitamin A deficiency, contact lens overwear, atopic keratitis, superior limbic keratitis, pterygium keratitis sicca, Sjogren's disease, acne rosacea, phylectenulosis, syphilis, Mycobacteria infections, lipid degeneration, chemical burns, bacterial ulcers, fungal ulcers, Herpes simplex infections, Herpes zoster infections, protozoan infections, Kaposi sarcoma, Mooren ulcer, Terrien's marginal degeneration, mariginal keratolysis, rheumatoid arthritis, systemic lupus, polyarteritis, trauma, Wegener's sarcoidosis, Scleritis, Steven's Johnson disease, periphigoid radial keratotomy, and corneal graph rejection.

Diseases associated with retinal/choroidal neovascularization include, but are not limited to, diabetic retinopathy, macular degeneration, sickle cell anemia, sarcoid, syphilis, pseudoxanthoma elasticum, Paget's disease, vein occlusion, artery occlusion, carotid obstructive disease, chronic uveitis/vitritis, mycobacterial infections, Lyme's disease, systemic lupus erythematosis, retinopathy of prematurity, retinitis pigmentosa, retina edema (including macular edema), Eale's disease, Bechet's disease, infections causing a retinitis or choroiditis, presumed ocular histoplasmosis, Best's disease, myopia, optic pits, Stargart's disease, pars planitis, chronic retinal detachment, hyperviscosity syndromes, toxoplasmosis, trauma and post-laser complications.

Other diseases include, but are not limited to, diseases associated with rubeosis (neovascularization of the angle) and diseases caused by the abnormal proliferation of fibrovascular or fibrous tissue including all forms of proliferative vitreoretinopathy.

Retinopathy of prematurity (ROP) is a disease of the eye that affects prematurely born babies. It is thought to be caused by disorganized growth of retinal blood vessels which may result in scarring and retinal detachment. ROP can be mild and may resolve spontaneously, but may lead to blindness in serious cases. As such, all preterm babies are at risk for ROP, and very low birth weight is an additional risk factor. Both oxygen toxicity and relative hypoxia can contribute to the development of ROP.

Macular degeneration is a medical condition predominantly found in elderly adults in which the center of the inner lining of the eye, known as the macula area of the retina, suffers thinning, atrophy, and in some cases, bleeding. This can result in loss of central vision, which entails inability to see fine details, to read, or to recognize faces. According to the American Academy of Ophthalmology, it is the leading cause of central vision loss (blindness) in the United States today for those over the age of fifty years. Although some macular dystrophies that affect younger individuals are sometimes referred to as macular degeneration, the term generally refers to age-related macular degeneration (AMD or ARMD).

Age-related macular degeneration begins with characteristic yellow deposits in the macula (central area of the retina which provides detailed central vision, called fovea) called drusen between the retinal pigment epithelium and the underlying choroid. Most people with these early changes (referred to as age-related maculopathy) have good vision. People with drusen can go on to develop advanced AMD. The risk is considerably higher when the drusen are large and numerous and associated with disturbance in the pigmented cell layer under the macula. Large and soft drusen are related to elevated cholesterol deposits and may respond to cholesterol lowering agents or the Rheo Procedure.

Advanced AMD, which is responsible for profound vision loss, has two forms: dry and wet. Central geographic atrophy, the dry form of advanced AMD, results from atrophy to the retinal pigment epithelial layer below the retina, which causes vision loss through loss of photoreceptors (rods and cones) in the central part of the eye. While no treatment is available for this condition, vitamin supplements with high doses of antioxidants, lutein and zeaxanthin, have been demonstrated by the National Eye Institute and others to slow the progression of dry macular degeneration and in some patients, improve visual acuity.

Retinitis pigmentosa (RP) is a group of genetic eye conditions. In the progression of symptoms for RP, night blindness generally precedes tunnel vision by years or even decades. Many people with RP do not become legally blind until their 40s or 50s and retain some sight all their life. Others go completely blind from RP, in some cases as early as childhood. Progression of RP is different in each case. RP is a type of hereditary retinal dystrophy, a group of inherited disorders in which abnormalities of the photoreceptors (rods and cones) or the retinal pigment epithelium (RPE) of the retina lead to progressive visual loss. Affected individuals first experience defective dark adaptation or nyctalopia (night blindness), followed by reduction of the peripheral visual field (known as tunnel vision) and, sometimes, loss of central vision late in the course of the disease.

Macular edema occurs when fluid and protein deposits collect on or under the macula of the eye, a yellow central area of the retina, causing it to thicken and swell. The swelling may distort a person's central vision, as the macula is near the center of the retina at the back of the eyeball. This area holds tightly packed cones that provide sharp, clear central vision to enable a person to see form, color, and detail that is directly in the line of sight. Cystoid macular edema is a type of macular edema that includes cyst formation.

## C. Exemplary Anti-IGF-IR Antibodies

In one aspect, the invention provides isolated antibodies that bind to IGF-1R.

In certain embodiments the anti-IGF-1R antibody has abolished FcRn-binding, i.e. it binds to human FcRn with an affinity comparable to or less than an antibody having the mutations I253A/H310A/H435A or H310A/H433A/Y436A or L251D/L314D/L432D or L251S/L314S/L432S or combinations thereof in the Fc-region (numbering according to Kabat EU index numbering system).

In one embodiment the anti-IGF-1R antibody has no (remaining) detectable FcRn binding using a surface plasmon resonance based determination method.

In one embodiment the anti-IGF-1R antibody binds to human FcRn with a $K_D$-value of more than 1.7 $\mu$M at pH 6, i.e. with low affinity.

In one embodiment of all aspects the antibody has the same or a shorter retention time on an FcRn affinity chromatography column as an antibody comprising an Fc-region with the mutations I253A/H310A/H435A or H310A/H433A/Y436A or L251D/L314D/L432D or combinations thereof (numbering according to Kabat EU index numbering system).

In one embodiment of all aspects the antibody has a retention time on an FcRn affinity chromatography column of three minutes or less.

In one embodiment the FcRn affinity column has the column dimensions of 50 mm x 5 mm, the bed height is 5 cm and the loading is 50 $\mu$g antibody. In one embodiment the equilibration buffer is 20 mM MES, with 150 mM NaCl, adjusted to pH 5.5, the elution buffer is 20 mM Tris/HCl, with 150 mM NaCl, adjusted to pH 8.8 and the elution is by applying 7.5 CV equilibration buffer, from 0 % to 100 % elution buffer in 30 CV, and thereafter 10 CV elution buffer.

In one embodiment the anti-IGF-1R antibody does not bind to a human FcRn column due to single or multiple point mutations in the CH2 and/or CH3 domain of the anti-IGF-1R antibody. In one embodiment the antibody has a comparable, i.e. within +/- 10 %, or shorter retention time on a FcRn column as an anti IGF-1R antibody with 4 point mutations (HC1: I253A, H310A, H435A; HC2: H310A).

In one embodiment the anti-IGF-1R antibody binds to human FcRn with about the same or lesser affinity than an antibody comprising an Fc-region with at least one of the mutations L251D, L251S, M252T, I253A, S254W, S254R, H310A, N434L, H435A, Y436A (numbering according to Kabat EU index numbering system).

In one embodiment the anti-IGF-1R antibody binds to human FcRn with about the same or lesser affinity than an antibody comprising an Fc-region with the mutations I253A/H310A/H435A or H310A/H433A/Y436A or L251D/L314D/L432D or L251S/L314S/L432S or combinations thereof (numbering according to Kabat EU index numbering system).

In one embodiment the anti-IGF-1R antibody binds to human FcRn with about the same or lesser affinity than an antibody with a heavy chain amino acid sequence of SEQ ID NO: 01 or SEQ ID NO: 96 and a light chain amino acid sequence of SEQ ID NO: 03, or than an antibody with a heavy chain amino acid sequence of SEQ ID NO: 02 or SEQ ID NO: 96 and a light chain amino acid sequence of SEQ ID NO: 04.

In one embodiment the anti-IGF-1R antibody has at least one of the mutations L251D, L251S, M252T, I253A, S254W, S254R, H310A, H433A, N434L, H435A, Y436A (numbering according to Kabat EU index numbering system) or a combination thereof.

In one embodiment the anti-IGF-1R antibody has at least one of the mutations L251D, L251S, M252T, I253A, S254W, S254R, H310A, H433A, N434L, H435A, Y436A (numbering according to Kabat EU index numbering system) in the first Fc-region polypeptide and at least one of the mutations L251D, L251S, M252T, I253A, S254W, S254R, H310A, H433A, N434L, H435A, Y436A (numbering according to Kabat EU index numbering system) in the second Fc-region polypeptide.

In one embodiment the anti-IGF-1R antibody has at least the mutations I253A/H310A/H435A or H310A/H433A/Y436A or L251D/L314D/L432D or L251S/L314S/L432S or combinations thereof (numbering according to Kabat EU index numbering system) in the first Fc-region polypeptide and at least one of the mutations L251D, L251S, M252T, I253A, S254W, S254R, H310A, H433A, N434G, N434L, H435A, Y436A (numbering according to Kabat EU index numbering system) in the second Fc-region polypeptide.

In one embodiment the anti-IGF-1R antibody has at least the mutations I253A/H310A/H435A or H310A/H433A/Y436A or L251D/L314D/L432D or L251S/L314S/L432S or combinations thereof (numbering according to Kabat EU index numbering system) in both Fc-region polypeptides.

In one embodiment the anti-IGF-1R antibody has

a) an antibody heavy chain comprising as HVRs amino acid residues 31-35 (HVR-H1), amino acid residues 50-66 (HVR-H2) and amino acid residues 99-107 (HVR-H3) of SEQ ID NO: 01 or SEQ ID NO: 02, and

b) an antibody light chain comprising as HVRs amino acid residues 24-34 (HVR-L1), amino acid residues 50-56 (HVR-L2) and amino acid residues 89-98 (HVR-L3) of SEQ ID NO: 03 or SEQ ID NO: 04.

In one preferred embodiment the anti-IGF-1R antibody has

a) an antibody heavy chain comprising as HVRs amino acid residues 31-35 (HVR-H1), amino acid residues 50-66 (HVR-H2) and amino acid residues 99-107 (HVR-H3) of SEQ ID NO: 01, and

b) an antibody light chain comprising as HVRs amino acid residues 24-34 (HVR-L1), amino acid residues 50-56 (HVR-L2) and amino acid residues 89-98 (HVR-L3) of SEQ ID NO: 03.

These are the sequences of AK18 (<IGF-1R> HUMAB Clone 18) which is described in detail in WO 2005/005635.

In another preferred embodiment the anti-IGF-1R antibody comprised a modified heavy chain variable domain (VH) and light chain variable domain (VL), and the respective HVRs, derived from AK18 (<IGF-1R> HUMAB Clone 18) with improved affinity as described in detail in WO 2013/041462. This affinity improved anti-IGF-1R antibodies in combination with the Fc-region mutations as described herein are especially useful for the treatment of the respective ocular vascular and inflammatory diseases.

Therefore, in one preferred embodiment the anti-IGF-1R antibody has

a) an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3), or

b) an antibody heavy chain comprising as HVRs the amino acid sequence of SEQ ID NO: 64 (HVR-H1), the amino acid sequence of SEQ ID NO: 65 (HVR-H2), and the amino acid sequence of SEQ ID NO: 66 (HVR-H3), and an antibody light chain comprising as HVRs the amino acid sequence of SEQ ID NO: 67 (HVR-L1), the amino acid sequence of SEQ ID NO: 68 (HVR-L2), and the amino acid sequence of SEQ ID NO: 69 (HVR-L3), or

c) an antibody heavy chain comprising as HVRs the amino acid sequence of SEQ ID NO: 72 (HVR-H1), the amino acid sequence of SEQ ID NO: 73 (HVR-H2), and the amino acid sequence of SEQ ID NO: 74 (HVR-H3), and an antibody light chain comprising as HVRs the amino acid sequence of SEQ ID NO: 75 (HVR-L1), the amino acid sequence of SEQ ID NO: 76 (HVR-L2), and the amino acid sequence of SEQ ID NO: 77 (HVR-L3), or

d) an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3), or

e) an antibody heavy chain comprising as HVRs the amino acid sequence of SEQ ID NO: 88 (HVR-H1), the amino acid sequence of SEQ ID NO: 89 (HVR-H2), and the amino acid sequence of SEQ ID NO: 90 (HVR-H3), and an antibody light chain comprising as HVRs the amino acid sequence of SEQ ID NO: 91 (HVR-L1), the amino acid sequence of SEQ ID NO: 92 (HVR-L2), and the amino acid sequence of SEQ ID NO: 93 (HVR-L3).

Therefore, in another preferred embodiment the anti-IGF-1R antibody comprises

a) a heavy chain variable domain VH of SEQ ID NO: 62, and a light chain variable domain VL of SEQ ID NO: 63, or
b) a heavy chain variable domain VH of SEQ ID NO: 70, and a light chain variable domain VL of SEQ ID NO: 71, or
c) a heavy chain variable domain VH of SEQ ID NO: 78, and a light chain variable domain VL of SEQ ID NO: 79, or
d) a heavy chain variable domain VH of SEQ ID NO: 86, and a light chain variable domain VL of SEQ ID NO: 87, or
e) a heavy chain variable domain VH of SEQ ID NO: 94, and a light chain variable domain VL of SEQ ID NO: 95.

Therefore, in one preferred embodiment the anti-IGF-1R antibody has

an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3).

Therefore, in another preferred embodiment the anti-IGF-1R antibody has

an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3).

Therefore, in one preferred embodiment the anti-IGF-1R antibody comprises

a) a heavy chain variable domain VH of SEQ ID NO: 62, and a light chain variable domain VL of SEQ ID NO: 63.

Therefore, in another preferred embodiment the anti-IGF-1R antibody comprises

d) a heavy chain variable domain VH of SEQ ID NO: 86, and a light chain variable domain VL of SEQ ID NO: 87.

In one embodiment the anti-IGF-1R antibody has a heterodimeric Fc-region.
In one embodiment the antibody is an isolated antibody.
In one embodiment the antibody is a full-length antibody.
In one embodiment the antibody is a monoclonal antibody.
In one embodiment the antibody is a human, humanized or chimeric antibody.
In one embodiment the antibody comprises a VH sequence of SEQ ID NO: 05 or SEQ ID NO: 06.
In one embodiment the antibody comprises a VL sequence of SEQ ID NO: 07 or SEQ ID NO: 08.
In one embodiment the antibody comprises a VH sequence of SEQ ID NO: 05 and a VL sequence of SEQ ID NO: 07.
In one embodiment the antibody comprises a VH sequence of SEQ ID NO: 06 and a VL sequence of SEQ ID NO: 08.
In one embodiment the antibody comprises an Fc-region comprising a first Fc-region polypeptide and a second Fc-region polypeptide wherein

i) the first and the second Fc-region polypeptide comprise the mutation Y436A, or

ii) the first and the second Fc-region polypeptide comprise the mutations 1253A, H310A and H435A, or

iii) the first and the second Fc-region polypeptide comprise the mutations H310A, H433A and Y436A, or

iv) the first and the second Fc-region polypeptide comprise the mutations L251D, L314D and L432D, or

v) the first and the second Fc-region polypeptide comprise the mutations L251S, L314S and L432S, or

vi) the first Fc-region polypeptide comprises the mutation Y436A and the second Fc-region polypeptide comprises

a) the mutations 1253A, H310A and H435A, or
b) the mutations H310A, H433A and Y436A, or
c) the mutations L251D, L314D and L432D, or
d) the mutations L251S, L314S and L432S,

or

vii) the first Fc-region polypeptide comprises the mutations I253A, H310A and H435A and the second Fc-region polypeptide comprises

a) the mutations H310A, H433A and Y436A, or
b) the mutations L251D, L314D and L432D, or
c) the mutations L251S, L314S and L432S,

or

viii) the first Fc-region polypeptide comprises the mutations H310A, H433A and Y436A and the second Fc-region polypeptide comprises

a) the mutations L251D, L314D and L432D, or
b) the mutations L251S, L314S and L432S,

or

ix) the first Fc-region polypeptide comprises the mutations L251D, L314D and L432D and the second Fc-region polypeptide comprises the mutations L251S, L314S and L432S.

In one embodiment of all aspects the antibody does not specifically bind to the human FcRn. In one embodiment of all aspects the antibody does not specifically bind to Staphylococcal protein A.

In one embodiment of all aspects the antibody does not specifically bind to the human FcRn. In one embodiment of all aspects the antibody does specifically bind to Staphylococcal protein A.

In one embodiment of all aspects the antibody comprises an Fc-region comprising a first and a second Fc-region polypeptide both of human IgG1 or human IgG4 subclass (derived from human origin) which comprise one or two of the mutations selected from i) the group I253A, H310A and H435A, or ii) the group H310A, H433A and Y436A, or iii) the group L251D, L314D and L432D, or iv) the group L251S, L314S and L432S (numbering according to Kabat EU index numbering system) in the first Fc-region polypeptide and one or two of the mutations selected from the group comprising the mutations L251D, L251S, I253A, H310A, L314D, L314S, L432D, L432S, H433A, H435A, and Y436A (numbering according to Kabat EU index numbering system) in the second Fc-region polypeptide so that all of the mutations i) I253A, H310A and H435A, or ii) H310A, H433A and Y436A, or iii) L251D, L314D and L432D, or iv) L251S, L314S and L432S are comprised in the variant (human) IgG class Fc-region.

In one embodiment of all aspects the antibody comprises an Fc-region comprising a first and a second Fc-region polypeptide both of human IgG1 or human IgG4 subclass (i.e. derived from human origin), which comprise the mutations I253A/H310A/H435A or H310A/H433A/Y436A or L251D/L314D/L432D or L251S/L314S/L432S or combinations thereof in the Fc-region (numbering according to Kabat EU index numbering system), whereby i) all mutations are in the first or the second Fc-region polypeptide, or ii) one or two mutations are in the first Fc-region polypeptide and one or two mutations are in the second Fc-region polypeptide so that all of the mutations i) I253A, H310A and H435A, or ii) H310A, H433A and Y436A, or iii) L251D, L314D and L432D, or iv) L251S, L314S and L432S are comprised in the Fc-region.

In one embodiment of all aspects the antibody comprises an Fc-region comprising a first and a second Fc-region polypeptide both of human IgG1 or human IgG4 subclass (i.e. derived from human origin), which comprise the mutations I253A/H310A/H435A or H310A/H433A/Y436A or L251D/L314D/L432D or L251S/L314S/L432S in the first as well as in the second Fc-region polypeptide (numbering according to Kabat EU index numbering system), or which comprise the mutations I253A/H310A/H435A in the first Fc-region polypeptide and the mutations H310A/H433A/Y436A in the second Fc-region polypeptide (numbering according to Kabat EU index numbering system).

In one embodiment of all aspects the second Fc-region polypeptide further comprises the mutations Y349C, T366S, L368A and Y407V ("hole") and the first Fc-region polypeptide further comprises the mutations S354C and T366W ("knob").

In one embodiment of all aspects the Fc-region polypeptides are of the human IgG1 subclass. In one embodiment the first Fc-region polypeptide and the second Fc-region polypeptide further comprise the mutations L234A and L235A. In one embodiment the first Fc-region polypeptide and the second Fc-region polypeptide further comprise the mutation P329G.

In one embodiment of all aspects the Fc-region polypeptides are of the human IgG4 subclass. In one embodiment the first Fc-region polypeptide and the second Fc-region polypeptide further comprise the mutations S228P and L235E. In one embodiment the first Fc-region polypeptide and the second Fc-region polypeptide further comprise the mutation P329G.

In one embodiment of all aspects the antibody is characterized in that the antibody

- shows a lower serum concentration compared to corresponding bispecific antibody without the mutations in the Fc-region polypeptides (96 hours after intravitreal application in mice, which are mouse FcRn deficient, but hemizygous transgenic for human FcRn) (determined in assays as described in Example 4), and/or

- shows a similar (factor 0.8 to 1.2) concentration in whole right eye lysates compared to corresponding bispecific antibody without the mutations in the Fc-region polypeptides (in mice, which are mouse FcRn deficient, but hemizygous transgenic for human FcRn, 96 hours after intravitreal application in the right eye) (determined in assays as described in Example 4), and/or

- has abolished binding to the human FcRn, and/or

- has no binding to Staphylococcal protein A (determined by SPR), and/or

- has maintained binding to Staphylococcal protein A (determined by SPR).

In another aspect, an anti-IGF-1R antibody comprises a heavy chain variable domain (VH) sequence having at least 90 %, 91 %, 92 %, 93 %, 94 %, 95 %, 96 %, 97 %, 98 %, 99 %, or 100 % sequence identity to the amino acid sequence of SEQ ID NO: 05 or 62 or 86. In certain embodiments, a VH sequence having at least 90 %, 91 %, 92 %, 93 %, 94 %, 95 %, 96 %, 97 %, 98 %, or 99 % identity contains substitutions (e.g. conservative substitutions), insertions, or deletions relative to the reference sequence, but an anti-IGF-1R antibody comprising that sequence retains the ability to bind to IGF-1R. In certain embodiments, a total of 1 to 10 amino acids have been substituted, inserted and/or deleted in SEQ ID NO: 05 or 62 or 86. In certain embodiments, substitutions, insertions, or deletions occur in regions outside the HVRs (i.e., in the FRs). Optionally, the anti-IGF-1R antibody comprises the VH sequence in SEQ ID NO: 05 or 62 or 86, including post-translational modifications of that sequence.

In another aspect, an anti-IGF-1R antibody is provided, wherein the antibody comprises a light chain variable domain (VL) having at least 90 %, 91 %, 92 %, 93 %, 94 %, 95 %, 96 %, 97 %, 98 %, 99 %, or 100 % sequence identity to the amino acid sequence of SEQ ID NO: 07 or 63 or 87. In certain embodiments, a VL sequence having at least 90 %, 91 %, 92 %, 93 %, 94 %, 95 %, 96 %, 97 %, 98 %, or 99 % identity contains substitutions (e.g. conservative substitutions), insertions, or deletions relative to the reference sequence, but an anti-IGF-1R antibody comprising that sequence retains the ability to bind to IGF-1R. In certain embodiments, a total of 1 to 10 amino acids have been substituted, inserted and/or deleted in SEQ ID NO: 07 or 63 or 87. In certain embodiments, the substitutions, insertions, or deletions occur in regions outside the HVRs (i.e., in the FRs). Optionally, the anti-IGF-1R antibody comprises the VL sequence in SEQ ID NO: 07 or 63 or 87, including post-translational modifications of that sequence.

In another aspect, an anti-IGF-1R antibody is provided, wherein the antibody comprises a VH as in any of the embodiments provided above, and a VL as in any of the embodiments provided above. In one embodiment, the antibody comprises the VH and VL sequences in SEQ ID NO: 05 and SEQ ID NO: 07, or in SEQ ID NO: 62 and SEQ ID NO: 63, or in SEQ ID NO: 86 or SEQ ID NO: 87, respectively, including post-translational modifications of those sequences.

In a further aspect, the invention provides an antibody that binds to the same epitope as an anti-IGF-1R antibody provided herein. For example, in certain embodiments, an antibody is provided that binds to the same epitope as an anti-IGF-1R antibody comprising a VH sequence of SEQ ID NO: 05 and a VL sequence of SEQ ID NO: 07.

In another embodiment, the antibody is a full length antibody, e.g. an intact IgG1 or IgG4 antibody, with the FcRn-binding specificity as defined above.

The antibody as reported herein is produced by recombinant means. Thus, one aspect of the current invention is a nucleic acid encoding the antibody as reported herein and a further aspect is a cell comprising the nucleic acid encoding an antibody as reported herein. Methods for recombinant production are widely known in the state of the art and comprise protein expression in prokaryotic and eukaryotic cells with subsequent isolation of the antibody and usually purification to a pharmaceutically acceptable purity. For the expression of the antibodies as aforementioned in a host cell, nucleic acids encoding the respective (modified) light and heavy chains are inserted into expression plasmids by standard methods. Expression is performed in appropriate prokaryotic or eukaryotic host cells like CHO cells, NS0 cells, SP2/0 cells, HEK293 cells, COS cells, PER.C6 cells, yeast, or E.coli cells, and the antibody is recovered from the cells (cultivation supernatant or cells after lysis). General methods for recombinant production of antibodies are well-known in the state of the art and described, for example, in the review articles of Makrides, S.C., Protein Expr. Purif. 17 (1999) 183-202, Geisse, S., et al., Protein Expr. Purif. 8 (1996) 271-282, Kaufman, R.J., Mol. Biotechnol. 16 (2000) 151-160, and Werner, R.G., Drug Res. 48 (1998) 870-880.

Accordingly one aspect of the current invention is a method for the preparation of an antibody as reported herein, comprising the steps of

a) transforming a host cell with plasmids comprising nucleic acids encoding the antibody,
b) culturing the host cell under conditions that allow synthesis of the antibody, and
c) recovering the antibody from the culture.

In one embodiment the recovering step under c) includes the use of a light chain constant domain specific capture reagent (which e.g. specific for the kappa or the lambda constant light chain, depending on whether a kappa or a lambda light chain is contained in the antibody). In one embodiment this light chain specific capture reagent is used in a bind-and-elute-mode. Examples of such light chain constant domain specific capture reagents are e.g. KappaSelect™ and LambdaFabSelect™ (available from GE Healthcare/BAC), which are based on a highly rigid agarose base matrix that allows high flow rates and low back pressure at large scale. These materials contain a ligand that binds to the constant region of the kappa or the lambda light chain respectively (i.e. fragments lacking the constant region of the light chain will not bind). Both are therefore capable of binding other target molecules containing the constant region of the light chain, for example, IgG, IgA and IgM. The ligands are attached to the matrix via a long hydrophilic spacer arm to make them easily available for binding to the target molecule. They are based on a single-chain antibody fragment that is screened for either human Ig kappa or lambda.

In one embodiment the recovering step under c) includes the use of an Fc-region specific capture reagent. In one embodiment the Fc-region specific capture reagent is used in a bind-and-elute-mode. Examples of such Fc-region specific capture reagents are e.g. Staphylococcus protein A-based affinity chromatography materials.

The antibodies are suitably separated from the culture medium by conventional immunoglobulin purification procedures such as, for example, affinity chromatography (protein A-Sepharose, KappaSelect™, LambdaFabSelect™), hydroxylapatite chromatography, gel electrophoresis, or dialysis.

DNA and RNA encoding the monoclonal antibodies is readily isolated and sequenced using conventional procedures. B-cells or hybridoma cells can serve as a source of such DNA and RNA. Once isolated, the DNA may be inserted into expression plasmids, which are then transfected into host cells such as HEK 293 cells, CHO cells, or myeloma cells that do not otherwise produce immunoglobulin protein, to obtain the synthesis of recombinant monoclonal antibodies in the host cells.

Some of the antibodies as reported herein provide ease of isolation/purification by comprising Fc-regions that are differentially modified, wherein at least one of the modifications results in i) a differential affinity of the antibody for protein A, and ii) a differential affinity of the antibody for the human FcRn, and the antibody is isolable from a disrupted cell, from medium, or from a mixture of antibodies based on its affinity for protein A.

Purification of antibodies is performed in order to eliminate cellular components or other contaminants, e.g. other cellular nucleic acids or proteins, by standard techniques, including alkaline/SDS treatment, CsCl banding, column chromatography, agarose gel electrophoresis, and others well known in the art (see e.g. Ausubel, F., et al., ed. Current Protocols in Molecular Biology, Greene Publishing and Wiley Interscience, New York (1987)). Different methods are well established and widespread used for protein purification, such as affinity chromatography with microbial proteins (e.g. protein A or protein G affinity chromatography), ion exchange chromatography (e.g. cation exchange (carboxymethyl resins), anion exchange (amino ethyl resins) and mixed-mode exchange), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and electrophoretical methods (such as gel electrophoresis, capillary electrophoresis) (Vijayalakshmi, M.A., Appl. Biochem. Biotech. 75 (1998) 93-102).

It is herewith expressly stated that the term "comprising" as used herein comprises the term "consisting of". Thus, all aspects and embodiments that contain the term "comprising" are likewise disclosed with the term "consisting of".

In a further aspect, an anti-IGF-1R antibody according to any of the above embodiments may incorporate any of the features, singly or in combination, as described in Sections 1-6 below:

## 1. Antibody Affinity

In certain embodiments, an antibody provided herein has a dissociation constant (Kd) of $\leq 100$ nM, $\leq 10$ nM (e.g. $10^{-7}$ M or less, e.g. from $10^{-7}$ M to $10^{-13}$ M, e.g., from $10^{-8}$ M to $10^{-13}$ M).

In one embodiment, Kd is measured using a BIACORE® surface plasmon resonance assay. For example, an assay using a BIACORE®-2000 or a BIACORE®-3000 (GE Healthcare Inc., Piscataway, NJ) is performed at 25 °C with immobilized antigen CM5 chips at ~10 response units (RU). In one embodiment, carboxymethylated dextran biosensor chips (CM5, GE Healthcare Inc.) are activated with $N$-ethyl-$N'$- (3-dimethylaminopropyl)-carbodiimide hydrochloride (EDC) and $N$-hydroxysuccinimide (NHS) according to the supplier's instructions. Antigen is diluted with 10 mM sodium acetate, pH 4.8, to 5 $\mu$g/mL ($\sim$ 0.2 $\mu$M) before injection at a flow rate of 5 $\mu$l/minute to achieve approximately 10 response units (RU) of coupled protein. Following the injection of antigen, 1 M ethanolamine is injected to block non-reacted groups. For kinetics measurements, two-fold serial dilutions (e.g. of Fab) (0.78 nM to 500 nM) are injected in PBS with 0.05 % polysorbate 20 (TWEEN-20™) surfactant (PBST) at 25 °C at a flow rate of approximately 25 $\mu$L/min. Association rates ($k_{on}$) and dissociation rates ($k_{off}$) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio $k_{off}/k_{on}$ (see, e.g., Chen, Y. et al., J. Mol. Biol. 293 (1999) 865-881). If the on-rate exceeds $10^6$ M$^{-1}$ s$^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25 °C of a 20 nM anti-antigen antibody (Fab form) in PBS, pH 7.2, in the presence of increasing concentrations of antigen as measured in a spectrometer, such as a stop-flow equipped spectrophotometer (Aviv Instruments) or a 8000-series SLM-AMINCO ™ spectrophotometer (ThermoSpectronic) with a stirred cuvette.

## 2. Chimeric and Humanized Antibodies

In certain embodiments, an antibody provided herein is a chimeric antibody. Certain chimeric antibodies are described, e.g., in US 4,816,567; and Morrison, S.L.et al., Proc. Natl. Acad. Sci. USA 81 (1984) 6851-6855). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro, J.C. and Fransson, J., Front. Biosci. 13 (2008) 1619-1633, and are further described, e.g., in Riechmann, I., et al., Nature 332 (1988) 323-329; Queen, C., et al., Proc. Natl. Acad. Sci. USA 86 (1989) 10029-10033; US 5,821,337, US 7,527,791, US 6,982,321, and US 7,087,409; Kashmiri, S.V., et al., Methods 36 (2005) 25-34 (describing specificity determining region (SDR) grafting); Padlan, E.A., Mol. Immunol. 28 (1991) 489-498 (describing "resurfacing"); Dall'Acqua, W.F. et al., Methods 36 (2005) 43-60 (describing "FR shuffling"); Osbourn, J. et al., Methods 36 (2005) 61-68; and Klimka, A. et al., Br. J. Cancer 83 (2000) 252-260 (describing the "guided selection" approach to FR shuffling).

Human framework regions that may be used for humanization include but are not limited to: framework regions selected using the "best-fit" method (see, e.g., Sims, M.J., et al., J. Immunol. 151 (1993) 2296-2308; framework regions derived from the consensus sequence of human antibodies of a particular subgroup of light or heavy chain variable regions (see, e.g., Carter, P., et al., Proc. Natl. Acad. Sci. USA 89 (1992) 4285-4289; and Presta, L.G., et al., J. Immunol. 151 (1993) 2623-2632); human mature (somatically mutated) framework regions or human germline framework regions (see, e.g., Almagro, J.C. and Fransson, J., Front. Biosci. 13 (2008) 1619-1633); and framework regions derived from screening FR libraries (see, e.g., Baca, M. et al., J. Biol. Chem. 272 (1997) 10678-10684 and Rosok, M.J. et al., J. Biol. Chem. 271 (19969 22611-22618).

## 3. Human Antibodies

In certain embodiments, an antibody provided herein is a human antibody. Human antibodies can be produced using various techniques known in the art. Human antibodies are described generally in van Dijk, M.A. and van de Winkel, J.G., Curr. Opin. Pharmacol. 5 (2001) 368-374 and Lonberg, N., Curr. Opin. Immunol. 20 (2008) 450-459.

Human antibodies maybe prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, N., Nat. Biotech. 23 (2005) 1117-1125 (see also, e.g., US 6,075,181 and US 6,150,584 describing XENOMOUSE™ technology; US 5,770,429 describing HUMAB® technology; US 7,041,870 describing K-M MOUSE® technology, and US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described (see, e.g., Kozbor, D.J., Immunol. 133 (1984) 3001-3005; Brodeur, B.R., et al., Monoclonal Antibody Production Techniques and Applications, Marcel Dekker, Inc., New York (1987), pp. 51-63; and Boerner, P., et al., J. Immunol. 147 (1991) 86-95). Human antibodies generated via human B-cell hybridoma technology are also described in Li, J.et al., Proc. Natl. Acad. Sci. USA 103 (2006) 3557-3562. Additional methods include those described, for example, in US 7,189,826 (describing production of monoclonal human IgM antibodies from hybridoma cell lines) and Ni, J., Xiandai Mianyixue 26 (2006) 265-268 (describing human-human hybridomas). Human hybridoma technology (Trioma technology) is also described in Vollmers, H.P. and Brandlein, S., Histology and Histopathology 20 (2005) 927-937 and Vollmers, H.P. and Brandlein, S., Methods and Findings in Experimental and Clinical Pharmacology 27 (2005) 185-191.

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

## 4. Library-Derived Antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom, H.R. et al., Methods in Molecular Biology 178 (2001) 1-37 and further described, e.g., in the McCafferty, J. et al., Nature348 (1990) 552-554; Clackson, T. et al., Nature 352 (1991) 624-628; Marks, J.D. et al., J. Mol. Biol. 222 (1992) 581-597; Marks, J.D. and Bradbury, A., Methods in Molecular Biology 248 (2003) 161-175; Sidhu, S.S. et al., J. Mol. Biol. 338 (2004) 299-310; Lee, C.V. et al., J. Mol. Biol. 340 (2004) 1073-1093; Fellouse, F.A., Proc. Natl. Acad. Sci. USA 101 (2004) 12467-12472; and Lee, C.V. et al., J. Immunol. Methods 284 (2004) 119-132.

In certain phage display methods, repertoires of VH and VL genes are separately cloned by polymerase chain reaction (PCR) and recombined randomly in phage libraries, which can then be screened for antigen-binding phage as described in Winter, G., et al., Ann. Rev. Immunol. 12 (1994) 433-455. Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self-antigens without any immunization as described by Griffiths, A.D., et al., EMBO J. 12 (1993) 725-734. Finally, naive libraries can also be made synthetically by cloning non-rearranged V-gene segments from stem cells, and using PCR primers containing random sequence to encode the highly variable CDR3 regions and to accomplish rearrangement *in vitro,* as described by Hoogenboom, H.R. and Winter, G., J. Mol. Biol. 227 (1992) 381-388. Patent publications describing human antibody phage libraries include, for example: US 5,750,373, and US 2005/0079574, US 2005/0119455, US 2005/0266000, US 2007/0117126, US 2007/0160598, US 2007/0237764, US 2007/0292936, and US 2009/0002360.

Antibodies or antibody fragments isolated from human antibody libraries are considered human antibodies or human antibody fragments herein.

**5. Multispecific Antibodies**

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for IGF-1Rand the other is for any other antigen. In certain embodiments, bispecific antibodies may bind to two different epitopes of IGF-1R.Bispecific antibodies may also be used to localize cytotoxic agents to cells which express IGF-1R.Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein, C. and Cuello, A.C., Nature 305 (1983) 537-540, WO 93/08829, and Traunecker, A., et al., EMBO J. 10 (1991) 3655-3659), and "knob-in-hole" engineering (see, e.g., US 5,731,168). Multi-specific antibodies may also be made by engineering electrostatic steering effects for making antibody Fc-heterodimeric molecules (WO 2009/089004); cross-linking two or more antibodies or fragments (see, e.g., US 4,676,980, and Brennan, M. et al., Science 229 (1985) 81-83); using leucine zippers to produce bi-specific antibodies (see, e.g., Kostelny, S.A., et al., J. Immunol. 148 (1992) 1547-1553; using "diabody" technology for making bispecific antibody fragments (see, e.g., Holliger, P. et al., Proc. Natl. Acad. Sci. USA 90 (1993) 6444-6448); and using single-chain Fv (sFv) dimers (see, e.g. Gruber, M et al., J. Immunol. 152 (1994) 5368-5374); and preparing trispecific antibodies as described, e.g., in Tutt, A. et al., J. Immunol. 147 (1991) 60-69.

Engineered antibodies with three or more functional antigen binding sites, including "Octopus antibodies," are also included herein (see, e.g. US 2006/0025576).

The antibody or fragment herein also includes a "Dual Acting Fab" or "DAF" comprising an antigen binding site that binds to IGF-1R as well as another, different antigen (see, US 2008/0069820, for example).

The antibody or fragment herein also includes multispecific antibodies described in WO 2009/080251, WO 2009/080252, WO 2009/080253, WO 2009/080254, WO 2010/112193, WO 2010/115589, WO 2010/136172, WO 2010/145792, and WO 2010/145793.

**6. Antibody Variants**

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

**a) Substitution, Insertion, and Deletion Variants**

In certain embodiments, antibody variants having one or more amino acid substitutions are provided. Sites of interest for substitutional mutagenesis include the HVRs and FRs. Conservative substitutions are shown in the following Table under the heading of "preferred substitutions". More substantial changes are provided in the following Table under the heading of "exemplary substitutions", and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**Table.**

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;

(2) neutral hydrophilic: Cys, Ser, Thr, Asn, Gln;

(3) acidic: Asp, Glu;

(4) basic: His, Lys, Arg;

(5) residues that influence chain orientation: Gly, Pro;

(6) aromatic: Trp, Tyr, Phe.

Non-conservative substitutions will entail exchanging a member of one of these classes for another class.
One type of substitutional variant involves substituting one or more hypervariable region residues of a parent antibody (*e.g.* a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody.

An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, P.S., Methods Mol. Biol. 207 (2008) 179-196), and/or residues that contact antigen, with the resulting variant VH or VL being tested for binding affinity. Affinity maturation by constructing and reselecting from secondary libraries has been described, e.g., in Hoogenboom, H.R. et al. in Methods in Molecular Biology 178 (2002) 1-37. In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham, B.C. and Wells, J.A., Science 244 (1989) 1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen can be used. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

**b) Glycosylation variants**

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc-region, the carbohydrate attached thereto may be altered. Native antibodies produced by mammalian cells typically comprise a branched, biantennary oligosaccharide that is generally attached by an N-linkage to Asn297 of the CH2 domain of the Fc-region (see, e.g., Wright, A. and Morrison, S.L., TIBTECH 15 (1997) 26-32). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc-region. For example, the amount of fucose in such antibody may be from 1% to 80%, from 1% to 65%, from 5 % to 65 % or from 20 % to 40 %. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc-region (EU numbering of Fc-region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US 2003/0157108; US 2004/0093621. Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO 2005/053742; WO 2002/031140; Okazaki, A. et al., J. Mol. Biol. 336 (2004) 1239-1249; Yamane-Ohnuki, N. et al., Biotech. Bioeng. 87 (2004) 614-622. Examples of cell lines capable of producing defucosylated antibodies include Lec13 CHO cells deficient in protein fucosylation (Ripka, J., et al., Arch. Biochem. Biophys. 249 (1986) 533-545; US 2003/0157108; and WO 2004/056312, especially at Example 11), and knockout cell lines, such as alpha-1,6-fucosyltransferase gene, *FUT8,* knockout CHO cells (see, e.g., Yamane-Ohnuki, N., et al., Biotech. Bioeng. 87 (2004) 614-622; Kanda, Y.et al., Biotechnol. Bioeng. 94 (2006) 680-688; and WO2003/085107).

Antibodies variants are further provided with bisected oligosaccharides, e.g., in which a biantennary oligosaccharide attached to the Fc-region of the antibody is bisected by GlcNAc. Such antibody variants may have reduced fucosylation and/or improved ADCC function. Examples of such antibody variants are described, e.g., in WO 2003/011878; US 6,602,684; and US 2005/0123546. Antibody variants with at least one galactose residue in the oligosaccharide attached to the Fc-region are also provided. Such antibody variants may have improved CDC function. Such antibody variants are described, e.g., in WO 1997/30087; WO 1998/58964; and WO 1999/22764.

### c) Fc-region variants

In certain embodiments, one or more further amino acid modifications may be introduced into the Fc-region of an antibody provided herein, thereby generating an Fc-region variant. The Fc-region variant may comprise a human Fc-region sequence (*e.g.*, a human IgG1, IgG2, IgG3 or IgG4 Fc-region) comprising an amino acid modification (*e.g.* a substitution/mutation) at one or more amino acid positions.

In certain embodiments, the invention contemplates an antibody variant that possesses some but not all effector functions, which make it a desirable candidate for applications in which the half-life of the antibody *in vivo* is important. FcRn binding and *in vivo* clearance/half-life determinations can also be performed using methods known in the art (see, e.g., Petkova, S.B. et al., Int. Immunol. 18 (2006) 1759-1769).

Antibodies with reduced effector function include those with substitution of one or more of Fc-region residues 238, 265, 269, 270, 297, 327 and 329 (US 6,737,056). Such Fc-region variants include Fc-regions with substitutions at two or more of amino acid positions 265, 269, 270, 297 and 327, including the so-called "DANA" Fc-region mutant with substitution of residues 265 and 297 to alanine (US 7,332,581).

Certain antibody variants with improved or diminished binding to FcRs are described (see, e.g., US 6,737,056; WO 2004/056312, and Shields, R.L. et al., J. Biol. Chem. 276 (2001) 6591-6604).

In certain embodiments, an antibody variant comprises an Fc-region with one or more amino acid substitutions which improve ADCC, e.g., substitutions at positions 298, 333, and/or 334 of the Fc-region (EU numbering of residues).

In some embodiments, alterations are made in the Fc-region that result in altered (*i.e.,* either improved or diminished) C1q binding and/or Complement Dependent Cytotoxicity (CDC), e.g., as described in US 6,194,551, WO 99/51642, and Idusogie, E.E. et al., J. Immunol. 164 (2000) 4178-4184.

See also Duncan, A.R. and Winter, G., Nature 322 (1988) 738-740; US 5,648,260; US 5,624,821; and WO 94/29351 concerning other examples of Fc-region variants.

### d) Cysteine engineered antibody variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc-region. Cysteine engineered antibodies may be generated as described, e.g., in US 7,521,541.

### e) Antibody Derivatives

In certain embodiments, an antibody provided herein may be further modified to contain additional non-proteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide copolymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or non-branched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and non-proteinaceous moiety that may be selectively heated by exposure to radiation are provided. In one embodiment, the non-proteinaceous moiety is a carbon nanotube (Kam, N.W. et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed.

**f) Heterodimerization**

There exist several approaches for CH3-modifications to enforce the heterodimerization, which are well described e.g. in WO 96/27011, WO 98/050431, EP 1870459, WO 2007/110205, WO 2007/147901, WO 2009/089004, WO 2010/129304, WO 2011/90754, WO 2011/143545, WO 2012058768, WO 2013157954, WO 2013096291. Typically in all such approaches the first CH3 domain and the second CH3 domains are both engineered in a complementary manner so that each CH3 domain (or the heavy chain comprising it) cannot longer homodimerize with itself but is forced to heterodimerize with the complementary engineered other CH3 domain (so that the first and second CH3 domain heterodimerize and no homodimers between the two first or the two second CH3 domains are formed). These different approaches for improved heavy chain heterodimerization are contemplated as different alternatives in combination with the heavy -light chain modifications (VH and VL exchange/replacement in one binding arm and the introduction of substitutions of charged amino acids with opposite charges in the CH1/CL interface) in the multispecific antibodies according to the invention which reduce light chain mispairing an Bence-Jones type side products.

In one preferred embodiment of the invention (in case the multispecific antibody comprises CH3 domains in the heavy chains) the CH3 domains of said multispecific antibody according to the invention can be altered by the "knob-into-holes" technology which is described in detail with several examples in e.g. WO 96/027011, Ridgway, J.B., et al., Protein Eng. 9 (1996) 617-621; and Merchant, A.M., et al., Nat. Biotechnol. 16 (1998) 677-681; WO 98/ 050431(. In this method the interaction surfaces of the two CH3 domains are altered to increase the heterodimerization of both heavy chains containing these two CH3 domains. Each of the two CH3 domains (of the two heavy chains) can be the "knob", while the other is the "hole". The introduction of a disulfide bridge further stabilizes the heterodimers (Merchant, A.M., et al., Nature Biotech. 16 (1998) 677-681; Atwell, S., et al., J. Mol. Biol. 270 (1997) 26-35) and increases the yield.

Thus in one embodiment of the invention said multispecific antibody (comprises a CH3 domain in each heavy chain and) is further characterized in that

the first CH3 domain of the first heavy chain of the antibody under a) and the second CH3 domain of the second heavy chain of the antibody under b) each meet at an interface which comprises an original interface between the antibody CH3 domains.

wherein said interface is altered to promote the formation of the multispecific antibody, wherein the alteration is characterized in that:

i) the CH3 domain of one heavy chain is altered,
so that within the original interface of the CH3 domain of one heavy chain that meets the original interface of the CH3 domain of the other heavy chain within the multispecific antibody,
an amino acid residue is replaced with an amino acid residue having a larger side chain volume, thereby generating a protuberance within the interface of the CH3 domain of one heavy chain which is positionable in a cavity within the interface of the CH3 domain of the other heavy chain
and
ii) the CH3 domain of the other heavy chain is altered,
so that within the original interface of the second CH3 domain that meets the original interface of the first CH3 domain within the multispecific antibody
an amino acid residue is replaced with an amino acid residue having a smaller side chain volume, thereby generating a cavity within the interface of the second CH3 domain within which a protuberance within the interface of the first CH3 domain is positionable.

Preferably said amino acid residue having a larger side chain volume is selected from the group consisting of arginine (R), phenylalanine (F), tyrosine (Y), tryptophan (W).

Preferably said amino acid residue having a smaller side chain volume is selected from the group consisting of alanine (A), serine (S), threonine (T), valine (V).

In one aspect of the invention both CH3 domains are further altered by the introduction of cysteine (C) as amino acid in the corresponding positions of each CH3 domain such that a disulfide bridge between both CH3 domains can be formed.

In one preferred embodiment, said multispecific antibody comprises a amino acid T366W mutation in the first CH3 domain of the "knobs chain" and amino acid T366S, L368A, Y407V mutations in the second CH3 domain of the "hole chain". An additional interchain disulfide bridge between the CH3 domains can also be used (Merchant, A.M., et al., Nature Biotech. 16 (1998) 677-681) e.g. by introducing an amino acid Y349C mutation into the CH3 domain of the "hole chain" and an amino acid E356C mutation or an amino acid S354C mutation into the CH3 domain of the "knobs chain".

In one preferred embodiment, said multispecific antibody (which comprises a CH3 domain in each heavy chain) comprises amino acid S354C, T366W mutations in one of the two CH3 domains and amino acid Y349C, T366S, L368A, Y407V mutations in the other of the two CH3 domains (the additional amino acid S354C mutation in one CH3 domain and the additional amino acid Y349C mutation in the other CH3 domain forming an interchain disulfide bridge) (numbering according to Kabat).

Other techniques for CH3-modifications to enforcing the heterodimerization are contemplated as alternatives of the invention and described e.g. in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954, WO 2013/096291.

In one embodiment the heterodimerization approach described in EP 1 870 459A1, can be used alternatively. This approach is based on the by the introduction of substitutions/mutations of charged amino acids with the opposite charge at specific amino acid positions of the in the CH3/ CH3 domain interface between both heavy chains. One preferred embodiment for said multispecific antibody are amino acid R409D; K370E mutations in the first CH3 domain of the (of the multispecific antibody) and amino acid D399K; E357K mutations in the seconds CH3 domain of the multispecific antibody (numbering according to Kabat).

In another embodiment said multispecific antibody comprises a amino acid T366W mutation in the CH3 domain of the "knobs chain" and amino acid T366S, L368A, Y407V mutations in the CH3 domain of the "hole chain" and additionally amino acid R409D; K370E mutations in the CH3 domain of the "knobs chain" and amino acid D399K; E357K mutations in the CH3 domain of the "hole chain".

In another embodiment said multispecific antibody comprises amino acid S354C, T366W mutations in one of the two CH3 domains and amino acid Y349C, T366S, L368A, Y407V mutations in the other of the two CH3 domains or said multispecific antibody comprises amino acid Y349C, T366W mutations in one of the two CH3 domains and amino acid S354C, T366S, L368A, Y407V mutations in the other of the two CH3 domains and additionally amino acid R409D; K370E mutations in the CH3 domain of the "knobs chain" and amino acid D399K; E357K mutations in the CH3 domain of the "hole chain".

In one embodiment the heterodimerization approach described in WO2013/157953 can be used alternatively. In one embodiment a first CH3 domain comprises amino acid T366K mutation and a second CH3 domain polypeptide comprises amino acid L351D mutation. In a further embodiment the first CH3 domain comprises further amino acid L351K mutation. In a further embodiment the second CH3 domain comprises further amino acid mutation selected from Y349E, Y349D and L368E (preferably L368E).

In one embodiment the heterodimerization approach described in WO2012/058768 can be used alternatively. In one embodiment a first CH3 domain comprises amino acid L351Y, Y407A mutations and a second CH3 domain comprises amino acid T366A, K409F mutations. In a further embodiment the second CH3 domain comprises a further amino acid mutation at position T411, D399, S400, F405, N390, or K392 e.g. selected from a) T411 N, T411 R, T411Q, T411 K, T411D, T411E or T411W, b) D399R, D399W, D399Y or D399K, c S400E, S400D, S400R, or S400K F405I, F405M, F405T, F405S, F405V or F405W N390R, N390K or N390D K392V, K392M, K392R, K392L, K392F or K392E. In a further embodiment a first CH3 domain comprises amino acid L351Y, Y407A mutations and a second CH3 domain comprises amino acid T366V, K409F mutations. In a further embodiment a first CH3 domain comprises amino acid Y407A mutations and a second CH3 domain comprises amino acid T366A, K409F mutations. In a further embodiment the second CH3 domain comprises a further amino acid K392E, T411E, D399R and S400R mutations.

In one embodiment the heterodimerization approach described in WO2011/143545 can be used alternatively e.g. with the amino acid modification at a position selected from the group consisting of 368 and 409.

In one embodiment the heterodimerization approach described in WO2011/090762 which also uses the knobs-into-holes technology described above can be used alternatively,. In one embodiment a first CH3 domain comprises amino acid T366W mutations and a second CH3 domain comprises amino acid Y407A mutations. In one embodiment a first CH3 domain comprises amino acid T366Y mutations and a second CH3 domain comprises amino acid Y407T mutations.

In one embodiment the multispecific antibody is of IgG2 isotype and the heterodimerization approach described in WO2010/129304 can be used alternatively.

In one embodiment the heterodimerization approach described in WO2009/089004 can be used alternatively. In one embodiment a first CH3 domain comprises amino acid substitution of K392 or N392 with a negative-charged amino acid (e.g. glutamic acid (E), or aspartic acid (D), preferably K392D or N392D) and a second CH3 domain comprises amino acid substitution of D399, E356, D356, or E357 with a positive-charged amino acid (e.g. Lysine (K) or arginine (R), preferably D399K, E356K, D356K, or E357K and more preferably D399K and E356K. In a further embodiment the first CH3 domain further comprises amino acid substitution of K409 or R409 with a negative-charged amino acid (e.g. glutamic acid (E), or aspartic acid (D), preferably K409D or R409D). In a further embodiment the first CH3 domain further or alternatively comprises amino acid substitution of K439 and/or K370 with a negative-charged amino acid (e.g. glutamic acid (E), or aspartic acid (D)).

In one embodiment the heterodimerization approach described in WO2007/147901 can be used alternatively. In one embodiment a first CH3 domain comprises amino acid K253E, D282K, and K322D mutations and a second CH3 domain comprises amino acid D239K, E240K, and K292D mutations.

In one embodiment the heterodimerization approach described in WO2007/110205 can be used alternatively.

## D. Recombinant Methods and Compositions

Antibodies may be produced using recombinant methods and compositions, e.g., as described in US 4,816,567. In one embodiment, isolated nucleic acid(s) encoding an anti-IGF-1R antibody described herein is(are) provided. Such nucleic acid may encode an amino acid sequence comprising the VL and/or an amino acid sequence comprising the VH of the antibody (e.g., the light and/or heavy chains of the antibody). In a further embodiment, one or more plasmids (e.g., expression plasmids) comprising such nucleic acid are provided. In a further embodiment, a host cell comprising such nucleic acid is provided. In one such embodiment, a host cell comprises (e.g., has been transformed with): (1) a plasmid comprising a nucleic acid that encodes an amino acid sequence comprising the VL of the antibody and an amino acid sequence comprising the VH of the antibody, or (2) a first plasmid comprising a nucleic acid that encodes an amino acid sequence comprising the VL of the antibody and a second plasmid comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp20 cell).In one embodiment, a method of making an anti-IGF-1R antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-IGF-1R antibody, nucleic acid encoding an antibody, e.g., as described above, is isolated and inserted into one or more plasmids for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding plasmids include prokaryotic or eukaryotic cells described herein. For example, antibodies may be produced in bacteria, in particular when glycosylation and Fc effector function are not needed. For expression of antibody fragments and polypeptides in bacteria, see, e.g., US 5,648,237, US 5,789,199, and US 5,840,523 (see also Charlton, K.A., In: Methods in Molecular Biology, Vol. 248, Lo, B.K.C. (ed.), Humana Press, Totowa, NJ (2003), pp. 245-254, describing expression of antibody fragments in *E. coli*.). After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding plasmids, including fungi and yeast strains whose glycosylation pathways have been "humanized", resulting in the production of an antibody with a partially or fully human glycosylation pattern. See Gerngross, T.U., Nat. Biotech. 22 (2004) 1409-1414; and Li, H. et al., Nat. Biotech. 24 (2006) 210-215.

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. See, e.g., US 5,959,177, US 6,040,498, US 6,420,548, US 7,125,978, and US 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (HEK293 or 293 cells as described, e.g., in Graham, F.L., et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor (MMT 060562); TRI cells, as described, e.g., in Mather, J.P., et al., Annals N.Y. Acad. Sci. 383 (1982) 44-68; MRC 5 cells; and FS4 cells. Other useful mammalian host cell lines include Chinese hamster ovary (CHO) cells, including DHFR⁻ CHO cells (Urlaub, G., et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

## E. Assays

Anti-IGF-1R antibodies provided herein may be identified, screened for, or characterized for their physical/chemical properties and/or biological activities by various assays known in the art.

In one aspect, an antibody of the invention is tested for its antigen binding activity, e.g., by known methods such as ELISA, Western blot, etc.

In one aspect, assays are provided for identifying anti-IGF-1R antibodies thereof having biological activity.

## F. Combination treatment

In certain embodiments the anti-IGF-1R antibody or pharmaceutical composition according to the invention is administered alone (without an additional therapeutic agent) for the treatment of one or more ocular vascular diseases described herein.

In other embodiments the anti-IGF-1R antibody or pharmaceutical composition according to the invention is administered in combination with one or more additional therapeutic agents or methods for the treatment of one or more vascular eye diseases described herein.

In other embodiments, the anti-IGF-1R antibody or pharmaceutical composition according to the invention is formulated in combination with one or more additional therapeutic agents and administered for the treatment of one or more vascular eye diseases described herein.

In certain embodiments, the combination treatments provided herein include that the anti-IGF-1R antibody or pharmaceutical composition according to the invention is administered sequentially with one or more additional therapeutic agents for the treatment of one or more ocular vascular diseases described herein.

The additional therapeutic agents include, but are not limited to, Tryptophanyl-tRNA synthetase (TrpRS), EyeOOl (anti-VEGF PEGylated aptamer), squalamine, RETAANE(TM) (anecortave acetate for depot suspension; Alcon, Inc.), Combretastatin A4 Prodrug (CA4P), MACUGEN(TM), MIFEPREX(TM) (mifepristone-ru486), subtenon triamcinolone acetonide, intravitreal crystalline triamcinolone acetonide, Prinomastat (AG3340- synthetic matrix metalloproteinase inhibitor, Pfizer), fluocinolone acetonide (including fluocinolone intraocular implant, Bausch & Lomb/Control Delivery Systems), VEGFR inhibitors (Sugen), VEGF-Trap (Regeneron/Aventis), VEGF receptor tyrosine kinase inhibitors such as 4-(4-bromo-2-fluoroanilino)-6-methoxy-7-(1-methylpiperidin-4-ylmethoxy)quinazoline (ZD6474), 4-(4-fluoro-2-methylindol-5- yloxy)-6-methoxy-7-(3- pyrrolidin-1-ylpropoxy)quinazoline (AZD2171), vatalanib (PTK787) and SU1 1248 (sunitinib), linomide, and inhibitors of integrin v.beta.3 function and angiostatin.

Other pharmaceutical therapies that can be used in combination with the anti-IGF-1R antibody or pharmaceutical composition according to the invention, including, but are not limited to, VISUDYNE(TM) with use of a non-thermal laser, PKC 412, Endovion (NeuroSearch A/S), neurotrophic factors, including by way of example Glial Derived Neurotrophic Factor and Ciliary Neurotrophic Factor, diatazem, dorzolamide, Phototrop, 9-cis-retinal, eye medication (including Echo Therapy) including phospholine iodide or echothiophate or carbonic anhydrase inhibitors, AE-941 (AEterna Laboratories, Inc.), Sirna-027 (Sima Therapeutics, Inc.), pegaptanib (NeXstar Pharmaceuticals/Gilead Sciences), neurotrophins (including, by way of example only, NT-4/5, Genentech), Cand5 (Acuity Pharmaceuticals), INS-37217 (Inspire Pharmaceuticals), integrin antagonists (including those from Jerini AG and Abbott Laboratories), EG-3306 (Ark Therapeutics Ltd.), BDM-E (BioDiem Ltd.), thalidomide (as used, for example, by EntreMed, Inc.), cardiotrophin-1 (Genentech), 2-methoxyestradiol (Allergan/Oculex), DL-8234 (Toray Industries), NTC-200 (Neurotech), tetrathiomolybdate (University of Michigan), LYN-002 (Lynkeus Biotech), microalgal compound (Aquasearch/Albany, Mera Pharmaceuticals), D-9120 (Celltech Group plc.), ATX-S10 (Hamamatsu Photonics), TGF-beta 2 (Genzyme/Celtrix), tyrosine kinase inhibitors (Allergan, SUGEN, Pfizer), NX-278-L (NeXstar Pharmaceuticals/Gilead Sciences), Opt-24 (OPTIS France SA), retinal cell ganglion neuroprotectants (Cogent Neurosciences), N-nitropyrazole derivatives (Texas A&M University System), KP-102 (Krenitsky Pharmaceuticals), cyclosporin A, Timited retinal translocation, photodynamic therapy, (including, by way of example only, receptor-targeted PDT, Bristol-Myers Squibb, Co.; porfimer sodium for injection with PDT; verteporfin, QLT Inc.; rostaporfin with PDT, Miravent Medical Technologies; talaporfin sodium with PDT, Nippon Petroleum; motexafin lutetium, Pharmacyclics, Inc.), antisense oligonucleotides (including, by way of example, products tested by Novagali Pharma SA and ISIS-13650, Isis Pharmaceuticals), laser photocoagulation, drusen lasering, macular hole surgery, macular translocation surgery, implantable miniature telescopes, Phi-Motion Angiography (also known as Micro-Laser Therapy and Feeder Vessel Treatment), Proton Beam Therapy, microstimulation therapy, Retinal Detachment and Vitreous Surgery, Scleral Buckle, Submacular Surgery, Transpupillary Thermotherapy, Photosystem I therapy, use of RNA interference (RNAi), extracorporeal rheopheresis (also known as membrane differential filtration and Rheotherapy), microchip implantation, stem cell therapy, gene replacement therapy, ribozyme gene therapy (including gene therapy for hypoxia response element, Oxford Biomedica; Lentipak, Genetix; PDEF gene therapy, GenVec), photoreceptor/retinal cells transplantation (including transplantable retinal epithelial cells, Diacrin, Inc.; retinal cell transplant, Cell Genesys, Inc.), and acupuncture.

Any anti-angiogenic agent can be used in combination with the anti-IGF-1R antibody or pharmaceutical composition according to the invention, including, but not limited to, those listed by Carmeliet and Jain, Nature 407 (2000) 249-257. In certain embodiments, the anti-angiogenic agent is another VEGF antagonist or a VEGF receptor antagonist such as VEGF variants, soluble VEGF receptor fragments, aptamers capable of blocking VEGF or VEGFR, neutralizing anti-VEGFR antibodies, low molecule weight inhibitors of VEGFR tyrosine kinases and any combinations thereof and these include anti-VEGF aptamers (e.g. Pegaptanib), soluble recombinant decoy receptors (e.g. VEGF Trap). In certain embodiments, the anti-angiogenic agent is include corticosteroids, angiostatic steroids, anecortave acetate, angiostatin, endostatin, small interfering RNA's decreasing expression of VEGFR or VEGF ligand, post-VEGFR blockade with tyrosine kinase inhibitors, MMP inhibitors, IGFBP3, SDF-1 blockers, PEDF, gamma-secretase, Delta-like ligand 4, integrin antagonists, HIF-1 alpha blockade, protein kinase CK2 blockade, and inhibition of stem cell (i.e. endothelial progenitor cell) homing to the site of neovascularization using vascular endothelial cadherin (CD-144) and stromal derived factor (SDF)-I antibodies. Small molecule RTK inhibitors targeting VEGF receptors including PTK787 can also be used. Agents that have activity against neovascularization that are not necessarily anti-VEGF compounds can also be used and include anti-inflammatory drugs, m-Tor inhibitors, rapamycin, everolismus, temsirolismus, cyclospohne, anti-TNF agents, anti-complement agents, and nonsteroidal anti-inflammatory agents. Agents that are neuroprotective and can potentially reduce the progression of dry macular degeneration can also be used, such as the class of drugs called the 'neurosteroids.' These include drugs such as dehydroepiandrosterone (DHEA) (Brand names: Prastera(R) and Fidelin(R)), dehydroepiandrosterone sulfate, and pregnenolone sulfate. Any AMD (age-related macular degeneration) therapeutic agent can be used in combination with the bispecific antibody or pharmaceutical composition according to the invention, including but not limited to verteporfin in combination with PDT, pegaptanib sodium, zinc, or an antioxidant(s), alone or in any combination.

## G. Pharmaceutical Formulations

Pharmaceutical formulations of an anti-IGF-1R antibody as described herein are prepared by mixing such antibody having the desired degree of purity with one or more optional pharmaceutically acceptable carriers (Remington's Pharmaceutical Sciences, 16th edition, Osol, A. (ed.) (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyl dimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as poly(vinylpyrrolidone); amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.* Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rhuPH20 (HYLENEX®, Baxter International, Inc.). Certain exemplary sHASEGPs and methods of use, including rhuPH20, are described in US 2005/0260186 and US 2006/0104968. In one aspect, a sHASEGP is combined with one or more additional glycosaminoglycanases such as chondroitinases.

Exemplary lyophilized antibody formulations are described in US 6,267,958. Aqueous antibody formulations include those described in US 6,171,586 and WO 2006/044908, the latter formulations including a histidine-acetate buffer.

The formulation herein may also contain more than one active ingredients as necessary for the particular indication being treated, preferably those with complementary activities that do not adversely affect each other.

Active ingredients may be entrapped in microcapsules prepared, for example, by coacervation techniques or by interfacial polymerization, for example, hydroxymethylcellulose or gelatin-microcapsules and poly-(methyl methacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semi-permeable matrices of solid hydrophobic polymers containing the antibody, which matrices are in the form of shaped articles, *e.g.* films, or microcapsules.

The formulations to be used for *in vivo* administration are generally sterile. Sterility may be readily accomplished, e.g., by filtration through sterile filtration membranes.

## H. Therapeutic Methods and Compositions

Any of the anti-IGF-1R antibodies provided herein may be used in therapeutic methods.

In one aspect, an anti-IGF-1R antibody for use as a medicament is provided. In further aspects, an anti-IGF-1R antibody for use in treating vascular eye diseases is provided. In certain embodiments, an anti-IGF-1R antibody for use in a method of treatment is provided. In certain embodiments, the invention provides an anti-IGF-1R antibody for use in a method of treating an individual having a vascular eye disease comprising administering to the individual an effective amount of the anti-IGF-1R antibody. In one such embodiment, the method further comprises administering to the individual an effective amount of at least one additional therapeutic agent, e.g., as described above in section D. In further embodiments, the invention provides an anti-IGF-1R antibody for use in inhibiting IGF-1R in the eye. In certain embodiments, the invention provides an anti-IGF-1R antibody for use in a method of inhibiting angiogenesis in an individual comprising administering to the individual an effective of the anti-IGF-1R antibody to inhibit IGF-1R signaling. An "individual" according to any of the above embodiments is in one preferred embodiment a human.

In a further aspect, the invention provides for the use of an anti-IGF-1R antibody in the manufacture or preparation of a medicament. In one embodiment, the medicament is for treatment of a vascular eye disease. In a further embodiment, the medicament is for use in a method of treating a vascular eye disease comprising administering to an individual having a vascular eye disease an effective amount of the medicament. In one such embodiment, the method further comprises administering to the individual an effective amount of at least one additional therapeutic agent, e.g., as described above. In a further embodiment, the medicament is for inhibiting angiogenesis. In a further embodiment, the medicament is for use in a method of inhibiting angiogenesis in an individual comprising administering to the individual an amount effective of the medicament to inhibit IGF-1R mediated signaling. An "individual" according to any of the above embodiments may be a human.

In a further aspect, the invention provides a method for treating a vascular eye disease. In one embodiment, the method comprises administering to an individual having such a vascular eye disease an effective amount of an anti-IGF-1R antibody. In one such embodiment, the method further comprises administering to the individual an effective amount of at least one additional therapeutic agent, as described below. An "individual" according to any of the above embodiments may be a human.

In a further aspect, the invention provides a method for inhibiting angiogenesis in the eye in an individual. In one embodiment, the method comprises administering to the individual an effective amount of an anti-IGF-1R antibody to inhibit IGF-1R mediated signaling. In one embodiment, an "individual" is a human.

In a further aspect, the invention provides pharmaceutical formulations comprising any of the anti-IGF-1R antibodies provided herein, e.g., for use in any of the above therapeutic methods. In one embodiment, a pharmaceutical formulation comprises any of the anti-IGF-1R antibodies provided herein and a pharmaceutically acceptable carrier. In another embodiment, a pharmaceutical formulation comprises any of the anti-IGF-1R antibodies provided herein and at least one additional therapeutic agent, e.g., as described below.

Antibodies of the invention can be used either alone or in combination with other agents in a therapy. For instance, an antibody of the invention may be co-administered with at least one additional therapeutic agent.

An antibody as reported herein (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies as reported herein would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99 % of the dosages described herein, or in any dosage and by any route that is empirically/clinically determined to be appropriate.

For the prevention or treatment of disease, the appropriate dosage of an antibody of the invention (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 μg/kg to 15 mg/kg (e.g. 0.5 mg/kg - 10 mg/kg) of antibody can be an initial candidate dosage for administration to the patient, whether, for example, by one or more separate administrations, or by continuous infusion. One typical daily dosage might range from about 1 μg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05mg/kg to about 10mg/kg. Thus, one or more doses of about 0.5mg/kg, 2.0mg/kg, 4.0mg/kg or 10mg/kg (or any combination thereof) may be administered to the patient. Such doses may be administered intermittently, e.g. every week or every three weeks (*e.g.* such that the patient receives from about two to about twenty, or *e.g.* about six doses of the antibody). An initial higher loading dose, followed by one or more lower doses may be administered. The progress of this therapy is easily monitored by conventional techniques and assays.

## III. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is used for treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the invention in place of or in addition to an anti-IGF-1R antibody.

## IV. SPECIFIC EMBODIMENTS

1. An anti-IGF-1R antibody that specifically binds to human IGF-1R and that has abolished FcRn binding.

2. The antibody according to embodiment 1, wherein the antibody binds to human FcRn with an affinity comparable to or less than an antibody having the mutations I253A/H310A/H435A or H310A/H433A/Y436A or L251D/L314D/L432D or combinations thereof in the Fc-region (numbering according to Kabat EU index numbering system).

3. The antibody according to any one of embodiments 1 to 2, wherein the anti-IGF-1R antibody has no (remaining) detectable FcRn binding using a surface plasmon resonance based determination method.

4. The antibody according to any one of embodiments 1 to 3, wherein the anti-IGF-1R antibody binds to human FcRn with a $K_D$-value of more than 1.7 μM at pH 6.

5. The antibody according to any one of embodiments 1 to 4, wherein the antibody has the same or a shorter retention time on an FcRn affinity chromatography column as an antibody comprising an Fc-region with the mutations I253A/H310A/H435A or H310A/H433A/Y436A or L251D/L314D/L432D or combinations thereof (numbering according to Kabat EU index numbering system).

6. The antibody according to any one of embodiments 1 to 5, wherein the antibody has a retention time on an FcRn affinity chromatography column of three minutes or less.

7. The antibody according to embodiment 6, wherein the FcRn affinity column has the column dimensions of 50 mm x 5 mm, the bed height is 5 cm and the loading is 50 μg antibody.

8. The antibody according to any one of embodiments 6 to 7, wherein for the FcRn affinity chromatography column the equilibration buffer is 20 mM MES, with 150 mM NaCl, adjusted to pH 5.5, the elution buffer is 20 mM Tris/HCl, with 150 mM NaCl, adjusted to pH 8.8 and the elution is by applying 7.5 column volumes (CV) equilibration buffer, from 0 % to 100 % elution buffer in 30 CV, and thereafter 10 CV elution buffer.

9. The antibody according to any one of embodiments 1 to 8, wherein the anti-IGF-1R antibody does not bind to a human FcRn column due to single or multiple point mutations in the CH2 and/or CH3 domain of the anti-IGF-1R antibody.

10. The antibody according to any one of embodiments 1 to 9, wherein the anti-IGF-1R antibody binds to human FcRn with about the same or lesser affinity than an antibody comprising an Fc-region with at least one of the mutations L251D, L251S, M252T, I253A, S254W, S254R, H310A, H433A, N434L, H435A, Y436A (numbering according to Kabat EU index numbering system).

11. The antibody according to any one of embodiments 1 to 10, wherein the anti-IGF-1R antibody binds to human FcRn with about the same or lesser affinity than an antibody comprising an Fc-region with the mutations I253A/H310A/H435A or H310A/H433A/Y436A or L251D/L314D/L432D or combinations thereof (numbering according to Kabat EU index numbering system).

12. The antibody according to any one of embodiments 1 to 11, wherein the anti-IGF-1R antibody binds to human FcRn with about the same or lesser affinity than an antibody with a heavy chain amino acid sequence of SEQ ID NO: 01 or SEQ ID NO: 96 and a light chain amino acid sequence of SEQ ID NO: 03, or then an antibody with a heavy chain amino acid sequence of SEQ ID NO: 02 or SEQ ID NO: 96 and a light chain amino acid sequence of SEQ ID NO: 04.

13. The antibody according to any one of embodiments 1 to 12, wherein the anti-IGF-1R antibody has at least one of the mutations L251D, L251S, M252T, I253A, S254W, S254R, H310A, H433A, N434L, H435A, Y436A (numbering according to Kabat EU index numbering system) or a combination thereof.

14. The antibody according to any one of embodiments 1 to 13, wherein the anti-IGF-1R antibody has at least one of the mutations L251D, L251S, M252T, I253A, S254W, S254R, H310A, H433A, N434L, H435A, Y436A (numbering according to Kabat EU index numbering system) in the first Fc-region polypeptide and at least one of the mutations L251D, L251S, M252T, I253A, S254W, S254R, H310A, H433A, N434L, H435A, Y436A (numbering according to Kabat EU index numbering system) in the second Fc-region polypeptide.

15. The antibody according to any one of embodiments 1 to 14, wherein the anti-IGF-1R antibody has at least the mutations I253A/H310A/H435A or H310A/H433A/Y436A or L251D/L314D/L432D or combinations thereof (numbering according to Kabat EU index numbering system) in the first Fc-region polypeptide and at least one of the mutations L251D, M252T, I253A, S254W, S254R, H310A, H433A, N434G, N434L, H435A, Y436A (numbering according to Kabat EU index numbering system) in the second Fc-region polypeptide.

16. The antibody according to any one of embodiments 1 to 15, wherein the anti-IGF-1R antibody has at least the mutations I253A/H310A/H435A or H310A/H433A/Y436A or L251D/L314D/L432D or combinations thereof (numbering according to Kabat EU index numbering system) in both Fc-region polypeptides.

17. The antibody according to any one of embodiments 1 to 16, wherein the anti-IGF-1R antibody has

a) an antibody heavy chain comprising as HVRs amino acid residues 31-35 (HVR-H1), amino acid residues 50-66 (HVR-H2) and amino acid residues 99-107 (HVR-H3) of SEQ ID NO: 01, and
b) an antibody light chain comprising as HVRs amino acid residues 24-34 (HVR-L1), amino acid residues 50-56 (HVR-L2) and amino acid residues 89-98 (HVR-L3) of SEQ ID NO: 03.

18. The antibody according to any one of embodiments 1 to 16, wherein embodiment the anti-IGF-1R antibody has

a) an antibody heavy chain comprising as HVRs amino acid residues 31-35 (HVR-H1), amino acid residues 50-66 (HVR-H2) and amino acid residues 99-107 (HVR-H3) of SEQ ID NO: 02,

b) an antibody light chain comprising as HVRs amino acid residues 24-34 (HVR-L1), amino acid residues 50-56 (HVR-L2) and amino acid residues 89-98 (HVR-L3) of SEQ ID NO: 04.

19. The antibody according to any one of embodiments 1 to 16, wherein the anti-IGF-1R antibody has

a) an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3), or

b) an antibody heavy chain comprising as HVRs the amino acid sequence of SEQ ID NO: 64 (HVR-H1), the amino acid sequence of SEQ ID NO: 65 (HVR-H2), and the amino acid sequence of SEQ ID NO: 66 (HVR-H3), and an antibody light chain comprising as HVRs the amino acid sequence of SEQ ID NO: 67 (HVR-L1), the amino acid sequence of SEQ ID NO: 68 (HVR-L2), and the amino acid sequence of SEQ ID NO: 69 (HVR-L3), or

c) an antibody heavy chain comprising as HVRs the amino acid sequence of SEQ ID NO: 72 (HVR-H1), the amino acid sequence of SEQ ID NO: 73 (HVR-H2), and the amino acid sequence of SEQ ID NO: 74 (HVR-H3), and an antibody light chain comprising as HVRs the amino acid sequence of SEQ ID NO: 75 (HVR-L1), the amino acid sequence of SEQ ID NO: 76 (HVR-L2), and the amino acid sequence of SEQ ID NO: 77 (HVR-L3), or

d) an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3), or

e) an antibody heavy chain comprising as HVRs the amino acid sequence of SEQ ID NO: 88 (HVR-H1), the amino acid sequence of SEQ ID NO: 89 (HVR-H2), and the amino acid sequence of SEQ ID NO: 90 (HVR-H3), and an antibody light chain comprising as HVRs the amino acid sequence of SEQ ID NO: 91 (HVR-L1), the amino acid sequence of SEQ ID NO: 92 (HVR-L2), and the amino acid sequence of SEQ ID NO: 93 (HVR-L3), or

f) an antibody heavy chain comprising as HVRs amino acid residues 31-35 (HVR-H1), amino acid residues 50-66 (HVR-H2) and amino acid residues 99-107 (HVR-H3) of SEQ ID NO: 02 and an antibody light chain comprising as HVRs amino acid residues 24-34 (HVR-L1), amino acid residues 50-56 (HVR-L2) and amino acid residues 89-98 (HVR-L3) of SEQ ID NO: 04, or

g) an antibody heavy chain comprising as HVRs amino acid residues 31-35 (HVR-H1), amino acid residues 50-66 (HVR-H2) and amino acid residues 99-107 (HVR-H3) of SEQ ID NO: 01 and an antibody light chain comprising as HVRs amino acid residues 24-34 (HVR-L1), amino acid residues 50-56 (HVR-L2) and amino acid residues 89-98 (HVR-L3) of SEQ ID NO: 03.

20. The antibody according to any one of embodiments 1 to 19, wherein the anti-IGF-1R antibody comprises

a) a heavy chain variable domain VH of SEQ ID NO: 62 and a light chain variable domain VL of SEQ ID NO: 63, or
b) a heavy chain variable domain VH of SEQ ID NO: 70 and a light chain variable domain VL of SEQ ID NO: 71, or
c) a heavy chain variable domain VH of SEQ ID NO: 78 and a light chain variable domain VL of SEQ ID NO: 79, or
d) a heavy chain variable domain VH of SEQ ID NO: 86 and a light chain variable domain VL of SEQ ID NO: 87, or
e) a heavy chain variable domain VH of SEQ ID NO: 94 and a light chain variable domain VL of SEQ ID NO: 95, or
f) a heavy chain variable domain VH of SEQ ID NO: 05 and a light chain variable domain VL of SEQ ID NO: 07, or

g) a heavy chain variable domain VH of SEQ ID NO: 06 and a light chain variable domain of SEQ ID NO: 08.

21. The antibody according to any one of embodiments 1 to 20, wherein the anti-IGF-1R antibody has an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3).

22. The antibody according to any one of embodiments 1 to 20, wherein the anti-IGF-1R antibody has an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3).

23. The antibody according to any one of embodiments 1 to 20, wherein the anti-IGF-1R antibody comprises a heavy chain variable domain VH of SEQ ID NO: 62 and a light chain variable domain VL of SEQ ID NO: 63.

24. The antibody according to any one of embodiments 1 to 20, wherein the anti-IGF-1R antibody comprises a heavy chain variable domain VH of SEQ ID NO: 86 and a light chain variable domain VL of SEQ ID NO: 87.

25. The antibody according to any one of embodiments 1 to 24, wherein the anti-IGF-1R has an Fc-region comprising a first Fc-region polypeptide and a second Fc-region polypeptide.

26. The antibody according to embodiment 25, wherein the anti-IGF-1R antibody has a heterodimeric Fc-region.

27. The antibody according to any one of embodiments 1 to 26, wherein

i) the first Fc-region polypeptide is selected from the group comprising

- human IgG1 Fc-region polypeptide,
- human IgG2 Fc-region polypeptide,
- human IgG3 Fc-region polypeptide,
- human IgG4 Fc-region polypeptide,
- human IgG1 Fc-region polypeptide with the mutations L234A, L235A,
- human IgG1 Fc-region polypeptide with the mutations Y349C, T366S, L368A, Y407V,
- human IgG1 Fc-region polypeptide with the mutations S354C, T366S, L368A, Y407V,
- human IgG1 Fc-region polypeptide with the mutations L234A, L235A, Y349C, T366S, L368A, Y407V,
- human IgG1 Fc-region polypeptide with the mutations L234A, L235A, S354C, T366S, L368A, Y407V,
- human IgG1 Fc-region polypeptide with the mutations P329G,
- human IgG1 Fc-region polypeptide with the mutations L234A, L235A, P329G,
- human IgG1 Fc-region polypeptide with the mutations P329G, Y349C, T366S, L368A, Y407V,
- human IgG1 Fc-region polypeptide with the mutations P329G, S354C, T366S, L368A, Y407V,
- human IgG1 Fc-region polypeptide with the mutations L234A, L235A, P329G, Y349C, T366S, L368A, Y407V,
- human IgG1 Fc-region polypeptide with the mutations L234A, L235A, P329G, S354C, T366S, L368A, Y407V,
- human IgG4 Fc-region polypeptide with the mutations S228P, L235E,
- human IgG4 Fc-region polypeptide with the mutations S228P, L235E, P329G,
- human IgG4 Fc-region polypeptide with the mutations Y349C, T366S, L368A, Y407V,
- human IgG4 Fc-region polypeptide with the mutations S354C, T366S, L368A, Y407V,
- human IgG4 Fc-region polypeptide with the mutations S228P, L235E, Y349C, T366S, L368A, Y407V,
- human IgG4 Fc-region polypeptide with the mutations S228P, L235E, S354C, T366S, L368A, Y407V,
- human IgG4 Fc-region polypeptide with the mutations P329G,
- human IgG4 Fc-region polypeptide with the mutations P329G, Y349C, T366S, L368A, Y407V,
- human IgG4 Fc-region polypeptide with the mutations P329G, S354C, T366S, L368A, Y407V,
- human IgG4 Fc-region polypeptide with the mutations S228P, L235E, P329G, Y349C, T366S, L368A, Y407V,
- human IgG4 Fc-region polypeptide with the mutations S228P, L235E, P329G, S354C, T366S, L368A, Y407V,
- human IgG1, IgG2 or IgG4 with the mutations K392D, and
- human IgG3 with the mutation N392D,

and
ii) the second Fc-region polypeptide is selected from the group comprising

- human IgG1 Fc-region polypeptide,
- human IgG2 Fc-region polypeptide,
- human IgG3 Fc-region polypeptide,
- human IgG4 Fc-region polypeptide,
- human IgG1 Fc-region polypeptide with the mutations L234A, L235A,
- human IgG1 Fc-region polypeptide with the mutations S354C, T366W,
- human IgG1 Fc-region polypeptide with the mutations Y349C, T366W,
- human IgG1 Fc-region polypeptide with the mutations L234A, L235A, S354C, T366W,
- human IgG1 Fc-region polypeptide with the mutations L234A, L235A, Y349C, T366W,
- human IgG1 Fc-region polypeptide with the mutations P329G,
- human IgG1 Fc-region polypeptide with the mutations L234A, L235A, P329G,
- human IgG1 Fc-region polypeptide with the mutations P329G, S354C, T366W,
- human IgG1 Fc-region polypeptide with the mutations P329G, Y349C, T366W,
- human IgG1 Fc-region polypeptide with the mutations L234A, L235A, P329G, S354C, T366W,
- human IgG1 Fc-region polypeptide with the mutations L234A, L235A, P329G, Y349C, T366W,
- human IgG4 Fc-region polypeptide with the mutations S228P, L235E,
- human IgG4 Fc-region polypeptide with the mutations S228P, L235E, P329G,
- human IgG4 Fc-region polypeptide with the mutations S354C, T366W,
- human IgG4 Fc-region polypeptide with the mutations Y349C, T366W,
- human IgG4 Fc-region polypeptide with the mutations S228P, L235E, S354C, T366W,
- human IgG4 Fc-region polypeptide with the mutations S228P, L235E, Y349C, T366W,
- human IgG4 Fc-region polypeptide with the mutations P329G,
- human IgG4 Fc-region polypeptide with the mutations P329G, S354C, T366W,
- human IgG4 Fc-region polypeptide with the mutations P329G, Y349C, T366W,
- human IgG4 Fc-region polypeptide with the mutations S228P, L235E, P329G, S354C, T366W,
- human IgG4 Fc-region polypeptide with the mutations S228P, L235E, P329G, Y349C, T366W,
- human IgG1 with the mutations D399K, D356K, and/or E357K, and
- human IgG2, IgG3 or IgG4 with the mutations D399K, E356K, and/or E357K.

28. The antibody according to any one of embodiments 1 to 27, wherein

i) the first Fc-region polypeptide has an amino acid sequence selected from the group comprising SEQ ID NO: 14, 15, 16, 17, 18, 19, 21, 23, 24, 25, 27, 29, 30, 32, 34, 35, 36, and 38, and

ii) the second Fc-region polypeptide has an amino acid sequence selected from the group comprising SEQ ID NO: 14, 15, 16, 17, 18, 20, 22, 23, 24, 26, 28, 29, 30, 31, 33, 35, 37, and 39.

29. The antibody according to any one of embodiments 1 to 27, wherein

i) the Fc-region polypeptide is a human IgG1 Fc-region polypeptide and the second Fc-region polypeptide is a human IgG1 Fc-region polypeptide, or

ii) the first Fc-region polypeptide is a human IgG1 Fc-region polypeptide with the mutations L234A, L235A and the second Fc-region polypeptide is a human IgG1 Fc-region polypeptide with the mutations L234A, L235A, or

iii) the first Fc-region polypeptide is a human IgG1 Fc-region polypeptide with the mutations L234A, L235A, P329G and the second Fc-region polypeptide is a human IgG1 Fc-region polypeptide with the mutations L234A, L235A, P329G, or

iv) the first Fc-region polypeptide is a human IgG1 Fc-region polypeptide with the mutations L234A, L235A, S354C, T366W and the second Fc-region polypeptide is a human IgG1 Fc-region polypeptide with the mutations L234A, L235A, Y349C, T366S, L368A, Y407V, or

v) the first Fc-region polypeptide is a human IgG1 Fc-region polypeptide with the mutations L234A, L235A, P329G, S354C, T366W and the second Fc-region polypeptide is a human IgG1 Fc-region polypeptide with the mutations L234A, L235A, P329G, Y349C, T366S, L368A, Y407V, or

vi) the first Fc-region polypeptide is a human IgG4 Fc-region polypeptide and the second Fc-region polypeptide is a human IgG4 Fc-region polypeptide, or

vii) the first Fc-region polypeptide is a human IgG4 Fc-region polypeptide with the mutations S228P, L235E and the second Fc-region polypeptide is a human IgG4 Fc-region polypeptide with the mutations S228P, L235E, or

viii) the first Fc-region polypeptide is a human IgG4 Fc-region polypeptide with the mutations S228P, L235E, P329G and the second Fc-region polypeptide is a human IgG4 Fc-region polypeptide with the mutations S228P, L235E, P329G, or

ix) the first Fc-region polypeptide is a human IgG4 Fc-region polypeptide with the mutations S228P, L235E, S354C, T366W and the second Fc-region polypeptide is a human IgG4 Fc-region polypeptide with the mutations S228P, L235E, Y349C, T366S, L368A, Y407V, or

x) the first Fc-region polypeptide is a human IgG4 Fc-region polypeptide with the mutations S228P, L235E, P329G, S354C, T366W and the second Fc-region polypeptide is a human IgG4 Fc-region polypeptide with the mutations S228P, L235E, P329G, Y349C, T366S, L368A, Y407V.

30. The antibody according to any one of embodiments 1 to 29, wherein

i) the first Fc-region polypeptide has the amino acid sequence of SEQ ID NO: 14 and the second Fc-region polypeptide has the amino acid sequence of SEQ ID NO: 14, or

ii) the first Fc-region polypeptide has the amino acid sequence of SEQ ID NO: 18 and the second Fc-region polypeptide has the amino acid sequence of SEQ ID NO: 18, or

iii) the first Fc-region polypeptide has the amino acid sequence of SEQ ID NO: 24 and the second Fc-region polypeptide has the amino acid sequence of SEQ ID NO: 24, or

iv) the first Fc-region polypeptide has the amino acid sequence of SEQ ID NO: 22 and the second Fc-region polypeptide has the amino acid sequence of SEQ ID NO: 21, or

v) the first Fc-region polypeptide has the amino acid sequence of SEQ ID NO: 28 and the second Fc-region polypeptide has the amino acid sequence of SEQ ID NO: 27, or

vi) the first Fc-region polypeptide has the amino acid sequence of SEQ ID NO: 17 and the second Fc-region polypeptide has the amino acid sequence of SEQ ID NO: 17, or

vii) the first Fc-region polypeptide has the amino acid sequence of SEQ ID NO: 29 and the second Fc-region polypeptide has the amino acid sequence of SEQ ID NO: 29, or

viii) the first Fc-region polypeptide has the amino acid sequence of SEQ ID NO: 30 and the second Fc-region polypeptide has the amino acid sequence of SEQ ID NO: 30, or

ix) the first Fc-region polypeptide is has the amino acid sequence of SEQ ID NO: 33 and the second Fc-region polypeptide has the amino acid sequence of SEQ ID NO: 34, or

x) the first Fc-region polypeptide has the amino acid sequence of SEQ ID NO: 39 and the second Fc-region polypeptide has the amino acid sequence of SEQ ID NO: 38.

31. The antibody according to any one of embodiments 1 to 30, wherein the antibody is an isolated antibody.

32. The antibody according to any one of embodiments 1 to 31, wherein the antibody is a full-length antibody.

33. The antibody according to any one of embodiments 1 to 32, wherein the antibody is a monoclonal antibody.

34. The antibody according to any one of embodiments 1 to 33, wherein the antibody is a human, humanized or chimeric antibody.

35. The antibody according to any one of embodiments 1 to 34, wherein the antibody comprises a VH sequence of SEQ ID NO: 05 or SEQ ID NO: 06.

36. The antibody according to any one of embodiments 1 to 34, wherein the antibody comprises a VL sequence of SEQ ID NO: 07 or SEQ ID NO: 08.

37. The antibody according to any one of embodiments 1 to 34, wherein the antibody comprises a VH sequence of SEQ ID NO: 05 and a VL sequence of SEQ ID NO: 07.

38. The antibody according to any one of embodiments 1 to 34, wherein the antibody comprises a VH sequence of SEQ ID NO: 06 and a VL sequence of SEQ ID NO: 08.

39. The antibody according to any one of embodiments 1 to 34, wherein the antibody comprises a VH sequence of SEQ ID NO: 62 and a VL sequence of SEQ ID NO: 63.

40. The antibody according to any one of embodiments 1 to 34, wherein the antibody comprises a VH sequence of SEQ ID NO: 86 and a VL sequence of SEQ ID NO: 87.

41. The antibody according to any one of embodiments 1 to 40, wherein the antibody comprises a first Fc-region polypeptide and a second Fc-region polypeptide wherein

i) the first and the second Fc-region polypeptide further comprise the mutation Y436A, or

ii) the first and the second Fc-region polypeptide further comprise the mutations 1253A, H310A and H435A, or

iii) the first and the second Fc-region polypeptide further comprise the mutations H310A, H433A and Y436A, or

iv) the first and the second Fc-region polypeptide further comprise the mutations L251D, L314D and L432D, or

v) the first Fc-region polypeptide further comprises the mutation Y436A and the second Fc-region polypeptide further comprises

    a) the mutations 1253A, H310A and H435A, or
    b) the mutations H310A, H433A and Y436A, or
    c) the mutations L251D, L314D and L432D,

or

vi) the first Fc-region polypeptide further comprises the mutations I253A, H310A and H435A and the second Fc-region polypeptide further comprises

    a) the mutations H310A, H433A and Y436A, or
    b) the mutations L251D, L314D and L432D,

or

vii) the first Fc-region polypeptide further comprises the mutations H310A, H433A and Y436A and the second Fc-region polypeptide further comprises

    a) the mutations L251D, L314D and L432D.

42. The antibody according to any one of embodiments 1 to 40, wherein the antibody comprises an Fc-region comprising a first and a second Fc-region polypeptide both of human IgG1 or human IgG4 subclass (derived from human origin) which comprise one or two of the mutations selected from i) the group I253A, H310A and H435A, or ii) the group H310A, H433A and Y436A, or iii) the group L251D, L314D and L432D (numbering according to Kabat EU index numbering system) in the first Fc-region polypeptide and one or two of the mutations selected from the group comprising the mutations L251D, I253A, H310A, L314D, L432D, H433A, H435A and Y436A (numbering according to Kabat EU index numbering system) in the second Fc-region polypeptide so that all of the mutations in the first and the second Fc-region polypeptide when taken together result in that the mutations i) I253A, H310A and H435A, or ii) H310A, H433A and Y436A, or iii) L251D, L314D and L432D are comprised in the Fc-region.

43. The antibody according to any one of embodiments 1 to 40, wherein the antibody comprises an Fc-region comprising a first and a second Fc-region polypeptide both of human IgG1 or human IgG4 subclass (i.e. derived from human origin), which both comprise the mutations I253A/H310A/H435A or H310A/H433A/Y436A or L251D/L314D/L432D or combinations thereof in the Fc-region (numbering according to Kabat EU index numbering system), whereby either all mutations are in the first or the second Fc-region polypeptide, or one or two mutations are in the first Fc-region polypeptide and one or two mutations are in the second Fc-region polypeptide so that all of the mutations in the first and the second Fc-region polypeptide when taken together result in that the mutations i) I253A, H310A and H435A, or ii) H310A, H433A and Y436A, or iii) L251D, L314D and L432D are comprised in the Fc-region.

44. The antibody according to any one of embodiments 1 to 40, wherein the antibody comprises an Fc-region comprising a first and a second Fc-region polypeptide both of human IgG1 or human IgG4 subclass (i.e. derived from human origin), which comprise the mutations I253A/H310A/H435A or H310A/H433A/Y436A or L251D/L314D/L432D in the first as well as in the second Fc-region polypeptide (numbering according to Kabat EU index numbering system), or comprises the combinations of the mutations I253A/H310A/H435A in the first Fc-region polypeptide and the combination of the mutations H310A/H433A/Y436A in the second Fc-region polypeptide (numbering according to Kabat EU index numbering system).

45. The antibody according to any one of embodiments 1 to 44, wherein i) the antibody does not specifically bind to the human FcRn and does specifically bind to Staphylococcal protein A, or ii) the antibody does not specifically bind to the human FcRn and does not specifically bind to Staphylococcal protein A.

46. The antibody according to any one of embodiments 1 to 45, wherein the antibody

- shows a lower serum concentration compared to corresponding bispecific antibody without the mutations in the Fc-region polypeptides (96 hours after intravitreal application in mice, which are mouse FcRn deficient, but hemizygous transgenic for human FcRn) (determined in assays as described in Example 4), and/or

- shows a similar (factor 0.8 to 1.2) concentration in whole right eye lysates compared to corresponding bispecific antibody without the mutations in the Fc-region polypeptides (in mice, which are mouse FcRn deficient, but hemizygous transgenic for human FcRn, 96 hours after intravitreal application in the right eye) (determined in assays as described in Example 4), and/or

- has abolished binding to the human FcRn, and/or

- has no binding to Staphylococcal protein A (determined by SPR), and/or

- has maintained binding to Staphylococcal protein A (determined by SPR).

47. The antibody according to any one of embodiments 1 to 46, wherein the antibody comprises a heavy chain variable domain (VH) sequence having at least 90 %, 91 %, 92 %, 93 %, 94 %, 95 %, 96 %, 97 %, 98 %, 99 %, or 100 % sequence identity to the amino acid sequence of SEQ ID NO: 05 or 62 or 86.

48. The antibody according to any one of embodiments 1 to 47, wherein the antibody comprises a light chain variable domain (VL) having at least 90 %, 91 %, 92 %, 93 %, 94 %, 95 %, 96 %, 97 %, 98 %, 99 %, or 100 % sequence identity to the amino acid sequence of SEQ ID NO: 07 or 63 or 87.

49. The use of an anti-IGF-1R antibody according to any one of embodiments 1 to 48 for inhibition of IGF-1R in the eye.

50. The use of an anti-IGF-1R antibody according to any one of embodiments 1 to 48 for the treatment of vascular eye diseases.

51. A method of treatment of a patient suffering from ocular vascular diseases by administering an anti-IGF-1R antibody according to any one of embodiments 1 to 48 to a patient in the need of such treatment.

52. An anti-IGF-1R antibody according to any one of embodiments 1 to 48 for intravitreal application.

53. An anti-IGF-1R antibody according to any one of embodiments 1 to 48 for the treatment of vascular eye diseases.

54. A pharmaceutical formulation comprising an anti-IGF-1R antibody according to any one of embodiments 1 to 48 and optionally a pharmaceutically acceptable carrier.

55. An anti-IGF-1R antibody according to any one of embodiments 1 to 48 for use as a medicament.

56. An anti-IGF-1R antibody according to any one of embodiments 1 to 48 for use in treating vascular eye diseases.

57. An anti-IGF-1R antibody according to any one of embodiments 1 to 48 for use in a method of treatment.

58. An anti-IGF-1R antibody according to any one of embodiments 1 to 48 for use in a method of treating an individual having a vascular eye disease comprising administering to the individual an effective amount of the anti-IGF-1R antibody according to any one of embodiments 1 to 48.

59. The antibody according to embodiment 58, wherein the method further comprises administering to the individual an effective amount of at least one additional therapeutic agent.

60. An anti-IGF-1R antibody according to any one of embodiments 1 to 48 for use in inhibiting IGF-1R in the eye.

61. An anti-IGF-1R antibody according to any one of embodiments 1 to 48 for use in a method of inhibiting angiogenesis in the eye in an individual comprising administering to the individual an effective amount of the anti-IGF-1R antibody according to any one of embodiments 1 to 48 to inhibit IGF-1R signaling.

62. Use of an anti-IGF-1R antibody according to any one of embodiments 1 to 48 in the manufacture of a medicament.

63. The use according to embodiment 62, wherein the medicament is for treatment of a vascular eye disease.

64. The use according to any one of embodiments 62 to 63, wherein the medicament is for use in a method of treating a vascular eye disease comprising administering to an individual having a vascular eye disease an effective amount of the medicament.

65. The use according to embodiment 64, wherein the method further comprises administering to the individual an effective amount of at least one additional therapeutic agent.

66. The use according to any one of embodiments 62 to 65, wherein the medicament is for inhibiting angiogenesis.

67. The use according to any one of embodiments 62 to 66, wherein the medicament is for use in a method of inhibiting angiogenesis in an individual comprising administering to the individual an amount effective of the medicament to inhibit IGF-1R mediated signaling.

68. A method for treating a vascular eye disease.

69. The method according to embodiment 68, wherein the method comprises administering to an individual having such a vascular eye disease an effective amount of an anti-IGF-1R antibody according to any one of embodiments 1 to 48.

70. The method according to embodiment 69, wherein the method further comprises administering to the individual an effective amount of at least one additional therapeutic agent.

71. A method for inhibiting angiogenesis in the eye in an individual.

72. The method according to embodiment 71, wherein the method comprises administering to the individual an effective amount of an anti-IGF-1R antibody according to any one of embodiments 1 to 48 to inhibit IGF-1R mediated signaling.

73. A pharmaceutical formulations comprising an anti-IGF-1R antibody according to any one of embodiments 1 to 48.

74. The pharmaceutical formulation according to embodiment 73, wherein the formulation is for use in any of the embodiments 49 to 72.

75. A pharmaceutical formulation comprising an anti-IGF-1R antibody according to any one of embodiments 1 to 48 and a pharmaceutically acceptable carrier.

76. A pharmaceutical formulation comprising an anti-IGF-1R antibody according to any one of embodiments 1 to 48 and at least one additional therapeutic agent.

77. The antibody or the use or the method according to any one of embodiments 49 to 72, wherein the administration is an intravitreal administration.

## V. EXAMPLES

The following are examples of methods and compositions of the invention. It is understood that various other embodiments may be practiced, given the general description provided above.

### Antibodies and their respective sequences

| Description | Sequences |
|---|---|
| <IGF-1R> IgG1 wt | SEQ ID NO: 42<br>SEQ ID NO: 43 |
| <IGF-1R> IgG1 with I253A, H310A, H435A | SEQ ID NO: 42<br>SEQ ID NO: 44 |
| <IGF-1R> IgG1 with M252Y, S254T, T256E | SEQ ID NO: 42<br>SEQ ID NO: 45 |
| <IgF-1R> IgG1 wt, KiH | SEQ ID NO: 42<br>SEQ ID NO: 46<br>SEQ ID NO: 47 |
| <IgF-1R> IgG1 knob wt, hole I253A, H310A, H435A | SEQ ID NO: 42<br>SEQ ID NO: 48<br>SEQ ID NO: 49 |
| <IGF-1R> IgG1 knob wt, hole H310A, H433A, Y436A | SEQ ID NO: 42<br>SEQ ID NO: 50<br>SEQ ID NO: 51 |
| <IGF-1R> IgG1 knob wt, hole M252Y, S254T, T256E | SEQ ID NO: 42<br>SEQ ID NO: 52<br>SEQ ID NO: 53 |
| <IGF-1R> IgG1 knob wt, hole L251D, L314D, L432D | SEQ ID NO: 42<br>SEQ ID NO: 54<br>SEQ ID NO: 55 |

(continued)

| Description | Sequences |
|---|---|
| <IGF-1R> IgG1 with H310A, H433A, Y436A | SEQ ID NO: 42<br>SEQ ID NO: 96 |

**Methods**

Electrospray ionization mass spectrometry (ESI-MS)

Protein aliquots (50 $\mu$g) were deglycosylated by adding 0.5 $\mu$L N-Glycanase plus (Roche) and sodium phosphate buffer (0.1 M, pH 7.1) to obtain a final sample volume of 115 $\mu$L. The mixture was incubated at 37 °C for 18 h. Afterwards for reduction and denaturing 60 $\mu$L 0.5 M TCEP (Pierce) in 4 M guanidine * HCl (Pierce) and 50 $\mu$L 8 M guanidine * HCl were added. The mixture was incubated at 37 °C for 30 min. Samples were desalted by size exclusion chromatography (Sepharose G-25, isocratic, 40 % acetonitrile with 2 % formic acid). ESI mass spectra (+ve) were recorded on a Q-TOF instrument (maXis, Bruker) equipped with a nano ESI source (TriVersa NanoMate, Advion). MS parameter settings were as follows: Transfer: Funnel RF, 400 Vpp; ISCID Energy, 0 eV; Multipole RF, 400 Vpp; Quadrupole: Ion Energy, 4.0 eV; Low Mass, 600 m/z; Source: Dry Gas, 8 L/min; Dry Gas Temperature, 160 °C; Collision Cell: Collision Energy, 10 eV; Collision RF: 2000 Vpp; Ion Cooler: Ion Cooler RF, 300 Vpp; Transfer Time: 120 $\mu$s; Pre Puls Storage, 10 $\mu$s; scan range m/z 600 to 2000. For data evaluation in-house developed software (MassAnalyzer) was used.

FcRn surface plasmon resonance (SPR) analysis

The binding properties of wild-type antibody and the mutants to FcRn were analyzed by surface plasmon resonance (SPR) technology using a BIAcore T100 instrument (BIAcore AB, Uppsala, Sweden). This system is well established for the study of molecular interactions. It allows a continuous real-time monitoring of ligand/analyte bindings and, thus, the determination of kinetic parameters in various assay settings. SPR-technology is based on the measurement of the refractive index close to the surface of a gold coated biosensor chip. Changes in the refractive index indicate mass changes on the surface caused by the interaction of immobilized ligand with analyte injected in solution. If molecules bind to an immobilized ligand on the surface the mass increases, in case of dissociation the mass decreases. In the current assay, the FcRn receptor was immobilized onto a BIAcore CM5-biosensor chip (GE Healthcare Bioscience, Uppsala, Sweden) via amine coupling to a level of 400 Response units (RU). The assay was carried out at room temperature with PBS, 0.05 % Tween-20TM pH 6.0 (GE Healthcare Bioscience) as running and dilution buffer. 200 nM of antibody samples were injected at a flow rate of 50 $\mu$L/min at room temperature. Association time was 180 seconds, dissociation phase took 360 seconds. Regeneration of the chip surface was reached by a short injection of HBS-P, pH 8.0. Evaluation of SPR-data was performed by comparison of the biological response signal height at 180 seconds after injection and at 300 seconds after injection. The corresponding parameters are the RU max level (180 seconds after injection) and late stability (300 seconds after end of injection).

Protein A surface plasmon resonance (SPR) analysis

The assay is based on surface plasmon resonance spectroscopy. Protein A is immobilized onto the surface of a SPR biosensor. By injecting the sample into the flow cells of the SPR spectrometer it forms a complex with the immobilized protein A resulting in an increasing mass on the sensor chip surface, and therefore to a higher response (as 1 RU is defined as 1 pg/mm$^2$). Afterwards the sensor chip is regenerated by dissolving the sample-protein A-complex. The gained responses are then evaluated for the signal high in response units (RU) and the dissociation behavior.

Around 3,500 response units (RU) of protein A (20 $\mu$g/mL) were coupled onto a CM5 chip (GE Healthcare) at pH 4.0 by using the amine coupling kit of GE Healthcare.

The sample and system buffer was HBS-P+ (0.01 M HEPES, 0.15 M NaCl, 0.005 % Surfactant P20 Sterile-filtered, pH 7.4). Flow cell temperature was set to 25 °C and sample compartment temperature to 12 °C. The system was primed with running buffer. Then, a 5 nM solutions of the sample constructs were injected for 120 seconds with a flow rate of 30 $\mu$L/min, followed by a 300 seconds dissociation phase. Then the sensor chip surface was regenerated by two 30 seconds long injections of Glycine-HCl pH 1.5 at a flow rate of 30 $\mu$L/min. Each sample was measured as a triplicate.

### Example 1

**Preparation of FcRn affinity column**

Expression of FcRn in HEK293 cells

FcRn was transiently expressed by transfection of HEK293 cells with two plasmids containing the coding sequence of FcRn and of beta-2-microglobulin. The transfected cells were cultured in shaker flasks at 36.5 °C, 120 rpm (shaker amplitude 5 cm), 80 % humidity and 7 % $CO_2$. The cells were diluted every 2 - 3 days to a density of 3 to $4*10^5$ cells/mL.

For transient expression, a 14 L stainless steel bioreactor was started with a culture volume of 8 L at 36.5 °C, pH 7.0 $\pm$ 0.2, $pO_2$ 35 % (gassing with $N_2$ and air, total gas flow 200 mL min$^{-1}$) and a stirrer speed of 100 - 400 rpm. When the cell density reached $20*10^5$ cells/mL, 10 mg plasmid DNA (equimolar amounts of both plasmids) was diluted in 400 ml Opti-MEM (Invitrogen). 20 mL of 293fectin (Invitrogen) was added to this mixture, which was then incubated for 15 minutes at room temperature and subsequently transferred into the fermenter. From the next day on, the cells were supplied with nutrients in continuous mode: a feed solution was added at a rate of 500 ml per day and glucose as needed to keep the level above 2 g/L. The supernatant was harvested 7 days after transfection using a swing head centrifuge with 1 L buckets: 4,000 rpm for 90 minutes. The supernatant (13 L) was cleared by a Sartobran P filter (0.45 $\mu$m + 0.2 $\mu$m, Sartorius) and the FcRn beta-2-microglobulin complex was purified therefrom.

Biotinylation of neonatal Fc receptor

A soluble extracellular domain of FcRn with His-Avi Tag that has been co-expressed with $\beta_2$-microglobulin in HEK293 cells was biotinylated after purification as follows:

Between 1.2 mg and 12 mg FcRn/$\beta_2$-microglobulin in 5 ml 20 mM sodium citrate buffer, pH 5.5 containing 150 mM KCl, 250 $\mu$l PBS and 1 tablet Complete protease inhibitor (Roche Diagnostics GmbH, Mannheim, Germany) were biotinylated using the biotinylation kit from Avidity according to the manufacturer instructions (Bulk BIRA). Biotinylation reaction was done at room temperature overnight. The modified protein was dialyzed against 20 mM sodium phosphate buffer comprising 150 mM NaCl, pH 7.5 at 4 °C over night to remove excess of biotin.

Coupling to streptavidin sepharose

One gram streptavidin sepharose (GE Healthcare) was added to the biotinylated and dialyzed receptor (between 1.2 and 12 mg FcRn/$\beta$2-microglobulin, for standard analytical application 3 mg were chosen) and incubated for two hours with shaking. The receptor derivatized sepharose was filled in a 1 ml XK column (GE Healthcare).

### Example 2

**Chromatography using the FcRn affinity column**

The receptor derivatized sepharose was filled in a 1 ml XK column (GE Healthcare) and the FcRn column then was equilibrated with 20 mM 2-(N-morpholine)-ethanesulfonic acid (MES) buffer containing 150 mM NaCl, pH 5.5.

Conditions:

| | |
|---|---|
| column dimensions: | 50 mm x 5 mm |
| bed height: | 5 cm |
| loading: | 50 $\mu$g sample |
| equilibration buffer: | 20 mM MES, with 150 mM NaCl, adjusted to pH 5.5 |
| elution buffer: | 20 mM Tris/HCl, with 150 mM NaCl, adjusted to pH 8.8 |
| elution: | 7.5 CV equilibration buffer, in 30 CV to 100 % elution buffer, 10 CV elution buffer |

Antibody samples containing 50 to 100 $\mu$g of protein were adjusted to pH 5.5 and applied to the FcRn column using ÄKTA explorer 10 XT or Dionex Summit (Dionex, Idstein, Germany). The column with 5 cm bed height was then washed with 5-10 column volumes of equilibration buffer 20 mM MES, 150 mM NaCl, pH 5.5. The affinity-bound antibodies were eluted with a pH gradient to 20 mM Tris/HCl, 150 mM NaCl, pH 8.8, in 30 column volumes. For complete elution of modified antibodies, the pH was increased in the gradient up to pH 8.8. The experiments were carried out at room temperature. The elution profile was obtained by continuous measurement of the absorbance at 280 nm. The time taken for an analyte peak, X, to reach the detector after sample injection was called the retention time.

### Example 3

**Expression of anti-IGF-1R antibodies**

Cell lines

The parental cell line used for the generation of a cell line for recombinant IgG expression is a Chinese hamster ovarian (CHO) cell line, CHO-DG44 (Flintoff, W.F. et al., Somat. Cell Genet. 2 (1976) 245-261; Flintoff et al., Mol. Cell. Biol. 2 (1982) 275-285; Urlaub, G. et al., Cell 33 (1983) 405-412; Urlaub, G. et al., Somat. Cell Mol. Genet. 12 (1986) 555-566). CHO-DG44 cells have lost both endogenous loci for the enzyme Dihydrofolate Reductase (DHFR).
CHO-DG44 cells were grown in MEM alpha Minus Medium (Gibco No. 22561), 10 % dialyzed FCS (Gibco No. 26400-044) and 2 mmol/L L-Glutamine, 100 $\mu$M Hypoxanthine, 16 $\mu$M Thymidine (HT supplement).

Plasmids

The expression system comprised the CMV promoter and is described in the following Table. As antibody an antibody against IGF-1R (WO 2005/005635; AK18 or AK22) was used.

**Table.**

| bp | plasmid element / DNA segment |
|---|---|
| 1-26 | Unique restriction sites: SgrAI, Sse83871 |
| 27-614 | Human cytomegalovirus (HCMV) promoter (CMV-Prom) including human CMV IE promoter including synthetic 5'-UTR |
| 615-641 | Linker |
| 642-780 | Murine Ig heavy chain leader sequence (L1, signal sequence intron, L2) |
| 642-686 | L1 |
| 687-768 | Signal intron (SS intron) |
| 769-780 | L2 |
| 781-1105 | Variable $\kappa$-light chain domain of IGF-1R antibody (AK18) |
| 1106-1140 | Linker |
| 1141-3134 | Human/mouse $\kappa$-light chain hybrid intron 2 |
| 2433-2913 | $\kappa$-enhancer fragment |
| 3135-3475 | Linker |
| 3476-3795 | $\kappa$-Light chain constant region (C-kappa) |
| 3796-4098 | Human Ig $\kappa$-light chain polyadenylation sequence (C-kappa pA) |
| 4099-4137 | Linker |
| 4138-5800 | Hygromycin resistance |
| 4138-4485 | SV40 promoter (SV40 Prom) incl. 72bp repeat, TATA, SV40 origin |
| 4486-4502 | Linker |
| 5403-5528 | Hygromycin-B-phosphotransferase (Hyg) |
| 5529-5535 | Linker |
| 5536-5795 | SV40 polyadenylation signal (SV40 pA) |
| 5796-5800 | Linker |
| 5801-6944 | Murine dihydrofolate reductase (DHFR) |
| 5801-6088 | SV40 promoter (SV40 Prom) incl. 72bp repeat shortened, SV40 origin |
| 6089-6105 | Linker |
| 6106-6672 | Murine DHFR gene (murine DHFR) |
| 6673-6679 | Linker |

(continued)

| bp | plasmid element / DNA segment |
|---|---|
| 6680-6944 | SV40 polyadenylation signal (SV40 pA) |
| 6945-7181 | Linker |
| 7182-8941 | Bacterial origin of replication and selective marker derived from plasmid pUC18 |
| 7182-7792 | Origin of replication ("pUC origin") |
| 7793-7939 | Linker |
| 7940-8847 | $\beta$-Lactamase gene (Ap(r)) |
| 8848-8941 | Linker |
| 8942-9529 | Human cytomegalovirus (HCMV) promoter (CMV-Prom) including human CMV IE promoter including synthetic 5'-UTR |
| 9530-9556 | Linker |
| 9557-9696 | Murine Ig heavy chain leader sequence (L1, signal sequence intron, L2) |
| 9557-9602 | L1 |
| 9603-9685 | Signal intron (SS intron) |
| 9686-9696 | L2 |
| 9697-10051 | Variable IgG1 heavy chain domain of IGF-1R antibody (AK18) |
| 10052-10085 | Linker |
| 10086-11682 | Human/mouse heavy chain hybrid intron 2 including the part of the mouse Ig heavy chain J-segment region including the Ig heavy chain enhancer element (part $JH_3$, $JH_4$) Mouse Ig heavy chain enhancer element |
| 11683-11909 | Linker |
| 11910-13504 | Human IgG1 heavy chain constant region ($CH_1$-Hinge-$CH_2$-$CH_3$) |
| 11910-12203 | CH1 |
| 12594-12638 | Hinge |
| 12757-13086 | CH2 |
| 13184-13504 | CH3 (alternative splice site deleted) |
| 13505-13967 | Human IgG1 heavy chain polyadenylation sequence (IgG1 pA) |
| 13968-13970 | SgrAI-Linker |

Transient transfections in HEK293 system

The antibodies were generated by transient transfection with the respective plasmids (e.g. encoding the heavy and/or modified heavy chain, as well as the corresponding light chain) using the HEK293 system (Invitrogen) according to the manufacturer's instruction. Briefly, HEK293 cells (Invitrogen) growing in suspension either in a shake flask or in a stirred fermenter in serum-free FreeStyle™ 293 expression medium (Invitrogen) were transfected with a mix of the 2 or 3 expression plasmids and 293fectin™ or fectin (Invitrogen). For 2 L shake flask (Corning) HEK293 cells were seeded at a density of $1*10^6$ cells/mL in 600 mL and incubated at 120 rpm, 8 % $CO_2$. The day after the cells were transfected at a cell density of approx. $1.5*10^6$ cells/mL with approx. 42 mL mix of A) 20 mL Opti-MEM (Invitrogen) with 600 $\mu$g total plasmid DNA (1 $\mu$g/mL) encoding the heavy or modified heavy chain, respectively and the corresponding light chain in an equimolar ratio and B) 20 ml Opti-MEM + 1.2 mL 293 fectin or fectin (2 $\mu$l/mL). According to the glucose consumption glucose solution was added during the course of the fermentation. The supernatant containing the secreted antibody was harvested after 5-10 days and antibodies were either directly purified from the supernatant or the supernatant was frozen and stored.

Purification

Antibodies were purified from cell culture supernatants by affinity chromatography using MabSelectSure-Sepharose™ (for non-AAA mutants) (GE Healthcare, Sweden) or KappaSelect-Agarose (for AAA mutants) (GE Healthcare, Sweden), hydrophobic interaction chromatography using butyl-Sepharose (GE Healthcare, Sweden) and Superdex 200 size exclusion (GE Healthcare, Sweden) chromatography.
Briefly, sterile filtered cell culture supernatants were captured on a MabSelectSuRe resin equilibrated with PBS buffer (10 mM $Na_2HPO_4$, 1 mM $KH_2PO_4$, 137 mM NaCl and 2.7 mM KCl, pH 7.4), washed with equilibration buffer and eluted with 25 mM sodium citrate at pH 3.0. The AAA mutants were captured on a KappaSelect resin equilibrated with 25 mM Tris, 50 mM NaCl, pH 7.2, washed with equilibration buffer and eluted with 25 mM sodium citrate pH 2.9. The eluted protein fractions were pooled and neutralized with 2M Tris, pH 9.0. The pooled antibody containing fractions were further purified by size exclusion chromatography using a Superdex 200 26/60 GL (GE Healthcare, Sweden) column equilibrated with 20 mM histidine, 140 mM NaCl, pH 6.0. The antibody containing fractions were pooled, concentrated to the required concentration using Vivaspin ultrafiltration devices (Sartorius Stedim Biotech S.A., France) and stored at -80 °C.

Purity and antibody integrity were analyzed after each purification step by CE-SDS using microfluidic Labchip technology (Caliper Life Science, USA). 5 μL of protein solution was prepared for CE-SDS analysis using the HT Protein Express Reagent Kit according manufacturer's instructions and analyzed on LabChip GXII system using a HT Protein Express Chip. Data were analyzed using LabChip GX Software.

**Example 4**

**Pharmacokinetic (PK) study of <IGF-1R> antibodies in mice transgenic for huFcRn**

In life phase

The study included female C57BL/6J mice (background); mouse FcRn deficient, but hemizygous transgenic for human FcRn (huFcRn, line 276 -/tg)

| | |
|---|---|
| Animals: | C57BL/6J-mice, knock-out for muFcRn, transgenic for huFcRn, line 276, heterozygous, male and female |
| Compounds: | HuMab<IGF-1R> wt, <IGF-1R_ YTE> and <IGF-1R_ AAA> |
| Dose: | 10 mg/kg body weight, single i.v. bolus administration (volume of administration: 10 mL/kg bodyweight) |
| Concentration: | 1 mg/mL for i.v. studies |

Part 1

All mice were injected once intravitreally into the right eye with 1 μL/animal of the appropriate solution of the <IGF-1R>, <IGF-1R_YTE> (anti-IGF-1R antibody with the YTE mutation) and <IGF-1R_AAA> (anti-IGF-1R antibody with the IHH-AAA mutation) antibodies.

Mice were allocated to 2 groups with 6 animals each. Blood samples are taken from group 1 at 2, 24 and 96 hours and from group 2 at 7, 48 and 168 hours after dosing.

Injection into the vitreous of the right mouse eye was performed by using the NanoFil Microsyringe system for nanoliter injection from World Precision Instruments, Inc., Berlin, Germany. Mice were anesthetized with 2.5 % Isoflurane and for visualization of the mouse eye a Leica MZFL 3 microscope with a 40 fold magnification and a ring-light with a Leica KL 2500 LCD lightning was used. Subsequently, 2 μL of the compound were injected using a 35-gauge needle.

Blood was collected via the retrobulbar venous plexus of the contralateral eye from each animal for the determination of the compound levels in serum.

Serum samples of at least 50 μl were obtained from blood after 1 hour at RT by centrifugation (9,300 x g) at 4 °C for 3 min. Serum samples were frozen directly after centrifugation and stored frozen at -80 °C until analysis. Treated eyes of the animals of group 1 were isolated 96 hours after treatment and of the animals of group 2 168 hours after treatment. Samples were stored frozen at -80 °C until analysis.

Part 2

All mice were injected once intravenously via the tail vein with the appropriate amount of antibody solution (i.e.1 mg/mL and a dose of 10 mg/kg body weight and an application volume of 200 μL).

Mice were allocated to 3 groups with 8 animals each. Blood samples are taken from group 1 at 0, 24 and 336 hours, from group 2 at 2, 168 and 504 hours and group 3 after 8, 48 and 672 hours after dosing. Blood was collected via the retrobulbar venous plexus from each animal for the determination of the compound levels in serum.

Serum samples of at least 50 μL were obtained from blood after 1 hour at RT by centrifugation (9,300 x g) at 4 °C for 3 min. Serum samples were frozen directly after centrifugation and stored frozen at -80 °C until analysis.

Preparation of whole eye lysates (mice)

The eye lysates were gained by physico-chemical disintegration of the whole eye from laboratory animals. For mechanical disruption, each eye was transferred into a 1.5 mL micro vial with conical bottom. After freeze and thawing, the eyes were washed with 1 mL cell washing buffer once (Bio-Rad, Bio-Plex Cell Lysis Kit, Cat. No. 171-304011). In the following step, 500 $\mu$L of freshly prepared cell lysis buffer were added and the eyes were grinded using a 1.5 mL tissue grinding pestle (Kimble Chase, 1.5 mL pestle, Art. No. 749521-1500). The mixture was then frozen and thawed five times and grinded again. To separate lysate from remaining tissue the samples were centrifuged for 4 minutes at 4,500 g. After centrifuging the supernatant was collected and stored at -20 °C until further analysis in the quantification ELISA.

Analysis

The concentrations of the <IGF-1R> antibodies in mice serum and eye lysates were determined with an enzyme linked immunosorbent assay (ELISA).
For quantification of <IGF-1R> antibodies in mouse serum samples and eye lysates, a standard solid-phase serial sandwich immunoassay with biotinylated and digoxigenated monoclonal antibodies used as capture and detection antibodies was performed. The bound immune complex of capture antibody, analyte and detection antibody on the solid phase of the streptavidin coated micro titer plate (SA-MTP) is then detected with a horseradish-peroxidase coupled to an anti-digoxigenin antibody. After washing unbound material from the SA-MTP and addition of ABTS-substrate, the gained signal is proportional to the amount of analyte bound on the solid phase of the SA-MTP. Quantification is then done by converting the measured signals of the samples into concentrations referring to calibrators analyzed in parallel.
In a first step the SA-MTP was coated with 100 $\mu$L/well of biotinylated capture antibody solution (mAb<hFC$_{\gamma PAN}$>IgG-Bi) on a MTP-shaker. Meanwhile calibrators, QC-samples and samples were prepared. After coating the SA-MTP with capture antibody, the plate was washed three times with washing buffer. Subsequently 100 $\mu$L/well of the calibrators, QC-samples and samples were pipetted on the SA-MTP and incubated again for one hour at 500 rpm. The analyte was now bound via the capture antibody to the solid phase of the SA-MTP. After incubation and removal of unbound analyte by washing the plate 100 $\mu$L/well of the first detection antibody (mAb<hFC$_{\gamma PAN}$>IgG-Dig) was added to the SA-MTP. Again, the plate was incubated for one hour at 500 rpm on a shaker. After washing, 100 $\mu$L/well of the second detection antibody (pAb<Digoxigenin>S-Fab-POD (poly)) at a concentration of 50 mU/mL was added to the wells of the SA-MTP and the plate was incubated again for one hour at 500 rpm. After a final washing step to remove excess of detection antibody, 100 $\mu$L/well substrate (ABTS®) is added. The antibody-enzyme conjugate catalyzes the color reaction of the ABTS® substrate. The signal was then measured by an ELISA reader at 405 nm wavelength (reference wavelength: 490 nm ([405/490] nm)).

Pharmacokinetic Evaluation

The pharmacokinetic parameters were calculated by non-compartmental analysis, using the pharmacokinetic evaluation program WinNonlinTM (Pharsight), version 5.2.1.

**Example 5**

**Pharmacokinetic (PK) properties of antibodies with HHY-AAA mutation**

PK data with FcRn mice transgenic for human FcRn

In life phase:

The study included female C57BL/6J mice (background); mouse FcRn deficient, but hemizygous transgenic for human FcRn (huFcRn, line 276 -/tg)

Part 1:

All mice were injected once intravitreally into the right eye with the appropriate solution of IGF-1R 0033, IGF-1R 0035, IGF-1R 0045 (i.e. 22.2 $\mu$g compound/animal of IGF-1R 0033, 24.4 $\mu$g compound/animal IGF-1R 0035, 32.0 $\mu$g compound/animal IGF-1R and 32.0 $\mu$g compound/animal of IGF-1R 0045).
Thirteen mice were allocated to 2 groups with 6 and 7, respectively, animals each. Blood samples are taken from group 1 at 2, 24 and 96 hours and from group 2 at 7, 48 and 168 hours after dosing.

Injection into the vitreous of the right mouse eye was performed by using the NanoFil Microsyringe system for nanoliter injection from World Precision Instruments, Inc., Berlin, Germany. Mice were anesthetized with 2.5 % Isoflurane and for visualization of the mouse eye a Leica MZFL 3 microscope with a 40 fold magnification and a ring-light with a Leica KL 2500 LCD lightning was used. Subsequently, 2 $\mu$L of the compound were injected using a 35-gauge needle.

Blood was collected via the retrobulbar venous plexus of the contralateral eye from each animal for the determination of the compound levels in serum.

Serum samples of at least 50 $\mu$L were obtained from blood after 1 hour at RT by centrifugation (9,300 x g) at 4 °C for 3 min. Serum samples were frozen directly after centrifugation and stored frozen at -80 °C until analysis. Treated eyes of the animals of group 1 were isolated 96 hours after treatment and of the animals of group 2 168 hours after treatment. Samples were stored frozen at -80 °C until analysis.

Part 2:

All mice were injected once intravenously via the tail vein with the appropriate solution of IGF-1R 0033, IGF-1R 0035, IGF-1R 0045 (i.e. 22.2 $\mu$g compound/animal of IGF-1R 0033, 24.4 $\mu$g compound/animal IGF-1R 0035, 32.0 $\mu$g compound/animal IGF-1R and 32.0 $\mu$g compound/animal of IGF-1R 0045).

Twelve mice were allocated to 2 groups with 6 animals each. Blood samples are taken from group 1 at 1, 24 and 96 hours and from group 2 at 7, 48 and 168 hours after dosing. Blood was collected via the retrobulbar venous plexus from each animal for the determination of the compound levels in serum.

Serum samples of at least 50 $\mu$L were obtained from blood after 1 hour at RT by centrifugation (9,300 x g) at 4 °C for 3 min. Serum samples were frozen directly after centrifugation and stored frozen at -80 °C until analysis.

Preparation of cell lysis buffer

Carefully mix 100 $\mu$L factor 1, 50 $\mu$L factor 2 and 24.73 mL Cell Lysis buffer (all from Bio-Rad, Bio-Plex Cell Lysis Kit, Cat. No. 171-304011) and add 125 $\mu$L PMSF- solution (174.4 mg phenylmethylsulfonylfluoride diluted in 2.0 mL DMSO).

Preparation of whole eye lysates (mice)

The eye lysates were gained by physico-chemical disintegration of the whole eye from laboratory animals. For mechanical disruption each eye was transferred into a 1.5 mL micro vial with conical bottom. After thawing, the eyes were washed with 1 mL cell washing buffer once (Bio-Rad, Bio-Plex Cell Lysis Kit, Cat. No. 171-304011). In the following step 500 $\mu$L of freshly prepared cell lysis buffer were added and the eyes were grinded using a 1.5 mL tissue grinding pestle (VWR Int., Art. No. 431-0098). The mixture was then frozen and thawed five times and grinded again. To separate lysate from remaining tissue the samples were centrifuged for 4 min. at 4500 x g. After centrifuging the supernatant was collected and stored at -20 °C until further analysis in the quantification ELISA.

Analysis (serum)

For quantification of antibodies in mouse serum sample, a standard solid-phase serial sandwich immunoassay with biotinylated and digoxigenated monoclonal antibodies used as capture and detection antibodies is performed. Serum accounts for about 50 % of the full blood sample volume.

More detailed, concentrations of the antibodies in mouse serum samples were determined by a human-IgG (Fab) specific enzyme linked immunosorbent assay. Streptavidin coated microtiter plates were incubated with the biotinylated anti-human Fab(kappa) monoclonal antibody M-1.7.10-IgG as capture antibody diluted in assay buffer for one hour at room temperature with agitation. After washing three times with phosphate-buffered saline-polysorbate 20 (Tween20), serum samples at various dilutions were added followed by second incubation for one hour at room temperature. After three repeated washings bound antibody was detected by subsequent incubation with the anti-human Fab(CH1) monoclonal antibody M-1.19.31-IgG conjugated to digoxigenin, followed by an anti-digoxigenin antibody conjugated to horseradish peroxidase (HRP). ABTS (2,2'-azino-bis(3-ethylbenzothiazoline-6-sulphonic acid); Roche Diagnostics GmbH, Mannheim, Germany) was used as HRP substrate to form a colored reaction product. Absorbance of the resulting reaction product was read at 405 nm (ABTS; reference wavelength: 490 nm).

All samples, positive and negative control samples were analyzed in replicates and calibrated against an antibody standard provided.

Analysis (eve lysate)

The concentrations of the analytes in mouse eye lysate samples were determined using a qualified electro-chemiluminescence immunoassay (ECLIA) method based on the ELECSYS® instrument platform (Roche Diagnostics GmbH, Mannheim, Germany) under non-GLP conditions.

The undiluted supernatant (eye lysates) was incubated with capture and detection molecules for 9 min. at 37 °C. Biotinylated anti-human-Fab(kappa) monoclonal antibody M-1.7.10-IgG was used as capture molecule and a ruthenium(II)tris(bispyridyl)$_3{}^{2+}$ labeled anti-human-Fab(CHl) monoclonal antibody M-1.19.31-IgG was used for detection. Streptavidin-coated magnetic microparticles were added and incubated for additional 9 min. at 37 °C to allow binding of preformed immune complexes due to biotin-streptavidin interactions. The microparticles were magnetically captured on an electrode and a chemiluminescent signal generated using the co-reactant tripropyl amine (TPA). The gained signal was measured by a photomultiplier detector.

**Table:** Standard chart IGF-1R 0033

| | concentration [ng/mL] | signal mean counts | standard deviation signal counts | serum-conc. [ng/mL] | recovery [%] |
|---|---|---|---|---|---|
| standard sample 9 | 0 | 1038 | 46 | - | - |
| standard sample 8 | 0.686 | 2682 | 105 | 0.675 | 98 |
| standard sample 7 | 2.06 | 6275 | 791 | 2.06 | 100 |
| standard sample 6 | 6.17 | 15907 | 316 | 6.23 | 101 |
| standard sample 5 | 18.5 | 45455 | 1238 | 18.8 | 102 |
| standard sample 4 | 55.6 | 133940 | 949 | 55.7 | 100 |
| standard sample 3 | 167 | 388069 | 2929 | 165 | 99 |
| standard sample 2 | 500 | 1129804 | 16777 | 503 | 101 |
| standard sample 1 | 1500 | 2956965 | 60287 | 1499 | 100 |

**Table:** Standard chart IGF-1R 0035

| | concentration [ng/mL] | signal mean counts | standard deviation signal counts | serum-conc. [ng/mL] | recovery [%] |
|---|---|---|---|---|---|
| standard sample 9 | 0 | 1024 | 63 | - | - |
| standard sample 8 | 0.686 | 2817 | 38 | 0.681 | 99 |
| standard sample 7 | 2.06 | 6451 | 39 | 2.08 | 101 |
| standard sample 6 | 6.17 | 17100 | 319 | 6.13 | 99 |

(continued)

|  | concentration [ng/mL] | signal mean counts | standard deviation signal counts | serum-conc. [ng/mL] | recovery [%] |
|---|---|---|---|---|---|
| standard sample 5 | 18.5 | 49693 | 713 | 18.6 | 100 |
| standard sample 4 | 55.6 | 146746 | 2575 | 56.1 | 101 |
| standard sample 3 | 167 | 423597 | 5068 | 165 | 99 |
| standard sample 2 | 500 | 1224244 | 11655 | 502 | 100 |
| standard sample 1 | 1500 | 3144901 | 44536 | 1499 | 100 |

**Table:** Standard chart IGF-1R 0045

|  | concentration [ng/mL] | signal mean counts | standard deviation signal counts | serum-conc. [ng/mL] | recovery [%] |
|---|---|---|---|---|---|
| standard sample 9 | 0 | 1339 | 545 | - | - |
| standard sample 8 | 0.686 | 3108 | 61 | 0.622 | 91 |
| standard sample 7 | 2.06 | 7032 | 189 | 1.93 | 94 |
| standard sample 6 | 6.17 | 19175 | 750 | 6.10 | 99 |
| standard sample 5 | 18.5 | 55526 | 823 | 18.7 | 101 |
| standard sample 4 | 55.6 | 158591 | 5412 | 55.7 | 100 |
| standard sample 3 | 167 | 456316 | 28759 | 167 | 100 |
| standard sample 2 | 500 | 1274801 | 47532 | 499 | 100 |
| standard sample 1 | 1500 | 3280452 | 239523 | 1501 | 100 |

Results:

A) Serum concentrations

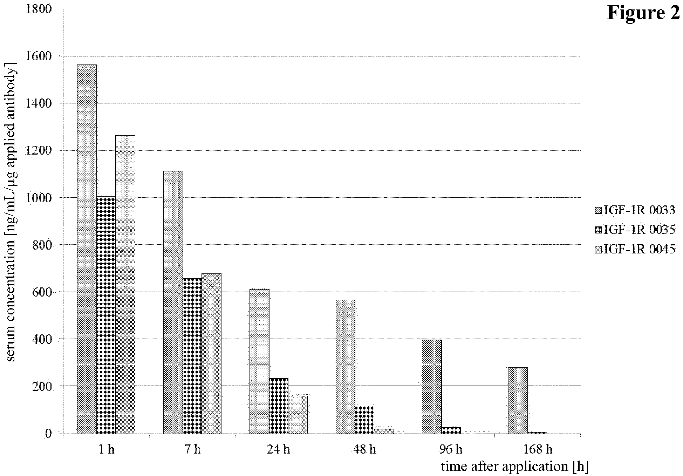

Results for serum concentrations are shown in the following Tables and Figure 2.

**Table:** IGF-1R 0033 (without HHY-AAA mutation): Comparison of serum concentrations after intravitreal and intravenous application (n.d. = not determined)

| | serum concentration after intravitreal application | serum concentration after intravenous application |
|---|---|---|
| ID | average conc. [$\mu$g/mL] | average conc. [$\mu$g/mL] |
| 1 h | n.d. | 34.7 |
| 2 h | 5.9 | n.d. |
| 7 h | 11.1 | 24.7 |
| 24 h | 4.4 | 13.6 |
| 48 h | 7.8 | 12.6 |
| 96 h | 2.1 | 8.9 |
| 168 h | 2.9 | 6.2 |

**Table:** IGF-1R 0035 (with HHY-AAA mutation in one Fc-region polypeptide): Comparison of serum concentrations after intravitreal and intravenous application

| | serum concentration after intravitreal application | serum concentration after intravenous application |
|---|---|---|
| ID | average conc. [$\mu$g/mL] | average conc. [$\mu$g/mL] |
| 1 h | n.d. | 24.5 |
| 2 h | 7.3 | n.d. |
| 7 h | 7.9 | 16.1 |
| 24 h | 2.3 | 5.7 |
| 48 h | 1.7 | 2.9 |
| 96 h | 0.3 | 0.6 |
| 168 h | 0.1 | 0.2 |

**Table:** IGF-1R 0045 (with HHY-AAA mutation in both Fc-region polypeptides): Comparison of serum concentrations after intravitreal and intravenous application (BLQ = below limit of quantitation)

| | serum concentration after intravitreal application | serum concentration after intravenous application |
|---|---|---|
| ID | average conc. [$\mu$g/mL] | average conc. [$\mu$g/mL] |
| 1 h | n.d. | 40.5 |
| 2 h | 13.2 | n.d. |
| 7 h | 9.6 | 21.7 |
| 24 h | 2.2 | 5.1 |
| 48 h | 0.9 | 0.7 |
| 96 h | 0.05 | 0.03 |
| 168 h | 0.01 | BLQ |

**Table:** Comparison of serum concentrations after intravenous application of antibodies IGF-1R 0033, 0035 and 0045 normalized to 1 μg applied antibody

|  | IGF-1R 0033 | IGF-1R 0035 | IGF-1R 0045 |
|---|---|---|---|
| ID | average conc. [ng/mL/μg applied antibody] | | |
| 1 h | 1564 | 1006 | 1266 |
| 7 h | 1114 | 659 | 679 |
| 24 h | 613 | 234 | 160 |
| 48 h | 569 | 118 | 21 |
| 96 h | 399 | 26 | 1 |
| 168 h | 280 | 7 | 0 |

Results:

B) Concentrations in eye-lysates of left and right eyes

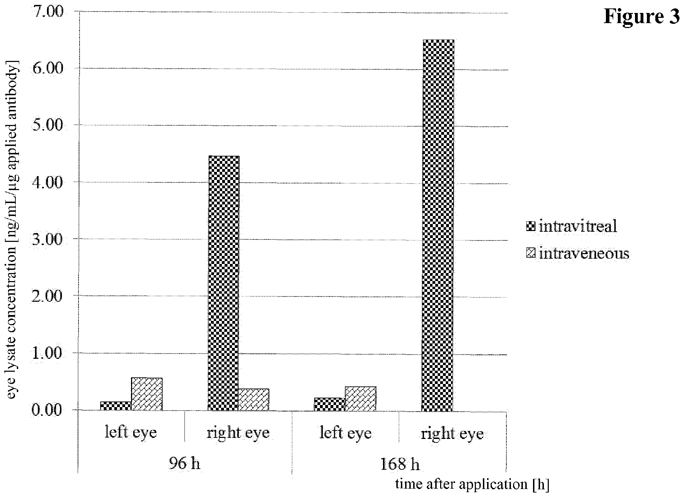

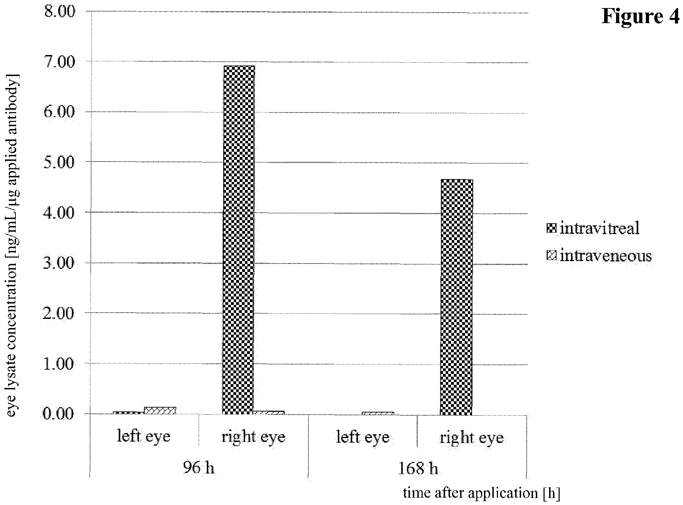

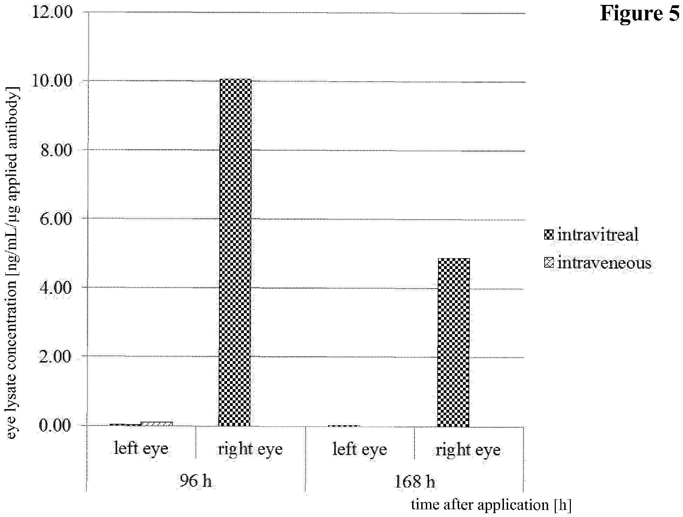

Results for concentrations in eye lysates are shown in the following Tables and Figures 3 to 5.

**Table:** Concentrations of IGF-1R 0033 (without HHY-AAA mutation) in eye lysates after intravitreal application into the right eye

| mean conc. values from n=7 (96 h) and n=6 (196 h) mice | | |
|---|---|---|
| ID | | mean conc. [ng/mL] |
| 96 h | left eye | 3.3 |
|  | right eye | 99.5 |
| 168 h | left eye | 5.2 |
|  | right eye | 144.9 |

**Table:** Concentrations of IGF-1R 0033 (without HHY-AAA mutation) in eye lysates after intravenous application (BLQ = below limit of quantitation)

| mean conc. values from n=5 (96 h) and n=6 (196 h) mice | | |
|---|---|---|
| ID | | mean conc. [ng/mL] |
| 96 h | left eye | 12.7 |
|  | right eye | 8.5 |
| 168 h | left eye | 9.7 |
|  | right eye | BLQ |

**Table:** Concentrations of IGF-1R 0035 (with the HHY-AAA mutation in one Fc-region polypeptide) in eye lysates after intravitreal application into the right eye

| mean conc. values from n=6 mice | | |
|---|---|---|
| ID | | mean conc. [ng/mL] |
| 96 h | left eye | 1.1 |
|  | right eye | 169.2 |
| 168 h | left eye | 0.3 |
|  | right eye | 114.7 |

**Table:** Concentrations of IGF-1R 0035 (with the HHY-AAA mutation in one Fc-region polypeptide) in eye lysates after intravenous application (BLQ = below limit of quantitation)

| mean conc. values from n=6 mice | | |
|---|---|---|
| ID | | mean conc. [ng/mL] |
| 96 h | left eye | 3.7 |
| | right eye | 1.7 |
| 168 h | left eye | 1.4 |
| | right eye | 0.3 |

**Table:** Concentrations of IGF-1R 0045 (with the HHY-AAA mutation in both Fc-region polypeptides) in eye lysates after intravitreal application into the right eye

| mean conc. values from n=6 mice | | |
|---|---|---|
| ID | | mean conc. [ng/mL] |
| 96 h | left eye | 1.4 |
| | right eye | 322.6 |
| 168 h | left eye | 1.4 |
| | right eye | 156.8 |

**Table:** Concentrations of IGF-1R 0045 (with the HHY-AAA mutation in both Fc-region polypeptides) in eye lysates after intravenous application (BLQ = below limit of quantitation)

| mean conc. values from n=6 (96 h) and n=5 (196 h) mice | | |
|---|---|---|
| ID | | mean conc. [ng/mL] |
| 96 h | left eye | 3.6 |
| | right eye | 1.3 |
| 168 h | left eye | 0.8 |
| | right eye | 0.4 |

**Table:** Concentrations of IGF-1R 0033, 0035 and 0045 in eye lysates after intravitreal application into the right eye normalized to 1 μg applied antibody

| | | IGF-1R 0033 | IGF-1R 0035 | IGF-1R 0045 |
|---|---|---|---|---|
| ID | | mean conc. [ng/mL] | | |
| 96 h | left eye | 0.15 | 0.05 | 0.04 |
| | right eye | 4.48 | 6.93 | 10.08 |
| 168 h | left eye | 0.24 | 0.01 | 0.04 |
| | right eye | 6.53 | 4.70 | 4.90 |

Summary of Results:

After intravitreal application the anti-IGF-1R antibodies 0035 and 0045 as reported herein (with one sided or both sided HHY-AAA mutation) shows similar concentrations (after 96 and 168 hours) in the eye lysates as compared to the anti-IGF-1R antibody without HHY-AAA mutation (IGF-1R 0033).

Also after intravitreal application the anti-IGF-1R antibodies 0035 and 0045 as reported herein (with one sided or both sided HHY-AAA mutation) shows in addition a faster clearance and shorter half-life in the serum as compared to the anti-IGF-1R antibody without HHY-AAA mutation (IGF-1R 0033).

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.

**Claims**

1. An anti-IGF-1R antibody that specifically binds to human IGF-1R and that has abolished FcRn binding, wherein the anti-IGF-1R antibody has at least one of the mutations L251D, M252T, I253A, S254W, S254R, H310A, H433A, N434L, H435A, Y436A (numbering according to Kabat EU index numbering system) or a combination thereof.

2. The antibody according to claim 1, wherein the anti-IGF-1R antibody has no (remaining) detectable FcRn binding using a surface plasmon resonance based determination method.

3. The antibody according to any one of claims 1 to 2, wherein the anti-IGF-1R antibody binds to human FcRn with a $K_D$-value of more than 1.7 $\mu$M at pH 6.

4. The antibody according to any one of claims 1 to 3, wherein the antibody has a retention time on an FcRn affinity chromatography column of three minutes or less.

5. The antibody according to any one of claims 1 to 4, wherein the anti-IGF-1R antibody has at least one of the mutations L251D, M252T, I253A, S254W, S254R, H310A, H433A, N434L, H435A, Y436A (numbering according to Kabat EU index numbering system) in the first Fc-region polypeptide and at least one of the mutations L251D, M252T, I253A, S254W, S254R, H310A, H433A, N434L, H435A, Y436A (numbering according to Kabat EU index numbering system) in the second Fc-region polypeptide.

6. The antibody according to any one of claims 1 to 5, wherein the anti-IGF-1R antibody has at least the mutations I253A/H310A/H435A or H310A/H433A/Y436A or L251D/L314D/L432D or combinations thereof (numbering according to Kabat EU index numbering system) in the first Fc-region polypeptide and at least one of the mutations L251D, M252T, I253A or S254W, S254R, H310A, H433A, N434G, N434L, H435A, Y436A (numbering according to Kabat EU index numbering system) in the second Fc-region polypeptide.

7. The antibody according to any one of claims 1 to 6, wherein the antibody comprises a first Fc-region polypeptide and a second Fc-region polypeptide wherein

> i) the first and the second Fc-region polypeptide further comprise the mutation Y436A, or
> ii) the first and the second Fc-region polypeptide further comprise the mutations I253A, H310A and H435A, or
> iii) the first and the second Fc-region polypeptide further comprise the mutations H310A, H433A and Y436A, or
> iv) the first and the second Fc-region polypeptide further comprise the mutations L251D, L314D and L432D, or
> v) the first Fc-region polypeptide further comprises the mutation Y436A and the second Fc-region polypeptide further comprises

>> a) the mutations I253A, H310A and H435A, or
>> b) the mutations H310A, H433A and Y436A, or
>> c) the mutations L251D, L314D and L432D,

> or
> vi) the first Fc-region polypeptide further comprises the mutations I253A, H310A and H435A and the second Fc-region polypeptide further comprises

>> a) the mutations H310A, H433A and Y436A, or
>> b) the mutations L251D, L314D and L432D,

> or
> vii) the first Fc-region polypeptide further comprises the mutations H310A, H433A and Y436A and the second Fc-region polypeptide further comprises

>> a) the mutations L251D, L314D and L432D.

8. The antibody according to any one of claims 1 to 7, wherein the antibody comprises an Fc-region comprising a first and a second Fc-region polypeptide both of human IgG1 or human IgG4 subclass (derived from human origin) which comprise one or two of the mutations selected from i) the group I253A, H310A, H435A, or ii) the group H310A, H433A, Y436A, or iii) the group L251D, L314D, L432D (numbering according to Kabat EU index numbering system) in the first Fc-region polypeptide and one or two of the mutations selected from the group comprising the mutations L251D, I253A, H310A, L314D, L432D, H433A, H435A and Y436A (numbering according to Kabat EU index numbering system) in the second Fc-region polypeptide so that all of the mutations in the first and the second Fc-region polypeptide when taken together result in that the mutations i) I253A, H310A and H435A, or ii) H310A, H433A and Y436A, or iii) L251D, L314D and L432D are comprised in the Fc-region.

9. The antibody according to any one of claims 1 to 7, wherein the antibody comprises an Fc-region comprising a first and a second Fc-region polypeptide both of human IgG1 or human IgG4 subclass (i.e. derived from human origin), which both comprise the combination of the mutations I253A/H310A/H435A or H310A/H433A/Y436A or L251D/L314D/L432D or combinations thereof in the Fc-region (numbering according to Kabat EU index numbering system), whereby either all mutations are in the first or the second Fc-region polypeptide or one or two mutations are in the first Fc-region polypeptide and one or two mutations are in the second Fc-region polypeptide so that all of the mutations in the first and the second Fc-region polypeptide when taken together result in that the mutations i) I253A, H310A and H435A, or ii) H310A, H433A and Y436A, or iii) L251D, L314D and L432D are comprised in the Fc-region.

10. The antibody according to any one of claims 1 to 7, wherein the antibody comprises an Fc-region comprising a first and a second Fc-region polypeptide both of human IgG1 or human IgG4 subclass (i.e. derived from human origin), which comprise the combination of the mutations I253A/H310A/H435A or H310A/H433A/Y436A or L251D/L314D/L432D in the first as well as in the second Fc-region polypeptide (numbering according to Kabat EU index numbering system), or comprises the combinations of the mutations I253A/H310A/H435A in the first Fc-region polypeptide and the combination of the mutations H310A/H433A/Y436A in the second Fc-region polypeptide (numbering according to Kabat EU index numbering system).

**11.** The antibody according to any one of claims 1 to 10, wherein the antibody does not specifically bind to the human FcRn and/or does specifically bind to Staphylococcal protein A.

**12.** The antibody according to any one of claims 1 to 11, wherein the antibody is **characterized in that** the antibody

- shows a lower serum concentration compared to corresponding bispecific antibody without the mutations in the Fc-region polypeptides (96 hours after intravitreal application in mice, which are mouse FcRn deficient, but hemizygous transgenic for human FcRn), and/or
- shows a similar (factor 0.8 to 1.2) concentration in whole right eye lysates compared to corresponding bispecific antibody without the mutations in the Fc-region polypeptides (in mice, which are mouse FcRn deficient, but hemizygous transgenic for human FcRn, 96 hours after intravitreal application in the right eye), and/or
- has abolished binding to the human FcRn, and/or
- has no binding to Staphylococcal protein A (determined by SPR), and/or
- has maintained binding to Staphylococcal protein A (determined by SPR).

**13.** The antibody according to any one of claims 1 to 12, wherein the anti-IGF-1R antibody has

a) an antibody heavy chain comprising as HVRs the amino acid sequence of SEQ ID NO: 56 (HVR-H1), the amino acid sequence of SEQ ID NO: 57 (HVR-H2), and the amino acid sequence of SEQ ID NO: 58 (HVR-H3), and an antibody light chain comprising as HVRs the amino acid sequence of SEQ ID NO: 59 (HVR-L1), the amino acid sequence of SEQ ID NO: 60 (HVR-L2), and the amino acid sequence of SEQ ID NO: 61 (HVR-L3), or
b) an antibody heavy chain comprising as HVRs the amino acid sequence of SEQ ID NO: 64 (HVR-H1), the amino acid sequence of SEQ ID NO: 65 (HVR-H2), and the amino acid sequence of SEQ ID NO: 66 (HVR-H3), and an antibody light chain comprising as HVRs the amino acid sequence of SEQ ID NO: 67 (HVR-L1), the amino acid sequence of SEQ ID NO: 68 (HVR-L2), and the amino acid sequence of SEQ ID NO: 69 (HVR-L3), or
c) an antibody heavy chain comprising as HVRs the amino acid sequence of SEQ ID NO: 72 (HVR-H1), the amino acid sequence of SEQ ID NO: 73 (HVR-H2), and the amino acid sequence of SEQ ID NO: 74 (HVR-H3), and an antibody light chain comprising as HVRs the amino acid sequence of SEQ ID NO: 75 (HVR-L1), the amino acid sequence of SEQ ID NO: 76 (HVR-L2), and the amino acid sequence of SEQ ID NO: 77 (HVR-L3), or
d) an antibody heavy chain comprising as HVRs the amino acid sequence of SEQ ID NO: 80 (HVR-H1), the amino acid sequence of SEQ ID NO: 81 (HVR-H2), and the amino acid sequence of SEQ ID NO: 82 (HVR-H3), and an antibody light chain comprising as HVRs the amino acid sequence of SEQ ID NO: 83 (HVR-L1), the amino acid sequence of SEQ ID NO: 84 (HVR-L2), and the amino acid sequence of SEQ ID NO: 85 (HVR-L3), or
e) an antibody heavy chain comprising as HVRs the amino acid sequence of SEQ ID NO: 88 (HVR-H1), the amino acid sequence of SEQ ID NO: 89 (HVR-H2), and the amino acid sequence of SEQ ID NO: 90 (HVR-H3), and an antibody light chain comprising as HVRs the amino acid sequence of SEQ ID NO: 91 (HVR-L1), the amino acid sequence of SEQ ID NO: 92 (HVR-L2), and the amino acid sequence of SEQ ID NO: 93 (HVR-L3), or
f) an antibody heavy chain comprising as HVRs amino acid residues 31-35 (HVR-H1), amino acid residues 50-66 (HVR-H2) and amino acid residues 99-107 (HVR-H3) of SEQ ID NO: 02, and an antibody light chain comprising as HVRs amino acid residues 24-34 (HVR-L1), amino acid residues 50-56 (HVR-L2) and amino acid residues 89-98 (HVR-L3) of SEQ ID NO: 04, or
g) an antibody heavy chain comprising as HVRs amino acid residues 31-35 (HVR-H1), amino acid residues 50-66 (HVR-H2) and amino acid residues 99-107 (HVR-H3) of SEQ ID NO: 01 and an antibody light chain comprising as HVRs amino acid residues 24-34 (HVR-L1), amino acid residues 50-56 (HVR-L2) and amino acid residues 89-98 (HVR-L3) of SEQ ID NO: 03.

**14.** The antibody according to any one of claims 1 to 13, wherein the anti-IGF-1R antibody comprises

a) a heavy chain variable domain VH of SEQ ID NO: 62 and a light chain variable domain VL of SEQ ID NO: 63, or
b) a heavy chain variable domain VH of SEQ ID NO: 70 and a light chain variable domain VL of SEQ ID NO: 71, or
c) a heavy chain variable domain VH of SEQ ID NO: 78 and a light chain variable domain VL of SEQ ID NO: 79, or
d) a heavy chain variable domain VH of SEQ ID NO: 86 and a light chain variable domain VL of SEQ ID NO: 87, or
e) a heavy chain variable domain VH of SEQ ID NO: 94 and a light chain variable domain VL of SEQ ID NO: 95, or
f) a heavy chain variable domain VH of SEQ ID NO: 05 and a light chain variable domain VL of SEQ ID NO: 07, or
g) a heavy chain variable domain VH of SEQ ID NO: 06 and a light chain variable domain of SEQ ID NO: 08.

**15.** An anti-IGF-1R antibody according to any one of claims 1 to 14 for the treatment of vascular eye diseases.

16. An anti-IGF-1R antibody according to any one of claims 1 to 14 for use as a medicament.

Figure 1

Figure 2

Figure 3

Figure 4

Figure 5

Europäisches
Patentamt

European
Patent Office

Office européen
des brevets

# EUROPEAN SEARCH REPORT

Application Number

EP 17 20 5688

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2008/077546 A1 (HOFFMANN LA ROCHE [CH]; KOLL HANS [DE]; KUENKELE KLAUS-PETER [DE]; MOS) 3 July 2008 (2008-07-03) | 1-11,15, 16 | INV. C07K16/22 C07K16/28 |
| A | * the whole document * | 13,14 | A61K39/00 A61K39/395 |
| Y | KIM HYUNCHEOL ET AL: "FcRn receptor-mediated pharmacokinetics of therapeutic IgG in the eye.", MOLECULAR VISION 2009, vol. 15, 2009, pages 2803-2812, XP002688851, ISSN: 1090-0535 | 1-11,15, 16 | A61K9/00 C07K16/40 |
| A | * the whole document * | 13,14 | |
| Y | KIM J-K ET AL: "Mapping the site on human IgG for binding of the MHC class I-related receptor, FcRn", EUROPEAN JOURNAL OF IMMUNOLOGY, WILEY - V C H VERLAG GMBH & CO. KGAA, DE, vol. 29, no. 9, 1 September 1999 (1999-09-01), pages 2819-2825, XP002300286, ISSN: 0014-2980, DOI: 10.1002/(SICI)1521-4141(199909)29:09<2819: :AID-IMMU2819>3.0.CO;2-6 | 1-11,15, 16 | |
| A | * the whole document * | 13,14 | TECHNICAL FIELDS SEARCHED (IPC) C07K A61K |
| Y,D | MEDESAN C ET AL: "Delineation of the amino acid residues involved in transcytosis and catabolism of mouse IgG1", THE JOURNAL OF IMMUNOLOGY, THE AMERICAN ASSOCIATION OF IMMUNOLOGISTS, US, vol. 158, no. 5, 1 March 1997 (1997-03-01) , pages 2211-2217, XP002532767, ISSN: 0022-1767 | 1-11,15, 16 | |
| A | * the whole document * | 13,14 | |

-/--

The present search report has been drawn up for all claims

| Place of search | Date of completion of the search | Examiner |
|---|---|---|
| The Hague | 19 March 2018 | Hix, Rebecca |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

page 1 of 3

Europäisches
Patentamt

European
Patent Office

Office européen
des brevets

**EUROPEAN SEARCH REPORT**

Application Number

EP 17 20 5688

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | TIMOTHY T KUO ET AL: "Neonatal Fc Receptor: From Immunity to Therapeutics", JOURNAL OF CLINICAL IMMUNOLOGY, KLUWER ACADEMIC PUBLISHERS-PLENUM PUBLISHERS, NE, vol. 30, no. 6, 1 October 2010 (2010-10-01), pages 777-789, XP019858481, ISSN: 1573-2592, DOI: 10.1007/S10875-010-9468-4 | 1-11,15,16 | |
| A | * the whole document * | 13,14 | |
| Y,D | S.-W. QIAO ET AL: "Dependence of antibody-mediated presentation of antigen on FcRn", PROCEEDINGS OF THE NATIONAL ACADEMY OF SCIENCES, vol. 105, no. 27, 1 January 2008 (2008-01-01), pages 9337-9342, XP055046753, ISSN: 0027-8424, DOI: 10.1073/pnas.0801717105 | 1-11,15,16 | |
| A | * the whole document * | 13,14 | TECHNICAL FIELDS SEARCHED (IPC) |
| Y,D | WO 2009/126304 A1 (BIOGEN IDEC INC [US]; HARIHARAN KANDASAMY [US]; DONG JIANYING [US]) 15 October 2009 (2009-10-15) | 1-11,15,16 | |
| A | * the whole document * | 13,14 | |
| Y,D | CHARLOTTE MAGDELAINE-BEUZELIN ET AL: "Therapeutic antibodies in ophthalmology: Old is new again", MABS, vol. 2, no. 2, 1 March 2010 (2010-03-01), pages 176-180, XP055079691, ISSN: 1942-0862, DOI: 10.4161/mabs.2.2.11205 | 1-11,15,16 | |
| A | * the whole document * | 13,14 | |

-/--

The present search report has been drawn up for all claims

| Place of search | Date of completion of the search | Examiner |
|---|---|---|
| The Hague | 19 March 2018 | Hix, Rebecca |

page 2 of 3

Europäisches
Patentamt

European
Patent Office

Office européen
des brevets

# EUROPEAN SEARCH REPORT

Application Number

EP 17 20 5688

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | ROBERT STEINBROOK: "The Price of Sight - Ranibizumab, Bevacizumab, and the Treatment of Macular Degeneration", NEW ENGLAND JOURNAL OF MEDICINE, vol. 355, no. 14, 5 October 2006 (2006-10-05), pages 1409-1412, XP055079675, ISSN: 0028-4793, DOI: 10.1056/NEJMp068185 | 1-11,15, 16 | |
| A | * the whole document * | 13,14 | |
| Y,D | TIMOTHY T. KUO ET AL: "Neonatal Fc receptor and IgG-based therapeutics", MABS, 1 September 2011 (2011-09-01), pages 422-430, XP055079703, | 1-11,15, 16 | |
| A | * the whole document * | 13,14 | |
| Y | GHETIE V ET AL: "FcRn: the MHC class I-related receptor that is more than an IgG transporter", IMMUNOLOGY TODAY, ELSEVIER PUBLICATIONS, CAMBRIDGE, GB, vol. 18, no. 12, 1 December 1997 (1997-12-01), pages 592-598, XP004097421, ISSN: 0167-5699, DOI: 10.1016/S0167-5699(97)01172-9 | 1-11,15, 16 | |
| A | * the whole document * | 13,14 | |
| Y,D | WO 2006/031370 A2 (GENENTECH INC [US]; LOWMAN HENRY B [US]; ADAMS CAMELLIA W [US]; MARVIN) 23 March 2006 (2006-03-23) | 1-11,15, 16 | |
| A | * the whole document * | 13,14 | |

TECHNICAL FIELDS
SEARCHED      (IPC)

The present search report has been drawn up for all claims

| Place of search | Date of completion of the search | Examiner |
|---|---|---|
| The Hague | 19 March 2018 | Hix, Rebecca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 20 5688

19-03-2018

| Patent document cited in search report | | Publication date | Patent family member(s) | | Publication date |
|---|---|---|---|---|---|
| WO 2008077546 | A1 | 03-07-2008 | AR | 064620 A1 | 15-04-2009 |
| | | | AU | 2007338402 A1 | 03-07-2008 |
| | | | BR | PI0722062 A2 | 01-04-2014 |
| | | | CA | 2672715 A1 | 03-07-2008 |
| | | | CL | 2007003726 A1 | 16-05-2008 |
| | | | CN | 101611059 A | 23-12-2009 |
| | | | CR | 10810 A | 12-08-2009 |
| | | | EC | SP099440 A | 31-07-2009 |
| | | | EP | 2102242 A1 | 23-09-2009 |
| | | | JP | 2010513352 A | 30-04-2010 |
| | | | KR | 20090088911 A | 20-08-2009 |
| | | | KR | 20120080663 A | 17-07-2012 |
| | | | MA | 31066 B1 | 04-01-2010 |
| | | | NZ | 576956 A | 29-07-2011 |
| | | | PE | 18322008 A1 | 27-12-2008 |
| | | | RU | 2009127846 A | 27-01-2011 |
| | | | TW | 200833712 A | 16-08-2008 |
| | | | US | 2008226635 A1 | 18-09-2008 |
| | | | US | 2012076778 A1 | 29-03-2012 |
| | | | WO | 2008077546 A1 | 03-07-2008 |
| | | | ZA | 200904324 B | 28-04-2010 |
| WO 2009126304 | A1 | 15-10-2009 | CN | 102065895 A | 18-05-2011 |
| | | | EP | 2274010 A1 | 19-01-2011 |
| | | | JP | 2011516549 A | 26-05-2011 |
| | | | US | 2009291088 A1 | 26-11-2009 |
| | | | WO | 2009126304 A1 | 15-10-2009 |
| WO 2006031370 | A2 | 23-03-2006 | AU | 2005285347 A1 | 23-03-2006 |
| | | | BR | PI0515230 A | 15-07-2008 |
| | | | CA | 2577133 A1 | 23-03-2006 |
| | | | CN | 101052654 A | 10-10-2007 |
| | | | EP | 1778728 A2 | 02-05-2007 |
| | | | JP | 2008510466 A | 10-04-2008 |
| | | | KR | 20070057839 A | 07-06-2007 |
| | | | KR | 20080080675 A | 04-09-2008 |
| | | | RU | 2367667 C2 | 20-09-2009 |
| | | | US | 2006067930 A1 | 30-03-2006 |
| | | | WO | 2006031370 A2 | 23-03-2006 |
| | | | ZA | 200701715 B | 30-07-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008077546 A
- WO 2009126304 A
- WO 2006031370 A
- WO 2005005635 A
- WO 2013041462 A
- US 7695936 B
- US 20030078385 A
- WO 2009089004 A
- WO 2011122011 A
- US 4816567 A
- US 5821337 A
- US 7527791 B
- US 6982321 B
- US 7087409 B
- US 6075181 A
- US 6150584 A
- US 5770429 A
- US 7041870 B
- US 20070061900 A
- US 7189826 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- WO 9308829 A
- US 5731168 A
- US 4676980 A
- US 20060025576 A
- US 20080069820 A
- WO 2009080251 A
- WO 2009080252 A
- WO 2009080253 A
- WO 2009080254 A
- WO 2010112193 A
- WO 2010115589 A
- WO 2010136172 A
- WO 2010145792 A
- WO 2010145793 A
- US 20030157108 A
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- WO 2004056312 A
- WO 2003085107 A
- WO 2003011878 A
- US 6602684 B
- US 20050123546 A
- WO 199730087 A
- WO 199858964 A
- WO 199922764 A
- US 6737056 B
- US 7332581 B
- US 6194551 B
- WO 9951642 A
- US 5648260 A
- US 5624821 A
- WO 9429351 A
- US 7521541 B
- WO 9627011 A
- WO 98050431 A
- EP 1870459 A
- WO 2007110205 A
- WO 2007147901 A
- WO 2010129304 A
- WO 201190754 A
- WO 2011143545 A
- WO 2012058768 A
- WO 2013157954 A
- WO 2013096291 A
- WO 96027011 A
- EP 1870459 A1
- WO 2013157953 A
- WO 2011090762 A
- US 5648237 A
- US 5789199 A
- US 5840523 A
- US 5959177 A
- US 6040498 A

- US 6420548 B
- US 7125978 B
- US 6417429 B
- US 20050260186 A

- US 20060104968 A
- US 6267958 B
- US 6171586 B
- WO 2006044908 A

**Non-patent literature cited in the description**

- **LEROITH, D. et al.** *Endocrin. Rev.,* 1995, vol. 16, 143-163
- **ADAMS, T.E. et al.** *Cell. Mol. Life Sci.,* 2000, vol. 57, 1050-1063
- **FOLKMAN, J. et al.** *J. Biol. Chem.,* 1992, vol. 267, 10931-10934
- **KLAGSBRUN, M. et al.** *Annu. Rev. Physiol.,* 1991, vol. 53, 217-239
- **GARNER, A.** Vascular diseases, in: Pathobiology of ocular disease, A dynamic approach. Marcel Dekker, 1994, 1625-1710
- **KIM, H. et al.** *Invest. Ophthalmol. Vis. Sci.,* 2008, vol. 49, 2025-2029
- **KIM, H. et al.** *Mol. Vis.,* 2009, vol. 15, 2803-2812
- **MAGDELAINE-BEUZELIN, C. et al.** report for therapeutic antibodies in ophthalmology that old is new again. *MABS,* 2010, vol. 2, 176-180
- **STEINBROOK, R.** report the price of sight - Ranibizumab, Bevacizumab, and the treatment of macular degeneration. *New Eng. J. Med.,* 2006, vol. 355, 1409-1412
- **KIM, J.K. et al.** report the mapping of the site on human IgG for binding of the MHC class I-related receptor, FcRn. *Eur. J. Immunol.,* 1999, vol. 29, 2819-2825
- **KUO, T.T. et al.** report about the neonatal Fc receptor: from immunity to therapeutics. *J. Clin. Immunol.,* 2010, vol. 30, 777-789
- **KUO, T.T et al.** report about neonatal Fc receptor and IgG-based therapeutics. *MABS,* 2011, vol. 3, 422-438
- **MEDESAN, C. et al.** report the delineation of the amino acid residues involved in transcytosis and catabolism of mouse IgG1. *J. Immunol.,* 1997, vol. 158, 2211-2217
- **QIAO, S.-W. et al.** report the dependence of antibody-mediated presentation of antigen on FcRn. *Proc. Natl. Acad. Sci. USA,* 2008, vol. 105, 9337-9342
- **KABAT, E.A. et al.** Sequences of Proteins of Immunological Interest. Public Health Service, National Institutes of Health, 1991
- **HUBER, A.H. et al.** *J. Mol. Biol.,* 1993, vol. 230, 1077-1083
- **KABAT, E.A. et al.** Sequences of Proteins of Immunological Interest. NIH, 1991, vol. 1-3
- **KABAT et al.** Sequences of Proteins of Immunological Interest. Public Health Service, National Institutes of Health, 1991

- **CHOTHIA, C. ; LESK, A.M.** *J. Mol. Biol.,* 1987, vol. 196, 901-917
- **MACCALLUM et al.** *J. Mol. Biol.,* 1996, vol. 262, 732-745
- **FLATMAN, S. et al.** *J. Chrom. B,* 2007, vol. 848, 79-87
- **KINDT, T.J. et al.** Kuby Immunology. W.H. Freeman and Co, 2007, 91
- **PORTOLANO, S. et al.** *J. Immunol.,* 1993, vol. 150, 880-887
- **CLACKSON, T. et al.** *Nature,* 1991, vol. 352, 624-628
- **GARNER, A.** Vascular diseases, In: Pathobiology of ocular disease, A dynamic approach. Marcel Dekker, 1994, 1625-1710
- **HUBER, A.H. et al.** *J. Mol. Biol.,* 1993, 1077-1083
- **KIM, J.K. et al.** *Eur. J. Immunol.,* 1999, vol. 29, 2819-2825
- **RAGHAVAN, M. et al.** *Biochem.,* 1995, vol. 34, 14649-146579
- **MEDESAN, C. et al.** *J Immunol.,* 1997, vol. 158, 2211-2217
- **KUO, T.T. et al.** *J. Clin. Immunol.,* 2010, vol. 30, 777-789
- **DALL'ACQUA, W.F. et al.** *J. Immunol,* 2002, vol. 169, 5171-5180
- **MEDESAN, C. et al.** *Eur. J. Immunol.,* 1996, vol. 26, 2533-2536
- **FIRAN, M. et al.** *Int. Immunol.,* 2001, vol. 13, 993-1002
- **KIM, J.K. et al.** *Eur. J. Immunol.,* 1994, vol. 24, 542-548
- **DALL'ACQUA, W.F. et al.** *J. Biol. Chem.,* 2006, vol. 281, 23514-23524
- **SHIELDS, R.L. et al.** *J. Biol. Chem.,* 2001, vol. 276, 6591-6604
- **YEUNG, Y.A. et al.** *J. Immunol.,* 2009, vol. 182, 7667-7671
- **KIM, J.K.** *Scand. J. Immunol.,* 1994, vol. 40, 457-465
- **GHETIE, V. ; WARD, E.S.** *Immunol. Today,* 1997, vol. 18, 592-598
- **MEDESAN, C. et al.** *J. Immunol.,* 1997, vol. 158, 2211-2217
- **KIM, J.K.** *Eur. J. Immunol.,* 1999, vol. 29, 2819-2825
- **DALL'ACQUA.** *J. Immunol.,* 2002, vol. 169, 5171-5180

- **HINTON, P.R. et al.** *J. Biol. Chem.,* 2004, vol. 279, 6213-6216
- **VACCARO, C. et al.** *Nat. Biotechnol.,* 2005, vol. 23, 1283-1288
- **POP, L.M. et al.** *Int. Immunopha rmacol.,* 2005, vol. 5, 1279-1290
- **PETKOVA, S.B. et al.** *Int. Immunol,* 2006, vol. 18, 1759-1769
- **HINTON, P.R. et al.** *J. Immunol.,* 2006, vol. 176, 346-356
- **MANNAN, A. et al.** *J. Biol. Chem.,* 2007, vol. 282, 1709-1717
- **MANNAN, A. et al.** *Drug Metab. Dispos.,* 2007, vol. 35, 86-94
- **ROPEENIAN, D.C. ; AKILESH, S.** *Nat. Rev. Immunol.,* 2007, vol. 7, 715-725
- **YEUNG, Y.A. et al.** *Cancer Res.,* 2010, vol. 70, 3269-3277
- **MAKRIDES, S.C.** *Protein Expr. Purif.,* 1999, vol. 17, 183-202
- **GEISSE, S. et al.** *Protein Expr. Purif.,* 1996, vol. 8, 271-282
- **KAUFMAN, R.J.** *Mol. Biotechnol.,* 2000, vol. 16, 151-160
- **WERNER, R.G.** *Drug Res.,* 1998, vol. 48, 870-880
- **AUSUBEL, F. et al.** Current Protocols in Molecular Biology. Greene Publishing and Wiley Interscience, 1987
- **VIJAYALAKSHMI, M.A.** *Appl. Biochem. Biotech.,* 1998, vol. 75, 93-102
- **CHEN, Y. et al.** *J. Mol. Biol.,* 1999, vol. 293, 865-881
- **MORRISON, S.L. et al.** *Proc. Natl. Acad. Sci. USA,* 1984, vol. 81, 6851-6855
- **ALMAGRO, J.C. ; FRANSSON, J.** *Front. Biosci.,* 2008, vol. 13, 1619-1633
- **RIECHMANN, I. et al.** *Nature,* 1988, vol. 332, 323-329
- **QUEEN, C. et al.** *Proc. Natl. Acad. Sci. USA,* 1989, vol. 86, 10029-10033
- **KASHMIRI, S.V. et al.** *Methods,* 2005, vol. 36, 25-34
- **PADLAN, E.A.** *Mol. Immunol.,* 1991, vol. 28, 489-498
- **DALL'ACQUA, W.F. et al.** *Methods,* 2005, vol. 36, 43-60
- **OSBOURN, J. et al.** *Methods,* 2005, vol. 36, 61-68
- **KLIMKA, A. et al.** *Br. J. Cancer,* 2000, vol. 83, 252-260
- **SIMS, M.J. et al.** *J. Immunol.,* 1993, vol. 151, 2296-2308
- **CARTER, P. et al.** *Proc. Natl. Acad. Sci. USA,* 1992, vol. 89, 4285-4289
- **PRESTA, L.G. et al.** *J. Immunol.,* 1993, vol. 151, 2623-2632
- **BACA, M. et al.** *J. Biol. Chem.,* 1997, vol. 272, 10678-10684
- **ROSOK, M.J. et al.** *J. Biol. Chem.,* vol. 271, 22611-22618
- **VAN DIJK, M.A. ; VAN DE WINKEL, J.G.** *Curr. Opin. Pharmacol.,* 2001, vol. 5, 368-374
- **LONBERG, N.** *Curr. Opin. Immunol.,* 2008, vol. 20, 450-459
- **LONBERG, N.** *Nat. Biotech.,* 2005, vol. 23, 1117-1125
- **KOZBOR, D.J.** *Immunol,* 1984, vol. 133, 3001-3005
- **BRODEUR, B.R. et al.** Monoclonal Antibody Production Techniques and Applications. Marcel Dekker, Inc, 1987, 51-63
- **BOERNER, P. et al.** J. Immunol. 1991, vol. 147, 86-95
- **LI, J. et al.** *Proc. Natl. Acad. Sci. USA,* 2006, vol. 103, 3557-3562
- **NI, J.** *Xiandai Mianyixue,* 2006, vol. 26, 265-268
- **VOLLMERS, H.P. ; BRANDLEIN, S.** *Histology and Histopathology,* 2005, vol. 20, 927-937
- **VOLLMERS, H.P. ; BRANDLEIN, S.** Methods and Findings. *Experimental and Clinical Pharmacology,* 2005, vol. 27, 185-191
- **HOOGENBOOM, H.R. et al.** *Methods in Molecular Biology,* 2001, vol. 178, 1-37
- **MCCAFFERTY, J. et al.** *Nature,* 1990, vol. 348, 552-554
- **MARKS, J.D. et al.** *J. Mol. Biol.,* 1992, vol. 222, 581-597
- **MARKS, J.D. ; BRADBURY, A.** *Methods in Molecular Biology,* 2003, vol. 248, 161-175
- **SIDHU, S.S. et al.** *J. Mol. Biol.,* 2004, vol. 338, 299-310
- **LEE, C.V. et al.** *J. Mol. Biol.,* 2004, vol. 340, 1073-1093
- **FELLOUSE, F.A.** *Proc. Natl. Acad. Sci. USA,* 2004, vol. 101, 12467-12472
- **LEE, C.V. et al.** *J. Immunol. Methods,* 2004, vol. 284, 119-132
- **WINTER, G. et al.** *Ann. Rev. Immunol.,* 1994, vol. 12, 433-455
- **GRIFFITHS, A.D. et al.** *EMBO J.,* 1993, vol. 12, 725-734
- **HOOGENBOOM, H.R. ; WINTER, G.** *J. Mol. Biol.,* 1992, vol. 227, 381-388
- **MILSTEIN, C. ; CUELLO, A.C.** *Nature,* 1983, vol. 305, 537-540
- **TRAUNECKER, A. et al.** *EMBO J.,* 1991, vol. 10, 3655-3659
- **BRENNAN, M. et al.** *Science,* 1985, vol. 229, 81-83
- **KOSTELNY, S.A. et al.** *J. Immunol.,* 1992, vol. 148, 1547-1553
- **HOLLIGER, P. et al.** *Proc. Natl. Acad. Sci. USA,* 1993, vol. 90, 6444-6448

- **GRUBER, M et al.** *J. Immunol.,* 1994, vol. 152, 5368-5374
- **TUTT, A. et al.** *J. Immunol.,* 1991, vol. 147, 60-69
- **CHOWDHURY, P.S.** *Methods Mol. Biol.,* 2008, vol. 207, 179-196
- **HOOGENBOOM, H.R. et al.** *Methods in Molecular Biology,* 2002, vol. 178, 1-37
- **CUNNINGHAM, B.C. ; WELLS, J.A.** *Science,* 1989, vol. 244, 1081-1085
- **WRIGHT, A. ; MORRISON, S.L.** *TIBTECH,* 1997, vol. 15, 26-32
- **OKAZAKI, A. et al.** *J. Mol. Biol.,* 2004, vol. 336, 1239-1249
- **YAMANE-OHNUKI, N. et al.** *Biotech. Bioeng.,* 2004, vol. 87, 614-622
- **RIPKA, J. et al.** *Arch. Biochem. Biophys.,* 1986, vol. 249, 533-545
- **KANDA, Y. et al.** *Biotechnol. Bioeng.,* 2006, vol. 94, 680-688
- **PETKOVA, S.B. et al.** *Int. Immunol.,* 2006, vol. 18, 1759-1769
- **IDUSOGIE, E.E. et al.** *J. Immunol.,* 2000, vol. 164, 4178-4184
- **DUNCAN, A.R. ; WINTER, G.** *Nature,* 1988, vol. 322, 738-740
- **KAM, N.W. et al.** *Proc. Natl. Acad. Sci. USA,* 2005, vol. 102, 11600-11605
- **RIDGWAY, J.B. et al.** *Protein Eng.,* 1996, vol. 9, 617-621
- **MERCHANT, A.M. et al.** *Nat. Biotechnol.,* 1998, vol. 16, 677-681
- **MERCHANT, A.M. et al.** *Nature Biotech.,* 1998, vol. 16, 677-681
- **ATWELL, S. et al.** *J. Mol. Biol.,* 1997, vol. 270, 26-35
- **CHARLTON, K.A.** Methods in Molecular Biology. Humana Press, 2003, vol. 248, 245-254
- **GERNGROSS, T.U.** *Nat. Biotech.,* 2004, vol. 22, 1409-1414
- **LI, H. et al.** *Nat. Biotech.,* 2006, vol. 24, 210-215
- **GRAHAM, F.L. et al.** *J. Gen Virol.,* 1977, vol. 36, 59-74
- **MATHER, J.P.** *Biol. Reprod.,* 1980, vol. 23, 243-252
- **MATHER, J.P et al.** *Annals N.Y. Acad. Sci.,* 1982, vol. 383, 44-68
- **URLAUB, G. et al.** *Proc. Natl. Acad. Sci. USA,* 1980, vol. 77, 4216-4220
- **YAZAKI, P. ; WU, A.M.** Methods in Molecular Biology. Humana Press, 2004, vol. 248, 255-268
- **CARMELIET ; JAIN.** *Nature,* 2000, vol. 407, 249-257
- Remington's Pharmaceutical Sciences. 1980
- **FLINTOFF, W.F. et al.** *Somat. Cell Genet.,* 1976, vol. 2, 245-261
- **FLINTOFF et al.** *Mol. Cell. Biol.,* 1982, vol. 2, 275-285
- **URLAUB, G. et al.** *Cell,* 1983, vol. 33, 405-412
- **URLAUB, G. et al.** *Somat. Cell Mol. Genet.,* 1986, vol. 12, 555-566

SEQUENCE LISTING

<110>  F. Hoffmann-La Roche

<120>  FcRn-binding abolished anti-IGF-1R antibodies and their use in
       the treatment of vascular eye diseases

<130>  P31561-EP-3

<150>  EP13165741.3
<151>  2013-04-29

<150>  EP14151318.4
<151>  2014-01-15

<160>  96

<170>  PatentIn version 3.5

<210>  1
<211>  448
<212>  PRT
<213>  Homo sapiens

<400>  1

Gln Val Glu Leu Val Glu Ser Gly Gly Gly Val Val Gln Pro Gly Arg
1               5                   10                  15


Ser Gln Arg Leu Ser Cys Ala Ala Ser Gly Phe Thr Phe Ser Ser Tyr
            20                  25                  30


Gly Met His Trp Val Arg Gln Ala Pro Gly Lys Gly Leu Glu Trp Val
            35                  40                  45


Ala Ile Ile Trp Phe Asp Gly Ser Ser Thr Tyr Tyr Ala Asp Ser Val
        50                  55                  60


Arg Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Phe Cys
                85                  90                  95


Ala Arg Glu Leu Gly Arg Arg Tyr Phe Asp Leu Trp Gly Arg Gly Thr
            100                 105                 110


Leu Val Ser Val Ser Ser Ala Ser Thr Lys Gly Pro Ser Val Phe Pro
            115                 120                 125


Leu Ala Pro Ser Ser Lys Ser Thr Ser Gly Gly Thr Ala Ala Leu Gly
        130                 135                 140


Cys Leu Val Lys Asp Tyr Phe Pro Glu Pro Val Thr Val Ser Trp Asn

```
         145                    150                    155                    160

         Ser Gly Ala Leu Thr Ser Gly Val His Thr Phe Pro Ala Val Leu Gln
                         165                    170                    175

         Ser Ser Gly Leu Tyr Ser Leu Ser Ser Val Val Thr Val Pro Ser Ser
                     180                    185                    190

         Ser Leu Gly Thr Gln Thr Tyr Ile Cys Asn Val Asn His Lys Pro Ser
                     195                    200                    205

         Asn Thr Lys Val Asp Lys Lys Val Glu Pro Lys Ser Cys Asp Lys Thr
             210                    215                    220

         His Thr Cys Pro Pro Cys Pro Ala Pro Glu Ala Ala Gly Gly Pro Ser
         225                    230                    235                    240

         Val Phe Leu Phe Pro Pro Lys Pro Lys Asp Thr Leu Met Ile Ser Arg
                         245                    250                    255

         Thr Pro Glu Val Thr Cys Val Val Val Asp Val Ser His Glu Asp Pro
                     260                    265                    270

         Glu Val Lys Phe Asn Trp Tyr Val Asp Gly Val Glu Val His Asn Ala
                     275                    280                    285

         Lys Thr Lys Pro Arg Glu Glu Gln Tyr Asn Ser Thr Tyr Arg Val Val
             290                    295                    300

         Ser Val Leu Thr Val Leu His Gln Asp Trp Leu Asn Gly Lys Glu Tyr
         305                    310                    315                    320

         Lys Cys Lys Val Ser Asn Lys Ala Leu Gly Ala Pro Ile Glu Lys Thr
                         325                    330                    335

         Ile Ser Lys Ala Lys Gly Gln Pro Arg Glu Pro Gln Val Tyr Thr Leu
                     340                    345                    350

         Pro Pro Ser Arg Asp Glu Leu Thr Lys Asn Gln Val Ser Leu Thr Cys
                     355                    360                    365

         Leu Val Lys Gly Phe Tyr Pro Ser Asp Ile Ala Val Glu Trp Glu Ser
             370                    375                    380

         Asn Gly Gln Pro Glu Asn Asn Tyr Lys Thr Thr Pro Pro Val Leu Asp
         385                    390                    395                    400
```

```
Ser Asp Gly Ser Phe Phe Leu Tyr Ser Lys Leu Thr Val Asp Lys Ser
            405             410             415

Arg Trp Gln Gln Gly Asn Val Phe Ser Cys Ser Val Met His Glu Ala
            420             425             430

Leu His Asn His Tyr Thr Gln Lys Ser Leu Ser Leu Ser Pro Gly Lys
            435             440             445


<210>  2
<211>  448
<212>  PRT
<213>  Homo sapiens

<400>  2

Gln Val Gln Leu Val Glu Ser Gly Gly Val Val Gln Pro Gly Arg
1               5               10              15

Ser Leu Arg Leu Ser Cys Ala Ala Ser Gly Phe Thr Phe Ser Ser Tyr
            20              25              30

Gly Met His Trp Val Arg Gln Ala Pro Gly Lys Gly Leu Glu Trp Met
            35              40              45

Ala Ile Ile Trp Phe Asp Gly Ser Ser Lys Tyr Tyr Gly Asp Ser Val
        50              55              60

Lys Gly Arg Phe Thr Ile Ser Arg Asp Asn Ser Lys Asn Thr Leu Tyr
65              70              75              80

Leu Gln Met Asn Ser Leu Arg Ala Asp Asp Thr Ala Val Tyr Tyr Cys
            85              90              95

Ala Arg Glu Leu Gly Arg Arg Tyr Phe Asp Leu Trp Gly Arg Gly Thr
            100             105             110

Leu Val Thr Val Ser Ser Ala Ser Thr Lys Gly Pro Ser Val Phe Pro
            115             120             125

Leu Ala Pro Ser Ser Lys Ser Thr Ser Gly Gly Thr Ala Ala Leu Gly
        130             135             140

Cys Leu Val Lys Asp Tyr Phe Pro Glu Pro Val Thr Val Ser Trp Asn
145             150             155             160

Ser Gly Ala Leu Thr Ser Gly Val His Thr Phe Pro Ala Val Leu Gln
            165             170             175
```

Ser Ser Gly Leu Tyr Ser Leu Ser Ser Val Val Thr Val Pro Ser Ser
            180                     185                 190

Ser Leu Gly Thr Gln Thr Tyr Ile Cys Asn Val Asn His Lys Pro Ser
            195                     200                 205

Asn Thr Lys Val Asp Lys Lys Val Glu Pro Lys Ser Cys Asp Lys Thr
            210                     215                 220

His Thr Cys Pro Pro Cys Pro Ala Pro Glu Ala Ala Gly Gly Pro Ser
225                     230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                     265                 270

Glu Val Lys Phe Asn Trp Tyr Val Asp Gly Val Glu Val His Asn Ala
            275                     280                 285

Lys Thr Lys Pro Arg Glu Glu Gln Tyr Asn Ser Thr Tyr Arg Val Val
            290                     295                 300

Ser Val Leu Thr Val Leu His Gln Asp Trp Leu Asn Gly Lys Glu Tyr
305                     310                 315                 320

Lys Cys Lys Val Ser Asn Lys Ala Leu Gly Ala Pro Ile Glu Lys Thr
                325                 330                 335

Ile Ser Lys Ala Lys Gly Gln Pro Arg Glu Pro Gln Val Tyr Thr Leu
            340                     345                 350

Pro Pro Ser Arg Asp Glu Leu Thr Lys Asn Gln Val Ser Leu Thr Cys
            355                     360                 365

Leu Val Lys Gly Phe Tyr Pro Ser Asp Ile Ala Val Glu Trp Glu Ser
            370                     375                 380

Asn Gly Gln Pro Glu Asn Asn Tyr Lys Thr Thr Pro Pro Val Leu Asp
385                     390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
                420                 425                 430

```
Leu His Asn His Tyr Thr Gln Lys Ser Leu Ser Leu Ser Pro Gly Lys
        435             440             445
```

<210> 3
<211> 215
<212> PRT
<213> Homo sapiens

<400> 3

```
Glu Ile Val Leu Thr Gln Ser Pro Ala Thr Leu Ser Leu Ser Pro Gly
1               5               10              15

Glu Arg Ala Thr Leu Ser Cys Arg Ala Ser Gln Ser Val Ser Ser Tyr
                20              25              30

Leu Ala Trp Tyr Gln Gln Lys Pro Gly Gln Ala Pro Arg Leu Leu Ile
            35              40              45

Tyr Asp Ala Ser Lys Arg Ala Thr Gly Ile Pro Ala Arg Phe Ser Gly
        50              55              60

Ser Gly Ser Gly Thr Asp Phe Thr Leu Thr Ile Ser Ser Leu Glu Pro
65              70              75              80

Glu Asp Phe Ala Val Tyr Tyr Cys Gln Gln Arg Ser Lys Trp Pro Pro
                85              90              95

Trp Thr Phe Gly Gln Gly Thr Lys Val Glu Ser Lys Arg Thr Val Ala
            100             105             110

Ala Pro Ser Val Phe Ile Phe Pro Pro Ser Asp Glu Gln Leu Lys Ser
            115             120             125

Gly Thr Ala Ser Val Val Cys Leu Leu Asn Asn Phe Tyr Pro Arg Glu
            130             135             140

Ala Lys Val Gln Trp Lys Val Asp Asn Ala Leu Gln Ser Gly Asn Ser
145             150             155             160

Gln Glu Ser Val Thr Glu Gln Asp Ser Lys Asp Ser Thr Tyr Ser Leu
                165             170             175

Ser Ser Thr Leu Thr Leu Ser Lys Ala Asp Tyr Glu Lys His Lys Val
            180             185             190

Tyr Ala Cys Glu Val Thr His Gln Gly Leu Ser Ser Pro Val Thr Lys
            195             200             205
```

Ser Phe Asn Arg Gly Glu Cys
    210                 215


<210>   4
<211>   215
<212>   PRT
<213>   Homo sapiens

<400>   4

Glu Ile Val Leu Thr Gln Ser Pro Ala Thr Leu Ser Leu Ser Pro Gly
1               5               10              15

Glu Arg Ala Thr Leu Ser Cys Arg Ala Ser Gln Ser Val Ser Ser Tyr
            20              25              30

Leu Ala Trp Tyr Gln Gln Lys Pro Gly Gln Ala Pro Arg Leu Leu Ile
        35              40              45

Tyr Asp Ala Ser Asn Arg Ala Thr Gly Ile Pro Ala Arg Phe Ser Gly
    50              55              60

Ser Gly Ser Gly Thr Asp Phe Thr Leu Thr Ile Ser Ser Leu Glu Pro
65              70              75              80

Glu Asp Phe Ala Val Tyr Tyr Cys Gln Gln Arg Ser Lys Trp Pro Pro
            85              90              95

Trp Thr Phe Gly Gln Gly Thr Lys Val Glu Ile Lys Arg Thr Val Ala
            100             105             110

Ala Pro Ser Val Phe Ile Phe Pro Pro Ser Asp Glu Gln Leu Lys Ser
        115             120             125

Gly Thr Ala Ser Val Val Cys Leu Leu Asn Asn Phe Tyr Pro Arg Glu
    130             135             140

Ala Lys Val Gln Trp Lys Val Asp Asn Ala Leu Gln Ser Gly Asn Ser
145             150             155             160

Gln Glu Ser Val Thr Glu Gln Asp Ser Lys Asp Ser Thr Tyr Ser Leu
            165             170             175

Ser Ser Thr Leu Thr Leu Ser Lys Ala Asp Tyr Glu Lys His Lys Val
        180             185             190

Tyr Ala Cys Glu Val Thr His Gln Gly Leu Ser Ser Pro Val Thr Lys
        195             200             205

```
Ser Phe Asn Arg Gly Glu Cys
    210                 215


<210>  5
<211>  118
<212>  PRT
<213>  Homo sapiens

<400>  5

Gln Val Glu Leu Val Glu Ser Gly Gly Gly Val Val Gln Pro Gly Arg
1               5                   10                  15


Ser Gln Arg Leu Ser Cys Ala Ala Ser Gly Phe Thr Phe Ser Ser Tyr
            20                  25                  30


Gly Met His Trp Val Arg Gln Ala Pro Gly Lys Gly Leu Glu Trp Val
        35                  40                  45


Ala Ile Ile Trp Phe Asp Gly Ser Ser Thr Tyr Tyr Ala Asp Ser Val
    50                  55                  60


Arg Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Phe Cys
                85                  90                  95


Ala Arg Glu Leu Gly Arg Arg Tyr Phe Asp Leu Trp Gly Arg Gly Thr
            100                 105                 110


Leu Val Ser Val Ser Ser
            115


<210>  6
<211>  118
<212>  PRT
<213>  Homo sapiens

<400>  6

Gln Val Gln Leu Val Glu Ser Gly Gly Gly Val Val Gln Pro Gly Arg
1               5                   10                  15


Ser Leu Arg Leu Ser Cys Ala Ala Ser Gly Phe Thr Phe Ser Ser Tyr
            20                  25                  30


Gly Met His Trp Val Arg Gln Ala Pro Gly Lys Gly Leu Glu Trp Met
        35                  40                  45
```

93

```
Ala Ile Ile Trp Phe Asp Gly Ser Ser Lys Tyr Tyr Gly Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Asp Asp Thr Ala Val Tyr Tyr Cys
                85                  90                  95

Ala Arg Glu Leu Gly Arg Arg Tyr Phe Asp Leu Trp Gly Arg Gly Thr
            100                 105                 110

Leu Val Thr Val Ser Ser
            115


<210>  7
<211>  108
<212>  PRT
<213>  Homo sapiens

<400>  7

Glu Ile Val Leu Thr Gln Ser Pro Ala Thr Leu Ser Leu Ser Pro Gly
1                   5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
            35                  40                  45

Tyr Asp Ala Ser Lys Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Lys Trp Pro Pro
                85                  90                  95

Trp Thr Phe Gly Gln Gly Thr Lys Val Glu Ser Lys
            100                 105


<210>  8
<211>  108
<212>  PRT
<213>  Homo sapiens

<400>  8

Glu Ile Val Leu Thr Gln Ser Pro Ala Thr Leu Ser Leu Ser Pro Gly
```

```
         1                  5                       10                      15

         Glu Arg Ala Thr Leu Ser Cys Arg Ala Ser Gln Ser Val Ser Ser Tyr
                     20                  25                  30

         Leu Ala Trp Tyr Gln Gln Lys Pro Gly Gln Ala Pro Arg Leu Leu Ile
                     35                  40                  45

         Tyr Asp Ala Ser Asn Arg Ala Thr Gly Ile Pro Ala Arg Phe Ser Gly
             50                  55                  60

         Ser Gly Ser Gly Thr Asp Phe Thr Leu Thr Ile Ser Ser Leu Glu Pro
         65                  70                  75                  80

         Glu Asp Phe Ala Val Tyr Tyr Cys Gln Gln Arg Ser Lys Trp Pro Pro
                     85                  90                  95

         Trp Thr Phe Gly Gln Gly Thr Lys Val Glu Ile Lys
                     100                 105
```

```
         <210>   9
         <211>   107
         <212>   PRT
         <213>   Homo sapiens

         <400>   9

         Ala Pro Glu Leu Leu Gly Gly Pro Ser Val Phe Leu Phe Pro Pro Lys
         1                   5                   10                  15

         Pro Lys Asp Thr Leu Met Ile Ser Arg Thr Pro Glu Val Thr Cys Val
                     20                  25                  30

         Trp Asp Val Ser His Glu Asp Pro Glu Val Lys Phe Asn Trp Tyr Val
                     35                  40                  45

         Asp Gly Val Glu Val His Asn Ala Lys Thr Lys Pro Arg Glu Glu Gln
             50                  55                  60

         Glu Ser Thr Tyr Arg Trp Ser Val Leu Thr Val Leu His Gln Asp Trp
         65                  70                  75                  80

         Leu Asn Gly Lys Glu Tyr Lys Cys Lys Val Ser Asn Lys Ala Leu Pro
                     85                  90                  95

         Ala Pro Ile Glu Lys Thr Ile Ser Lys Ala Lys
                     100                 105
```

```
         <210>   10
```

<211> 106
<212> PRT
<213> Homo sapiens

<400> 10

Gly Gln Pro Arg Glu Pro Gln Val Tyr Thr Leu Pro Pro Ser Arg Asp
1               5                   10                  15

Glu Leu Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
    50                  55                  60

Phe Leu Tyr Ser Lys Leu Thr Val Asp Lys Ser Arg Trp Gln Gln Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Pro Gly
            100                 105

<210> 11
<211> 1337
<212> PRT
<213> Homo sapiens

<400> 11

Glu Ile Cys Gly Pro Gly Ile Asp Ile Arg Asn Asp Tyr Gln Gln Leu
1               5                   10                  15

Lys Arg Leu Glu Asn Cys Thr Val Ile Glu Gly Tyr Leu His Ile Leu
            20                  25                  30

Leu Ile Ser Lys Ala Glu Asp Tyr Arg Ser Tyr Arg Phe Pro Lys Leu
        35                  40                  45

Thr Val Ile Thr Glu Tyr Leu Leu Leu Phe Arg Val Ala Gly Leu Glu
        50                  55                  60

Ser Leu Gly Asp Leu Phe Pro Asn Leu Thr Val Ile Arg Gly Trp Lys
65                  70                  75                  80

Leu Phe Tyr Asn Tyr Ala Leu Val Ile Phe Glu Met Thr Asn Leu Lys
                85                  90                  95

Asp Ile Gly Leu Tyr Asn Leu Arg Asn Ile Thr Arg Gly Ala Ile Arg
        100                    105                110

Ile Glu Lys Asn Ala Asp Leu Cys Tyr Leu Ser Thr Val Asp Trp Ser
        115                    120                125

Leu Ile Leu Asp Ala Val Ser Asn Asn Tyr Ile Val Gly Asn Lys Pro
        130                    135                140

Pro Lys Glu Cys Gly Asp Leu Cys Pro Gly Thr Met Glu Glu Lys Pro
145                    150                155                160

Met Cys Glu Lys Thr Thr Ile Asn Asn Glu Tyr Asn Tyr Arg Cys Trp
                165                    170                175

Thr Thr Asn Arg Cys Gln Lys Met Cys Pro Ser Thr Cys Gly Lys Arg
            180                    185                190

Ala Cys Thr Glu Asn Asn Glu Cys Cys His Pro Glu Cys Leu Gly Ser
            195                    200                205

Cys Ser Ala Pro Asp Asn Asp Thr Ala Cys Val Ala Cys Arg His Tyr
    210                    215                220

Tyr Tyr Ala Gly Val Cys Val Pro Ala Cys Pro Pro Asn Thr Tyr Arg
225                    230                235                240

Phe Glu Gly Trp Arg Cys Val Asp Arg Asp Phe Cys Ala Asn Ile Leu
            245                    250                255

Ser Ala Glu Ser Ser Asp Ser Glu Gly Phe Val Ile His Asp Gly Glu
            260                    265                270

Cys Met Gln Glu Cys Pro Ser Gly Phe Ile Arg Asn Gly Ser Gln Ser
            275                    280                285

Met Tyr Cys Ile Pro Cys Glu Gly Pro Cys Pro Lys Val Cys Glu Glu
        290                    295                300

Glu Lys Lys Thr Lys Thr Ile Asp Ser Val Thr Ser Ala Gln Met Leu
305                    310                315                320

Gln Gly Cys Thr Ile Phe Lys Gly Asn Leu Leu Ile Asn Ile Arg Arg
            325                    330                335

Gly Asn Asn Ile Ala Ser Glu Leu Glu Asn Phe Met Gly Leu Ile Glu

```
                340                          345                          350

        Val Val Thr Gly Tyr Val Lys Ile Arg His Ser His Ala Leu Val Ser
                355                  360                  365

        Leu Ser Phe Leu Lys Asn Leu Arg Leu Ile Leu Gly Glu Glu Gln Leu
                370                  375                  380

        Glu Gly Asn Tyr Ser Phe Tyr Val Leu Asp Asn Gln Asn Leu Gln Gln
        385                  390                  395                  400

        Leu Trp Asp Trp Asp His Arg Asn Leu Thr Ile Lys Ala Gly Lys Met
                        405                  410                  415

        Tyr Phe Ala Phe Asn Pro Lys Leu Cys Val Ser Glu Ile Tyr Arg Met
                        420                  425                  430

        Glu Glu Val Thr Gly Thr Lys Gly Arg Gln Ser Lys Gly Asp Ile Asn
                435                  440                  445

        Thr Arg Asn Asn Gly Glu Arg Ala Ser Cys Glu Ser Asp Val Leu His
                450                  455                  460

        Phe Thr Ser Thr Thr Thr Ser Lys Asn Arg Ile Ile Ile Thr Trp His
        465                  470                  475                  480

        Arg Tyr Arg Pro Pro Asp Tyr Arg Asp Leu Ile Ser Phe Thr Val Tyr
                        485                  490                  495

        Tyr Lys Glu Ala Pro Phe Lys Asn Val Thr Glu Tyr Asp Gly Gln Asp
                        500                  505                  510

        Ala Cys Gly Ser Asn Ser Trp Asn Met Val Asp Val Asp Leu Pro Pro
                        515                  520                  525

        Asn Lys Asp Val Glu Pro Gly Ile Leu Leu His Gly Leu Lys Pro Trp
                530                  535                  540

        Thr Gln Tyr Ala Val Tyr Val Lys Ala Val Thr Leu Thr Met Val Glu
        545                  550                  555                  560

        Asn Asp His Ile Arg Gly Ala Lys Ser Glu Ile Leu Tyr Ile Arg Thr
                        565                  570                  575

        Asn Ala Ser Val Pro Ser Ile Pro Leu Asp Val Leu Ser Ala Ser Asn
                        580                  585                  590
```

Ser Ser Ser Gln Leu Ile Val Lys Trp Asn Pro Pro Ser Leu Pro Asn
        595                 600                 605

Gly Asn Leu Ser Tyr Tyr Ile Val Arg Trp Gln Arg Gln Pro Gln Asp
        610                 615                 620

Gly Tyr Leu Tyr Arg His Asn Tyr Cys Ser Lys Asp Lys Ile Pro Ile
625                     630                 635                 640

Arg Lys Tyr Ala Asp Gly Thr Ile Asp Ile Glu Glu Val Thr Glu Asn
                645                 650                 655

Pro Lys Thr Glu Val Cys Gly Gly Glu Lys Gly Pro Cys Cys Ala Cys
        660                 665                 670

Pro Lys Thr Glu Ala Glu Lys Gln Ala Glu Lys Glu Glu Ala Glu Tyr
        675                 680                 685

Arg Lys Val Phe Glu Asn Phe Leu His Asn Ser Ile Phe Val Pro Arg
        690                 695                 700

Pro Glu Arg Lys Arg Arg Asp Val Met Gln Val Ala Asn Thr Thr Met
705                     710                 715                 720

Ser Ser Arg Ser Arg Asn Thr Thr Ala Ala Asp Thr Tyr Asn Ile Thr
                725                 730                 735

Asp Pro Glu Glu Leu Glu Thr Glu Tyr Pro Phe Phe Glu Ser Arg Val
        740                 745                 750

Asp Asn Lys Glu Arg Thr Val Ile Ser Asn Leu Arg Pro Phe Thr Leu
        755                 760                 765

Tyr Arg Ile Asp Ile His Ser Cys Asn His Glu Ala Glu Lys Leu Gly
        770                 775                 780

Cys Ser Ala Ser Asn Phe Val Phe Ala Arg Thr Met Pro Ala Glu Gly
785                     790                 795                 800

Ala Asp Asp Ile Pro Gly Pro Val Thr Trp Glu Pro Arg Pro Glu Asn
                805                 810                 815

Ser Ile Phe Leu Lys Trp Pro Glu Pro Glu Asn Pro Asn Gly Leu Ile
        820                 825                 830

Leu Met Tyr Glu Ile Lys Tyr Gly Ser Gln Val Glu Asp Gln Arg Glu
        835                 840                 845

99

```
Cys Val Ser Arg Gln Glu Tyr Arg Lys Tyr Gly Gly Ala Lys Leu Asn
    850                 855             860

Arg Leu Asn Pro Gly Asn Tyr Thr Ala Arg Ile Gln Ala Thr Ser Leu
865                 870             875                 880

Ser Gly Asn Gly Ser Trp Thr Asp Pro Val Phe Phe Tyr Val Gln Ala
                885             890                 895

Lys Thr Gly Tyr Glu Asn Phe Ile His Leu Ile Ile Ala Leu Pro Val
            900             905                 910

Ala Val Leu Leu Ile Val Gly Gly Leu Val Ile Met Leu Tyr Val Phe
            915             920             925

His Arg Lys Arg Asn Asn Ser Arg Leu Gly Asn Gly Val Leu Tyr Ala
    930                 935             940

Ser Val Asn Pro Glu Tyr Phe Ser Ala Ala Asp Val Tyr Val Pro Asp
945                 950             955                 960

Glu Trp Glu Val Ala Arg Glu Lys Ile Thr Met Ser Arg Glu Leu Gly
            965             970                 975

Gln Gly Ser Phe Gly Met Val Tyr Glu Gly Val Ala Lys Gly Val Val
            980             985                 990

Lys Asp Glu Pro Glu Thr Arg Val  Ala Ile Lys Thr Val  Asn Glu Ala
            995             1000                1005

Ala Ser  Met Arg Glu Arg Ile  Glu Phe Leu Asn Glu  Ala Ser Val
    1010                1015                1020

Met Lys  Glu Phe Asn Cys His  His Val Val Arg Leu  Leu Gly Val
    1025                1030                1035

Val Ser  Gln Gly Gln Pro Thr  Leu Val Ile Met Glu  Leu Met Thr
    1040                1045                1050

Arg Gly  Asp Leu Lys Ser Tyr  Leu Arg Ser Leu Arg  Pro Glu Met
    1055                1060                1065

Glu Asn  Asn Pro Val Leu Ala  Pro Pro Ser Leu Ser  Lys Met Ile
    1070                1075                1080

Gln Met  Ala Gly Glu Ile Ala  Asp Gly Met Ala Tyr  Leu Asn Ala
    1085                1090                1095
```

```
Asn Lys Phe Val His Arg Asp  Leu Ala Ala Arg Asn  Cys Met Val
    1100            1105                1110

Ala Glu Asp Phe Thr Val Lys  Ile Gly Asp Phe Gly  Met Thr Arg
    1115            1120                1125

Asp Ile Tyr Glu Thr Asp Tyr  Tyr Arg Lys Gly Gly  Lys Gly Leu
    1130            1135                1140

Leu Pro Val Arg Trp Met Ser  Pro Glu Ser Leu Lys  Asp Gly Val
    1145            1150                1155

Phe Thr Thr Tyr Ser Asp Val  Trp Ser Phe Gly Val  Val Leu Trp
    1160            1165                1170

Glu Ile Ala Thr Leu Ala Glu  Gln Pro Tyr Gln Gly  Leu Ser Asn
    1175            1180                1185

Glu Gln Val Leu Arg Phe Val  Met Glu Gly Gly Leu  Leu Asp Lys
    1190            1195                1200

Pro Asp Asn Cys Pro Asp Met  Leu Phe Glu Leu Met  Arg Met Cys
    1205            1210                1215

Trp Gln Tyr Asn Pro Lys Met  Arg Pro Ser Phe Leu  Glu Ile Ile
    1220            1225                1230

Ser Ser Ile Lys Glu Glu Met  Glu Pro Gly Phe Arg  Glu Val Ser
    1235            1240                1245

Phe Tyr Tyr Ser Glu Glu Asn  Lys Leu Pro Glu Pro  Glu Glu Leu
    1250            1255                1260

Asp Leu Glu Pro Glu Asn Met  Glu Ser Val Pro Leu  Asp Pro Ser
    1265            1270                1275

Ala Ser Ser Ser Ser Leu Pro  Leu Pro Asp Arg His  Ser Gly His
    1280            1285                1290

Lys Ala Glu Asn Gly Pro Gly  Pro Gly Val Leu Val  Leu Arg Ala
    1295            1300                1305

Ser Phe Asp Glu Arg Gln Pro  Tyr Ala His Met Asn  Gly Gly Arg
    1310            1315                1320

Lys Asn Glu Arg Ala Leu Pro  Leu Pro Gln Ser Ser  Thr Cys
```

1325            1330           1335

```
<210>  12
<211>  330
<212>  PRT
<213>  Homo sapiens

<400>  12

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
            115                 120                 125

Lys Pro Lys Asp Thr Leu Met Ile Ser Arg Thr Pro Glu Val Thr Cys
            130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
                180                 185                 190

His Gln Asp Trp Leu Asn Gly Lys Glu Tyr Lys Cys Lys Val Ser Asn
                195                 200                 205

Lys Ala Leu Pro Ala Pro Ile Glu Lys Thr Ile Ser Lys Ala Lys Gly
```

```
          210                    215                    220

Gln Pro Arg Glu Pro Gln Val Tyr Thr Leu Pro Pro Ser Arg Asp Glu
225                 230                 235                 240


Leu Thr Lys Asn Gln Val Ser Leu Thr Cys Leu Val Lys Gly Phe Tyr
                245                 250                 255


Pro Ser Asp Ile Ala Val Glu Trp Glu Ser Asn Gly Gln Pro Glu Asn
                260                 265                 270


Asn Tyr Lys Thr Thr Pro Pro Val Leu Asp Ser Asp Gly Ser Phe Phe
                275                 280                 285


Leu Tyr Ser Lys Leu Thr Val Asp Lys Ser Arg Trp Gln Gln Gly Asn
                290                 295                 300


Val Phe Ser Cys Ser Val Met His Glu Ala Leu His Asn His Tyr Thr
305                 310                 315                 320


Gln Lys Ser Leu Ser Leu Ser Pro Gly Lys
                325                 330
```

<210> 13
<211> 327
<212> PRT
<213> Homo sapiens

<400> 13

```
Ala Ser Thr Lys Gly Pro Ser Val Phe Pro Leu Ala Pro Cys Ser Arg
1                   5                   10                  15


Ser Thr Ser Glu Ser Thr Ala Ala Leu Gly Cys Leu Val Lys Asp Tyr
                20                  25                  30


Phe Pro Glu Pro Val Thr Val Ser Trp Asn Ser Gly Ala Leu Thr Ser
                35                  40                  45


Gly Val His Thr Phe Pro Ala Val Leu Gln Ser Ser Gly Leu Tyr Ser
                50                  55                  60


Leu Ser Ser Val Val Thr Val Pro Ser Ser Ser Leu Gly Thr Lys Thr
65                  70                  75                  80


Tyr Thr Cys Asn Val Asp His Lys Pro Ser Asn Thr Lys Val Asp Lys
                85                  90                  95


Arg Val Glu Ser Lys Tyr Gly Pro Pro Cys Pro Ser Cys Pro Ala Pro
```

```
                100                    105                    110

    Glu Phe Leu Gly Gly Pro Ser Val Phe Leu Phe Pro Pro Lys Pro Lys
            115                 120             125

    Asp Thr Leu Met Ile Ser Arg Thr Pro Glu Val Thr Cys Val Val Val
            130                 135             140

    Asp Val Ser Gln Glu Asp Pro Glu Val Gln Phe Asn Trp Tyr Val Asp
    145                 150             155                 160

    Gly Val Glu Val His Asn Ala Lys Thr Lys Pro Arg Glu Glu Gln Phe
                165             170                 175

    Asn Ser Thr Tyr Arg Val Val Ser Val Leu Thr Val Leu His Gln Asp
            180                 185             190

    Trp Leu Asn Gly Lys Glu Tyr Lys Cys Lys Val Ser Asn Lys Gly Leu
            195                 200             205

    Pro Ser Ser Ile Glu Lys Thr Ile Ser Lys Ala Lys Gly Gln Pro Arg
    210                 215             220

    Glu Pro Gln Val Tyr Thr Leu Pro Pro Ser Gln Glu Glu Met Thr Lys
    225                 230             235                 240

    Asn Gln Val Ser Leu Thr Cys Leu Val Lys Gly Phe Tyr Pro Ser Asp
                245             250                 255

    Ile Ala Val Glu Trp Glu Ser Asn Gly Gln Pro Glu Asn Asn Tyr Lys
                260             265                 270

    Thr Thr Pro Pro Val Leu Asp Ser Asp Gly Ser Phe Phe Leu Tyr Ser
            275                 280             285

    Arg Leu Thr Val Asp Lys Ser Arg Trp Gln Glu Gly Asn Val Phe Ser
        290                 295             300

    Cys Ser Val Met His Glu Ala Leu His Asn His Tyr Thr Gln Lys Ser
    305                 310             315                 320

    Leu Ser Leu Ser Leu Gly Lys
                325


    <210>  14
    <211>  227
    <212>  PRT
    <213>  Homo sapiens
```

<400> 14

Asp Lys Thr His Thr Cys Pro Pro Cys Pro Ala Pro Glu Leu Leu Gly
1               5               10              15

Gly Pro Ser Val Phe Leu Phe Pro Pro Lys Pro Lys Asp Thr Leu Met
              20              25              30

Ile Ser Arg Thr Pro Glu Val Thr Cys Val Val Val Asp Val Ser His
              35              40              45

Glu Asp Pro Glu Val Lys Phe Asn Trp Tyr Val Asp Gly Val Glu Val
      50              55              60

His Asn Ala Lys Thr Lys Pro Arg Glu Glu Gln Tyr Asn Ser Thr Tyr
65              70              75              80

Arg Val Val Ser Val Leu Thr Val Leu His Gln Asp Trp Leu Asn Gly
              85              90              95

Lys Glu Tyr Lys Cys Lys Val Ser Asn Lys Ala Leu Pro Ala Pro Ile
          100             105             110

Glu Lys Thr Ile Ser Lys Ala Lys Gly Gln Pro Arg Glu Pro Gln Val
          115             120             125

Tyr Thr Leu Pro Pro Ser Arg Asp Glu Leu Thr Lys Asn Gln Val Ser
      130             135             140

Leu Thr Cys Leu Val Lys Gly Phe Tyr Pro Ser Asp Ile Ala Val Glu
145             150             155             160

Trp Glu Ser Asn Gly Gln Pro Glu Asn Asn Tyr Lys Thr Thr Pro Pro
              165             170             175

Val Leu Asp Ser Asp Gly Ser Phe Phe Leu Tyr Ser Lys Leu Thr Val
          180             185             190

Asp Lys Ser Arg Trp Gln Gln Gly Asn Val Phe Ser Cys Ser Val Met
          195             200             205

His Glu Ala Leu His Asn His Tyr Thr Gln Lys Ser Leu Ser Leu Ser
210             215             220

Pro Gly Lys
225

<210> 15
<211> 223
<212> PRT
<213> Homo sapiens

<400> 15

Val Glu Cys Pro Pro Cys Pro Ala Pro Pro Val Ala Gly Pro Ser Val
1               5                   10                  15

Phe Leu Phe Pro Pro Lys Pro Lys Asp Thr Leu Met Ile Ser Arg Thr
                20                  25                  30

Pro Glu Val Thr Cys Val Val Val Asp Val Ser His Glu Asp Pro Glu
            35                  40                  45

Val Gln Phe Asn Trp Tyr Val Asp Gly Val Glu Val His Asn Ala Lys
            50                  55                  60

Thr Lys Pro Arg Glu Glu Gln Phe Asn Ser Thr Phe Arg Val Val Ser
65                  70                  75                  80

Val Leu Thr Val Val His Gln Asp Trp Leu Asn Gly Lys Glu Tyr Lys
                85                  90                  95

Cys Lys Val Ser Asn Lys Gly Leu Pro Ala Pro Ile Glu Lys Thr Ile
                100                 105                 110

Ser Lys Thr Lys Gly Gln Pro Arg Glu Pro Gln Val Tyr Thr Leu Pro
            115                 120                 125

Pro Ser Arg Glu Glu Met Thr Lys Asn Gln Val Ser Leu Thr Cys Leu
            130                 135                 140

Val Lys Gly Phe Tyr Pro Ser Asp Ile Ser Val Glu Trp Glu Ser Asn
145                 150                 155                 160

Gly Gln Pro Glu Asn Asn Tyr Lys Thr Thr Pro Pro Met Leu Asp Ser
                165                 170                 175

Asp Gly Ser Phe Phe Leu Tyr Ser Lys Leu Thr Val Asp Lys Ser Arg
                180                 185                 190

Trp Gln Gln Gly Asn Val Phe Ser Cys Ser Val Met His Glu Ala Leu
            195                 200                 205

His Asn His Tyr Thr Gln Lys Ser Leu Ser Leu Ser Pro Gly Lys
    210                 215                 220

<210> 16
<211> 227
<212> PRT
<213> Homo sapiens

<400> 16

Asp Thr Pro Pro Pro Cys Pro Arg Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Gln Phe Lys Trp Tyr Val Asp Gly Val Glu Val
        50                  55                  60

His Asn Ala Lys Thr Lys Pro Arg Glu Glu Gln Tyr Asn Ser Thr Phe
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Thr Lys Gly Gln Pro Arg Glu Pro Gln Val
            115                 120                 125

Tyr Thr Leu Pro Pro Ser Arg Glu Glu Met Thr Lys Asn Gln Val Ser
        130                 135                 140

Leu Thr Cys Leu Val Lys Gly Phe Tyr Pro Ser Asp Ile Ala Val Glu
145                 150                 155                 160

Trp Glu Ser Ser Gly Gln Pro Glu Asn Asn Tyr Asn Thr Thr Pro Pro
                165                 170                 175

Met Leu Asp Ser Asp Gly Ser Phe Phe Leu Tyr Ser Lys Leu Thr Val
                180                 185                 190

Asp Lys Ser Arg Trp Gln Gln Gly Asn Ile Phe Ser Cys Ser Val Met
            195                 200                 205

His Glu Ala Leu His Asn Arg Phe Thr Gln Lys Ser Leu Ser Leu Ser
        210                 215                 220

Pro Gly Lys
225

<210> 17
<211> 229
<212> PRT
<213> Homo sapiens

<400> 17

Glu Ser Lys Tyr Gly Pro Pro Cys Pro Ser Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
            35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
            115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
            195                 200                 205

```
Val Met His Glu Ala Leu His Asn His Tyr Thr Gln Lys Ser Leu Ser
    210                 215                 220

Leu Ser Leu Gly Lys
225


<210>  18
<211>  227
<212>  PRT
<213>  Artificial Sequence

<220>
<223>  human IgG1 Fc-region derived Fc-region polypeptide with the
       mutations L234A, L235A

<400>  18

Asp Lys Thr His Thr Cys Pro Pro Cys Pro Ala Pro Glu Ala Ala Gly
1                 5                 10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
            35                  40                  45

Glu Asp Pro Glu Val Lys Phe Asn Trp Tyr Val Asp Gly Val Glu Val
        50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
            115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175
```

```
Val Leu Asp Ser Asp Gly Ser Phe Phe Leu Tyr Ser Lys Leu Thr Val
            180                 185                 190


Asp Lys Ser Arg Trp Gln Gln Gly Asn Val Phe Ser Cys Ser Val Met
            195                 200                 205


His Glu Ala Leu His Asn His Tyr Thr Gln Lys Ser Leu Ser Leu Ser
    210                 215                 220


Pro Gly Lys
225



<210> 19
<211> 227
<212> PRT
<213> Artificial Sequence

<220>
<223> human IgG1 Fc-region derived Fc-region polypeptide with Y349C,
      T366S, L368A and Y407V mutations

<400> 19

Asp Lys Thr His Thr Cys Pro Pro Cys Pro Ala Pro Glu Leu Leu Gly
1               5                 10                  15


Gly Pro Ser Val Phe Leu Phe Pro Pro Lys Pro Lys Asp Thr Leu Met
            20                  25                  30


Ile Ser Arg Thr Pro Glu Val Thr Cys Val Val Val Asp Val Ser His
            35                  40                  45


Glu Asp Pro Glu Val Lys Phe Asn Trp Tyr Val Asp Gly Val Glu Val
    50                  55                  60


His Asn Ala Lys Thr Lys Pro Arg Glu Glu Gln Tyr Asn Ser Thr Tyr
65                  70                  75                  80


Arg Val Val Ser Val Leu Thr Val Leu His Gln Asp Trp Leu Asn Gly
            85                  90                  95


Lys Glu Tyr Lys Cys Lys Val Ser Asn Lys Ala Leu Pro Ala Pro Ile
            100                 105                 110


Glu Lys Thr Ile Ser Lys Ala Lys Gly Gln Pro Arg Glu Pro Gln Val
            115                 120                 125


Cys Thr Leu Pro Pro Ser Arg Asp Glu Leu Thr Lys Asn Gln Val Ser
    130                 135                 140
```

```
Leu Ser Cys Ala Val Lys Gly Phe Tyr Pro Ser Asp Ile Ala Val Glu
145             150             155             160

Trp Glu Ser Asn Gly Gln Pro Glu Asn Asn Tyr Lys Thr Thr Pro Pro
                165             170             175

Val Leu Asp Ser Asp Gly Ser Phe Phe Leu Val Ser Lys Leu Thr Val
                180             185             190

Asp Lys Ser Arg Trp Gln Gln Gly Asn Val Phe Ser Cys Ser Val Met
            195             200             205

His Glu Ala Leu His Asn His Tyr Thr Gln Lys Ser Leu Ser Leu Ser
    210             215             220

Pro Gly Lys
225
```

```
<210>  20
<211>  227
<212>  PRT
<213>  Artificial Sequence

<220>
<223>  human IgG1 Fc-region derived Fc-region polypeptide with S354C,
       T366W mutations

<400>  20
```

```
Asp Lys Thr His Thr Cys Pro Pro Cys Pro Ala Pro Glu Leu Leu Gly
1               5               10              15

Gly Pro Ser Val Phe Leu Phe Pro Pro Lys Pro Lys Asp Thr Leu Met
                20              25              30

Ile Ser Arg Thr Pro Glu Val Thr Cys Val Val Val Asp Val Ser His
            35              40              45

Glu Asp Pro Glu Val Lys Phe Asn Trp Tyr Val Asp Gly Val Glu Val
        50              55              60

His Asn Ala Lys Thr Lys Pro Arg Glu Glu Gln Tyr Asn Ser Thr Tyr
65              70              75              80

Arg Val Val Ser Val Leu Thr Val Leu His Gln Asp Trp Leu Asn Gly
                85              90              95

Lys Glu Tyr Lys Cys Lys Val Ser Asn Lys Ala Leu Pro Ala Pro Ile
                100             105             110
```

111

```
Glu Lys Thr Ile Ser Lys Ala Lys Gly Gln Pro Arg Glu Pro Gln Val
        115                 120                 125


Tyr Thr Leu Pro Pro Cys Arg Asp Glu Leu Thr Lys Asn Gln Val Ser
    130                 135                 140


Leu Trp Cys Leu Val Lys Gly Phe Tyr Pro Ser Asp Ile Ala Val Glu
145                 150                 155                 160


Trp Glu Ser Asn Gly Gln Pro Glu Asn Asn Tyr Lys Thr Thr Pro Pro
                165                 170                 175


Val Leu Asp Ser Asp Gly Ser Phe Phe Leu Tyr Ser Lys Leu Thr Val
            180                 185                 190


Asp Lys Ser Arg Trp Gln Gln Gly Asn Val Phe Ser Cys Ser Val Met
        195                 200                 205


His Glu Ala Leu His Asn His Tyr Thr Gln Lys Ser Leu Ser Leu Ser
    210                 215                 220


Pro Gly Lys
225


<210>  21
<211>  227
<212>  PRT
<213>  Artificial Sequence

<220>
<223>  human IgG1 Fc-region derived Fc-region polypeptide with L234A,
       L235A mutations and Y349C, T366S, L368A, Y407V mutations

<400>  21

Asp Lys Thr His Thr Cys Pro Pro Cys Pro Ala Pro Glu Ala Ala Gly
1               5                   10                  15


Gly Pro Ser Val Phe Leu Phe Pro Pro Lys Pro Lys Asp Thr Leu Met
            20                  25                  30


Ile Ser Arg Thr Pro Glu Val Thr Cys Val Val Val Asp Val Ser His
        35                  40                  45


Glu Asp Pro Glu Val Lys Phe Asn Trp Tyr Val Asp Gly Val Glu Val
    50                  55                  60


His Asn Ala Lys Thr Lys Pro Arg Glu Glu Gln Tyr Asn Ser Thr Tyr
65                  70                  75                  80
```

112

```
Arg Val Val Ser Val Leu Thr Val Leu His Gln Asp Trp Leu Asn Gly
              85                  90                  95


Lys Glu Tyr Lys Cys Lys Val Ser Asn Lys Ala Leu Pro Ala Pro Ile
             100                 105                 110


Glu Lys Thr Ile Ser Lys Ala Lys Gly Gln Pro Arg Glu Pro Gln Val
         115                 120                 125


Cys Thr Leu Pro Pro Ser Arg Asp Glu Leu Thr Lys Asn Gln Val Ser
     130                 135                 140


Leu Ser Cys Ala Val Lys Gly Phe Tyr Pro Ser Asp Ile Ala Val Glu
145                 150                 155                 160


Trp Glu Ser Asn Gly Gln Pro Glu Asn Asn Tyr Lys Thr Thr Pro Pro
             165                 170                 175


Val Leu Asp Ser Asp Gly Ser Phe Phe Leu Val Ser Lys Leu Thr Val
             180                 185                 190


Asp Lys Ser Arg Trp Gln Gln Gly Asn Val Phe Ser Cys Ser Val Met
         195                 200                 205


His Glu Ala Leu His Asn His Tyr Thr Gln Lys Ser Leu Ser Leu Ser
     210                 215                 220


Pro Gly Lys
225


<210>   22
<211>   227
<212>   PRT
<213>   Artificial Sequence

<220>
<223>   human IgG1 Fc-region derived Fc-region polypeptide with a L234A,
        L235A and S354C, T366W mutations

<400>   22

Asp Lys Thr His Thr Cys Pro Pro Cys Pro Ala Pro Glu Ala Ala Gly
1               5                   10                  15


Gly Pro Ser Val Phe Leu Phe Pro Pro Lys Pro Lys Asp Thr Leu Met
             20                  25                  30


Ile Ser Arg Thr Pro Glu Val Thr Cys Val Val Val Asp Val Ser His
         35                  40                  45
```

```
Glu Asp Pro Glu Val Lys Phe Asn Trp Tyr Val Asp Gly Val Glu Val
    50              55              60

His Asn Ala Lys Thr Lys Pro Arg Glu Glu Gln Tyr Asn Ser Thr Tyr
65              70              75              80

Arg Val Val Ser Val Leu Thr Val Leu His Gln Asp Trp Leu Asn Gly
            85              90              95

Lys Glu Tyr Lys Cys Lys Val Ser Asn Lys Ala Leu Pro Ala Pro Ile
            100             105             110

Glu Lys Thr Ile Ser Lys Ala Lys Gly Gln Pro Arg Glu Pro Gln Val
        115             120             125

Tyr Thr Leu Pro Pro Cys Arg Asp Glu Leu Thr Lys Asn Gln Val Ser
    130             135             140

Leu Trp Cys Leu Val Lys Gly Phe Tyr Pro Ser Asp Ile Ala Val Glu
145             150             155             160

Trp Glu Ser Asn Gly Gln Pro Glu Asn Asn Tyr Lys Thr Thr Pro Pro
            165             170             175

Val Leu Asp Ser Asp Gly Ser Phe Phe Leu Tyr Ser Lys Leu Thr Val
            180             185             190

Asp Lys Ser Arg Trp Gln Gln Gly Asn Val Phe Ser Cys Ser Val Met
        195             200             205

His Glu Ala Leu His Asn His Tyr Thr Gln Lys Ser Leu Ser Leu Ser
    210             215             220

Pro Gly Lys
225
```

<210> 23
<211> 227
<212> PRT
<213> Artificial Sequence

<220>
<223> human IgG1 Fc-region derived Fc-region polypeptide with a P329G
      mutation

<400> 23

```
Asp Lys Thr His Thr Cys Pro Pro Cys Pro Ala Pro Glu Leu Leu Gly
1               5               10              15
```

```
Gly Pro Ser Val Phe Leu Phe Pro Pro Lys Pro Lys Asp Thr Leu Met
            20                  25              30

Ile Ser Arg Thr Pro Glu Val Thr Cys Val Val Val Asp Val Ser His
            35              40              45

Glu Asp Pro Glu Val Lys Phe Asn Trp Tyr Val Asp Gly Val Glu Val
        50                  55              60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Gly Ala Pro Ile
            100                 105                 110

Glu Lys Thr Ile Ser Lys Ala Lys Gly Gln Pro Arg Glu Pro Gln Val
            115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
            195                 200                 205

His Glu Ala Leu His Asn His Tyr Thr Gln Lys Ser Leu Ser Leu Ser
    210                 215                 220

Pro Gly Lys
225
```

```
<210>   24
<211>   227
<212>   PRT
<213>   Artificial Sequence

<220>
<223>   human IgG1 Fc-region derived Fc-region polypeptide with L234A,
```

L235A mutations and P329G mutation

<400>    24

Asp Lys Thr His Thr Cys Pro Pro Cys Pro Ala Pro Glu Ala Ala Gly
1                   5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
            35                  40                  45

Glu Asp Pro Glu Val Lys Phe Asn Trp Tyr Val Asp Gly Val Glu Val
        50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
            85                  90                  95

Lys Glu Tyr Lys Cys Lys Val Ser Asn Lys Ala Leu Gly Ala Pro Ile
            100                 105                 110

Glu Lys Thr Ile Ser Lys Ala Lys Gly Gln Pro Arg Glu Pro Gln Val
            115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
            165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
            195                 200                 205

His Glu Ala Leu His Asn His Tyr Thr Gln Lys Ser Leu Ser Leu Ser
    210                 215                 220

Pro Gly Lys
225

<210> 25
<211> 227
<212> PRT
<213> Artificial Sequence

<220>
<223> human IgG1 Fc-region derived Fc-region polypeptide with a P239G
mutation and Y349C, T366S, L368A, Y407V mutations

<400> 25

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Gly Ala Pro Ile
            100                 105                 110

Glu Lys Thr Ile Ser Lys Ala Lys Gly Gln Pro Arg Glu Pro Gln Val
        115                 120                 125

Cys Thr Leu Pro Pro Ser Arg Asp Glu Leu Thr Lys Asn Gln Val Ser
    130                 135                 140

Leu Ser Cys Ala Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Val Ser Lys Leu Thr Val
            180                 185                 190

Asp Lys Ser Arg Trp Gln Gln Gly Asn Val Phe Ser Cys Ser Val Met
            195                 200                 205

```
His Glu Ala Leu His Asn His Tyr Thr Gln Lys Ser Leu Ser Leu Ser
    210             215             220

Pro Gly Lys
225


<210>  26
<211>  227
<212>  PRT
<213>  Artificial Sequence

<220>
<223>  human IgG1 Fc-region derived Fc-region polypeptide with a P329G
       mutation and S354C, T366W mutation

<400>  26

Asp Lys Thr His Thr Cys Pro Pro Cys Pro Ala Pro Glu Leu Leu Gly
1               5               10              15

Gly Pro Ser Val Phe Leu Phe Pro Pro Lys Pro Lys Asp Thr Leu Met
            20              25              30

Ile Ser Arg Thr Pro Glu Val Thr Cys Val Val Val Asp Val Ser His
            35              40              45

Glu Asp Pro Glu Val Lys Phe Asn Trp Tyr Val Asp Gly Val Glu Val
        50              55              60

His Asn Ala Lys Thr Lys Pro Arg Glu Glu Gln Tyr Asn Ser Thr Tyr
65              70              75              80

Arg Val Val Ser Val Leu Thr Val Leu His Gln Asp Trp Leu Asn Gly
            85              90              95

Lys Glu Tyr Lys Cys Lys Val Ser Asn Lys Ala Leu Gly Ala Pro Ile
            100             105             110

Glu Lys Thr Ile Ser Lys Ala Lys Gly Gln Pro Arg Glu Pro Gln Val
        115             120             125

Tyr Thr Leu Pro Pro Cys Arg Asp Glu Leu Thr Lys Asn Gln Val Ser
    130             135             140

Leu Trp Cys Leu Val Lys Gly Phe Tyr Pro Ser Asp Ile Ala Val Glu
145             150             155             160

Trp Glu Ser Asn Gly Gln Pro Glu Asn Asn Tyr Lys Thr Thr Pro Pro
                165             170             175
```

```
Val Leu Asp Ser Asp Gly Ser Phe Phe Leu Tyr Ser Lys Leu Thr Val
        180             185             190

Asp Lys Ser Arg Trp Gln Gln Gly Asn Val Phe Ser Cys Ser Val Met
        195             200             205

His Glu Ala Leu His Asn His Tyr Thr Gln Lys Ser Leu Ser Leu Ser
    210             215             220

Pro Gly Lys
225
```

<210> 27
<211> 227
<212> PRT
<213> Artificial Sequence

<220>
<223> human IgG1 Fc-region derived Fc-region polypeptide with L234A,
      L235A, P329G and Y349C, T366S, L368A, Y407V mutations

<400> 27

```
Asp Lys Thr His Thr Cys Pro Pro Cys Pro Ala Pro Glu Ala Ala Gly
1               5               10              15

Gly Pro Ser Val Phe Leu Phe Pro Pro Lys Pro Lys Asp Thr Leu Met
        20              25              30

Ile Ser Arg Thr Pro Glu Val Thr Cys Val Val Val Asp Val Ser His
        35              40              45

Glu Asp Pro Glu Val Lys Phe Asn Trp Tyr Val Asp Gly Val Glu Val
    50              55              60

His Asn Ala Lys Thr Lys Pro Arg Glu Glu Gln Tyr Asn Ser Thr Tyr
65              70              75              80

Arg Val Val Ser Val Leu Thr Val Leu His Gln Asp Trp Leu Asn Gly
            85              90              95

Lys Glu Tyr Lys Cys Lys Val Ser Asn Lys Ala Leu Gly Ala Pro Ile
        100             105             110

Glu Lys Thr Ile Ser Lys Ala Lys Gly Gln Pro Arg Glu Pro Gln Val
        115             120             125

Cys Thr Leu Pro Pro Ser Arg Asp Glu Leu Thr Lys Asn Gln Val Ser
    130             135             140
```

```
Leu Ser Cys Ala Val Lys Gly Phe Tyr Pro Ser Asp Ile Ala Val Glu
145             150             155             160

Trp Glu Ser Asn Gly Gln Pro Glu Asn Asn Tyr Lys Thr Thr Pro Pro
            165             170             175

Val Leu Asp Ser Asp Gly Ser Phe Phe Leu Val Ser Lys Leu Thr Val
            180             185             190

Asp Lys Ser Arg Trp Gln Gln Gly Asn Val Phe Ser Cys Ser Val Met
            195             200             205

His Glu Ala Leu His Asn His Tyr Thr Gln Lys Ser Leu Ser Leu Ser
    210             215             220

Pro Gly Lys
225


<210>  28
<211>  227
<212>  PRT
<213>  Artificial Sequence

<220>
<223>  human IgG1 Fc-region derived Fc-region polypeptide with L234A,
       L235A, P329G mutations and S354C, T366W mutations

<400>  28

Asp Lys Thr His Thr Cys Pro Pro Cys Pro Ala Pro Glu Ala Ala Gly
1               5               10              15

Gly Pro Ser Val Phe Leu Phe Pro Pro Lys Pro Lys Asp Thr Leu Met
            20              25              30

Ile Ser Arg Thr Pro Glu Val Thr Cys Val Val Val Asp Val Ser His
            35              40              45

Glu Asp Pro Glu Val Lys Phe Asn Trp Tyr Val Asp Gly Val Glu Val
    50              55              60

His Asn Ala Lys Thr Lys Pro Arg Glu Glu Gln Tyr Asn Ser Thr Tyr
65              70              75              80

Arg Val Val Ser Val Leu Thr Val Leu His Gln Asp Trp Leu Asn Gly
            85              90              95

Lys Glu Tyr Lys Cys Lys Val Ser Asn Lys Ala Leu Gly Ala Pro Ile
            100             105             110
```

Glu Lys Thr Ile Ser Lys Ala Lys Gly Gln Pro Arg Glu Pro Gln Val
        115                 120                 125

Tyr Thr Leu Pro Pro Cys Arg Asp Glu Leu Thr Lys Asn Gln Val Ser
        130                 135                 140

Leu Trp Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
                180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
        210                 215                 220

Pro Gly Lys
225

<210>  29
<211>  229
<212>  PRT
<213>  Artificial Sequence

<220>
<223>  human IgG4 Fc-region derived Fc-region polypeptide with S228P and
       L235E mutations

<400>  29

Glu Ser Lys Tyr Gly Pro Pro Cys Pro Pro Cys Pro Ala Pro Glu Phe
1               5               10              15

Glu Gly Gly Pro Ser Val Phe Leu Phe Pro Pro Lys Pro Lys Asp Thr
                20              25              30

Leu Met Ile Ser Arg Thr Pro Glu Val Thr Cys Val Val Val Asp Val
        35              40              45

Ser Gln Glu Asp Pro Glu Val Gln Phe Asn Trp Tyr Val Asp Gly Val
        50              55              60

Glu Val His Asn Ala Lys Thr Lys Pro Arg Glu Glu Gln Phe Asn Ser
65              70              75              80

```
Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225
```

<210> 30
<211> 229
<212> PRT
<213> Artificial Sequence

<220>
<223> human IgG4 Fc-region derived Fc-region polypeptide with S228P,
      L235E mutations and P329G mutation

<400> 30

```
Glu Ser Lys Tyr Gly Pro Pro Cys Pro Pro Cys Pro Ala Pro Glu Phe
1               5                   10                  15

Glu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45
```

122

```
Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
            85                  90                  95

Asn Gly Lys Glu Tyr Lys Cys Lys Val Ser Asn Lys Gly Leu Gly Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
            165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225
```

<210> 31
<211> 229
<212> PRT
<213> Artificial Sequence

<220>
<223> human IgG4 Fc-region derived Fc-region polypeptide with S354C,
      T366W mutations

<400> 31

```
Glu Ser Lys Tyr Gly Pro Pro Cys Pro Ser Cys Pro Ala Pro Glu Phe
1                   5                   10                  15
```

```
Leu Gly Gly Pro Ser Val Phe Leu Phe Pro Pro Lys Pro Lys Asp Thr
            20              25              30

Leu Met Ile Ser Arg Thr Pro Glu Val Thr Cys Val Val Val Asp Val
        35              40              45

Ser Gln Glu Asp Pro Glu Val Gln Phe Asn Trp Tyr Val Asp Gly Val
    50              55              60

Glu Val His Asn Ala Lys Thr Lys Pro Arg Glu Glu Gln Phe Asn Ser
65              70              75              80

Thr Tyr Arg Val Val Ser Val Leu Thr Val Leu His Gln Asp Trp Leu
            85              90              95

Asn Gly Lys Glu Tyr Lys Cys Lys Val Ser Asn Lys Gly Leu Pro Ser
        100             105             110

Ser Ile Glu Lys Thr Ile Ser Lys Ala Lys Gly Gln Pro Arg Glu Pro
    115             120             125

Gln Val Tyr Thr Leu Pro Pro Cys Gln Glu Glu Met Thr Lys Asn Gln
130             135             140

Val Ser Leu Trp Cys Leu Val Lys Gly Phe Tyr Pro Ser Asp Ile Ala
145             150             155             160

Val Glu Trp Glu Ser Asn Gly Gln Pro Glu Asn Asn Tyr Lys Thr Thr
            165             170             175

Pro Pro Val Leu Asp Ser Asp Gly Ser Phe Phe Leu Tyr Ser Arg Leu
        180             185             190

Thr Val Asp Lys Ser Arg Trp Gln Glu Gly Asn Val Phe Ser Cys Ser
    195             200             205

Val Met His Glu Ala Leu His Asn His Tyr Thr Gln Lys Ser Leu Ser
    210             215             220

Leu Ser Leu Gly Lys
225
```

```
<210>  32
<211>  229
<212>  PRT
<213>  Artificial Sequence

<220>
```

<223>   human IgG4 Fc-region derived Fc-region polypeptide with Y349C, T366S, L368A, Y407V mutations

<400>   32

Glu Ser Lys Tyr Gly Pro Pro Cys Pro Ser Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
            85                  90                  95

Asn Gly Lys Glu Tyr Lys Cys Lys Val Ser Asn Lys Gly Leu Pro Ser
        100                 105                 110

Ser Ile Glu Lys Thr Ile Ser Lys Ala Lys Gly Gln Pro Arg Glu Pro
        115                 120                 125

Gln Val Cys Thr Leu Pro Pro Ser Gln Glu Glu Met Thr Lys Asn Gln
    130                 135                 140

Val Ser Leu Ser Cys Ala Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
            165                 170                 175

Pro Pro Val Leu Asp Ser Asp Gly Ser Phe Phe Leu Val Ser Arg Leu
        180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225

<210> 33
<211> 229
<212> PRT
<213> Artificial Sequence

<220>
<223> human IgG4 Fc-region derived Fc-region polypeptide with a S228P,
L235E and S354C, T366W mutations

<400> 33

Glu Ser Lys Tyr Gly Pro Pro Cys Pro Pro Cys Pro Ala Pro Glu Phe
1               5               10              15

Glu Gly Gly Pro Ser Val Phe Leu Phe Pro Pro Lys Pro Lys Asp Thr
            20              25              30

Leu Met Ile Ser Arg Thr Pro Glu Val Thr Cys Val Val Val Asp Val
            35              40              45

Ser Gln Glu Asp Pro Glu Val Gln Phe Asn Trp Tyr Val Asp Gly Val
    50              55              60

Glu Val His Asn Ala Lys Thr Lys Pro Arg Glu Glu Gln Phe Asn Ser
65              70              75              80

Thr Tyr Arg Val Val Ser Val Leu Thr Val Leu His Gln Asp Trp Leu
            85              90              95

Asn Gly Lys Glu Tyr Lys Cys Lys Val Ser Asn Lys Gly Leu Pro Ser
            100             105             110

Ser Ile Glu Lys Thr Ile Ser Lys Ala Lys Gly Gln Pro Arg Glu Pro
            115             120             125

Gln Val Tyr Thr Leu Pro Pro Cys Gln Glu Glu Met Thr Lys Asn Gln
    130             135             140

Val Ser Leu Trp Cys Leu Val Lys Gly Phe Tyr Pro Ser Asp Ile Ala
145             150             155             160

Val Glu Trp Glu Ser Asn Gly Gln Pro Glu Asn Asn Tyr Lys Thr Thr
            165             170             175

Pro Pro Val Leu Asp Ser Asp Gly Ser Phe Phe Leu Tyr Ser Arg Leu
            180             185             190

Thr Val Asp Lys Ser Arg Trp Gln Glu Gly Asn Val Phe Ser Cys Ser
            195             200             205

```
Val Met His Glu Ala Leu His Asn His Tyr Thr Gln Lys Ser Leu Ser
    210                 215             220

Leu Ser Leu Gly Lys
225


<210>  34
<211>  229
<212>  PRT
<213>  Artificial Sequence

<220>
<223>  human IgG4 Fc-region derived Fc-region polypeptide with a S228P,
       L235E and Y349C, T366S, L368A, Y407V mutations

<400>  34

Glu Ser Lys Tyr Gly Pro Pro Cys Pro Pro Cys Pro Ala Pro Glu Phe
1                   5               10              15

Glu Gly Gly Pro Ser Val Phe Leu Phe Pro Pro Lys Pro Lys Asp Thr
                20              25              30

Leu Met Ile Ser Arg Thr Pro Glu Val Thr Cys Val Val Val Asp Val
            35              40              45

Ser Gln Glu Asp Pro Glu Val Gln Phe Asn Trp Tyr Val Asp Gly Val
    50              55              60

Glu Val His Asn Ala Lys Thr Lys Pro Arg Glu Glu Gln Phe Asn Ser
65              70              75              80

Thr Tyr Arg Val Val Ser Val Leu Thr Val Leu His Gln Asp Trp Leu
            85              90              95

Asn Gly Lys Glu Tyr Lys Cys Lys Val Ser Asn Lys Gly Leu Pro Ser
            100             105             110

Ser Ile Glu Lys Thr Ile Ser Lys Ala Lys Gly Gln Pro Arg Glu Pro
        115             120             125

Gln Val Cys Thr Leu Pro Pro Ser Gln Glu Glu Met Thr Lys Asn Gln
    130             135             140

Val Ser Leu Ser Cys Ala Val Lys Gly Phe Tyr Pro Ser Asp Ile Ala
145             150             155             160

Val Glu Trp Glu Ser Asn Gly Gln Pro Glu Asn Asn Tyr Lys Thr Thr
                165             170             175
```

```
        Pro Pro Val Leu Asp Ser Asp Gly Ser Phe Phe Leu Val Ser Arg Leu
                    180                 185                 190


        Thr Val Asp Lys Ser Arg Trp Gln Glu Gly Asn Val Phe Ser Cys Ser
                    195                 200                 205


        Val Met His Glu Ala Leu His Asn His Tyr Thr Gln Lys Ser Leu Ser
                    210                 215                 220


        Leu Ser Leu Gly Lys
        225
```

```
<210>  35
<211>  229
<212>  PRT
<213>  Artificial Sequence

<220>
<223>  human IgG4 Fc-region derived Fc-region polypeptide with a P329G
       mutation

<400>  35
```

```
        Glu Ser Lys Tyr Gly Pro Pro Cys Pro Ser Cys Pro Ala Pro Glu Phe
        1                   5                   10                  15


        Leu Gly Gly Pro Ser Val Phe Leu Phe Pro Pro Lys Pro Lys Asp Thr
                    20                  25                  30


        Leu Met Ile Ser Arg Thr Pro Glu Val Thr Cys Val Val Val Asp Val
                    35                  40                  45


        Ser Gln Glu Asp Pro Glu Val Gln Phe Asn Trp Tyr Val Asp Gly Val
                50                  55                  60


        Glu Val His Asn Ala Lys Thr Lys Pro Arg Glu Glu Gln Phe Asn Ser
        65                  70                  75                  80


        Thr Tyr Arg Val Val Ser Val Leu Thr Val Leu His Gln Asp Trp Leu
                        85                  90                  95


        Asn Gly Lys Glu Tyr Lys Cys Lys Val Ser Asn Lys Gly Leu Gly Ser
                    100                 105                 110


        Ser Ile Glu Lys Thr Ile Ser Lys Ala Lys Gly Gln Pro Arg Glu Pro
                    115                 120                 125


        Gln Val Tyr Thr Leu Pro Pro Ser Gln Glu Glu Met Thr Lys Asn Gln
                130                 135                 140
```

128

```
Val Ser Leu Thr Cys Leu Val Lys Gly Phe Tyr Pro Ser Asp Ile Ala
145                 150             155                 160


Val Glu Trp Glu Ser Asn Gly Gln Pro Glu Asn Asn Tyr Lys Thr Thr
                165             170                 175


Pro Pro Val Leu Asp Ser Asp Gly Ser Phe Phe Leu Tyr Ser Arg Leu
            180             185                 190


Thr Val Asp Lys Ser Arg Trp Gln Glu Gly Asn Val Phe Ser Cys Ser
        195             200                 205


Val Met His Glu Ala Leu His Asn His Tyr Thr Gln Lys Ser Leu Ser
    210             215                 220


Leu Ser Leu Gly Lys
225
```

```
<210>  36
<211>  229
<212>  PRT
<213>  Artificial Sequence

<220>
<223>  human IgG4 Fc-region derived Fc-region polypeptide with a P239G
       and Y349C, T366S, L368A, Y407V mutations

<400>  36
```

```
Glu Ser Lys Tyr Gly Pro Pro Cys Pro Ser Cys Pro Ala Pro Glu Phe
1               5               10              15


Leu Gly Gly Pro Ser Val Phe Leu Phe Pro Pro Lys Pro Lys Asp Thr
            20              25              30


Leu Met Ile Ser Arg Thr Pro Glu Val Thr Cys Val Val Val Asp Val
        35              40              45


Ser Gln Glu Asp Pro Glu Val Gln Phe Asn Trp Tyr Val Asp Gly Val
    50              55              60


Glu Val His Asn Ala Lys Thr Lys Pro Arg Glu Glu Gln Phe Asn Ser
65              70              75              80


Thr Tyr Arg Val Val Ser Val Leu Thr Val Leu His Gln Asp Trp Leu
            85              90                  95


Asn Gly Lys Glu Tyr Lys Cys Lys Val Ser Asn Lys Gly Leu Gly Ser
            100             105                 110
```

```
Ser Ile Glu Lys Thr Ile Ser Lys Ala Lys Gly Gln Pro Arg Glu Pro
    115                 120                 125

Gln Val Cys Thr Leu Pro Pro Ser Gln Glu Glu Met Thr Lys Asn Gln
    130                 135                 140

Val Ser Leu Ser Cys Ala Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Val Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225


<210>  37
<211>  229
<212>  PRT
<213>  Artificial Sequence

<220>
<223>  human IgG4 Fc-region derived Fc-region polypeptide with a P329G
       and S354C, T366W mutations

<400>  37

Glu Ser Lys Tyr Gly Pro Pro Cys Pro Ser Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80
```

```
Thr Tyr Arg Val Val Ser Val Leu Thr Val Leu His Gln Asp Trp Leu
                85                  90                  95


Asn Gly Lys Glu Tyr Lys Cys Lys Val Ser Asn Lys Gly Leu Gly Ser
            100                 105                 110


Ser Ile Glu Lys Thr Ile Ser Lys Ala Lys Gly Gln Pro Arg Glu Pro
            115                 120                 125


Gln Val Tyr Thr Leu Pro Pro Cys Gln Glu Glu Met Thr Lys Asn Gln
    130                 135                 140


Val Ser Leu Trp Cys Leu Val Lys Gly Phe Tyr Pro Ser Asp Ile Ala
145                 150                 155                 160


Val Glu Trp Glu Ser Asn Gly Gln Pro Glu Asn Asn Tyr Lys Thr Thr
                165                 170                 175


Pro Pro Val Leu Asp Ser Asp Gly Ser Phe Phe Leu Tyr Ser Arg Leu
            180                 185                 190


Thr Val Asp Lys Ser Arg Trp Gln Glu Gly Asn Val Phe Ser Cys Ser
            195                 200                 205


Val Met His Glu Ala Leu His Asn His Tyr Thr Gln Lys Ser Leu Ser
    210                 215                 220


Leu Ser Leu Gly Lys
225
```

<210> 38
<211> 229
<212> PRT
<213> Artificial Sequence

<220>
<223> human IgG4 Fc-region derived Fc-region polypeptide with a S228P,
      L235E, P329G and Y349C, T366S, L368A, Y407V mutations

<400> 38

```
Glu Ser Lys Tyr Gly Pro Pro Cys Pro Pro Cys Pro Ala Pro Glu Phe
1               5               10                  15


Glu Gly Gly Pro Ser Val Phe Leu Phe Pro Pro Lys Pro Lys Asp Thr
            20                  25                  30


Leu Met Ile Ser Arg Thr Pro Glu Val Thr Cys Val Val Val Asp Val
            35                  40                  45
```

```
Ser Gln Glu Asp Pro Glu Val Gln Phe Asn Trp Tyr Val Asp Gly Val
    50              55              60

Glu Val His Asn Ala Lys Thr Lys Pro Arg Glu Glu Gln Phe Asn Ser
65              70              75              80

Thr Tyr Arg Val Val Ser Val Leu Thr Val Leu His Gln Asp Trp Leu
            85              90              95

Asn Gly Lys Glu Tyr Lys Cys Lys Val Ser Asn Lys Gly Leu Gly Ser
        100             105             110

Ser Ile Glu Lys Thr Ile Ser Lys Ala Lys Gly Gln Pro Arg Glu Pro
        115             120             125

Gln Val Cys Thr Leu Pro Pro Ser Gln Glu Glu Met Thr Lys Asn Gln
    130             135             140

Val Ser Leu Ser Cys Ala Val Lys Gly Phe Tyr Pro Ser Asp Ile Ala
145             150             155             160

Val Glu Trp Glu Ser Asn Gly Gln Pro Glu Asn Asn Tyr Lys Thr Thr
            165             170             175

Pro Pro Val Leu Asp Ser Asp Gly Ser Phe Phe Leu Val Ser Arg Leu
        180             185             190

Thr Val Asp Lys Ser Arg Trp Gln Glu Gly Asn Val Phe Ser Cys Ser
        195             200             205

Val Met His Glu Ala Leu His Asn His Tyr Thr Gln Lys Ser Leu Ser
    210             215             220

Leu Ser Leu Gly Lys
225


<210> 39
<211> 229
<212> PRT
<213> Artificial Sequence

<220>
<223> human IgG4 Fc-region derived Fc-region polypeptide with a S228P,
      L235E, P329G and S354C, T366W mutations

<400> 39

Glu Ser Lys Tyr Gly Pro Pro Cys Pro Pro Cys Pro Ala Pro Glu Phe
1               5               10              15
```

```
Glu Gly Gly Pro Ser Val Phe Leu Phe Pro Pro Lys Pro Lys Asp Thr
            20              25              30

Leu Met Ile Ser Arg Thr Pro Glu Val Thr Cys Val Val Val Asp Val
            35              40              45

Ser Gln Glu Asp Pro Glu Val Gln Phe Asn Trp Tyr Val Asp Gly Val
    50              55              60

Glu Val His Asn Ala Lys Thr Lys Pro Arg Glu Glu Gln Phe Asn Ser
65              70              75              80

Thr Tyr Arg Val Val Ser Val Leu Thr Val Leu His Gln Asp Trp Leu
            85              90              95

Asn Gly Lys Glu Tyr Lys Cys Lys Val Ser Asn Lys Gly Leu Gly Ser
            100             105             110

Ser Ile Glu Lys Thr Ile Ser Lys Ala Lys Gly Gln Pro Arg Glu Pro
            115             120             125

Gln Val Tyr Thr Leu Pro Pro Cys Gln Glu Glu Met Thr Lys Asn Gln
    130             135             140

Val Ser Leu Trp Cys Leu Val Lys Gly Phe Tyr Pro Ser Asp Ile Ala
145             150             155             160

Val Glu Trp Glu Ser Asn Gly Gln Pro Glu Asn Asn Tyr Lys Thr Thr
            165             170             175

Pro Pro Val Leu Asp Ser Asp Gly Ser Phe Phe Leu Tyr Ser Arg Leu
            180             185             190

Thr Val Asp Lys Ser Arg Trp Gln Glu Gly Asn Val Phe Ser Cys Ser
            195             200             205

Val Met His Glu Ala Leu His Asn His Tyr Thr Gln Lys Ser Leu Ser
    210             215             220

Leu Ser Leu Gly Lys
225


<210>  40
<211>  105
<212>  PRT
<213>  homo sapiens
```

<400> 40

Gln Pro Lys Ala Ala Pro Ser Val Thr Leu Phe Pro Pro Ser Ser Glu
1               5                   10                  15

Glu Leu Gln Ala Asn Lys Ala Thr Leu Val Cys Leu Ile Ser Asp Phe
                20                  25                  30

Tyr Pro Gly Ala Val Thr Val Ala Trp Lys Ala Asp Ser Ser Pro Val
        35                  40                  45

Lys Ala Gly Val Glu Thr Thr Thr Pro Ser Lys Gln Ser Asn Asn Lys
        50                  55                  60

Tyr Ala Ala Ser Ser Tyr Leu Ser Leu Thr Pro Glu Gln Trp Lys Ser
65                  70                  75                  80

His Arg Ser Tyr Ser Cys Gln Val Thr His Glu Gly Ser Thr Val Glu
                85                  90                  95

Lys Thr Val Ala Pro Thr Glu Cys Ser
                100                 105

<210> 41
<211> 107
<212> PRT
<213> Homo sapiens

<400> 41

Arg Thr Val Ala Ala Pro Ser Val Phe Ile Phe Pro Pro Ser Asp Glu
1               5                   10                  15

Gln Leu Lys Ser Gly Thr Ala Ser Val Val Cys Leu Leu Asn Asn Phe
                20                  25                  30

Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Ser
        50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                85                  90                  95

Pro Val Thr Lys Ser Phe Asn Arg Gly Glu Cys
                100                 105

<210> 42
<211> 215
<212> PRT
<213> Artificial Sequence

<220>
<223> IGF-1R LC

<400> 42

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
                20                  25                  30

Leu Ala Trp Tyr Gln Gln Lys Pro Gly Gln Ala Pro Arg Leu Leu Ile
                35                  40                  45

Tyr Asp Ala Ser Lys Arg Ala Thr Gly Ile Pro Ala Arg Phe Ser Gly
        50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Lys Trp Pro Pro
                85                  90                  95

Trp Thr Phe Gly Gln Gly Thr Lys Val Glu Ser Lys Arg Thr Val Ala
                100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
        130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
                180                 185                 190

Tyr Ala Cys Glu Val Thr His Gln Gly Leu Ser Ser Pro Val Thr Lys
        195                 200                 205

```
Ser Phe Asn Arg Gly Glu Cys
    210                 215
```

<210> 43
<211> 447
<212> PRT
<213> Artificial Sequence

<220>
<223> IGF-1R wt

<400> 43

```
Gln Val Glu Leu Val Glu Ser Gly Gly Gly Val Val Gln Pro Gly Arg
1               5               10                  15

Ser Gln Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Ile Ile Trp Phe Asp Gly Ser Ser Thr Tyr Tyr Ala Asp Ser Val
        50                  55                  60

Arg Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Phe Cys
                85                  90                  95

Ala Arg Glu Leu Gly Arg Arg Tyr Phe Asp Leu Trp Gly Arg Gly Thr
            100                 105                 110

Leu Val Ser Val Ser Ser Ala Ser Thr Lys Gly Pro Ser Val Phe Pro
            115                 120                 125

Leu Ala Pro Ser Ser Lys Ser Thr Ser Gly Gly Thr Ala Ala Leu Gly
        130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190
```

```
Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
                260                 265                 270

Glu Val Lys Phe Asn Trp Tyr Val Asp Gly Val Glu Val His Asn Ala
            275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly
            435                 440                 445
```

<210> 44
<211> 447
<212> PRT
<213> Artificial Sequence

<220>
<223> IGF-1R AAA

<400> 44

Gln Val Glu Leu Val Glu Ser Gly Gly Gly Val Val Gln Pro Gly Arg
1               5                   10                  15

Ser Gln Arg Leu Ser Cys Ala Ala Ser Gly Phe Thr Phe Ser Ser Tyr
                20                  25                  30

Gly Met His Trp Val Arg Gln Ala Pro Gly Lys Gly Leu Glu Trp Val
            35                  40                  45

Ala Ile Ile Trp Phe Asp Gly Ser Ser Thr Tyr Tyr Ala Asp Ser Val
        50                  55                  60

Arg Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Phe Cys
                85                  90                  95

Ala Arg Glu Leu Gly Arg Arg Tyr Phe Asp Leu Trp Gly Arg Gly Thr
                100                 105                 110

Leu Val Ser Val Ser Ser Ala Ser Thr Lys Gly Pro Ser Val Phe Pro
            115                 120                 125

Leu Ala Pro Ser Ser Lys Ser Thr Ser Gly Gly Thr Ala Ala Leu Gly
        130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
            195                 200                 205

138

```
Asn Thr Lys Val Asp Lys Lys Val Glu Pro Lys Ser Cys Asp Lys Thr
    210                 215             220

His Thr Cys Pro Pro Cys Pro Ala Pro Glu Leu Leu Gly Gly Pro Ser
    225             230             235                 240

Val Phe Leu Phe Pro Pro Lys Pro Lys Asp Thr Leu Met Ala Ser Arg
            245             250                 255

Thr Pro Glu Val Thr Cys Val Val Val Asp Val Ser His Glu Asp Pro
            260             265             270

Glu Val Lys Phe Asn Trp Tyr Val Asp Gly Val Glu Val His Asn Ala
        275             280             285

Lys Thr Lys Pro Arg Glu Glu Gln Tyr Asn Ser Thr Tyr Arg Val Val
    290             295             300

Ser Val Leu Thr Val Leu Ala Gln Asp Trp Leu Asn Gly Lys Glu Tyr
305             310             315                 320

Lys Cys Lys Val Ser Asn Lys Ala Leu Pro Ala Pro Ile Glu Lys Thr
            325             330             335

Ile Ser Lys Ala Lys Gly Gln Pro Arg Glu Pro Gln Val Tyr Thr Leu
        340             345             350

Pro Pro Ser Arg Asp Glu Leu Thr Lys Asn Gln Val Ser Leu Thr Cys
        355             360             365

Leu Val Lys Gly Phe Tyr Pro Ser Asp Ile Ala Val Glu Trp Glu Ser
    370             375             380

Asn Gly Gln Pro Glu Asn Asn Tyr Lys Thr Thr Pro Pro Val Leu Asp
385             390             395                 400

Ser Asp Gly Ser Phe Phe Leu Tyr Ser Lys Leu Thr Val Asp Lys Ser
            405             410                 415

Arg Trp Gln Gln Gly Asn Val Phe Ser Cys Ser Val Met His Glu Ala
        420             425             430

Leu His Asn Ala Tyr Thr Gln Lys Ser Leu Ser Leu Ser Pro Gly
        435             440             445


<210>   45
<211>   447
<212>   PRT
```

<213> Artificial Sequence

<220>
<223> IGF-1R YTE

<400> 45

Gln Val Glu Leu Val Glu Ser Gly Gly Gly Val Val Gln Pro Gly Arg
1               5                   10                  15

Ser Gln Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Ile Ile Trp Phe Asp Gly Ser Ser Thr Tyr Tyr Ala Asp Ser Val
        50                  55                  60

Arg Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Phe Cys
                85                  90                  95

Ala Arg Glu Leu Gly Arg Arg Tyr Phe Asp Leu Trp Gly Arg Gly Thr
            100                 105                 110

Leu Val Ser Val Ser Ser Ala Ser Thr Lys Gly Pro Ser Val Phe Pro
            115                 120                 125

Leu Ala Pro Ser Ser Lys Ser Thr Ser Gly Gly Thr Ala Ala Leu Gly
        130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
            195                 200                 205

Asn Thr Lys Val Asp Lys Lys Val Glu Pro Lys Ser Cys Asp Lys Thr
        210                 215                 220

```
His Thr Cys Pro Pro Cys Pro Ala Pro Glu Leu Leu Gly Gly Pro Ser
225             230             235             240

Val Phe Leu Phe Pro Pro Lys Pro Lys Asp Thr Leu Tyr Ile Thr Arg
            245             250             255

Glu Pro Glu Val Thr Cys Val Val Val Asp Val Ser His Glu Asp Pro
            260             265             270

Glu Val Lys Phe Asn Trp Tyr Val Asp Gly Val Glu Val His Asn Ala
            275             280             285

Lys Thr Lys Pro Arg Glu Glu Gln Tyr Asn Ser Thr Tyr Arg Val Val
    290             295             300

Ser Val Leu Thr Val Leu His Gln Asp Trp Leu Asn Gly Lys Glu Tyr
305             310             315             320

Lys Cys Lys Val Ser Asn Lys Ala Leu Pro Ala Pro Ile Glu Lys Thr
            325             330             335

Ile Ser Lys Ala Lys Gly Gln Pro Arg Glu Pro Gln Val Tyr Thr Leu
            340             345             350

Pro Pro Ser Arg Asp Glu Leu Thr Lys Asn Gln Val Ser Leu Thr Cys
            355             360             365

Leu Val Lys Gly Phe Tyr Pro Ser Asp Ile Ala Val Glu Trp Glu Ser
    370             375             380

Asn Gly Gln Pro Glu Asn Asn Tyr Lys Thr Thr Pro Pro Val Leu Asp
385             390             395             400

Ser Asp Gly Ser Phe Phe Leu Tyr Ser Lys Leu Thr Val Asp Lys Ser
            405             410             415

Arg Trp Gln Gln Gly Asn Val Phe Ser Cys Ser Val Met His Glu Ala
            420             425             430

Leu His Asn His Tyr Thr Gln Lys Ser Leu Ser Leu Ser Pro Gly
    435             440             445
```

<210> 46
<211> 448
<212> PRT
<213> Artificial Sequence

<220>
<223> IgG1 wtKiH

<400> 46

Gln Val Glu Leu Val Glu Ser Gly Gly Gly Val Val Gln Pro Gly Arg
1               5                   10                  15

Ser Gln Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Ile Ile Trp Phe Asp Gly Ser Ser Thr Tyr Tyr Ala Asp Ser Val
        50                  55                  60

Arg Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Phe Cys
                85                  90                  95

Ala Arg Glu Leu Gly Arg Arg Tyr Phe Asp Leu Trp Gly Arg Gly Thr
            100                 105                 110

Leu Val Ser Val Ser Ser Ala Ser Thr Lys Gly Pro Ser Val Phe Pro
            115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
            195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

```
Val Phe Leu Phe Pro Pro Lys Pro Lys Asp Thr Leu Met Ile Ser Arg
            245             250                 255

Thr Pro Glu Val Thr Cys Val Val Val Asp Val Ser His Glu Asp Pro
            260             265                 270

Glu Val Lys Phe Asn Trp Tyr Val Asp Gly Val Glu Val His Asn Ala
            275             280                 285

Lys Thr Lys Pro Arg Glu Glu Gln Tyr Asn Ser Thr Tyr Arg Val Val
    290             295                 300

Ser Val Leu Thr Val Leu His Gln Asp Trp Leu Asn Gly Lys Glu Tyr
305             310                 315                 320

Lys Cys Lys Val Ser Asn Lys Ala Leu Pro Ala Pro Ile Glu Lys Thr
            325             330                 335

Ile Ser Lys Ala Lys Gly Gln Pro Arg Glu Pro Gln Val Tyr Thr Leu
            340             345                 350

Pro Pro Cys Arg Asp Glu Leu Thr Lys Asn Gln Val Ser Leu Trp Cys
            355             360                 365

Leu Val Lys Gly Phe Tyr Pro Ser Asp Ile Ala Val Glu Trp Glu Ser
    370             375                 380

Asn Gly Gln Pro Glu Asn Asn Tyr Lys Thr Thr Pro Pro Val Leu Asp
385             390                 395                 400

Ser Asp Gly Ser Phe Phe Leu Tyr Ser Lys Leu Thr Val Asp Lys Ser
            405             410                 415

Arg Trp Gln Gln Gly Asn Val Phe Ser Cys Ser Val Met His Glu Ala
            420             425                 430

Leu His Asn His Tyr Thr Gln Lys Ser Leu Ser Leu Ser Pro Gly Lys
            435             440                 445


<210>   47
<211>   448
<212>   PRT
<213>   Artificial Sequence

<220>
<223>   VH-IGG1-FCSSHOLE

<400>   47

Gln Val Glu Leu Val Glu Ser Gly Gly Gly Val Val Gln Pro Gly Arg
```

```
1                    5                        10                            15

Ser Gln Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
            35                  40                  45

Ala Ile Ile Trp Phe Asp Gly Ser Ser Thr Tyr Tyr Ala Asp Ser Val
            50                  55                  60

Arg Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Phe Cys
                85                  90                  95

Ala Arg Glu Leu Gly Arg Arg Tyr Phe Asp Leu Trp Gly Arg Gly Thr
            100                 105                 110

Leu Val Ser Val Ser Ser Ala Ser Thr Lys Gly Pro Ser Val Phe Pro
            115                 120                 125

Leu Ala Pro Ser Ser Lys Ser Thr Ser Gly Gly Thr Ala Ala Leu Gly
            130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
            195                 200                 205

Asn Thr Lys Val Asp Lys Lys Val Glu Pro Lys Ser Cys Asp Lys Thr
            210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255
```

144

```
Thr Pro Glu Val Thr Cys Val Val Val Asp Val Ser His Glu Asp Pro
        260             265             270

Glu Val Lys Phe Asn Trp Tyr Val Asp Gly Val Glu Val His Asn Ala
        275             280             285

Lys Thr Lys Pro Arg Glu Glu Gln Tyr Asn Ser Thr Tyr Arg Val Val
        290             295             300

Ser Val Leu Thr Val Leu His Gln Asp Trp Leu Asn Gly Lys Glu Tyr
305             310             315             320

Lys Cys Lys Val Ser Asn Lys Ala Leu Pro Ala Pro Ile Glu Lys Thr
                325             330             335

Ile Ser Lys Ala Lys Gly Gln Pro Arg Glu Pro Gln Val Cys Thr Leu
            340             345             350

Pro Pro Ser Arg Asp Glu Leu Thr Lys Asn Gln Val Ser Leu Ser Cys
            355             360             365

Ala Val Lys Gly Phe Tyr Pro Ser Asp Ile Ala Val Glu Trp Glu Ser
    370             375             380

Asn Gly Gln Pro Glu Asn Asn Tyr Lys Thr Thr Pro Pro Val Leu Asp
385             390             395             400

Ser Asp Gly Ser Phe Phe Leu Val Ser Lys Leu Thr Val Asp Lys Ser
            405             410             415

Arg Trp Gln Gln Gly Asn Val Phe Ser Cys Ser Val Met His Glu Ala
            420             425             430

Leu His Asn His Tyr Thr Gln Lys Ser Leu Ser Leu Ser Pro Gly Lys
        435             440             445
```

```
<210>   48
<211>   448
<212>   PRT
<213>   Artificial Sequence

<220>
<223>   I253A, H310A, H435A

<400>   48
```

```
Gln Val Glu Leu Val Glu Ser Gly Gly Gly Val Val Gln Pro Gly Arg
1               5               10              15

Ser Gln Arg Leu Ser Cys Ala Ala Ser Gly Phe Thr Phe Ser Ser Tyr
```

```
                  20                      25                       30

Gly Met His Trp Val Arg Gln Ala Pro Gly Lys Gly Leu Glu Trp Val
        35                  40                  45


Ala Ile Ile Trp Phe Asp Gly Ser Ser Thr Tyr Tyr Ala Asp Ser Val
        50                  55                  60


Arg Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Phe Cys
                85                  90                  95


Ala Arg Glu Leu Gly Arg Arg Tyr Phe Asp Leu Trp Gly Arg Gly Thr
            100                 105                 110


Leu Val Ser Val Ser Ser Ala Ser Thr Lys Gly Pro Ser Val Phe Pro
        115                 120                 125


Leu Ala Pro Ser Ser Lys Ser Thr Ser Gly Gly Thr Ala Ala Leu Gly
        130                 135                 140


Cys Leu Val Lys Asp Tyr Phe Pro Glu Pro Val Thr Val Ser Trp Asn
145                 150                 155                 160


Ser Gly Ala Leu Thr Ser Gly Val His Thr Phe Pro Ala Val Leu Gln
                165                 170                 175


Ser Ser Gly Leu Tyr Ser Leu Ser Ser Val Val Thr Val Pro Ser Ser
            180                 185                 190


Ser Leu Gly Thr Gln Thr Tyr Ile Cys Asn Val Asn His Lys Pro Ser
            195                 200                 205


Asn Thr Lys Val Asp Lys Lys Val Glu Pro Lys Ser Cys Asp Lys Thr
210                 215                 220


His Thr Cys Pro Pro Cys Pro Ala Pro Glu Leu Leu Gly Gly Pro Ser
225                 230                 235                 240


Val Phe Leu Phe Pro Pro Lys Pro Lys Asp Thr Leu Met Ile Ser Arg
                245                 250                 255


Thr Pro Glu Val Thr Cys Val Val Val Asp Val Ser His Glu Asp Pro
                260                 265                 270
```

```
Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
        290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Cys Arg Asp Glu Leu Thr Lys Asn Gln Val Ser Leu Trp Cys
            355                 360                 365

Leu Val Lys Gly Phe Tyr Pro Ser Asp Ile Ala Val Glu Trp Glu Ser
        370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
            435                 440                 445
```

```
<210>   49
<211>   448
<212>   PRT
<213>   Artificial Sequence

<220>
<223>   VH-AK18-IGG1-FCSSHOLE-AAA1

<400>   49
```

```
Gln Val Glu Leu Val Glu Ser Gly Gly Gly Val Val Gln Pro Gly Arg
1               5                   10                  15

Ser Gln Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
```

|    | 35  |     |     |     |     | 40  |     |     |     |     | 45  |     |     |     |
|----|-----|-----|-----|-----|-----|-----|-----|-----|-----|-----|-----|-----|-----|-----|

Ala Ile Ile Trp Phe Asp Gly Ser Ser Thr Tyr Tyr Ala Asp Ser Val
        50              55              60

Arg Gly Arg Phe Thr Ile Ser Arg Asp Asn Ser Lys Asn Thr Leu Tyr
65              70              75              80

Leu Gln Met Asn Ser Leu Arg Ala Glu Asp Thr Ala Val Tyr Phe Cys
            85              90              95

Ala Arg Glu Leu Gly Arg Arg Tyr Phe Asp Leu Trp Gly Arg Gly Thr
            100             105             110

Leu Val Ser Val Ser Ser Ala Ser Thr Lys Gly Pro Ser Val Phe Pro
            115             120             125

Leu Ala Pro Ser Ser Lys Ser Thr Ser Gly Gly Thr Ala Ala Leu Gly
    130             135             140

Cys Leu Val Lys Asp Tyr Phe Pro Glu Pro Val Thr Val Ser Trp Asn
145             150             155             160

Ser Gly Ala Leu Thr Ser Gly Val His Thr Phe Pro Ala Val Leu Gln
            165             170             175

Ser Ser Gly Leu Tyr Ser Leu Ser Ser Val Val Thr Val Pro Ser Ser
        180             185             190

Ser Leu Gly Thr Gln Thr Tyr Ile Cys Asn Val Asn His Lys Pro Ser
        195             200             205

Asn Thr Lys Val Asp Lys Lys Val Glu Pro Lys Ser Cys Asp Lys Thr
    210             215             220

His Thr Cys Pro Pro Cys Pro Ala Pro Glu Leu Leu Gly Gly Pro Ser
225             230             235             240

Val Phe Leu Phe Pro Pro Lys Pro Lys Asp Thr Leu Met Ala Ser Arg
            245             250             255

Thr Pro Glu Val Thr Cys Val Val Val Asp Val Ser His Glu Asp Pro
        260             265             270

Glu Val Lys Phe Asn Trp Tyr Val Asp Gly Val Glu Val His Asn Ala
        275             280             285

Lys Thr Lys Pro Arg Glu Glu Gln Tyr Asn Ser Thr Tyr Arg Val Val
290                 295                 300

Ser Val Leu Thr Val Leu Ala Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Cys Thr Leu
                340                 345                 350

Pro Pro Ser Arg Asp Glu Leu Thr Lys Asn Gln Val Ser Leu Ser Cys
                355                 360                 365

Ala Val Lys Gly Phe Tyr Pro Ser Asp Ile Ala Val Glu Trp Glu Ser
                370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Val Ser Lys Leu Thr Val Asp Lys Ser
                405                 410                 415

Arg Trp Gln Gln Gly Asn Val Phe Ser Cys Ser Val Met His Glu Ala
                420                 425                 430

Leu His Asn Ala Tyr Thr Gln Lys Ser Leu Ser Leu Ser Pro Gly Lys
                435                 440                 445

<210>    50
<211>    448
<212>    PRT
<213>    Artificial Sequence

<220>
<223>    H310A, H433A, Y436A

<400>    50

Gln Val Glu Leu Val Glu Ser Gly Gly Gly Val Val Gln Pro Gly Arg
1                 5                 10                 15

Ser Gln Arg Leu Ser Cys Ala Ala Ser Gly Phe Thr Phe Ser Ser Tyr
                20                 25                 30

Gly Met His Trp Val Arg Gln Ala Pro Gly Lys Gly Leu Glu Trp Val
                35                 40                 45

Ala Ile Ile Trp Phe Asp Gly Ser Ser Thr Tyr Tyr Ala Asp Ser Val

```
                50                          55                          60


            Arg Gly Arg Phe Thr Ile Ser Arg Asp Asn Ser Lys Asn Thr Leu Tyr
            65              70              75              80


            Leu Gln Met Asn Ser Leu Arg Ala Glu Asp Thr Ala Val Tyr Phe Cys
                        85              90              95


            Ala Arg Glu Leu Gly Arg Arg Tyr Phe Asp Leu Trp Gly Arg Gly Thr
                    100             105             110


            Leu Val Ser Val Ser Ser Ala Ser Thr Lys Gly Pro Ser Val Phe Pro
                    115             120             125


            Leu Ala Pro Ser Ser Lys Ser Thr Ser Gly Gly Thr Ala Ala Leu Gly
                130             135             140


            Cys Leu Val Lys Asp Tyr Phe Pro Glu Pro Val Thr Val Ser Trp Asn
            145             150             155             160


            Ser Gly Ala Leu Thr Ser Gly Val His Thr Phe Pro Ala Val Leu Gln
                        165             170             175


            Ser Ser Gly Leu Tyr Ser Leu Ser Ser Val Val Thr Val Pro Ser Ser
                    180             185             190


            Ser Leu Gly Thr Gln Thr Tyr Ile Cys Asn Val Asn His Lys Pro Ser
                    195             200             205


            Asn Thr Lys Val Asp Lys Lys Val Glu Pro Lys Ser Cys Asp Lys Thr
                210             215             220


            His Thr Cys Pro Pro Cys Pro Ala Pro Glu Leu Leu Gly Gly Pro Ser
            225             230             235             240


            Val Phe Leu Phe Pro Pro Lys Pro Lys Asp Thr Leu Met Ile Ser Arg
                        245             250             255


            Thr Pro Glu Val Thr Cys Val Val Val Asp Val Ser His Glu Asp Pro
                    260             265             270


            Glu Val Lys Phe Asn Trp Tyr Val Asp Gly Val Glu Val His Asn Ala
                    275             280             285


            Lys Thr Lys Pro Arg Glu Glu Gln Tyr Asn Ser Thr Tyr Arg Val Val
                290             295             300
```

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Cys Arg Asp Glu Leu Thr Lys Asn Gln Val Ser Leu Trp Cys
            355                 360                 365

Leu Val Lys Gly Phe Tyr Pro Ser Asp Ile Ala Val Glu Trp Glu Ser
        370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
            435                 440                 445

<210>    51
<211>    448
<212>    PRT
<213>    Artificial Sequence

<220>
<223>    VH-IGG1-FCSSHOLE-AAA2

<400>    51

Gln Val Glu Leu Val Glu Ser Gly Gly Gly Val Val Gln Pro Gly Arg
1                   5                   10                  15

Ser Gln Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Ile Ile Trp Phe Asp Gly Ser Ser Thr Tyr Tyr Ala Asp Ser Val
        50                  55                  60

Arg Gly Arg Phe Thr Ile Ser Arg Asp Asn Ser Lys Asn Thr Leu Tyr

151

|  | 65 |  |  |  | 70 |  |  |  | 75 |  |  |  | 80 |

Leu Gln Met Asn Ser Leu Arg Ala Glu Asp Thr Ala Val Tyr Phe Cys
              85                      90                      95

Ala Arg Glu Leu Gly Arg Arg Tyr Phe Asp Leu Trp Gly Arg Gly Thr
             100                     105                     110

Leu Val Ser Val Ser Ser Ala Ser Thr Lys Gly Pro Ser Val Phe Pro
             115                     120                     125

Leu Ala Pro Ser Ser Lys Ser Thr Ser Gly Gly Thr Ala Ala Leu Gly
         130                     135                     140

Cys Leu Val Lys Asp Tyr Phe Pro Glu Pro Val Thr Val Ser Trp Asn
145                     150                     155                     160

Ser Gly Ala Leu Thr Ser Gly Val His Thr Phe Pro Ala Val Leu Gln
                 165                     170                     175

Ser Ser Gly Leu Tyr Ser Leu Ser Ser Val Val Thr Val Pro Ser Ser
             180                     185                     190

Ser Leu Gly Thr Gln Thr Tyr Ile Cys Asn Val Asn His Lys Pro Ser
         195                     200                     205

Asn Thr Lys Val Asp Lys Lys Val Glu Pro Lys Ser Cys Asp Lys Thr
    210                     215                     220

His Thr Cys Pro Pro Cys Pro Ala Pro Glu Leu Leu Gly Gly Pro Ser
225                     230                     235                     240

Val Phe Leu Phe Pro Pro Lys Pro Lys Asp Thr Leu Met Ile Ser Arg
                 245                     250                     255

Thr Pro Glu Val Thr Cys Val Val Val Asp Val Ser His Glu Asp Pro
             260                     265                     270

Glu Val Lys Phe Asn Trp Tyr Val Asp Gly Val Glu Val His Asn Ala
         275                     280                     285

Lys Thr Lys Pro Arg Glu Glu Gln Tyr Asn Ser Thr Tyr Arg Val Val
         290                     295                     300

Ser Val Leu Thr Val Leu Ala Gln Asp Trp Leu Asn Gly Lys Glu Tyr
305                     310                     315                     320

```
Lys Cys Lys Val Ser Asn Lys Ala Leu Pro Ala Pro Ile Glu Lys Thr
            325                 330                 335

Ile Ser Lys Ala Lys Gly Gln Pro Arg Glu Pro Gln Val Cys Thr Leu
            340                 345                 350

Pro Pro Ser Arg Asp Glu Leu Thr Lys Asn Gln Val Ser Leu Ser Cys
            355                 360                 365

Ala Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Val Ser Lys Leu Thr Val Asp Lys Ser
            405                 410                 415

Arg Trp Gln Gln Gly Asn Val Phe Ser Cys Ser Val Met His Glu Ala
            420                 425                 430

Leu Ala Asn His Ala Thr Gln Lys Ser Leu Ser Leu Ser Pro Gly Lys
            435                 440                 445
```

```
<210>  52
<211>  448
<212>  PRT
<213>  Artificial Sequence

<220>
<223>  M252Y, S254T, T256E

<400>  52
```

```
Gln Val Glu Leu Val Glu Ser Gly Gly Gly Val Val Gln Pro Gly Arg
1               5                   10                  15

Ser Gln Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
            35                  40                  45

Ala Ile Ile Trp Phe Asp Gly Ser Ser Thr Tyr Tyr Ala Asp Ser Val
            50                  55                  60

Arg Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Phe Cys
```

                    85                          90                          95


Ala Arg Glu Leu Gly Arg Arg Tyr Phe Asp Leu Trp Gly Arg Gly Thr
            100                 105                 110


Leu Val Ser Val Ser Ser Ala Ser Thr Lys Gly Pro Ser Val Phe Pro
            115                 120                 125


Leu Ala Pro Ser Ser Lys Ser Thr Ser Gly Gly Thr Ala Ala Leu Gly
        130                 135                 140


Cys Leu Val Lys Asp Tyr Phe Pro Glu Pro Val Thr Val Ser Trp Asn
145                 150                 155                 160


Ser Gly Ala Leu Thr Ser Gly Val His Thr Phe Pro Ala Val Leu Gln
                165                 170                 175


Ser Ser Gly Leu Tyr Ser Leu Ser Ser Val Val Thr Val Pro Ser Ser
            180                 185                 190


Ser Leu Gly Thr Gln Thr Tyr Ile Cys Asn Val Asn His Lys Pro Ser
            195                 200                 205


Asn Thr Lys Val Asp Lys Lys Val Glu Pro Lys Ser Cys Asp Lys Thr
        210                 215                 220


His Thr Cys Pro Pro Cys Pro Ala Pro Glu Leu Leu Gly Gly Pro Ser
225                 230                 235                 240


Val Phe Leu Phe Pro Pro Lys Pro Lys Asp Thr Leu Met Ile Ser Arg
                245                 250                 255


Thr Pro Glu Val Thr Cys Val Val Val Asp Val Ser His Glu Asp Pro
            260                 265                 270


Glu Val Lys Phe Asn Trp Tyr Val Asp Gly Val Glu Val His Asn Ala
            275                 280                 285


Lys Thr Lys Pro Arg Glu Glu Gln Tyr Asn Ser Thr Tyr Arg Val Val
        290                 295                 300


Ser Val Leu Thr Val Leu His Gln Asp Trp Leu Asn Gly Lys Glu Tyr
305                 310                 315                 320


Lys Cys Lys Val Ser Asn Lys Ala Leu Pro Ala Pro Ile Glu Lys Thr
                325                 330                 335

```
        Ile Ser Lys Ala Lys Gly Gln Pro Arg Glu Pro Gln Val Tyr Thr Leu
                    340                 345                 350

        Pro Pro Cys Arg Asp Glu Leu Thr Lys Asn Gln Val Ser Leu Trp Cys
                    355                 360                 365

        Leu Val Lys Gly Phe Tyr Pro Ser Asp Ile Ala Val Glu Trp Glu Ser
                    370                 375                 380

        Asn Gly Gln Pro Glu Asn Asn Tyr Lys Thr Thr Pro Pro Val Leu Asp
        385                 390                 395                 400

        Ser Asp Gly Ser Phe Phe Leu Tyr Ser Lys Leu Thr Val Asp Lys Ser
                        405                 410                 415

        Arg Trp Gln Gln Gly Asn Val Phe Ser Cys Ser Val Met His Glu Ala
                    420                 425                 430

        Leu His Asn His Tyr Thr Gln Lys Ser Leu Ser Leu Ser Pro Gly Lys
                    435                 440                 445
```

```
<210>  53
<211>  448
<212>  PRT
<213>  Artificial Sequence

<220>
<223>  VH-IGG1-FCSSHOLE-YTE

<400>  53
```

```
        Gln Val Glu Leu Val Glu Ser Gly Gly Gly Val Val Gln Pro Gly Arg
        1                   5                   10                  15

        Ser Gln Arg Leu Ser Cys Ala Ala Ser Gly Phe Thr Phe Ser Ser Tyr
                    20                  25                  30

        Gly Met His Trp Val Arg Gln Ala Pro Gly Lys Gly Leu Glu Trp Val
                    35                  40                  45

        Ala Ile Ile Trp Phe Asp Gly Ser Ser Thr Tyr Tyr Ala Asp Ser Val
                50                  55                  60

        Arg Gly Arg Phe Thr Ile Ser Arg Asp Asn Ser Lys Asn Thr Leu Tyr
        65                  70                  75                  80

        Leu Gln Met Asn Ser Leu Arg Ala Glu Asp Thr Ala Val Tyr Phe Cys
                        85                  90                  95

        Ala Arg Glu Leu Gly Arg Arg Tyr Phe Asp Leu Trp Gly Arg Gly Thr
```

```
                    100                      105                         110

        Leu Val Ser Val Ser Ser Ala Ser Thr Lys Gly Pro Ser Val Phe Pro
                115                     120                     125

        Leu Ala Pro Ser Ser Lys Ser Thr Ser Gly Gly Thr Ala Ala Leu Gly
                130                     135                     140

        Cys Leu Val Lys Asp Tyr Phe Pro Glu Pro Val Thr Val Ser Trp Asn
        145                 150                     155                 160

        Ser Gly Ala Leu Thr Ser Gly Val His Thr Phe Pro Ala Val Leu Gln
                        165                     170                     175

        Ser Ser Gly Leu Tyr Ser Leu Ser Ser Val Val Thr Val Pro Ser Ser
                        180                     185                     190

        Ser Leu Gly Thr Gln Thr Tyr Ile Cys Asn Val Asn His Lys Pro Ser
                        195                     200                     205

        Asn Thr Lys Val Asp Lys Lys Val Glu Pro Lys Ser Cys Asp Lys Thr
                210                     215                     220

        His Thr Cys Pro Pro Cys Pro Ala Pro Glu Leu Leu Gly Gly Pro Ser
        225                     230                     235                 240

        Val Phe Leu Phe Pro Pro Lys Pro Lys Asp Thr Leu Tyr Ile Thr Arg
                        245                     250                     255

        Glu Pro Glu Val Thr Cys Val Val Val Asp Val Ser His Glu Asp Pro
                        260                     265                     270

        Glu Val Lys Phe Asn Trp Tyr Val Asp Gly Val Glu Val His Asn Ala
                        275                     280                     285

        Lys Thr Lys Pro Arg Glu Glu Gln Tyr Asn Ser Thr Tyr Arg Val Val
                290                     295                     300

        Ser Val Leu Thr Val Leu His Gln Asp Trp Leu Asn Gly Lys Glu Tyr
        305                     310                     315                 320

        Lys Cys Lys Val Ser Asn Lys Ala Leu Pro Ala Pro Ile Glu Lys Thr
                        325                     330                     335

        Ile Ser Lys Ala Lys Gly Gln Pro Arg Glu Pro Gln Val Cys Thr Leu
                340                     345                     350
```

```
Pro Pro Ser Arg Asp Glu Leu Thr Lys Asn Gln Val Ser Leu Ser Cys
    355                 360             365

Ala Val Lys Gly Phe Tyr Pro Ser Asp Ile Ala Val Glu Trp Glu Ser
    370             375             380

Asn Gly Gln Pro Glu Asn Asn Tyr Lys Thr Thr Pro Pro Val Leu Asp
385             390             395                         400

Ser Asp Gly Ser Phe Phe Leu Val Ser Lys Leu Thr Val Asp Lys Ser
            405             410                     415

Arg Trp Gln Gln Gly Asn Val Phe Ser Cys Ser Val Met His Glu Ala
            420             425             430

Leu His Asn His Tyr Thr Gln Lys Ser Leu Ser Leu Ser Pro Gly Lys
            435             440             445
```

<210> 54
<211> 448
<212> PRT
<213> Artificial Sequence

<220>
<223> DDD

<400> 54

```
Gln Val Glu Leu Val Glu Ser Gly Gly Gly Val Val Gln Pro Gly Arg
1               5               10                      15

Ser Gln Arg Leu Ser Cys Ala Ala Ser Gly Phe Thr Phe Ser Ser Tyr
            20              25              30

Gly Met His Trp Val Arg Gln Ala Pro Gly Lys Gly Leu Glu Trp Val
        35              40              45

Ala Ile Ile Trp Phe Asp Gly Ser Ser Thr Tyr Tyr Ala Asp Ser Val
    50              55              60

Arg Gly Arg Phe Thr Ile Ser Arg Asp Asn Ser Lys Asn Thr Leu Tyr
65              70              75                      80

Leu Gln Met Asn Ser Leu Arg Ala Glu Asp Thr Ala Val Tyr Phe Cys
            85              90              95

Ala Arg Glu Leu Gly Arg Arg Tyr Phe Asp Leu Trp Gly Arg Gly Thr
            100             105             110

Leu Val Ser Val Ser Ser Ala Ser Thr Lys Gly Pro Ser Val Phe Pro
```

```
                 115                      120                      125

      Leu Ala Pro Ser Ser Lys Ser Thr Ser Gly Gly Thr Ala Ala Leu Gly
          130               135                   140

      Cys Leu Val Lys Asp Tyr Phe Pro Glu Pro Val Thr Val Ser Trp Asn
      145               150                   155                   160

      Ser Gly Ala Leu Thr Ser Gly Val His Thr Phe Pro Ala Val Leu Gln
                    165               170                   175

      Ser Ser Gly Leu Tyr Ser Leu Ser Ser Val Val Thr Val Pro Ser Ser
                    180               185                   190

      Ser Leu Gly Thr Gln Thr Tyr Ile Cys Asn Val Asn His Lys Pro Ser
                    195               200                   205

      Asn Thr Lys Val Asp Lys Lys Val Glu Pro Lys Ser Cys Asp Lys Thr
      210               215                   220

      His Thr Cys Pro Pro Cys Pro Ala Pro Glu Leu Leu Gly Gly Pro Ser
      225               230                   235                   240

      Val Phe Leu Phe Pro Pro Lys Pro Lys Asp Thr Leu Met Ile Ser Arg
                    245               250                   255

      Thr Pro Glu Val Thr Cys Val Val Val Asp Val Ser His Glu Asp Pro
                    260               265                   270

      Glu Val Lys Phe Asn Trp Tyr Val Asp Gly Val Glu Val His Asn Ala
                    275               280                   285

      Lys Thr Lys Pro Arg Glu Glu Gln Tyr Asn Ser Thr Tyr Arg Val Val
                    290               295                   300

      Ser Val Leu Thr Val Leu His Gln Asp Trp Leu Asn Gly Lys Glu Tyr
      305               310                   315                   320

      Lys Cys Lys Val Ser Asn Lys Ala Leu Pro Ala Pro Ile Glu Lys Thr
                    325               330                   335

      Ile Ser Lys Ala Lys Gly Gln Pro Arg Glu Pro Gln Val Tyr Thr Leu
                    340               345                   350

      Pro Pro Cys Arg Asp Glu Leu Thr Lys Asn Gln Val Ser Leu Trp Cys
                    355               360                   365
```

```
Leu Val Lys Gly Phe Tyr Pro Ser Asp Ile Ala Val Glu Trp Glu Ser
    370                 375             380

Asn Gly Gln Pro Glu Asn Asn Tyr Lys Thr Thr Pro Pro Val Leu Asp
    385                 390             395                 400

Ser Asp Gly Ser Phe Phe Leu Tyr Ser Lys Leu Thr Val Asp Lys Ser
                405             410                 415

Arg Trp Gln Gln Gly Asn Val Phe Ser Cys Ser Val Met His Glu Ala
                420             425             430

Leu His Asn His Tyr Thr Gln Lys Ser Leu Ser Leu Ser Pro Gly Lys
            435             440             445


<210>  55
<211>  448
<212>  PRT
<213>  Artificial Sequence

<220>
<223>  VH-IGG1-FCSSHOLE-DDD

<400>  55

Gln Val Glu Leu Val Glu Ser Gly Gly Gly Val Val Gln Pro Gly Arg
1               5               10              15

Ser Gln Arg Leu Ser Cys Ala Ala Ser Gly Phe Thr Phe Ser Ser Tyr
            20              25              30

Gly Met His Trp Val Arg Gln Ala Pro Gly Lys Gly Leu Glu Trp Val
        35              40              45

Ala Ile Ile Trp Phe Asp Gly Ser Ser Thr Tyr Tyr Ala Asp Ser Val
        50              55              60

Arg Gly Arg Phe Thr Ile Ser Arg Asp Asn Ser Lys Asn Thr Leu Tyr
65              70              75              80

Leu Gln Met Asn Ser Leu Arg Ala Glu Asp Thr Ala Val Tyr Phe Cys
                85              90              95

Ala Arg Glu Leu Gly Arg Arg Tyr Phe Asp Leu Trp Gly Arg Gly Thr
            100             105             110

Leu Val Ser Val Ser Ser Ala Ser Thr Lys Gly Pro Ser Val Phe Pro
            115             120             125

Leu Ala Pro Ser Ser Lys Ser Thr Ser Gly Gly Thr Ala Ala Leu Gly
```

130 135 140

Cys Leu Val Lys Asp Tyr Phe Pro Glu Pro Val Thr Val Ser Trp Asn
145 150 155 160

Ser Gly Ala Leu Thr Ser Gly Val His Thr Phe Pro Ala Val Leu Gln
165 170 175

Ser Ser Gly Leu Tyr Ser Leu Ser Ser Val Val Thr Val Pro Ser Ser
180 185 190

Ser Leu Gly Thr Gln Thr Tyr Ile Cys Asn Val Asn His Lys Pro Ser
195 200 205

Asn Thr Lys Val Asp Lys Lys Val Glu Pro Lys Ser Cys Asp Lys Thr
210 215 220

His Thr Cys Pro Pro Cys Pro Ala Pro Glu Leu Leu Gly Gly Pro Ser
225 230 235 240

Val Phe Leu Phe Pro Pro Lys Pro Lys Asp Thr Asp Met Ile Ser Arg
245 250 255

Thr Pro Glu Val Thr Cys Val Val Val Asp Val Ser His Glu Asp Pro
260 265 270

Glu Val Lys Phe Asn Trp Tyr Val Asp Gly Val Glu Val His Asn Ala
275 280 285

Lys Thr Lys Pro Arg Glu Glu Gln Tyr Asn Ser Thr Tyr Arg Val Val
290 295 300

Ser Val Leu Thr Val Leu His Gln Asp Trp Asp Asn Gly Lys Glu Tyr
305 310 315 320

Lys Cys Lys Val Ser Asn Lys Ala Leu Pro Ala Pro Ile Glu Lys Thr
325 330 335

Ile Ser Lys Ala Lys Gly Gln Pro Arg Glu Pro Gln Val Cys Thr Leu
340 345 350

Pro Pro Ser Arg Asp Glu Leu Thr Lys Asn Gln Val Ser Leu Ser Cys
355 360 365

Ala Val Lys Gly Phe Tyr Pro Ser Asp Ile Ala Val Glu Trp Glu Ser
370 375 380

```
Asn Gly Gln Pro Glu Asn Asn Tyr Lys Thr Thr Pro Pro Val Leu Asp
385                 390             395                     400
```

```
Ser Asp Gly Ser Phe Phe Leu Val Ser Lys Leu Thr Val Asp Lys Ser
                405             410                 415
```

```
Arg Trp Gln Gln Gly Asn Val Phe Ser Cys Ser Val Met His Glu Ala
            420             425             430
```

```
Asp His Asn His Tyr Thr Gln Lys Ser Leu Ser Leu Ser Pro Gly Lys
        435             440             445
```

```
<210>  56
<211>  5
<212>  PRT
<213>  Artificial

<220>
<223>  heavy chain CDR1, <IGF-1R> F13B5 (modified <IGF-1R> HUMAB-Clone
       18)

<400>  56
```

```
Ser Tyr Gly Met His
1               5
```

```
<210>  57
<211>  17
<212>  PRT
<213>  Artificial

<220>
<223>  heavy chain CDR2, <IGF-1R> F13B5

<400>  57
```

```
Ile Ile Trp Phe Asp Gly Ser Ser Thr Tyr Tyr Ala Asp Ser Val Arg
1               5               10              15
```

```
Gly
```

```
<210>  58
<211>  9
<212>  PRT
<213>  Artificial

<220>
<223>  heavy chain CDR3, <IGF-1R> F13B5

<400>  58
```

```
Glu Leu Gly Arg Arg Tyr Phe Asp Leu
1               5
```

<210> 59
<211> 11
<212> PRT
<213> Artificial

<220>
<223> light chain CDR1, <IGF-1R> F13B5

<400> 59

Arg Ala Ser Gln Ser Val Ser Ser Tyr Leu Ala
1               5                   10


<210> 60
<211> 7
<212> PRT
<213> Artificial

<220>
<223> light chain CDR2, <IGF-1R> F13B5

<400> 60

Gln Ala Ser Lys Arg Ala Thr
1               5


<210> 61
<211> 10
<212> PRT
<213> Artificial

<220>
<223> light chain CDR3, <IGF-1R> F13B5

<400> 61

Gln Gln Arg Ser Lys Tyr Pro Pro Trp Thr
1               5                   10


<210> 62
<211> 118
<212> PRT
<213> Artificial

<220>
<223> heavy chain variable domain, <IGF-1R> F13B5

<400> 62

Gln Val Glu Leu Val Glu Ser Gly Gly Gly Val Val Gln Pro Gly Arg
1               5                   10                  15


Ser Gln Arg Leu Ser Cys Ala Ala Ser Gly Phe Thr Phe Ser Ser Tyr
            20                  25                  30


Gly Met His Trp Val Arg Gln Ala Pro Gly Lys Gly Leu Glu Trp Val
            35                  40                  45

Ala Ile Ile Trp Phe Asp Gly Ser Ser Thr Tyr Tyr Ala Asp Ser Val
50                    55                  60

Arg Gly Arg Phe Thr Ile Ser Arg Asp Asn Ser Lys Asn Thr Leu Tyr
65              70                  75                  80

Leu Gln Met Asn Ser Leu Arg Ala Glu Asp Thr Ala Val Tyr Phe Cys
              85                  90                  95

Ala Arg Glu Leu Gly Arg Arg Tyr Phe Asp Leu Trp Gly Arg Gly Thr
          100                 105                 110

Leu Val Ser Val Ser Ser
          115


<210>   63
<211>   108
<212>   PRT
<213>   Artificial

<220>
<223>   light chain variable domain, <IGF-1R> F13B5

<400>   63

Glu Ile Val Leu Thr Gln Ser Pro Ala Thr Leu Ser Leu Ser Pro Gly
1                   5                   10                  15

Glu Arg Ala Thr Leu Ser Cys Arg Ala Ser Gln Ser Val Ser Ser Tyr
              20                  25                  30

Leu Ala Trp Tyr Gln Gln Lys Pro Gly Gln Ala Pro Arg Leu Leu Ile
          35                  40                  45

Tyr Gln Ala Ser Lys Arg Ala Thr Gly Ile Pro Ala Arg Phe Ser Gly
      50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Lys Tyr Pro Pro
              85                  90                  95

Trp Thr Phe Gly Gln Gly Thr Lys Val Glu Ser Lys
          100                 105


<210>   64
<211>   5
<212>   PRT
<213>   Artificial

<220>
<223> heavy chain CDR1, <IGF-1R> L37F7 (modified <IGF-1R> HUMAB-Clone 18)

<400> 64

Ser Tyr Gly Met His
1               5

<210> 65
<211> 17
<212> PRT
<213> Artificial

<220>
<223> heavy chain CDR2, <IGF-1R> L37F7

<400> 65

Ile Ile Trp Phe Asp Gly Ser Ser Thr Tyr Tyr Ala Asp Ser Val Arg
1               5               10              15

Gly

<210> 66
<211> 9
<212> PRT
<213> Artificial

<220>
<223> heavy chain CDR3, <IGF-1R> L37F7

<400> 66

Glu Leu Gly Arg Arg Tyr Phe Asp Leu
1               5

<210> 67
<211> 11
<212> PRT
<213> Artificial

<220>
<223> light chain CDR1, <IGF-1R> L37F7

<400> 67

Arg Ala Ser Gln Ser Val Ser Ser Gln Leu Ala
1               5                   10

<210> 68
<211> 7
<212> PRT
<213> Artificial

<220>

<223> light chain CDR2, <IGF-1R> L37F7

<400> 68

Lys Ala Thr Asn Arg Ala Thr
1               5


<210> 69
<211> 10
<212> PRT
<213> Artificial

<220>
<223> light chain CDR3, <IGF-1R> L37F7

<400> 69

Gln Gln Arg Ser Lys Tyr Pro Pro Trp Thr
1               5               10


<210> 70
<211> 118
<212> PRT
<213> Artificial

<220>
<223> heavy chain variable domain, <IGF-1R> L37F7

<400> 70

Gln Val Glu Leu Val Glu Ser Gly Gly Gly Val Val Gln Pro Gly Arg
1               5               10              15

Ser Gln Arg Leu Ser Cys Ala Ala Ser Gly Phe Thr Phe Ser Ser Tyr
            20              25              30

Gly Met His Trp Val Arg Gln Ala Pro Gly Lys Gly Leu Glu Trp Val
        35              40              45

Ala Ile Ile Trp Phe Asp Gly Ser Ser Thr Tyr Tyr Ala Asp Ser Val
    50              55              60

Arg Gly Arg Phe Thr Ile Ser Arg Asp Asn Ser Lys Asn Thr Leu Tyr
65              70              75              80

Leu Gln Met Asn Ser Leu Arg Ala Glu Asp Thr Ala Val Tyr Phe Cys
            85              90              95

Ala Arg Glu Leu Gly Arg Arg Tyr Phe Asp Leu Trp Gly Arg Gly Thr
            100             105             110

Leu Val Ser Val Ser Ser
        115

<210> 71
<211> 108
<212> PRT
<213> Artificial

<220>
<223> light chain variable domain, <IGF-1R> L37F7

<400> 71

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Gln
            20                  25                  30

Leu Ala Trp Tyr Gln Gln Lys Pro Gly Gln Ala Pro Arg Leu Leu Ile
        35                  40                  45

Tyr Lys Ala Thr Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Lys Tyr Pro Pro
                85                  90                  95

Trp Thr Phe Gly Gln Gly Thr Lys Val Glu Ser Lys
            100                 105

<210> 72
<211> 5
<212> PRT
<213> Artificial

<220>
<223> heavy chain CDR1, <IGF-1R> L39D7 (modified <IGF-1R> HUMAB-Clone
      18)

<400> 72

Ser Tyr Gly Met His
1               5

<210> 73
<211> 17
<212> PRT
<213> Artificial

<220>
<223> heavy chain CDR2, <IGF-1R> L39D7

<400> 73

Ile Ile Trp Phe Asp Gly Ser Ser Thr Tyr Tyr Ala Asp Ser Val Arg
1               5                   10                  15

Gly

<210> 74
<211> 9
<212> PRT
<213> Artificial

<220>
<223> heavy chain CDR3, <IGF-1R> L39D7

<400> 74

Glu Leu Gly Arg Arg Tyr Phe Asp Leu
1               5

<210> 75
<211> 11
<212> PRT
<213> Artificial

<220>
<223> light chain CDR1, <IGF-1R> L39D7

<400> 75

Arg Ala Ser Gln Ser Val Ser Lys Gln Leu Ala
1               5                   10

<210> 76
<211> 7
<212> PRT
<213> Artificial

<220>
<223> light chain CDR2, <IGF-1R> L39D7

<400> 76

Asn Ala Ser Lys Arg Ala Thr
1               5

<210> 77
<211> 10
<212> PRT
<213> Artificial

<220>
<223> light chain CDR3, <IGF-1R> L39D7

<400> 77

Gln Gln Arg Ser Lys Tyr Pro Pro Trp Thr
1               5                   10

<210> 78
<211> 118
<212> PRT
<213> Artificial

<220>
<223> heavy chain variable domain, <IGF-1R> L39D7

<400> 78

Gln Val Glu Leu Val Glu Ser Gly Gly Gly Val Val Gln Pro Gly Arg
1               5               10              15

Ser Gln Arg Leu Ser Cys Ala Ala Ser Gly Phe Thr Phe Ser Ser Tyr
            20              25              30

Gly Met His Trp Val Arg Gln Ala Pro Gly Lys Gly Leu Glu Trp Val
        35              40              45

Ala Ile Ile Trp Phe Asp Gly Ser Ser Thr Tyr Tyr Ala Asp Ser Val
    50              55              60

Arg Gly Arg Phe Thr Ile Ser Arg Asp Asn Ser Lys Asn Thr Leu Tyr
65              70              75              80

Leu Gln Met Asn Ser Leu Arg Ala Glu Asp Thr Ala Val Tyr Phe Cys
                85              90              95

Ala Arg Glu Leu Gly Arg Arg Tyr Phe Asp Leu Trp Gly Arg Gly Thr
            100             105             110

Leu Val Ser Val Ser Ser
            115

<210> 79
<211> 108
<212> PRT
<213> Artificial

<220>
<223> light chain variable domain, <IGF-1R> L39D7

<400> 79

Glu Ile Val Leu Thr Gln Ser Pro Ala Thr Leu Ser Leu Ser Pro Gly
1               5               10              15

Glu Arg Ala Thr Leu Ser Cys Arg Ala Ser Gln Ser Val Ser Lys Gln
            20              25              30

Leu Ala Trp Tyr Gln Gln Lys Pro Gly Gln Ala Pro Arg Leu Leu Ile

                  35                    40                    45

        Tyr Asn Ala Ser Lys Arg Ala Thr Gly Ile Pro Ala Arg Phe Ser Gly
            50                  55                  60

        Ser Gly Ser Gly Thr Asp Phe Thr Leu Thr Ile Ser Ser Leu Glu Pro
        65                  70                  75                  80

        Glu Asp Phe Ala Val Tyr Tyr Cys Gln Gln Arg Ser Lys Tyr Pro Pro
                        85                  90                  95

        Trp Thr Phe Gly Gln Gly Thr Lys Val Glu Ser Lys
                        100                 105


        <210>   80
        <211>   5
        <212>   PRT
        <213>   Artificial

        <220>
        <223>   heavy chain CDR1, <IGF-1R> L31D11 (modified <IGF-1R> HUMAB-Clone
                18)

        <400>   80

        Ser Tyr Gly Met His
        1                   5


        <210>   81
        <211>   17
        <212>   PRT
        <213>   Artificial

        <220>
        <223>   heavy chain CDR2, <IGF-1R> L31D11

        <400>   81

        Ile Ile Trp Phe Asp Gly Ser Ser Thr Tyr Tyr Ala Asp Ser Val Arg
        1                   5                   10                  15

        Gly


        <210>   82
        <211>   9
        <212>   PRT
        <213>   Artificial

        <220>
        <223>   heavy chain CDR3, <IGF-1R> L31D11

        <400>   82

        Glu Leu Gly Arg Arg Tyr Phe Asp Leu

1                    5


<210> 83
<211> 11
<212> PRT
<213> Artificial

<220>
<223> light chain CDR1, <IGF-1R> L31D11

<400> 83

Arg Ala Ser Arg Ser Val Tyr Ser Ser Leu Ala
1                   5                   10


<210> 84
<211> 7
<212> PRT
<213> Artificial

<220>
<223> light chain CDR2, <IGF-1R> L31D11

<400> 84

Lys Ala Ser Ser Arg Ala Thr
1                   5


<210> 85
<211> 10
<212> PRT
<213> Artificial

<220>
<223> light chain CDR3, <IGF-1R> L31D11

<400> 85

Gln Gln Arg Ser Lys Trp Pro Pro Trp Thr
1                   5                   10


<210> 86
<211> 118
<212> PRT
<213> Artificial

<220>
<223> heavy chain variable domain, <IGF-1R> L31D11

<400> 86

Gln Val Glu Leu Val Glu Ser Gly Gly Gly Val Val Gln Pro Gly Arg
1                   5                   10                  15


Ser Gln Arg Leu Ser Cys Ala Ala Ser Gly Phe Thr Phe Ser Ser Tyr
                20                  25                  30

```
Gly Met His Trp Val Arg Gln Ala Pro Gly Lys Gly Leu Glu Trp Val
        35              40              45
```

```
Ala Ile Ile Trp Phe Asp Gly Ser Ser Thr Tyr Tyr Ala Asp Ser Val
        50              55              60
```

```
Arg Gly Arg Phe Thr Ile Ser Arg Asp Asn Ser Lys Asn Thr Leu Tyr
65              70              75              80
```

```
Leu Gln Met Asn Ser Leu Arg Ala Glu Asp Thr Ala Val Tyr Phe Cys
            85              90              95
```

```
Ala Arg Glu Leu Gly Arg Arg Tyr Phe Asp Leu Trp Gly Arg Gly Thr
        100             105             110
```

```
Leu Val Ser Val Ser Ser
            115
```

```
<210>   87
<211>   108
<212>   PRT
<213>   Artificial

<220>
<223>   light chain variable domain, <IGF-1R> L31D11

<400>   87
```

```
Glu Ile Val Leu Thr Gln Ser Pro Ala Thr Leu Ser Leu Ser Pro Gly
1               5               10              15
```

```
Glu Arg Ala Thr Leu Ser Cys Arg Ala Ser Arg Ser Val Tyr Ser Ser
            20              25              30
```

```
Leu Ala Trp Tyr Gln Gln Lys Pro Gly Gln Ala Pro Arg Leu Leu Ile
        35              40              45
```

```
Tyr Lys Ala Ser Ser Arg Ala Thr Gly Ile Pro Ala Arg Phe Ser Gly
    50              55              60
```

```
Ser Gly Ser Gly Thr Asp Phe Thr Leu Thr Ile Ser Ser Leu Glu Pro
65              70              75              80
```

```
Glu Asp Phe Ala Val Tyr Tyr Cys Gln Gln Arg Ser Lys Trp Pro Pro
            85              90              95
```

```
Trp Thr Phe Gly Gln Gly Thr Lys Val Glu Ser Lys
            100             105
```

```
<210>   88
```

171

```
<211>  5
<212>  PRT
<213>  Artificial

<220>
<223>  heavy chain CDR1, <IGF-1R> L31D7 (modified <IGF-1R> HUMAB-Clone
       18)

<400>  88

Ser Tyr Gly Met His
1               5


<210>  89
<211>  17
<212>  PRT
<213>  Artificial

<220>
<223>  heavy chain CDR2, <IGF-1R> L31D7

<400>  89

Ile Ile Trp Phe Asp Gly Ser Ser Thr Tyr Tyr Ala Asp Ser Val Arg
1               5                   10                  15

Gly


<210>  90
<211>  9
<212>  PRT
<213>  Artificial

<220>
<223>  heavy chain CDR3, <IGF-1R> L31D7

<400>  90

Glu Leu Gly Arg Arg Tyr Phe Asp Leu
1               5


<210>  91
<211>  10
<212>  PRT
<213>  Artificial

<220>
<223>  light chain CDR1, <IGF-1R> L31D7

<400>  91

Arg Ala Ser Gln Ser Val Ile Ser Leu Ala
1               5                   10


<210>  92
<211>  7
<212>  PRT
```

172

<213>    Artificial

<220>
<223>    light chain CDR2, <IGF-1R> L31D7

<400>    92

Arg Ala Ser Lys Arg Ala Thr
1               5


<210>    93
<211>    10
<212>    PRT
<213>    Artificial

<220>
<223>    light chain CDR3, <IGF-1R> L31D7

<400>    93

Gln Gln Arg Ser Lys Trp Pro Pro Trp Thr
1               5                   10


<210>    94
<211>    118
<212>    PRT
<213>    Artificial

<220>
<223>    heavy chain variable domain, <IGF-1R> L31D7

<400>    94

Gln Val Glu Leu Val Glu Ser Gly Gly Gly Val Val Gln Pro Gly Arg
1               5                   10                  15


Ser Gln Arg Leu Ser Cys Ala Ala Ser Gly Phe Thr Phe Ser Ser Tyr
            20                  25                  30


Gly Met His Trp Val Arg Gln Ala Pro Gly Lys Gly Leu Glu Trp Val
            35                  40                  45


Ala Ile Ile Trp Phe Asp Gly Ser Ser Thr Tyr Tyr Ala Asp Ser Val
        50                  55                  60


Arg Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Phe Cys
                85                  90                  95


Ala Arg Glu Leu Gly Arg Arg Tyr Phe Asp Leu Trp Gly Arg Gly Thr
            100                 105                 110

Leu Val Ser Val Ser Ser
115

<210> 95
<211> 108
<212> PRT
<213> Artificial

<220>
<223> light chain variable domain, <IGF-1R> L31D7

<400> 95

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ile Gln Ser
            20                  25                  30

Leu Ala Trp Tyr Gln Gln Lys Pro Gly Gln Ala Pro Arg Leu Leu Ile
            35                  40                  45

Tyr Arg Ala Ser Lys Arg Ala Thr Gly Ile Pro Ala Arg Phe Ser Gly
        50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Lys Trp Pro Pro
                85                  90                  95

Trp Thr Phe Gly Gln Gly Thr Lys Val Glu Ser Lys
            100                 105

<210> 96
<211> 447
<212> PRT
<213> Artificial Sequence

<220>
<223> IGF-1R wt

<400> 96

Gln Val Glu Leu Val Glu Ser Gly Gly Gly Val Val Gln Pro Gly Arg
1               5                   10                  15

Ser Gln Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
            35                  40                  45

174

Ala Ile Ile Trp Phe Asp Gly Ser Ser Thr Tyr Tyr Ala Asp Ser Val
        50              55              60

Arg Gly Arg Phe Thr Ile Ser Arg Asp Asn Ser Lys Asn Thr Leu Tyr
65              70              75              80

Leu Gln Met Asn Ser Leu Arg Ala Glu Asp Thr Ala Val Tyr Phe Cys
            85              90              95

Ala Arg Glu Leu Gly Arg Arg Tyr Phe Asp Leu Trp Gly Arg Gly Thr
        100             105             110

Leu Val Ser Val Ser Ser Ala Ser Thr Lys Gly Pro Ser Val Phe Pro
        115             120             125

Leu Ala Pro Ser Ser Lys Ser Thr Ser Gly Gly Thr Ala Ala Leu Gly
        130             135             140

Cys Leu Val Lys Asp Tyr Phe Pro Glu Pro Val Thr Val Ser Trp Asn
145             150             155             160

Ser Gly Ala Leu Thr Ser Gly Val His Thr Phe Pro Ala Val Leu Gln
            165             170             175

Ser Ser Gly Leu Tyr Ser Leu Ser Ser Val Val Thr Val Pro Ser Ser
        180             185             190

Ser Leu Gly Thr Gln Thr Tyr Ile Cys Asn Val Asn His Lys Pro Ser
        195             200             205

Asn Thr Lys Val Asp Lys Lys Val Glu Pro Lys Ser Cys Asp Lys Thr
        210             215             220

His Thr Cys Pro Pro Cys Pro Ala Pro Glu Leu Leu Gly Gly Pro Ser
225             230             235             240

Val Phe Leu Phe Pro Pro Lys Pro Lys Asp Thr Leu Met Ile Ser Arg
            245             250             255

Thr Pro Glu Val Thr Cys Val Val Val Asp Val Ser His Glu Asp Pro
            260             265             270

Glu Val Lys Phe Asn Trp Tyr Val Asp Gly Val Glu Val His Asn Ala
        275             280             285

Lys Thr Lys Pro Arg Glu Glu Gln Tyr Asn Ser Thr Tyr Arg Val Val
        290             295             300

```
Ser Val Leu Thr Val Leu Ala Gln Asp Trp Leu Asn Gly Lys Glu Tyr
305             310             315             320

Lys Cys Lys Val Ser Asn Lys Ala Leu Pro Ala Pro Ile Glu Lys Thr
                325             330             335

Ile Ser Lys Ala Lys Gly Gln Pro Arg Glu Pro Gln Val Tyr Thr Leu
        340             345             350

Pro Pro Ser Arg Asp Glu Leu Thr Lys Asn Gln Val Ser Leu Thr Cys
        355             360             365

Leu Val Lys Gly Phe Tyr Pro Ser Asp Ile Ala Val Glu Trp Glu Ser
        370             375             380

Asn Gly Gln Pro Glu Asn Asn Tyr Lys Thr Thr Pro Pro Val Leu Asp
385             390             395             400

Ser Asp Gly Ser Phe Phe Leu Tyr Ser Lys Leu Thr Val Asp Lys Ser
        405             410             415

Arg Trp Gln Gln Gly Asn Val Phe Ser Cys Ser Val Met His Glu Ala
        420             425             430

Leu Ala Asn His Ala Thr Gln Lys Ser Leu Ser Leu Ser Pro Gly
        435             440             445
```